# EUROPEAN PATENT APPLICATION

(11) **EP 4 574 844 A1**
(43) Date of publication of application: **25.06.2025**
(21) Application number: 23220069.1
(22) Date of filing: 22.12.2023
(51) Int. Cl.: C07K 16/28

(54) **MULTISPECIFIC ANTIBODIES HAVING SPECIFICITY FOR CD47 AND PDL1**

(71) Applicant: Numab Therapeutics AG, 8810 Horgen (CH)
(72) Inventor: The designation of the inventor has not yet been filed
(74) Representative: Virnekäs, Bernhard

(57) **Abstract**

The present invention relates to a multispecific antibody comprising one binding domain, which specifically binds to CD47 (CD47-BD), one binding domain, which specifically binds to PDL1 (PDL1-BD), and optionally one or two further binding domains having specificity for an antigen different from CD47 and PDL1. The present invention further relates to nucleic acid sequences encoding said multispecific antibody, vector(s) comprising said nucleic acid sequences, host cell(s) comprising said nucleic acid sequences or said vector(s), and a method of producing said multispecific antibody. Additionally, the present invention relates to pharmaceutical compositions comprising said multispecific antibody and methods of use thereof.

## Description

### FIELD OF THE INVENTION

The present invention relates to a multispecific antibody comprising one binding domain, which specifically binds to CD47 (CD47-BD), one binding domain, which specifically binds to PDL1 (PDL1-BD), and optionally one or two further binding domains having specificity for an antigen different from CD47 and PDL1. The present invention further relates to nucleic acid sequences encoding said multispecific antibody, vector(s) comprising said nucleic acid sequences, host cell(s) comprising said nucleic acid sequences or said vector(s), and a method of producing said multispecific antibody. Additionally, the present invention relates to pharmaceutical compositions comprising said multispecific antibody and methods of use thereof.

### BACKGROUND OF THE INVENTION

CD47 is a 50 kDa membrane receptor belonging to the immunoglobulin superfamily. CD47 consists of an extracellular N-terminal IgV domain, five transmembrane domains, and a short C-terminal intracellular tail. CD47 interacts with membrane integrins, the ligand thrombospondin-1 (TSP-1) and signal-regulatory protein alpha (SIRPα), an innate immune checkpoint protein, which is expressed on myeloid cells such as macrophages and dendritic cells (DCs) to suppress their activity. CD47 is found to be overexpressed in many different tumor types (Sick E, et al., Br. J. Pharmacol., 167 (7): 1415-1430 (2012); Chao MP, et al., Curr. Opin. Immunol, 24 (2): 225-232 (2012)) and has been correlated with poor survival in patients. Through the interaction with SIRPα, CD47 provides a so called "don't eat me signal" to macrophages and the dendritic cells (DCs) of the immune system, enabling CD47 expressing tumor cells to escape the innate immune surveillance.

Therefore, CD47 represents an interesting target in cancer therapy. However, CD47 is also ubiquitously expressed on healthy cells such as red blood cells (RBCs) and platelets in peripheral blood. Blockade of the CD47-SIRPα interaction by monoclonal antibodies has been shown to deplete those cells, often resulting in dose limiting toxicities.

PDL1 (CD274, B7-H1) is a 40 kDa type I transmembrane protein. PDL1 is a surface glycoprotein ligand for PD-1, a key immune checkpoint receptor expressed by activated T and B cells, and mediates immunosuppression. PDL1 is found on both antigen-presenting cells and human cancer cells, such as squamous cell carcinoma of the head and neck, melanoma, and brain tumor, thyroid, thymus, esophagus, lung, breast, gastrointestinal tract, colorectum, liver, pancreas, kidney, adrenal cortex, bladder, urothelium, ovary, and skin (Katsuya Y, et al., Lung Cancer.88(2):154-159 (2015); Nakanishi J, et al., Cancer Immunol Immunother. 56(8):1173-1182 (2007); Nomi T, et al., Clin Cancer Res. 13(7):2151-2157 (2007); Fay AP, et al., J Immunother Cancer. 3:3 (2015); Strome SE, et al., Cancer Res. 63(19):6501-6505 (2003); Jacobs JF, et al. Neuro Oncol.11(4):394-402 (2009); Wilmotte R, et al. Neuroreport. 16(10):1081-1085 (2005)). PDL1 is rarely expressed on normal tissues but inducibly expressed on tumor site (Dong H, et al., Nat Med. 8(8):793-800 (2002); Wang et al., Onco Targets Ther. 9: 5023-5039 (2016)). PDL1 downregulates T cell activation and cytokine secretion by binding to PD-1 (Freeman et al., 2000; Latchman et al, 2001). PD-1, activated by PDL1, potentially provides an immune-tolerant environment for tumor development and growth. PDL1 also negatively regulates T-cell function through interaction with another receptor, B7.1 (B7-1, CD80).

Various antibodies against PDL1 have been developed (see, for example, WO 2013/079174 and WO 2017/118321) to be used in the treatment of PDL1 sensitive tumor types, such as those indicated above. However, while encouraging results have been obtained for some patients with these PDL1 sensitive tumor types, there is still a large fraction of patients that do not respond to anti-PDL1 antibody treatment. Besides, many tumor types with low anti-PDL1 sensitivity are also enriched with CD47.

To address at least some of the abovementioned issues, several bispecific antibody therapeutics targeting CD47 and PDL1 have been developed that are currently in pre-clinical state or are tested in early clinical trials.

For example, WO 2021124073 (A1) discloses IgG-based bispecific heterodimeric antibodies having one CD47 binding domain and one PDL1 binding domain that are located in the Fab arms. One of these bispecific antibodies, i.e. PF-07257876, is currently tested in phase I clinical trials. WO 2019129054 (A1) discloses IgG-like, Fc-silenced triple-chain antibodies having one CD47 binding domain located in one Fab arm and two VHH PDL1 binding domains that are replacing the second Fab arm. One of these bispecific antibodies, i.e. IBI-322, is currently tested in phase II clinical trials.

It is generally expected that these anti-CD47xPD-L1 bispecific antibodies have greater selectivity towards PDL1- and CD47-expressing tumor cells and/or PDL1- and CD47-expressing immune cells in a tumor microenvironment than anti-CD47 monospecific antibodies. However, while most of these bispecific antibodies are reported to exhibit reduced or no binding to CD47 on red blood cells, adverse effects caused by red blood cell agglutination are still observed in clinical trials. Some of these bispecific antibodies also suffer from suboptimal efficacy. In this regard, the effect of the Fc region on the efficacy of these bispecific antibodies appears to be unclear, as the presence of active or silenced Fc does not correlate well with the observed clinical efficacy.

There is thus a need in the field to generate improved antibody therapeutics targeting CD47 and PDL1.

Said antibody therapeutics should have a high potency against CD47- and PDL1-expressing cancer cells and at the same time a tolerable safety profile to keep dose limiting side effects, such as in particular red blood cell agglutination, at a low level. Furthermore, it is desirable that said therapeutic antibodies have superior biophysical properties, such as a high stability, in order to facilitate developability and producibility in high yields.

### SUMMARY OF THE INVENTION

It is an object of the present invention to provide novel and improved therapeutic antibodies that can efficiently eliminate CD47- and PDL1-expressing cancer cells while exhibiting a low level of dose limiting side effects, in particular a low level of red blood cell agglutination. It is a further object of the present invention that said novel and improved therapeutic antibodies exhibit high stability to facilitate developability and producibility in high yields.

The inventors have now surprisingly found that multispecific antibodies having one highly affine PDL1-BD and one moderately affine CD47-BD, in combination with a well-defined IgG-like antibody architecture, wherein the CD47-BD is present in the form of an scFv, exhibit efficient killing of CD47- and PDL1-expressing cancer cells, while causing virtually no agglutination or phagocytosis of red blood cells, even at higher nanomolar concentrations. This was particularly unexpected, since alternative antibody architectures, e.g. antibody architectures wherein said CD47-BD is not in scFv format and/or the PDL1-BD and the CD47-BD are not located next to each other in the Fab arms, do not exhibit the desired combined properties, *i*. *e*. potent cancer cell lysis and at the same time low or no agglutination/lysis of red blood cells.

It has further been found that the distinct architecture of these multispecific antibodies allows for the addition of further binding domains at the C-terminus of the Fc region, which enables the incorporation of additional functionalities, such as the blockage of other escape mechanisms or activation of additional immune response pathways, for example by adding LILRB2-BDs to foster further activation of immune cells, such as the activation of macrophages and proliferative activation of T-cells.

Said multispecific antibodies also exhibit very advantageous biophysical properties, in particular an outstanding stability, which allows high yield production and the preparation of storage stable formulations. For example, the loss of monomeric content for PRO4672, PRO5052, PRO4687 and PRO5055, which are representative multispecific antibodies of the present invention, is less than 1 % when stored for two months at 4°C at a concentration of 10 mg/ml in a 20 mM histidine buffer (pH 6.0) with 125 mM NaCl and 0.02 % Poloxamer 188. This high stability is particularly surprising in view of the fact that the multispecific antibodies of the invention comprise up to four scFv antibody fragments.

Accordingly, in a first aspect, the present invention relates to a multispecific antibody comprising:
a) one binding domain, which specifically binds to CD47 (CD47-BD); wherein said CD47-BD comprises:
   i. the HCDR1 sequence of SEQ ID NO: 1,
      the HCDR2 sequence of SEQ ID NO: 2,
      the HCDR3 sequence of SEQ ID NO: 3,
      the LCDR1 sequence of SEQ ID NO: 4,
      the LCDR2 sequence of SEQ ID NO: 6, and
      the LCDR3 sequence of SEQ ID NO: 7; or
   ii. the HCDR1 sequence of SEQ ID NO: 1,
      the HCDR2 sequence of SEQ ID NO: 2,
      the HCDR3 sequence of SEQ ID NO: 3,
      the LCDR1 sequence of SEQ ID NO: 4,
      the LCDR2 sequence of SEQ ID NO: 5, and
      the LCDR3 sequence of SEQ ID NO: 7.
b) one binding domain, which specifically binds to PDL1 (PDL1-BD); and
c) an IgG-like region comprising an immunoglobulin Fc region, a first Fab arm and a second Fab arm, wherein:
   - the CD47 BD is an scFv fragment replacing the first Fab arm, and
   - the PDL1 BD is the binding domain of the second Fab arm or is an scFv fragment replacing said second Fab arm.

In a second aspect, the present invention relates to a nucleic acid or two nucleic acids or three nucleic acids encoding the multispecific antibody of the present invention.

In a third aspect, the present invention relates to a vector or two vectors or three vectors comprising the nucleic acid or two nucleic acids or three nucleic acids.

In a fourth aspect, the present invention relates to a host cell or host cells comprising the vector or the two vectors or the three vectors of the present invention.

In a fifth aspect, the present invention relates to a method for producing the multispecific antibody of the present invention, comprising (i) providing the nucleic acid or two nucleic acids or three nucleic acids of the present invention, or the vector or the two vectors or the three vectors of the present invention, expressing said nucleic acid or nucleic acids, or said vector or vectors, and collecting said multispecific antibody from the expression system, or (ii) providing a host cell or host cells of the present invention, culturing said host cell or said host cells; and collecting said multispecific antibody from the cell culture.

In a sixth aspect, the present invention relates to a pharmaceutical composition comprising the multispecific antibody of the present invention and a pharmaceutically acceptable carrier.

In a seventh aspect, the present invention relates to a pharmaceutical composition comprising:
a) a first multispecific antibody that specifically binds to CD47 and PDL1;
b) a second antibody that specifically binds to LILRB2; and
c) a pharmaceutically acceptable carrier.

In an eighth aspect, the present invention relates to a multispecific antibody of the present invention for use as a medicament.

In a ninth aspect, the present invention relates to a multispecific antibody of the present invention for use in the treatment of a disease, particularly a human disease, more particularly a human disease selected from cancer.

In a tenth aspect, the present invention relates to a method for the treatment of a disease, particularly a human disease, more particularly a human disease selected from cancer, comprising the step of administering the multispecific antibody of the present invention or the pharmaceutical composition of the present invention to a patient in need thereof.

In an eleventh aspect, the present invention relates an antibody binding domain, having specificity for CD47 (CD47-BD), as defined above, wherein said CD47-BD is in a format selected from an Fv, a dsFv, an scFv and a dsscFv, particularly from an Fv and an scFv, particularly wherein said CD47-BD is in the form of an scFv.

The aspects, advantageous features and preferred embodiments of the present invention summarized in the following items, respectively alone or in combination, further contribute to solving the object of the invention:
1. A multispecific antibody comprising:
   a) one binding domain, which specifically binds to CD47 (CD47-BD), wherein said CD47-BD comprises:
      i. the HCDR1 sequence of SEQ ID NO: 1,
         the HCDR2 sequence of SEQ ID NO: 2,
         the HCDR3 sequence of SEQ ID NO: 3,
         the LCDR1 sequence of SEQ ID NO: 4,
         the LCDR2 sequence of SEQ ID NO: 6, and
         the LCDR3 sequence of SEQ ID NO: 7; or
      ii. the HCDR1 sequence of SEQ ID NO: 1,
         the HCDR2 sequence of SEQ ID NO: 2,
         the HCDR3 sequence of SEQ ID NO: 3,
         the LCDR1 sequence of SEQ ID NO: 4,
         the LCDR2 sequence of SEQ ID NO: 5, and
         the LCDR3 sequence of SEQ ID NO: 7;
   b) one binding domain, which specifically binds to PDL1 (PDL1-BD); and
   c) an IgG-like region comprising an immunoglobulin Fc region, a first Fab arm and a second Fab arm, wherein:
      - the CD47 BD is an scFv fragment replacing the first Fab arm, and
      - the PDL1 BD is in the binding domain of the second Fab arm or is an scFv fragment replacing said second Fab arm.
2. The multispecific antibody of item 1, wherein said CD47-BD comprises:
   the HCDR1 sequence of SEQ ID NO: 1,
   the HCDR2 sequence of SEQ ID NO: 2,
   the HCDR3 sequence of SEQ ID NO: 3,
   the LCDR1 sequence of SEQ ID NO: 4,
   the LCDR2 sequence of SEQ ID NO: 6, and
   the LCDR3 sequence of SEQ ID NO: 7.
3. The multispecific antibody of item 1 or 2, wherein said PDL1-BD comprises:
   i. the HCDR1 sequence of SEQ ID NO: 74,
      the HCDR2 sequence of SEQ ID NO: 75,
      the HCDR3 sequence of SEQ ID NO: 76,
      the LCDR1 sequence of SEQ ID NO: 77,
      the LCDR2 sequence of SEQ ID NO: 78, and
      the LCDR3 sequence of SEQ ID NO: 80; or
   ii. the HCDR1 sequence of SEQ ID NO: 74,
      the HCDR2 sequence of SEQ ID NO: 75,
      the HCDR3 sequence of SEQ ID NO: 76,
      the LCDR1 sequence of SEQ ID NO: 77,
      the LCDR2 sequence of SEQ ID NO: 79, and
      the LCDR3 sequence of SEQ ID NO: 80; or
   iii. the HCDR1 sequence of SEQ ID NO: 20,
      the HCDR2 sequence of SEQ ID NO: 21,
      the HCDR3 sequence of SEQ ID NO: 23,
      the LCDR1 sequence of SEQ ID NO: 24, the LCDR2 sequence of SEQ ID NO: 25, and
      the LCDR3 sequence of SEQ ID NO: 27; or
   iv. the HCDR1 sequence of SEQ ID NO: 20,
      the HCDR2 sequence of SEQ ID NO: 21,
      the HCDR3 sequence of SEQ ID NO: 23,
      the LCDR1 sequence of SEQ ID NO: 24,
      the LCDR2 sequence of SEQ ID NO: 26, and
      the LCDR3 sequence of SEQ ID NO: 27; or
   v. the HCDR1 sequence of SEQ ID NO: 20,
      the HCDR2 sequence of SEQ ID NO: 22,
      the HCDR3 sequence of SEQ ID NO: 23,
      the LCDR1 sequence of SEQ ID NO: 24,
      the LCDR2 sequence of SEQ ID NO: 25, and
      the LCDR3 sequence of SEQ ID NO: 27; or
   vi. the HCDR1 sequence of SEQ ID NO: 20,
      the HCDR2 sequence of SEQ ID NO: 22,
      the HCDR3 sequence of SEQ ID NO: 23,
      the LCDR1 sequence of SEQ ID NO: 24,
      the LCDR2 sequence of SEQ ID NO: 26, and
      the LCDR3 sequence of SEQ ID NO: 27.
4. The multispecific antibody of items 1 or 2, wherein said PDL1-BD comprises:
   i. the HCDR1 sequence of SEQ ID NO: 74,
      the HCDR2 sequence of SEQ ID NO: 75,
      the HCDR3 sequence of SEQ ID NO: 76,
      the LCDR1 sequence of SEQ ID NO: 77,
      the LCDR2 sequence of SEQ ID NO: 78, and
      the LCDR3 sequence of SEQ ID NO: 80; or
   ii. the HCDR1 sequence of SEQ ID NO: 74,
      the HCDR2 sequence of SEQ ID NO: 75,
      the HCDR3 sequence of SEQ ID NO: 76,
      the LCDR1 sequence of SEQ ID NO: 77,
      the LCDR2 sequence of SEQ ID NO: 79, and
      the LCDR3 sequence of SEQ ID NO: 80.
5. The multispecific antibody of any of the preceding items, wherein said CD47-BD, when in scFv format, binds to human CD47 with a monovalent dissociation constant (KD) of 1 to 100 nM, particularly with a KD of 2 to 50 nM, particularly of 5 to 25 nM, as measured by surface plasmon resonance (SPR).
6. The multispecific antibody of any of the preceding items, wherein said CD47-BD:
   a. when in scFv format, has a melting temperature (Tm), determined by differential scanning fluorimetry, in the range of 50°C to 70°C, particularly in the range of 55°C to 65°C, in particular wherein said antibody is formulated in 50 mM phosphate-citrate buffer (NaCiP) at pH 6.4, 150 mM NaCl;
   b. when in scFv format, has a loss in monomer content, after storage for four weeks at -80°C, of less than 3 %, less than 2 %, less than 1 %, particularly less than 0.5 %, when the antibody of the invention is at a starting concentration of 10 mg/ml, and in particular wherein said antibody is formulated in 50 mM phosphate citrate buffer (NaCiP) with 150 mM NaCl at pH 6.4;
   c. when in scFv format, has a loss in monomer content, after storage for four weeks at 4°C, of less than 3 %, less than 2 %, less than 1 %, particularly less than 0.5 %, when the antibody of the invention is at a starting concentration of 10 mg/ml, and in particular wherein said antibody is formulated in 50 mM phosphate citrate buffer (NaCiP) with 150 mM NaCl at pH 6.4; and/or
   d. when in scFv format, has a loss in monomer content, after storage for four weeks at 40°C, of less than 3 %, less than 2 %, less than 1 %, particularly less than 0.5 %, when the antibody of the invention is at a starting concentration of 10 mg/ml, and in particular wherein said antibody is formulated in 50 mM phosphate citrate buffer (NaCiP) with 150 mM NaCl at pH 6.4.
7. The multispecific antibody of any of the preceding items, wherein said PDL1-BD:
   a. when in scFv format, binds to human PDL1 with a monovalent dissociation constant (KD) of 1 to 100 pM, particularly with a KD of 1 to 50 pM, particularly of 1 to 40 pM, as measured by surface plasmon resonance (SPR);
   b. when in scFv format, is cross-reactive with *Macaca fascicularis* (cynomolgus) PDL1, in particular binds to cynomolgus PDL1 with a monovalent KD of 1 to 100 pM, particularly with a KD of 1 to 50 pM, particularly of 1 to 40 pM, as measured by SPR;
   c. when in scFv format, has the ability to neutralize PDL1/PD-1 interaction, as measured in an NFAT reporter gene assay, with an EC₅₀ value of 0.1 to 10 nM, particularly of 0.1 to 5 pM, particularly of 0.1 to 3 nM;
   d. is non-cross-reactive to *Mus musculus* PDL1, as measured by SPR; and/or
   e. does not bind to human PDL2, as measured by SPR.
8. The multispecific antibody of any of the preceding items, wherein said PDL1-BD:
   a. when in scFv format, has a melting temperature (Tm), determined by differential scanning fluorimetry, in the range of 60°C to 85°C, particularly in the range of 65°C to 85°C, particularly in the range of 70°C to 85°C, in particular wherein said antibody is formulated in 50 mM phosphate-citrate buffer at pH 6.4, 150 mM NaCl;
   b. when in scFv format, has a loss in monomer content, after five consecutive freeze-thaw cycles, of less than 5 %, preferably less than 3 %, more preferably less than 1 %, when the antibody of the invention is at a starting concentration of 10 mg/ml, and in particular wherein the antibody of the invention is formulated in 50 mM phosphate citrate buffer with 150 mM NaCl at pH 6.4; and/or
   c. when in scFv format, has a loss in monomer content, after storage for four weeks at 4°C, of less than 15 %, e.g. less than 12 %, less than 10 %, less than 7 %, less than 5 %, less than 4 %, less than 3 %, less than 2 %, preferably less than 1 %, when the antibody of the invention is at a starting concentration of 10 mg/ml, and in particular wherein said antibody is formulated in 50 mM phosphate citrate buffer with 150 mM NaCl at pH 6.4.
9. The multispecific antibody of any of the preceding items, wherein the KD of the PDL1-BD of said multispecific antibody for binding to human PDL1 is at least 500 fold lower, in particular at least 1000 fold lower, in particular at least 1500 fold lower, in particular at least 2000 fold lower, than the KD of the CD47 BD of said multispecific antibody for binding to human CD47.
10. The multispecific antibody of any of the preceding items, wherein said multispecific antibody does not trigger any visible red blood cell agglutination at a concentration of 5 nM, particularly of 20 nM, particularly of 50 nM, particularly of 100 nM, particularly of 200 nM, particularly of 500 nM, as determined in a standard hemagglutination assay as described in Example 4.2; and/or does not cause the lysis of red blood cells at a concentration of 5 nM, particularly of 20 nM, particularly of 50 nM, as determined in a standard phagocytosis assay, as described in Example 5.4.
11. The multispecific antibody of item 1, wherein the immunoglobulin Fc region is selected from an immunoglobulin Fc region of an IgG subclass, particularly an immunoglobulin Fc region of human IgG1, IgG2 (IgG2A, IgG2B), IgG3 or IgG4, particularly of human IgG1 or IgG2, particularly of human IgG1.
12. The multispecific antibody of item 1, wherein the immunoglobulin Fc region is an active Fc region.
13. The multispecific antibody of any one of the preceding items, wherein the immunoglobulin Fc region has one or more amino acid modifications selected from the group consisting of:
   a) modifications to enhance heterodimerization of heavy chains, such as the knob-into-hole (KiH) technology;
   b) modifications that increase or decrease Fc receptor binding, when compared to the respective unmodified immunoglobulin Fc region thereof;
   c) modifications that increase serum half-life, when compared to the respective unmodified immunoglobulin Fc region thereof.
14. The multispecific antibody of any one of the preceding items, wherein said CD47-BD comprises VH3 or VH4 domain framework sequences FR1 to FR4; particularly VH3 domain framework sequences FR1 to FR4.
15. The multispecific antibody of item 14, wherein said CD47-BD comprises a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is a Vλ FR4, particularly is a Vλ FR4 comprising an amino acid sequence having at least 80, 90 percent identity to any of SEQ ID NOs: 161 to 169, more particularly a Vλ FR4 selected from any of SEQ ID NOs: 161 to 169, particularly a Vλ FR4 according to SEQ ID NO: 161 or 168.
16. The multispecific antibody of any one of the preceding items, wherein said CD47-BD comprises
   a) a VH sequence at least 90, 91, 92, 93, 94, 95, 96, 97, 98 or 99 percent identical to any one of the amino acid sequences selected from SEQ ID NOs: 8 and 9; and
      a VL sequence at least 90, 91, 92, 93, 94, 95, 96, 97, 98 or 99 percent identical to any one of the amino acid sequences selected from SEQ ID NOs: 11 and 12, particularly to SEQ ID NO: 12; or
   b) a VH sequence at least 90, 91, 92, 93, 94, 95, 96, 97, 98 or 99 percent identical to the amino acid sequence of SEQ ID NO: 10; and
      a VL sequence at least 90, 91, 92, 93, 94, 95, 96, 97, 98 or 99 percent identical to the amino acid sequence of SEQ ID NO: 13.
17. The multispecific antibody of any one of the preceding items, wherein said CD47-BD comprises
   a) a VH sequence selected from any one of SEQ ID NOs: 8 and 9; and
      a VL sequence selected from any one of SEQ ID NOs: 11 and 12, particularly the VL sequence of SEQ ID NO: 12; or
   b) the VH sequence of SEQ ID NO: 10; and
      the VL sequence of SEQ ID NO: 13.
18. The multispecific antibody of any one of the preceding items, wherein said PDL1-BD comprises VH3 or VH4 domain framework sequences FR1 to FR4; particularly VH3 domain framework sequences FR1 to FR4.
19. The multispecific antibody of item 18, wherein said PDL1-BD comprises
   a) a VL domain comprising a VL framework comprising framework regions FR1, FR2, FR3 and FR4, which are selected from Vκ subtypes, particularly from the Vκ1 and Vκ3 subtypes, particularly are of the Vκ1 subtype; or
   b) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vλ FR4, particularly is a Vλ FR4 comprising an amino acid sequence having at least 80, 90 percent identity to any of SEQ ID NOs: 161 to 169, more particularly a Vλ FR4 selected from any of SEQ ID NOs: 161 to 169, particularly a Vλ FR4 according to SEQ ID NO: 161 or 168.
20. The multispecific antibody of any one of the preceding items, wherein said PDL1-BD comprises
   a) a VH sequence at least 90, 91, 92, 93, 94, 95, 96, 97, 98 or 99 percent identical to the amino acid sequence of SEQ ID NO: 28; and
      a VL sequence at least 90, 91, 92, 93, 94, 95, 96, 97, 98 or 99 percent identical to any one of the amino acid sequences selected from SEQ ID NOs: 34, 36 and 37, particularly selected from SEQ ID NOs: 34 and 37, particularly to SEQ ID NO:37; or
   b) a VH sequence at least 90, 91, 92, 93, 94, 95, 96, 97, 98 or 99 percent identical to any one of the amino acid sequences selected from SEQ ID NOs: 29 and 31; and a VL sequence at least 90, 91, 92, 93, 94, 95, 96, 97, 98 or 99 percent identical to any one of the amino acid sequences selected from SEQ ID NOs: 33 and 35, particularly to SEQ ID NO: 35; or
   c) a VH sequence at least 90, 91, 92, 93, 94, 95, 96, 97, 98 or 99 percent identical to the amino acid sequence of SEQ ID NO: 30; and
      a VL sequence at least 90, 91, 92, 93, 94, 95, 96, 97, 98 or 99 percent identical to any one of the amino acid sequences selected from SEQ ID NOs: 32 and 34, particularly to SEQ ID NO: 34; or
   d) a VH sequence at least 90, 91, 92, 93, 94, 95, 96, 97, 98 or 99 percent identical to any one of the amino acid sequences selected from SEQ ID NOs: 38 and 42; and a VL sequence at least 90, 91, 92, 93, 94, 95, 96, 97, 98 or 99 percent identical to any one of the amino acid sequences selected from SEQ ID NOs: 48, 50 and 51, particularly selected from SEQ ID NOs: 48 and 51, particularly to SEQ ID NO: 51; or
   e) a VH sequence at least 90, 91, 92, 93, 94, 95, 96, 97, 98 or 99 percent identical to any one of the amino acid sequences selected from SEQ ID NOs: 39, 41, 43 and 45; and
      a VL sequence at least 90, 91, 92, 93, 94, 95, 96, 97, 98 or 99 percent identical to any one of the amino acid sequences selected from SEQ ID NOs: 47 and 49, particularly to SEQ ID NO: 49; or
   f) a VH sequence at least 90, 91, 92, 93, 94, 95, 96, 97, 98 or 99 percent identical to any one of the amino acid sequences selected from SEQ ID NOs: 40 and 44; and a VL sequence at least 90, 91, 92, 93, 94, 95, 96, 97, 98 or 99 percent identical to any one of the amino acid sequences selected from SEQ ID NOs: 46 and 48, particularly to SEQ ID NO: 48; or
   g) a VH sequence at least 90, 91, 92, 93, 94, 95, 96, 97, 98 or 99 percent identical to the amino acid sequence of SEQ ID NO: 52; and
      a VL sequence at least 90, 91, 92, 93, 94, 95, 96, 97, 98 or 99 percent identical to any one of the amino acid sequences selected from SEQ ID NOs: 58, 60 and 61, particularly selected from SEQ ID NOs: 58 and 61, particularly to SEQ ID NO: 61; or
   h) a VH sequence at least 90, 91, 92, 93, 94, 95, 96, 97, 98 or 99 percent identical to any one of the amino acid sequences selected from SEQ ID NOs: 53 and 55; and a VL sequence at least 90, 91, 92, 93, 94, 95, 96, 97, 98 or 99 percent identical to any one of the amino acid sequences selected from SEQ ID NOs: 57 and 59, particularly to SEQ ID NO: 59; or
   i) a VH sequence at least 90, 91, 92, 93, 94, 95, 96, 97, 98 or 99 percent identical to the amino acid sequence of SEQ ID NO: 54; and
      a VL sequence at least 90, 91, 92, 93, 94, 95, 96, 97, 98 or 99 percent identical to any one of the amino acid sequences selected from SEQ ID NOs: 56 and 58, particularly to SEQ ID NO: 58; or
   j) a VH sequence at least 90, 91, 92, 93, 94, 95, 96, 97, 98 or 99 percent identical to the amino acid sequence of SEQ ID NO: 81; and
      a VL sequence at least 90, 91, 92, 93, 94, 95, 96, 97, 98 or 99 percent identical to any one of the amino acid sequences selected from SEQ ID NOs: 86, 88 and 89, particularly selected from SEQ ID NOs: 86 and 89, particularly to SEQ ID NO: 89; or
   k) a VH sequence at least 90, 91, 92, 93, 94, 95, 96, 97, 98 or 99 percent identical to the amino acid sequence of SEQ ID NO: 83; and
      a VL sequence at least 90, 91, 92, 93, 94, 95, 96, 97, 98 or 99 percent identical to any one of the amino acid sequences selected from SEQ ID NOs: 85 and 86,
      particularly to SEQ ID NO: 86; or
   l) a VH sequence at least 90, 91, 92, 93, 94, 95, 96, 97, 98 or 99 percent identical to any one of the amino acid sequences selected from SEQ ID NOs: 82 and 84, particularly to SEQ ID NO: 84; and
      a VL sequence at least 90, 91, 92, 93, 94, 95, 96, 97, 98 or 99 percent identical to the amino acid sequences of SEQ ID NO: 86.
21. The multispecific antibody of any one of the preceding items, wherein said PDL1-BD comprises
   a) the VH sequence of SEQ ID NO: 28; and
      a VL sequence selected from any one of SEQ ID NOs: 34, 36 and 37, particularly selected from any one of SEQ ID NOs: 34 and 37, particularly the VL sequence of SEQ ID NO: 37; or
   b) a VH sequence selected from any one of SEQ ID NOs: 29 and 31; and
      a VL sequence selected from any one of SEQ ID NOs: 33 and 35, particularly the VL sequence of SEQ ID NO: 35; or
   c) the VH sequence of SEQ ID NO: 30; and
      a VL sequence selected from any one of SEQ ID NOs: 32 and 34, particularly the VL sequence of SEQ ID NO: 34; or
   d) a VH sequence selected from any one of SEQ ID NOs: 38 and 42; and
      a VL sequence selected from any one of SEQ ID NOs: 48, 50 and 51, particularly from SEQ ID NOs: 48 and 51, particularly the VL sequence of SEQ ID NO: 51; or
   e) a VH sequence selected from any one of SEQ ID NOs: 39, 41, 43 and 45; and
      a VL sequence selected from any one of SEQ ID NOs: 47 and 49, particularly the VL sequence of SEQ ID NO: 49; or
   f) a VH sequence selected from any one of SEQ ID NOs: 40 and 44; and
      a VL sequence selected from any one of SEQ ID NOs: 46 and 48, particularly the VL sequence of SEQ ID NO: 48; or
   g) the VH sequence of SEQ ID NO: 52; and
      a VL sequence selected from any one of SEQ ID NOs: 58, 60 and 61, particularly from SEQ ID NOs: 58 and 61, particularly the VL sequence of SEQ ID NO:61; or
   h) a VH sequence selected from any one of SEQ ID NO: 53 and 55; and
      a VL sequence selected from any one of SEQ ID NOs: 57 and 59, particularly the VL sequence of SEQ ID NO: 59; or
   i) the VH sequence of SEQ ID NO: 54; and
      a VL sequence selected from any one of SEQ ID NOs: 56 and 58, particularly the VL sequence of SEQ ID NO: 58; or
   j) the VH sequence of SEQ ID NO: 81; and
      a VL sequence selected from any one of SEQ ID NOs: 86, 88 and 89, particularly from SEQ ID NOs: 86 and 89, particularly the VL sequence of SEQ ID NO: 89; or
   k) the VH sequence of SEQ ID NO: 83; and
      a VL sequence selected from any one of SEQ ID NOs: 85 and 86, particularly the VL sequence of SEQ ID NO: 86; or
   l) a VH sequence selected from any one of SEQ ID NOs: 82 and 84, particularly the VH sequence of SEQ ID NO: 84; and
      the VL sequence of SEQ ID NO: 86.
22. The multispecific antibody of any one of the preceding items, wherein said PDL1-BDs comprises CDR regions derived from a parental rabbit antibody.
23. The multispecific antibody of any one of the preceding items,
   wherein said CD47-BD comprises:
   a) a VH sequence selected from any one of SEQ ID NOs: 8 and 9; and
      the VL sequence of SEQ ID NO: 12; or
   b) the VH sequence of SEQ ID NO: 10; and
      the VL sequence of SEQ ID NO: 13;
      and wherein said PDL1-BD comprises:
   a) the VH sequence of SEQ ID NO: 52; and
      a VL sequence selected from any one of SEQ ID NOs: 58 and 61, particularly the VL sequence of SEQ ID NO:61; or
   b) the VH sequence of SEQ ID NO: 54; and
      the VL sequence of SEQ ID NO: 58; or
   d) the VH sequence of SEQ ID NO: 81; and
      a VL sequence selected from any one of SEQ ID NOs: 86 and 89, particularly the VL sequence of SEQ ID NO: 89; or
   e) the VH sequence of SEQ ID NO: 83; and
      the VL sequence of SEQ ID NO: 86.
24. The multispecific antibody of any one of the preceding items, which has amino acid sequences selected from:
   - SEQ ID NO: 174 and SEQ ID NO: 175;
   - SEQ ID NO: 176 SEQ ID NO: 177 and SEQ ID NO: 178;
   - SEQ ID NO: 179, SEQ ID NO: 180 and SEQ ID NO: 181;
   - SEQ ID NO: 182 and SEQ ID NO: 183;
   - SEQ ID NO: 184, SEQ ID NO: 185 and SEQ ID NO: 186;
   - SEQ ID NO: 197 and SEQ ID NO: 198;
   - SEQ ID NO: 199, SEQ ID NO: 200 and SEQ ID NO: 201;
   - SEQ ID NO: 202 and SEQ ID NO: 203; and
   - SEQ ID NO: 204, SEQ ID NO: 205 and SEQ ID NO: 206.
25. The multispecific antibody of any one of the preceding items, wherein said multispecific antibody further comprises:
   d) one further binding domain, which has specificity for an antigen different from CD47 and PDL1,
   wherein said one further binding domain is attached to one of the C-termini of the heavy chains of the immunoglobulin Fc region.
26. The multispecific antibody of any one of the items 1 to 24, wherein the multispecific antibody further comprises:
   d) two further binding domains, which have specificity for an antigen different from CD47 and PDL1, wherein said two further binding domains are attached to each of the C-termini of the heavy chains of the immunoglobulin Fc region.
27. The multispecific antibody of item 25 or 26, wherein the one or two further binding domains specifically bind to a tumor associated antigen (TAA) different from CD47 and PDL1.
28. The multispecific antibody of item 27, wherein the TAA is selected from ADRB3, AFP, ALK, BCMA, beta human chorionic gonadotropin, CA-125 (MUC16), CAIX, CD123, CD133, CD135, CD135 (FLT3), CD138, CD171, CD19, CD20, CD22, CD24, CD276, CD33, CD33, CD38, CD44v6, CD79b, CD97, CDH3 (cadherin 3), CEA, CEACAM6, CLDN6, CLEC12A (CLL1), CSPG4, CYP1B1, EGFR, EGFRvlll, EPCAM, EPHA2, Ephrin B2, ERBBs (e. g. ERBB2), FAP, FGFR1, folate receptor alpha, folate receptor beta, Fos-related antigen, GA733, GD2, GD3, GFRalpha4, globoH, GPC3, GPR20, GPRC5D, HAVCR1, Her2/neu (HER2), HLA-A2, HMWMAA, HPV E6 or E7, human telomerase reverse transcriptase, IL-11Ra, IL-13Ra2, intestinal carboxyl esterase, KIT, Legumain, LewisY, LMP2, Ly6k, MAD-CT-1, MAD-CT-2, ML-IAP, MN-CA IX, MSLN, MUC1, mut hsp 70-2, NA-17, NCAM, neutrophil elastase, NY-BR-1, NY-ESO-1, o-acetyl-GD2, OR51E2, PANX3, PDGFR-beta, PLAC1, Polysialic acid, PSCA, PSMA, RAGE1, ROR1, sLe, sperm protein 17, SSEA-4, SSTR2, sTn antigen, sTn-O-Glycopeptides, TAG72, TARP, TEM1/CD248, TEM7R, thyroglobulin, Tn antigen, Tn-O-Glycopeptides, TPBG (5T4), TRP-2, TSHR, UPK2 and VEGFR2.
29. The multispecific antibody of item 25 or 26, wherein the one or two further binding domains specifically bind to an NK cell receptor and/or an immunomodulatory antigen different from PDL1.
30. The multispecific antibody of any one of items 25, 26 and 29, wherein at least one of the further binding domains specifically binds to an NK cell receptor.
31. The multispecific antibody of item 29 or 30, wherein the immunomodulatory antigen different from PDL1 is selected from B7-1 (CD80), B7-2 (CD86), B7-DC (CD273), B7-H1 (CD274), B7-H2 (CD275), B7-H3 (CD276), B7-H4 (VTCN1), B7-H5 (VISTA), BTLA (CD272), 4-1BB (CD137), CD137L, CD24, CD27, CD28, CD38, CD40, CD40L (CD154), CD54, CD59, CD70, CTLA-1, CTLA-4 (CD152), CXCL9, GITR (CD357), GITR, HVEM (CD270), ICAM-1 (CD54), ICOS (CD278), LAG-3 (CD223), OX40 (CD134), OX40L (CD252), PD-1 (CD279), TIGIT, CD314, CD334, CD335, CD337, VISTA and TIM-3 (CD366).
32. The multispecific antibody of item 26, wherein both of the further binding domains specifically bind to an NK cell receptor.
33. The multispecific antibody of any one of the items 29 to 32, wherein the NK cell receptor is selected from KIR2DL1, KIR2DL2/3, KIR2DL4, KIR2DL5A, KIR2DL5B, KIR3DL1, KIR3DL2, KIR3DL3, KIR2DS4, KIR3DS, NKG2A, NKG2C, NKG2D, NKG2E, NKp30, NKp44, NKp46, CD2, CD16, CD69, DNAX accessory molecule-1 (DNAM-1), 2B4, NK1.1; a killer immunoglobulin (Ig)-like activating receptors (KAR); ILTs/LIRs (CD85) such as LILRA1, LlLRA2, LlLRA3, LILRA4, LILRA5, LILRA6, LILRB1, LILRB2, LILRB3, LILRB4, LILRB5, ILT8, ILT10, ILT11; NKRP-1; CD94/NKG2A, CD94/NKG2C and CD94/NKG2E heterodimers.
34. The multispecific antibody of any one of the items 29 to 32, wherein the NK cell receptor is selected from NKp46, LILRB1, LILRB2, LILRB3, LILRB4 and LILRB5, particularly LILRB1, LILRB2, and LILRB4, particularly LILRB1 and LILRB2, particularly LILRB2.
35. The multispecific antibody of item 25, wherein said further binding domain specifically binds to LILRB2 (LILRB2-BD).
36. The multispecific antibody of item 25, wherein said two further binding domains specifically bind to LILRB2 (LILRB2-BDs).
37. The multispecific antibody of item 36, wherein the LILRB2-BDs bind to different epitopes on the extracellular domain of LILRB2.
38. The multispecific antibody of item 36, wherein the LILRB2-BDs bind to the same epitope on the extracellular domain of LILRB2.
39. The multispecific antibody of item 36, wherein the LILRB2-BDs are identical.
40. The multispecific antibody of any one of the items 35 to 39, wherein said LILRB2-BD or said LILRB2-BDs comprise:
   i. the HCDR1 sequence of SEQ ID NO: 93,
      the HCDR2 sequence of SEQ ID NO: 94,
      the HCDR3 sequence of SEQ ID NO: 95,
      the LCDR1 sequence of SEQ ID NO: 96,
      the LCDR2 sequence of SEQ ID NO: 97, and
      the LCDR3 sequence of SEQ ID NO: 99; or
   ii. the HCDR1 sequence of SEQ ID NO: 93,
      the HCDR2 sequence of SEQ ID NO: 94,
      the HCDR3 sequence of SEQ ID NO: 95,
      the LCDR1 sequence of SEQ ID NO: 96,
      the LCDR2 sequence of SEQ ID NO: 98, and
      the LCDR3 sequence of SEQ ID NO: 99; or
   iii. the HCDR1 sequence of SEQ ID NO: 112,
      the HCDR2 sequence of SEQ ID NO: 113,
      the HCDR3 sequence of SEQ ID NO: 114,
      the LCDR1 sequence of SEQ ID NO: 115,
      the LCDR2 sequence of SEQ ID NO: 116, and
      the LCDR3 sequence of SEQ ID NO: 118; or
   iv. the HCDR1 sequence of SEQ ID NO: 112,
      the HCDR2 sequence of SEQ ID NO: 113,
      the HCDR3 sequence of SEQ ID NO: 114,
      the LCDR1 sequence of SEQ ID NO: 115,
      the LCDR2 sequence of SEQ ID NO: 117, and
      the LCDR3 sequence of SEQ ID NO: 118 or
   v. the HCDR1 sequence of SEQ ID NO: 128,
      the HCDR2 sequence of SEQ ID NO: 129,
      the HCDR3 sequence of SEQ ID NO: 130,
      the LCDR1 sequence of SEQ ID NO: 131,
      the LCDR2 sequence of SEQ ID NO: 132, and
      the LCDR3 sequence of SEQ ID NO: 134; and/or
   vi. the HCDR1 sequence of SEQ ID NO: 128,
      the HCDR2 sequence of SEQ ID NO: 129,
      the HCDR3 sequence of SEQ ID NO: 130,
      the LCDR1 sequence of SEQ ID NO: 131,
      the LCDR2 sequence of SEQ ID NO: 133, and
      the LCDR3 sequence of SEQ ID NO: 134.
41. The multispecific antibody of any one of the items 35 to 39, wherein said LILRB2-BD or said LILRB2-BDs comprise:
   i. the HCDR1 sequence of SEQ ID NO: 93,
      the HCDR2 sequence of SEQ ID NO: 94,
      the HCDR3 sequence of SEQ ID NO: 95,
      the LCDR1 sequence of SEQ ID NO: 96,
      the LCDR2 sequence of SEQ ID NO: 98, and
      the LCDR3 sequence of SEQ ID NO: 99; or
   ii. the HCDR1 sequence of SEQ ID NO: 112,
      the HCDR2 sequence of SEQ ID NO: 113,
      the HCDR3 sequence of SEQ ID NO: 114,
      the LCDR1 sequence of SEQ ID NO: 115,
      the LCDR2 sequence of SEQ ID NO: 117, and
      the LCDR3 sequence of SEQ ID NO: 118 and/or
   iii. the HCDR1 sequence of SEQ ID NO: 128,
      the HCDR2 sequence of SEQ ID NO: 129,
      the HCDR3 sequence of SEQ ID NO: 130,
      the LCDR1 sequence of SEQ ID NO: 131,
      the LCDR2 sequence of SEQ ID NO: 133, and
      the LCDR3 sequence of SEQ ID NO: 134.
42. The multispecific antibody of any one of the items 35 to 39, wherein said LILRB2-BD or said LILRB2-BDs comprise:
   i. the HCDR1 sequence of SEQ ID NO: 93,
      the HCDR2 sequence of SEQ ID NO: 94,
      the HCDR3 sequence of SEQ ID NO: 95,
      the LCDR1 sequence of SEQ ID NO: 96,
      the LCDR2 sequence of SEQ ID NO: 97, and
      the LCDR3 sequence of SEQ ID NO: 99; and/or
   ii. the HCDR1 sequence of SEQ ID NO: 93,
      the HCDR2 sequence of SEQ ID NO: 94,
      the HCDR3 sequence of SEQ ID NO: 95,
      the LCDR1 sequence of SEQ ID NO: 96,
      the LCDR2 sequence of SEQ ID NO: 98, and
      the LCDR3 sequence of SEQ ID NO: 99.
43. The multispecific antibody of any one of the items 35 to 39, wherein said LILRB2-BD or both said LILRB2-BDs comprise:
   i. the HCDR1 sequence of SEQ ID NO: 93,
      the HCDR2 sequence of SEQ ID NO: 94,
      the HCDR3 sequence of SEQ ID NO: 95,
      the LCDR1 sequence of SEQ ID NO: 96,
      the LCDR2 sequence of SEQ ID NO: 98, and
      the LCDR3 sequence of SEQ ID NO: 99.
44. The multispecific antibody of any one of the items 35 to 43, wherein said LILRB2-BD or said LILRB2-BDs:
   a. when in scFv format, bind to human LILRB2 with a monovalent dissociation constant (KD) of 10 pM to 5 nM, particularly with a KD of 20 pM to 3 nM, particularly of 50 pM to 1 nM, as measured by surface plasmon resonance (SPR);
   b. block the interaction of LILRB2 to its ligand HLA-G with an IC₅₀ of 100 pM to 10 nM, particularly of 100 pM to 5 nM, particularly of 100 pM to 2 nM, as measured by flow cytometry;
   c. block the interaction of LILRB2 to its ligand HLA-A3 with an IC₅₀ of 100 pM to 10 nM, particularly of 100 pM to 5 nM, particularly of 100 pM to 2 nM, as measured by flow cytometry;
   d. are not cross-reactive with members of leukocyte immunoglobulin-like receptor subfamily A (LILRA); and/or
   e. are not cross reactive with members of leukocyte immunoglobulin-like receptor subfamily LILRB3, LILRB4 and LILRB5.
45. The multispecific antibody of any one of the items 35 to 44, wherein said LILRB2-BD or said LILRB2-BDs:
   a. when in scFv format, have a melting temperature (Tm), determined by differential scanning fluorimetry, in the range of 55°C to 85°C, particularly in the range of 60°C to 85°C, in particular wherein said antibodies are formulated in 50 mM phosphate-citrate buffer at pH 6.4, 150 mM NaCl;
   b. when in scFv format, have a loss in monomer content, after storage for four weeks, at -80°C, of less than 5 %, less than 4 %, less than 3 %, less than 2 %, particularly less than 1.5 %, when said antibodies are at a starting concentration of 10 mg/ml, and in particular wherein said antibody is formulated in 50 mM phosphate citrate buffer with 150 mM NaCl at pH 6.4;
   c. when in scFv format, has a loss in monomer content, after storage for four weeks, at 4°C, of less than 15 %, e.g. less than 12 %, less than 10 %, less than 9 %, less than 8 %, particularly less than 7 %, when said antibodies are at a starting concentration of 10 mg/ml, and in particular wherein said antibodies are formulated in 50 mM phosphate citrate buffer with 150 mM NaCl at pH 6.4;
   d. when in scFv format, has a loss in monomer content, after storage for four weeks, at 40°C, of less than 15 %, e.g. less than 12 %, less than 10 %, less than 9 %, less than 8 %, particularly less than 7 %, when said antibodies are at a starting concentration of 10 mg/ml, and in particular wherein said antibodies are formulated in 50 mM phosphate citrate buffer with 150 mM NaCl at pH 6.4.
46. The multispecific antibody of any one of the items 35 to 45, wherein said LILRB2-BD or said LILRB2-BDs are in a format independently selected from a Fab, an scFab, an Fv, a dsFv, an scFv and a dsscFv, particularly from a Fab, Fv and scFv.
47. The multispecific antibody of any one of the items 35 to 46, wherein said LILRB2-BD or said LILRB2-BDs comprise VH3 or VH4 domain framework sequences FR1 to FR4; particularly VH3 domain framework sequences FR1 to FR4.
48. The multispecific antibody of any one of the items 35 to 47, wherein said LILRB2-BD or said LILRB2-BDs independently comprise
   a) a VL domain comprising a VL framework comprising framework regions FR1, FR2, FR3 and FR4, which are selected from Vκ subtypes, particularly from the Vκ1 and Vκ3 subtypes, particularly are of the Vκ1 subtype; or
   b) a VL domain comprising a VL framework comprising framework regions FR1, FR2 and FR3, which are selected from Vκ subtypes, particularly from the Vκ1 and Vκ3 subtypes, particularly are of the Vκ1 subtype, and a framework FR4, which is selected from a Vλ FR4, particularly is a Vλ FR4 comprising an amino acid sequence having at least 80, 90 percent identity to any of SEQ ID NOs: 161 to 169, more particularly a Vλ FR4 selected from any of SEQ ID NOs: 161 to 169, particularly a Vλ FR4 according to SEQ ID NO: 161 or 168.
49. The multispecific antibody of any one of the items 35 to 48, wherein said LILRB2-BD or said LILRB2-BDs independently comprise
   a) a VH sequence at least 90, 91, 92, 93, 94, 95, 96, 97, 98 or 99 percent identical to the amino acid sequence of SEQ ID NO: 100; and
      a VL sequence at least 90, 91, 92, 93, 94, 95, 96, 97, 98 or 99 percent identical to any one of the amino acid sequences selected from SEQ ID NOs: 104, 106 and 107, particularly selected from SEQ ID NOs: 104 and 107, particularly to SEQ ID NO: 107; or
   b) a VH sequence at least 90, 91, 92, 93, 94, 95, 96, 97, 98 or 99 percent identical to the amino acid sequence of SEQ ID NO: 101; and
      a VL sequence at least 90, 91, 92, 93, 94, 95, 96, 97, 98 or 99 percent identical to any one of the amino acid sequences selected from SEQ ID NOs: 103 and 104, particularly to SEQ ID NO: 104; or
   c) a VH sequence at least 90, 91, 92, 93, 94, 95, 96, 97, 98 or 99 percent identical to the amino acid sequence of SEQ ID NO: 102; and
      a VL sequence at least 90, 91, 92, 93, 94, 95, 96, 97, 98 or 99 percent identical to the amino acid sequence of SEQ ID NO: 105; or
   d) a VH sequence at least 90, 91, 92, 93, 94, 95, 96, 97, 98 or 99 percent identical to the amino acid sequence of SEQ ID NO: 119; and
      a VL sequence at least 90, 91, 92, 93, 94, 95, 96, 97, 98 or 99 percent identical to any one of the amino acid sequences selected from SEQ ID NOs: 122, 123 and 124, particularly selected from SEQ ID NOs: 122 and 124, particularly to SEQ ID NO: 124; or
   e) a VH sequence at least 90, 91, 92, 93, 94, 95, 96, 97, 98 or 99 percent identical to the amino acid sequence of SEQ ID NO: 120; and
      a VL sequence at least 90, 91, 92, 93, 94, 95, 96, 97, 98 or 99 percent identical to any one of the amino acid sequences selected from SEQ ID NOs: 121 and 122, particularly to SEQ ID NO: 122; or
   f) a VH sequence at least 90, 91, 92, 93, 94, 95, 96, 97, 98 or 99 percent identical to the amino acid sequence of SEQ ID NO: 135; and
      a VL sequence at least 90, 91, 92, 93, 94, 95, 96, 97, 98 or 99 percent identical to any one of the amino acid sequences selected from SEQ ID NOs: 138, 139 and 140, particularly selected from SEQ ID NOs: 138 and 140, particularly to SEQ ID NO: 140; or
   g) a VH sequence at least 90, 91, 92, 93, 94, 95, 96, 97, 98 or 99 percent identical to the amino acid sequence of SEQ ID NO: 136; and
      a VL sequence at least 90, 91, 92, 93, 94, 95, 96, 97, 98 or 99 percent identical to the amino acid sequence of SEQ ID NO: 138.
50. The multispecific antibody of any one of the items 35 to 48, wherein said LILRB2-BD or said LILRB2-BDs independently comprise
   a) a VH sequence of SEQ ID NO: 100; and
      a VL sequence selected from SEQ ID NOs: 104, 106 and 107, particularly selected from SEQ ID NOs: 104 and 107, particularly from SEQ ID NO: 107; or
   b) a VH sequence of SEQ ID NO: 101; and
      a VL sequence selected from SEQ ID NOs: 103 and 104, particularly from SEQ ID NO: 104; or
   c) a VH sequence of SEQ ID NO: 102; and
      a VL sequence of SEQ ID NO: 105; or
   d) a VH sequence of SEQ ID NO: 119; and
      a VL sequence selected from SEQ ID NOs: 122, 123 and 124, particularly selected from SEQ ID NOs: 122 and 124, particularly from SEQ ID NO: 124; or
   e) a VH sequence of SEQ ID NO: 120; and
      a VL sequence selected from SEQ ID NOs: 121 and 122, particularly from SEQ ID NO: 122; or
   f) a VH sequence of SEQ ID NO: 135; and
      a VL sequence selected from SEQ ID NOs: 138, 139 and 140, particularly from SEQ ID NOs: 138 and 140, particularly from SEQ ID NO: 140; or
   g) a VH sequence of SEQ ID NO: 136; and
      a VL sequence of SEQ ID NO: 138.
51. The multispecific antibody of any one of the items 35 to 50, wherein said multispecific antibody is further characterized by one or more of the following features:
   a) said LILRB2-BD comprises CDR regions derived from a parental rabbit antibody;
   b) binds to human CD47 with a monovalent dissociation constant (KD) of 1 to 100 nM, particularly with a KD of 5 to 70 nM, particularly of 10 to 50 nM, as measured by surface plasmon resonance (SPR);
   c) binds to human PDL1 with a monovalent dissociation constant (KD) of 1 to 200 pM, particularly with a KD of 1 to 150 pM, particularly of 1 to 100 pM, as measured by surface plasmon resonance (SPR);
   d) binds to human LILRB2 with a monovalent dissociation constant (KD) of 10 pM to 5 nM, particularly with a KD of 20 pM to 3 nM, particularly of 50 pM to 1 nM, as measured by surface plasmon resonance (SPR);
   e) does not cause the lysis of red blood cells at a concentration of 10 nM, particularly of 50 nM, particularly of 100 nM as determined in a standard phagocytosis assay, as described in Example 5.4;
   f) blocks the interaction of CD47 to SIRPα with an IC₅₀ of 10 to 500 pM, particularly of 10 to 300 pM, particularly of 10 to 200 pM, as measured by flow cytometry using NCI-H292 cells;
   g) has a second melting temperature (Tm2), determined by differential scanning fluorimetry, in the range of 60°C to 80°C, particularly in the range of 63°C to 80°C, particularly in the range of 65°C to 80°C, in particular wherein said multispecific antibody is formulated in 20 mM Histidine buffer at pH 6.0, 125 mM NaCl and 0.02 % Poloxamer 188;
   h) has a loss in monomer content, after five consecutive freeze-thaw cycles, of less than 2 %, preferably less than 1 %, more preferably less than 0.5 %, when the antibody of the invention is at a starting concentration of 10 mg/ml, and in particular wherein the antibody of the invention is formulated in 20 mM Histidine buffer at pH 6.0, 125 mM NaCl and 0.02 % Poloxamer 188;
   i) has a loss in monomer content, after storage for 8 weeks at 4°C, of less than 5 %, e.g. less than 3 %, less than 2 %, less than 1 %, when the antibody of the invention is at a starting concentration of 10 mg/ml, and in particular wherein said antibody is formulated in 20 mM Histidine buffer at pH 6.0, 125 mM NaCl and 0.02 % Poloxamer 188;
   j) has a loss in monomer content, after storage for 4 weeks at 25°C, of less than 5 %, e.g. less than 3 %, less than 2 %, less than 1 %, when the antibody of the invention is at a starting concentration of 10 mg/ml, and in particular wherein said antibody is formulated in 20 mM Histidine buffer at pH 6.0, 125 mM NaCl and 0.02 % Poloxamer 188; and/or
   k) has a loss in monomer content, after storage for 4 weeks at 40°C, of less than 7 %, e.g. less than 6 %, less than 5 %, when the antibody of the invention is at a starting concentration of 10 mg/ml, and in particular wherein said antibody is formulated in 20 mM Histidine buffer at pH 6.0, 125 mM NaCl and 0.02 % Poloxamer 188.
52. The multispecific antibody of any one of the items 35 to 51, wherein the LILRB2-BD and the CD47-BD are capable of binding simultaneously to their respective antigens.
53. The multispecific antibody of any one of the items 35 to 52, which has amino acid sequences selected from:
   - SEQ ID NO: 187 and SEQ ID NO: 188 (PRO4672);
   - SEQ ID NO: 189, SEQ ID NO: 190 and SEQ ID NO: 191 (PRO4687);
   - SEQ ID NO: 192 and SEQ ID NO: 193 (PRO5052); and
   - SEQ ID NO: 194, SEQ ID NO: 195 and SEQ ID NO: 196 (PRO5055).
54. A nucleic acid or two nucleic acids or three nucleic acids encoding the multispecific antibody of any one of items 1 to 53.
55. A vector or two vectors or three vectors comprising the nucleic acid or two nucleic acids or three nucleic acids of item 54.
56. A host cell or host cells comprising the vector or the two vectors or the three vectors of item 55.
57. A method for producing the multispecific antibody of any one of items 1 to 53, comprising
   (i) providing the nucleic acid or two nucleic acids or three nucleic acids of item 54, or the vector or the two vectors or the three vectors of item 55, expressing said nucleic acid or nucleic acids, or said vector or vectors, and collecting said multispecific antibody from the expression system, or (ii) providing a host cell or host cells according to item 56, culturing said host cell or said host cells; and collecting said multispecific antibody from the cell culture.
58. A pharmaceutical composition comprising the multispecific antibody of any one of items 1 to 53 and a pharmaceutically acceptable carrier.
59. A pharmaceutical composition comprising:
   a) a first multispecific antibody that specifically binds to CD47 and PDL1;
   b) a second antibody that specifically binds to LILRB2; and
   c) a pharmaceutically acceptable carrier.
60. The pharmaceutical composition of item 59, wherein the first antibody that specifically binds to CD47 and PDL1 is selected from anti-CD47xPDL1 antibodies that are currently tested or have been tested in clinical trials, preferably wherein the first antibody that specifically binds to CD47 and PDL1 is selected from the group consisting of: IBI-322 by Innovent Biologics Inc.; PF-07257876 by Pfizer Inc.; 6MW-3211 by Mabwell (Shanghai) Bioscience Co. Ltd.; BAT7104 by Bio-Thera Solutions, Ltd.; QL401 by QLSF Biotherapeutics, Inc.; LB-101 by Centessa Pharmaceuticals plc; Imm2520 by ImmuneOnco Biopharmaceuticals Shanghai Inc.; SHS-009 by Nanjing Sanhome Pharmaceutical Co Ltd.; BCD-106 by Biocad Biopharmaceutical Co.; TJL-1C4 by I-MAB Biopharma Co. Ltd.; SG12473 by Hangzhou Sumgen Biotech Co. Ltd.; XB014 by Exelixis Inc.; PMC-122 by PharmAbcine Inc.; ABP-160 by Abpro Corp.; papiliximab by Shaperon Inc.; JY-207 by Shenzhen Enduring Biotech Ltd.; NY2601 and NY2901 by Light Chain Bioscience AG..
61. The pharmaceutical composition of item 59, wherein the first antibody that specifically binds to CD47 and PDL1 is selected from a multispecific antibody as defined in any one of the items 1 to 24.
62. The pharmaceutical composition of any one of the items 59 to 61, wherein the second antibody that specifically binds to LILRB2 is selected from anti-LILRB2 antibodies that are currently tested or have been tested in clinical trials, preferably wherein the second antibody that specifically binds to LILRB2 is selected from the group consisting of: JTX-8064 by Jounce Therapeutics Inc.; MK-4830 by Merck & Co. Inc.; IO-108 by Immune-One Therapeutics Inc.; NGM-707 by NGM Biopharmaceuticals Inc.; BMS-986406 by Bristol Myers Squibb Company; IOS-1002 by ImmunOs Therapeutics AG; CDX-585 by Celldex Therapeutics Inc.; SPX-303 by SparX Biopharmaceutical Corp.; CHS-1000 by Coherus BioSciences Inc.; ES009 by Elpiscience (Suzhou) Biopharma Ltd.; XBH41 by GV20 Therapeutics and IVS-5001 by Invectys.
63. The pharmaceutical composition of any one of the items 59 to 61, wherein the second antibody that specifically binds to LILRB2 comprises at least one LILRB2-BD as defined in any one of the items 40 to 50.
64. The pharmaceutical composition of any one of the items 59 to 61 and 63, wherein the second antibody comprises two LILRB2-BDs.
65. The pharmaceutical composition of item 64, wherein the two LILRB2-BDs bind to different epitopes on the extracellular domain of LILRB2.
66. The pharmaceutical composition of item 64, wherein the two LILRB2-BDs bind to the same epitope on the extracellular domain of LILRB2.
67. The pharmaceutical composition of item 66, wherein the LILRB2-BDs are identical.
68. The pharmaceutical composition of any one of the items 63 to 67, wherein the second antibody is a fragment-based antibody further comprising one hSA binding domain.
69. The pharmaceutical composition of item 68, wherein the format of the second antibody is selected from a tandem scDb (Tandab), a linear dimeric scDb (LD-scDb), a circular dimeric scDb (CD-scDb), a tandem tri-scFv, a tribody (Fab-(scFv)₂), a Fab-Fv₂, a triabody, an scDb-scFv, a tetrabody, a didiabody, a tandem-di-scFv and a MATCH.
70. The pharmaceutical composition of any one of the items 63 to 67, wherein the second antibody comprises an Fc region, as defined in items 11 and 12.
71. The pharmaceutical composition of item 70, wherein the immunoglobulin Fc region is selected from
   a) an immunoglobulin Fc region of an IgG subclass, particularly an immunoglobulin Fc region of human IgG1, IgG2 (IgG2A, IgG2B), IgG3 or IgG4, particularly of human IgG1 or IgG4;
   b) an immunoglobulin Fc region of a) having 1, 2 or 3 amino acid modifications, which exhibits increased or decreased Fc receptor binding when compared to the respective unmodified immunoglobulin Fc region of a).
72. The pharmaceutical composition of item 70 or 71, wherein the format of the second antibody is selected from an IgG format.
73. The multispecific antibody of any one of items 1 to 53 for use as a medicament.
74. The multispecific antibody of any one of items 1 to 53 or the pharmaceutical composition of any one of items 58 to 72 for use in the treatment of a disease, particularly a human disease, more particularly a human disease selected from cancer.
75. The multispecific antibody of item 74, wherein the cancer is selected from
   a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.
76. A method for the treatment of a disease, particularly a human disease, more particularly a human disease selected from cancer, comprising the step of administering the multispecific antibody of any one of items 1 to 53 or the pharmaceutical composition of any one of items 58 to 72 to a patient in need thereof.
77. The method of item 76, wherein the cancer is selected from
   a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.
78. An antibody binding domain, having specificity for CD47 (CD47-BD), as defined in any one of items 1, 2, 5, 6, 13 - 16, 21 and 23.
79. The antibody binding domain of item 78, wherein said CD47-BD is in a format selected from an Fv, a dsFv, an scFv and a dsscFv, particularly from an Fv and an scFv, particularly wherein said CD47-BD is in the form of an scFv.
80. An antibody comprising one or two, preferably one, antibody binding domains according to item 78 or 79.
81. An antibody binding domain, having specificity for LILRB2 (LILRB2-BD), as defined in any one of items 40 to 51.
82. The antibody binding domain of item 81, wherein said LILRB2-BD is in a format selected from an Fv, a dsFv, an scFv and a dsscFv, particularly from an Fv and an scFv, particularly wherein said LILRB2-BD is in the form of an scFv.
83. An antibody comprising one, two, three or four, preferably one or two, antibody binding domains according to item 80.
84. An antibody according to item 81, which has a sequence selected from:
   - SEQ ID NO: 207 and SEQ ID NO: 208;
   - SEQ ID NO: 209 and SEQ ID NO: 210; and
   - SEQ ID NO: 211 and SEQ ID NO: 212.

### BRIEF DESCRIPTION OF THE DRAWINGS

**Figure 1** shows the binding properties of PRO4710 (IgG4 of scFv PRO3744, 68-18-E11-sc07) to members of LILRBA and LILRB family expressed by CHO cells.
**Figure 2** shows the binding properties of PRO3635 (IgG of scFv PRO3064, 68-18-E11-sc02) and the references PRO3483 (NGM), PRO2518 (J19) and PRO3848 (Biond) to members of LILRA family expressed by CHO cells. An anti-V5 tag antibody served as positive control as all constructs contain V5 tag at N-terminus.
**Figure 3** shows the binding properties of PRO3635 (IgG of scFv PRO3064, 68-18-E11-sc02) and the references PRO3483 (NGM), PRO2518 (J19) and PRO3848 (Biond) to members of LILRB family expressed by CHO cells. An anti-V5 tag antibody served as positive control as all constructs contain V5 tag at N-terminus.
**Figure 4** shows the binding of the scFv PRO3153 (69-01-E02-sc03) applied at concentrations of 150 nM (A) and 50 nM (B) to members of LILRBA and LILRB family expressed by CHO cells.
**Figure 5** shows the binding properties of the scFv PRO3697 (69-06-G04-sc05), cross-linked with an anti-framework antibody (x-linked), to members of LILRBA and LILRB family expressed by CHO cells.
**Figure 6** shows the dose-dependent increase of the M1 marker CD86 and the dose-dependent decrease of the M2 marker CD163 in the presence of increasing concentrations of PRO3744, PRO3696 and PRO3697.
**Figure 7** shows phagocytosis of CFSE-labelled H292 tumor cells by M2-like HMDMs in the presence of increasing concentrations of PRO2777, PRO3599 and PRO3790. Assessment of phagocytosis = % CFSE-positive HMDMs.
**Figure 8** shows the results of LPS-driven secretion of IL-1β, TNF-α, and IL-6 in presence of increasing concentrations of multispecific antibodies PRO4672, PRO5052, PRO5053, PRO4687, PRO5055, PRO5056 and anti-LILRB2 antibody PRO4710.
**Figure 9** shows the results of LPS-driven secretion of IL-1β, TNF-α, and IL-6 in presence of increasing concentrations of anti-LILRB2 antibody, multispecific antibodies PRO4710, PRO4672, PRO4673, PRO4687, PRO4688, PRO5052, and reference antibodies atezolizumab (Atezo), avelumab, J19.H1 (PRO2518), PRO3599 (Pfizer), and magrolimab (PRO2777).
**Figure 10** shows the phagocytosis potency of trispecific antibodies PRO4672, PRO4687, PRO5052 and PRO5055 and of the reference compounds PRO2777, PRO3599 and PRO3851 for H292 cancer cells (A) and (C), expressing PDL1 and CD47, and for NALM6 cancer cells (B) and A375 cancer cells (D), expressing only CD47.
**Figure 11** shows the phagocytosis potency of trispecific antibodies PRO5052 and PRO5055 and of the reference compounds PRO2777, avelumab, PRO2777 + avelumab combination, PRO5134 (silenced Fc), PRO5054 (no CD47-BD) and PRO5057 (no CD47-BD) for H292 cancer cells (A) and (C), expressing PDL1 and CD47, and for A375 cancer cells (B) and (D), expressing only CD47.
**Figure 12** shows the phagocytosis potency of trispecific antibodies PRO5052, PRO5053, PRO5055 and PRO5056 and of the reference compounds PRO2777, PRO2777 + PRO2734 combination, PRO2777 + PRO2734 + avelumab combination, PRO5134 (silenced Fc), PRO5054 (no CD47-BD) and PRO5057 (no CD47-BD) for H292 cancer cells (A) and (C), expressing PDL1 and CD47, and for A375 cancer cells (B) and (D), expressing only CD47.
**Figure 13** shows the phagocytosis potency of the trispecific antibodies PRO4672, PRO4687, PRO5055, of the bispecific antibody PRO3790, of the comparative antibodies PRO4682 (CD47-BD in Fab), PRO4711 (CD47-BD from PRO3599), PRO4677 (inverse Morrison) and of the reference compounds PRO2777, PRO3599, for H292 cancer cells (A and C) and red blood cells (RBCs) (B C and D).
**Figure 14** shows the phagocytosis potency of the trispecific antibodies PRO4672, PRO4673, PRO4687, PRO4688, of the comparative antibodies PRO4682 and PRO4683 (CD47-BD in Fab), and of the reference antibodies PRO2777, PRO3609 and PRO3613, for H292 cancer cells (A and C) and red blood cells (RBCs) (B and D).
**Figure 15** shows the phagocytosis potency of the multispecific antibodies PRO5052, PRO5053, PRO5055 and PRO5056 towards H292 tumor cell and red blood cells (RBCs) in parallel to illustrates the potential therapeutic window.
**Figure 16** shows the T-cell proliferation potency of the anti-LILRB2 antibody PRO4710, the trispecific antibodies PRO5052, PRO5053 (no-LILRB2-BD), PRO5055, PRO5056 (no-LILRB2-BD), PRO5054 (no CD47-BD), and the reference antibodies avelumab and magrolimab.
**Figure 17** shows the release of TNFα and IL-1β mediated by the anti-LILRB2 antibody PRO4710, the trispecific antibodies PRO4672, PRO4673 (no LILRB2-BD), PRO4687, PRO4688 (no LILRB-BD), PRO5052, PRO5055, PRO5056 (no LILRB2-BD), by the comparative antibodies PRO5054 (no CD47-BD), PRO3613 (medium affinity CD47-BD), and by the reference antibodies magrolimab, atezolizumab (Atezo), PRO3599, PRO3851 and PRO2777.
**Figure 18** shows the hemagglutination assay result for trispecific antibodies PRO5052, PRO5055, and reference antibodies PRO2777, PRO3599.
**Figure 19** shows the hemagglutination assay result for the trispecific antibodies PRO5052, PRO5055, PRO4672, PRO4687, and reference antibodies PRO2777, PRO3851 and PRO3599.
**Figure 20** shows the binding of PRO5052, PRO5055 to cancer cell lines HT-29 (A), the binding of PRO5052 and PRO4672 to cancer cell line HT-1080 (B), the binding of PRO4687, PRO5055 and reference antibodies PRO3851 and PRO2777 to cancer cell lines HT-29 (C) and HT-1080 (D). PRO2777 and PRO3851 were applied as reference antibodies.
**Figure 21** shows the inhibition of CD47/SIRPα interaction on CHO-CD47 cells (A), NCI-H292 cells (B) and NCI-H292 cells treated with 10 ng/ml INFy (C) for PRO4672, PRO5052 and the reference antibodies PRO2777 and PRO3599.
**Figure 22** shows the inhibition of CD47/SIRPα interaction on CHO-CD47 cells (A), NCI-H292 cells (B), NCI-H292 cells treated with 10 ng/ml INFy (C) for PRO4687, PRO5055 and the reference antibodies PRO2777 and PRO3851.
**Figure 23** shows the binding properties of PRO5052 (A) and PRO5055 (B) to members of LILRBA and LILRB family expressed by CHO cells.

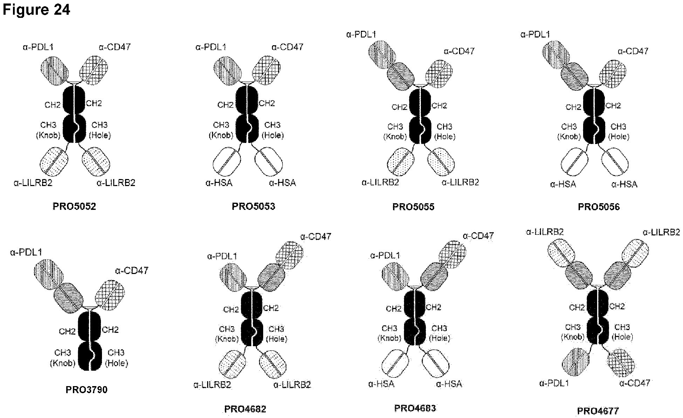

**Figure 24** shows the formats of the multispecific antibodies PRO3790, PRO5052, PRO5053, PRO5055, PRO5056 and the comparative antibodies PRO4677, PRO4682 and PRO4683.

### DETAILED DESCRIPTION OF THE INVENTION

Known antibody therapeutics targeting CD47 often suffer from dose limiting toxicities, in particular dose limiting toxicities caused by agglutination of red blood cells. Antibody therapeutics targeting PDL1 often lack killing potency even for PDL1-expressing tumor types. Consequently, the development of anti-CD47xPDL1 multispecific antibodies to target cancer cells that use CD47- and PDL1-based escape mechanisms is challenging. There is a need in the medical field to provide anti-CD47xPDL1 antibody-based therapeutics, which provide efficient killing of CD47- and PDL1-expressing cancer cells, while exhibiting low or no dose limiting toxicities.

The present invention provides multispecific antibodies comprising one CD47-BD and one PDL1-BD, and an IgG-like region comprising an immunoglobulin Fc region, a first Fab arm and a second Fab arm, wherein the CD47 BD is an scFv fragment replacing the first Fab arm, and the PDL1 BD is located in the second Fab arm or is an scFv fragment replacing said second Fab arm. These multispecific antibodies do not cause phagocytosis of red blood cells or trigger any visible red blood cell agglutination even at high nanomolar concentrations. At the same time, they exhibit a high potency in killing cancer cells that express CD47 and PDL1.

It is believed by the present inventors that the observed selectivity, and in particular the absence of red blood cell agglutination and red blood cell lysis at nanomolar concentrations, is due to the presence of moderately affine CD47-BD, in combination with a well-defined IgG-like antibody architecture, wherein the CD47-BD is present in the form of an scFv.

Alternative antibody architectures, e.g. antibody architectures wherein said CD47-BD is not in scFv format and/or the PDL1-BD and the CD47-BD are not located next to each other in the Fab arms, do not exhibit the desired combined properties, *i*. *e*. potent cancer cell lysis and at the same time low or no agglutination/lysis of red blood cells. For example, antibody formats wherein the CD47-BD is in Fab format exhibit significant red blood cell agglutination. IgG like anti-PDL1 × CD47 antibody formats, where the PDL1-BD and CD47-BD is placed at the C-terminus of the Fc region, still exhibit low to no red blood cell agglutination, but lose the ability to kill cancer cells. Likewise, Fc silenced IgG-like formats or Fc less antibody formats, e.g. Numab's MATCH formats, do not provide the desired cancer cell killing potency. The presence of a Fc region, which has at least some Fc receptor binding functionality, is apparently important for efficient cancer cell killing.

It has further been found that the exemplified multispecific antibodies of the present invention, *e*. *g*. PRO3790, PRO4672, PRO4673, PRO4687, PRO4688, PRO5052, PRO5053, PRO5055, PRO5056, exhibit lower red blood cell agglutination and/or red blood cell lysis than the prior art anti-CD47xPDL1 antibodies PF-07257876 (Pfizer Inc.) and IBI-322 (Innovent Biologics Inc), while exhibiting similar or even better *in-vitro* cancer cell killing potency. It is thus expected that the multispecific antibodies of the present invention will provide a large therapeutic window and thus provide distinct therapeutic advantages over conventional therapies.

In addition, the multispecific antibodies of the present invention exhibit very advantageous biophysical properties, in particular high storage stability. For example, the loss of monomeric content for PRO4672, PRO5052, PRO4687 and PRO5055, which are representative multispecific antibodies of the present invention, is less than 1 % when stored for two months at 4°C at a concentration of 10 mg/ml in a 20 mM Histidine buffer (pH 6.0) with 125 mM NaCl and 0.02 % Poloxamer 188. This high stability is particularly surprising in view of the fact that the multispecific antibodies of the invention comprise up to four scFv antibody fragments.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

The term "antibody" and the like, as used herein, includes whole antibodies or single chains thereof; and any antigen-binding fragment (*i*. *e*., "antigen-binding portion") or single chains thereof; and molecules comprising antibody CDRs, VH regions or VL regions (including without limitation multispecific antibodies). A naturally occurring "whole antibody" is a glycoprotein comprising at least two heavy (H) chains and two light (L) chains interconnected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs), flanked by regions that are more conserved, termed framework regions (FRs). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e. g*., effector cells) and the first component (C1q) of the classical complement system.

The term "immunoglobulin Fc region" or "Fc region", as used herein, is used to define a C-terminal region of an immunoglobulin heavy chain, *i*. *e*. the CH2 and CH3 domains of the heavy chain constant regions. The Fc region provides linkage to humoral and cellular components of the immune system, mostly through interaction with C1q and Fc receptors (FcR). The term "Fc region" includes Fc regions of an IgG subclass, particularly an immunoglobulin Fc region of human IgG1, IgG2 (lgG2A, IgG2B), IgG3 or IgG4. Further included are Fc regions that are engineered, *i*. *e*. Fc regions that have one or more amino acid modifications, to exhibit certain desired properties. Such amino acid modifications are for example modifications to enhance heterodimerization of heavy chains, such as knob-into-hole (KiH) technology (see for example Ridgway et al., Protein Eng. 9:617-21 (1996) and Spiess et al., J Biol Chem. 288(37):26583-93 (2013)). Other amino acid modifications are modifications that alter, i.e. increase or decrease Fc receptor binding, when compared to the respective unmodified immunoglobulin Fc region thereof (see for example the modifications disclosed in WO 2004/42072 (Presta) and in Wang et al., Protein Cell. 9(1):63-732018 (2018)) and/or increase serum half-life (see for example the modifications disclosed in Liu et al., Antibodies (Basel). 9(4):64 (2020)).

The term "Fc receptor" or "FcR" describes a receptor that binds to the Fc region of an antibody. Particularly, the Fc receptors are native sequence human FcRs, which binds an IgG antibody. These include for example the Fc gamma receptors (FcγRs). Five activating FcyRs are known, *i. e*. the high affinity receptors FcγRI, and the lower affinity receptors FcγRIIA, FcγRIIC, FcγRIIIA and FcγRIIIB. Besides, one inhibiting receptor is known, *i*. *e*. FcγRIIB, which have similar amino acid sequences to FcγRIIA that differ primarily in the cytoplasmic domains thereof. Activating receptors contain an immunoreceptor tyrosine-based activation motif (ITAM) in its cytoplasmic domain. The inhibiting receptor FcγRIIB contains an immunoreceptor tyrosine-based inhibition motif (ITIM) in its cytoplasmic domain, (see M. Daeron, Annu. Rev. Immunol. 5:203-234 (1997). FcRs are reviewed for example in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor FcRn, which plays a central role in the cellular trafficking and serum half-life of IgGs. High-affinity binding to FcRn *in vivo* is reported to prolong serum half-life of human IgGs (see for example Dall'Acqua et al., J Biol Chem. 281:23514-23524(2006)).

The term "silenced Fc", "Fc silenced" or "silenced Fc region", as used herein, refers to an Fc region, which has been engineered to exhibit insignificant or negligible Fc receptor-binding functionality, in particular insignificant or negligible binding affinity to FcyRs, when compared to its unmodified version. Antibodies having such silenced Fc regions do not or do not significantly trigger cellular immune responses through Fc receptor binding.

The term "active Fc" or "active Fc region", as used herein, refers to an Fc region, which has significant Fc receptor binding functionality, in particular significant binding affinity to FcyRs. This Fc receptor binding functionality may be present naturally and/or may be introduced or enhanced by engineering. Antibodies having such active Fc regions trigger cellular immune responses through Fc receptor binding.

In particular embodiments, the immunoglobulin Fc region comprised in the multispecific antibodies of the invention is selected from an Fc region of an IgG subclass, particularly an immunoglobulin Fc region of human IgG1, IgG2 (IgG2A, IgG2B), IgG3 or IgG4, particularly of human IgG1 or IgG2, particularly of human IgG1.

In some embodiments, the immunoglobulin Fc region comprised in the multispecific antibodies of the invention is an active Fc region.

In further embodiments, the immunoglobulin Fc region comprised in the multispecific antibodies of the invention has one or more amino acid modifications selected from the group consisting of:
a) modifications to enhance heterodimerization of heavy chains, such as the knob-into-hole (KiH) technology;
b) modifications that increase or decrease, in particular increase, Fc receptor binding, when compared to the respective unmodified immunoglobulin Fc region thereof;
c) modifications that increase serum half-life, when compared to the respective unmodified immunoglobulin Fc region thereof.

In particular embodiments, the immunoglobulin Fc region comprised in the multispecific antibodies of the invention has one or more amino acid modifications to enhance heterodimerization of the heavy chains, such as the knob-into-hole (KiH) technology.

The terms "binding domain", "antigen-binding fragment thereof", "antigen-binding portion" of an antibody, and the like, as used herein, refer to one or more fragments of an intact antibody that retain the ability to specifically bind to a given antigen (*e. g*., CD47, PDL1, LILRB2). Antigen-binding functions of an antibody can be performed by fragments of an intact antibody. Specifically, in case of the multispecific antibodies of the present invention, the terms "binding domain", "antigen-binding fragment", "antigen-binding fragment thereof", "antigen-binding portion", and the like, as used herein, refer to a Fab fragment, *i. e*. a monovalent fragment consisting of the VL, VH, CL and CH1 domains; a single chain Fab, *i*. *e*. a Fab fragment, where the heavy chain and the light chain are connected via a linker; an Fv fragment consisting of the VL and VH domains of a single arm of an antibody; a disulfide stabilized Fv fragment (dsFv); and a single chain Fv fragment (scFv). Preferably, the binding domains of the antibodies of the present invention are independently of each other selected from a Fab fragment, an Fv fragment, a dsFv fragment and a single-chain Fv fragment (scFv). In particular embodiments, the binding domains of the antibodies of the present invention are independently of each other selected from a Fab fragment and a single-chain Fv fragment (scFv). In other particular embodiments, the VL and VH domains of the scFv fragment are stabilized by an interdomain disulfide bond (dsscFv), in particular said VH domain comprises a single cysteine residue in position 51 (AHo numbering) and said VL domain comprises a single cysteine residue in position 141 (AHo numbering).

The term "fragment-based" or "fragment-based antibody", as used herein, refers to an antibody that comprises at least one antigen-binding fragment, as defined above, but no immunoglobulin Fc region. Due to the lack of the Fc region, these antibodies typically comprise and hSA binding domain for half-life extension.

The term "Complementarity Determining Regions" ("CDRs") refers to amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273, 927-948 ("Chothia" numbering scheme); ImMunoGenTics (IMGT) numbering (Lefranc, M.-P., The Immunologist, 7, 132-136 (1999); Lefranc, M.-P. et al., Dev. Comp. Immunol., 27, 55-77 (2003)) ("IMGT" numbering scheme); and the numbering scheme described in Honegger & Plückthun, J. Mol. Biol. 309 (2001) 657-670 ("AHo" numbering). For example, for classic formats, under Kabat, the CDR amino acid residues in the heavy chain variable region (VH) are numbered 31-35 (HCDR1), 50-65 (HCDR2), and 95-102 (HCDR3); and the CDR amino acid residues in the light chain variable region (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Plückthun ("AHo") is used (Honegger & Plückthun, J. Mol. Biol. 309 (2001) 657-670), unless specifically mentioned otherwise. In particular, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable region usually not all positions 1 to 149 will be occupied by an amino acid residue.

The term "binding specificity" as used herein refers to the ability of an individual antibody to react with one antigenic determinant and not with a different antigenic determinant. As used herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the antibody to discriminate between the target of interest and an unrelated molecule, as determined, for example, in accordance with specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard color development (*e*. *g*. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, particularly at least 100-fold difference between a background and signal indicates on specific binding. Typically, determination of binding specificity is performed by using not a single reference molecule, but a set of about three to five unrelated molecules, such as milk powder, transferrin or the like.

Suitably, the antibodies of the invention are isolated antibodies. The term "isolated antibody", as used herein, refers to an antibody that is substantially free of other antibodies having different antigenic specificities (*e*.*g.*, an isolated antibody that specifically binds CD47 and PDL1 is substantially free of antibodies that specifically bind antigens other than CD47 and PDL1. Moreover, an isolated antibody may be substantially free of other cellular material and/or chemicals.

Suitably, the antibodies of the invention are monoclonal antibodies. The term "monoclonal antibody" as used herein refers to antibodies that have substantially identical amino acid sequences or are derived from the same genetic source. A monoclonal antibody displays a binding specificity and affinity for a particular epitope, or binding specificities and affinities for specific epitopes.

Antibodies of the invention include, but are not limited to, chimeric, human and humanized antibodies.

The term "chimeric antibody" (or antigen-binding fragment thereof), as used herein, refers to an antibody molecule (or antigen-binding fragment thereof) in which (a) the constant region, or a portion thereof, is altered, replaced or exchanged so that the antigen-binding site (variable region) is linked to a constant region of a different or altered class, effector function and/or species; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" (or antigen-binding fragment thereof), as used herein, is intended to include antibodies (and antigen-binding fragments thereof) having variable regions in which both the framework and CDR regions are derived from sequences of human origin. Furthermore, if the antibody contains a constant region, the constant region also is derived from such human sequences, *e. g*., human germline sequences, or mutated versions of human germline sequences. The human antibodies and antigen-binding fragments thereof of the invention may include amino acid residues not encoded by human sequences (*e*. *g.,* mutations introduced by random or site-specific mutagenesis *in vitro* or by somatic mutation *in vivo*). This definition of a human antibody specifically excludes a humanized antibody comprising non-human antigen-binding residues. Human antibodies can be produced using various techniques known in the art, including phage-display libraries (Hoogenboom and Winter, J. Mol. Biol, 227:381 (1992); Marks et al, J. Mol. Biol, 222:581 (1991)). Also available for the preparation of human monoclonal antibodies are methods described in Cole et al, Monoclonal Antibodies and Cancer Therapy, Alan R. Liss, p. 77 (1985); Boemer et al, J. Immunol, 147(I):86-95 (1991). See also van Dijk and van de Winkel, Curr. Opin. Pharmacol, 5: 368-74 (2001). Human antibodies can be prepared by administering the antigen to a transgenic animal that has been modified to produce such antibodies in response to antigenic challenge, but whose endogenous loci have been disabled, *e*. *g.,* immunized xenomice (see, *e*. *g.,* U.S. Pat. Nos. 6,075,181 and 6,150,584 regarding XENOMOUSE^{™} technology). See also, for example, Li et al, Proc. Natl. Acad. Sci. USA, 103:3557- 3562 (2006) regarding human antibodies generated via a human B-cell hybridoma technology.

The term "humanized" antibody (or antigen-binding fragment thereof), as used herein, refers to an antibody (or antigen-binding fragment thereof) that retains the reactivity of a non-human antibody while being less immunogenic in humans. This can be achieved, for instance, by retaining the non-human CDR regions and replacing the remaining parts of the antibody with their human counterparts (*i. e*., the constant region as well as the framework portions of the variable region). Additional framework region modifications may be made within the human framework sequences as well as within the CDR sequences derived from the germline of another mammalian species. In particular, it is known that CDR1 of VH and CDR2 of VL are less important for binding specificity, as evidenced by the fact that in many antibodies obtained by immunization, those CDRs are identical to the CDR encoded by the corresponding germline gene segment. Thus, in one embodiment of the invention, the antibody or antigen-binding fragment thereof comprises in CDR1 of VH and/or in CDR2 of VL one or two amino acid residues that differ from the amino acid residues of the parental antibody. The humanized antibodies of the invention may include amino acid residues not encoded by human sequences (*e. g*., mutations introduced by random or site-specific mutagenesis *in vitro* or by somatic mutation *in vivo*, or a conservative substitution to promote stability or manufacturing). See, *e. g*., Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include but are not limited to the Xoma technology disclosed in U.S. Pat. No. 5,766,886.

The term "recombinant humanized antibody" as used herein, includes all human antibodies that are prepared, expressed, created or isolated by recombinant means, such as antibodies isolated from a host cell transformed to express the humanized antibody, *e*.*g.*, from a transfectoma, and antibodies prepared, expressed, created or isolated by any other means that involve splicing of all or a portion of a human immunoglobulin gene, sequences to other DNA sequences.

Preferably, the multispecific antibodies of the invention are humanized. More preferably, the multispecific antibodies of the invention are humanized and comprises rabbit derived CDRs.

The term "multispecific antibody" as used herein, refers to an antibody that binds to two or more different epitopes on at least two or more different targets (*e. g*., CD47, PDL1, hSA and LILRB2). Preferably, the multispecific antibodies of the invention are bispecific or trispecific. The term "bispecific antibody" as used herein, refers to an antibody that binds to at least two different epitopes on two different targets (*e. g*., CD47 and PDL1). The term "trispecific antibody" as used herein, refers to an antibody that binds to at least three different epitopes on three different targets (*e. g*., CD47, PDL1, hSA and LILRB2).

The term "epitope" means a protein determinant capable of specific binding to an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope, wherein all points of interaction between the protein and the interacting molecule (such as an antibody) occurring linearly along the primary amino acid sequence of the protein (continuous).

The term "recognize" as used herein refers to an antibody antigen-binding fragment thereof that finds and interacts (*e. g*., binds) with its conformational epitope.

The term "avidity" as used herein refers to an informative measure of the overall stability or strength of the antibody-antigen complex. It is controlled by three major factors: antibody epitope affinity; the valency of both the antigen and antibody; and the structural arrangement of the interacting parts. Ultimately these factors define the specificity of the antibody, that is, the likelihood that the particular antibody is binding to a precise antigen epitope.

Suitably, the multispecific antibodies of the invention comprise one CD47 biding domain.

The term "CD47" refers in particular to human CD47 with UniProt ID number Q08722. Suitably, the CD47-BD of the present invention targets CD47, in particular human CD47. Particularly, the multispecific antibodies of the invention comprises one CD47-BD that target human and cynomolgus (*Macaca fascicularis*) CD47.

Suitably, the CD47-BD, when in scFv format, binds to human CD47 with a monovalent dissociation constant (KD) of 1 to 100 nM, particularly with a KD of 2 to 50 nM, particularly of 5 to 25 nM, as measured by surface plasmon resonance (SPR).

As used herein, the term "affinity" refers to the strength of interaction between the antibody and the antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the total sum of non-covalent interactions between a single binding site of a molecule (*e. g*., of an antibody) and its binding partner (*e*. *g.,* an antigen or, more specifically, an epitope on an antigen). Unless indicated otherwise, as used herein, "binding affinity", "bind to", "binds to" or "binding to" refers to intrinsic binding affinity that reflects a 1:1 interaction between members of a binding pair (*e*. *g*., an antibody fragment and an antigen). The affinity of a molecule X for its partner Y can generally be represented by the dissociation constant (K_{D}). Affinity can be measured by common methods known in the art, including those described herein. Low-affinity antibodies generally bind antigens slowly and tend to dissociate readily, whereas high-affinity antibodies generally bind antigens faster and tend to remain bound longer. A variety of methods of measuring binding affinity are known in the art, any of which can be used for purposes of the present invention. Specific illustrative and exemplary embodiments for measuring binding affinity, *i. e*. binding strength are described in the following.

The term "K_{assoc}", "Kₐ" or "Kₒₙ", as used herein, are intended to refer to the association rate of a particular antibody-antigen interaction, whereas the term "K_{dis}", "K_{d}" or "K_{off}", as used herein, is intended to refer to the dissociation rate of a particular antibody-antigen interaction. In one embodiment, the term "K_{D}", as used herein, is intended to refer to the dissociation constant, which is obtained from the ratio of K_{d} to Kₐ (*i.e*. K_{d}/Kₐ) and is expressed as a molar concentration (M). The "K_{D}" or "K_{D} value" or "KD" or "KD value" according to this invention is in one embodiment measured by using surface plasmon resonance assays.

Affinity to recombinant human CD47 and recombinant cynomolgus CD47 was determined by surface plasmon resonance (SPR) measurements, as described in Example 2.6 (scFvs) and Examples 4.2 and 5.3 (multispecific molecules). Affinity to recombinant human PDL1 and recombinant cynomolgus PDL1 was determined by surface plasmon resonance (SPR) measurements, as described in Example 1.4 (scFvs) and Examples 4.2 and 5.3 (multispecific molecules). Likewise, affinity to recombinant human LILRB2 and recombinant cynomolgus LILRB2 was determined by surface plasmon resonance (SPR) measurements, as described in Example 3.6 (scFvs) and Example 5.3 (multispecific molecules).

Suitably, the multispecific antibody of the invention acts as a blocker of the CD47 interaction with SIRPα. In other words, the CD47-BD of the multispecific antibody of the invention is an inhibitor of CD47 signaling. The term "blocker" or "inhibitor" or "antagonist", as used herein, refers to an antibody or binding domain thereof that inhibits or reduces a biological activity of the antigen it binds to. The CD47-BD of the multispecific antibody of the invention bind to CD47, thereby blocking the binding of CD47 to SIRPα, which leads to reduced CD47 function.

Suitably, the CD47-BD of the multispecific antibody of the present invention is further characterized by one or more of the following parameters:
a. when in scFv format, has a melting temperature (Tm), determined by differential scanning fluorimetry, in the range of 50°C to 70°C, particularly in the range of 55°C to 65°C, in particular wherein said antibody is formulated in 50 mM phosphate-citrate buffer (NaCiP) at pH 6.4, 150 mM NaCl;
b. when in scFv format, has a loss in monomer content, after storage for four weeks at -80°C, of less than 3 %, less than 2 %, less than 1 %, particularly less than 0.5 %, when the antibody of the invention is at a starting concentration of 10 mg/ml, and in particular wherein said antibody is formulated in 50 mM phosphate citrate buffer (NaCiP) with 150 mM NaCl at pH 6.4;
c. when in scFv format, has a loss in monomer content, after storage for four weeks at 4°C, of less than 3 %, less than 2 %, less than 1 %, particularly less than 0.5 %, when the antibody of the invention is at a starting concentration of 10 mg/ml, and in particular wherein said antibody is formulated in 50 mM phosphate citrate buffer (NaCiP) with 150 mM NaCl at pH 6.4; and/or
d. when in scFv format, has a loss in monomer content, after storage for four weeks at 40°C, of less than 3 %, less than 2 %, less than 1 %, particularly less than 0.5 %, when the antibody of the invention is at a starting concentration of 10 mg/ml, and in particular wherein said antibody is formulated in 50 mM phosphate citrate buffer (NaCiP) with 150 mM NaCl at pH 6.4.

Differential scanning fluorimetry (DSF) measurements are performed as described earlier (Egan, et al., MAbs, 9(1) (2017), 68-84; Niesen, et al., Nature Protocols, 2(9) (2007) 2212-2221). In this DSF measurement procedure, the midpoint of transition for the thermal unfolding of the scFv constructs is determined by using the fluorescence dye SYPRO Orange (see Wong & Raleigh, Protein Science 25 (2016) 1834-1840). Typically, samples in phosphate-citrate buffer at pH 6.4 are prepared at a final protein concentration of 50 µg/ml and containing a final concentration of 5x SYPRO Orange in a total volume of 100 µl. Twenty-five microliters of prepared samples are added in triplicate to white-walled AB gene PCR plates. The assay is performed in a qPCR machine used as a thermal cycler, and the fluorescence emission is detected using the software's custom dye calibration routine. The PCR plate containing the test samples is subjected to a temperature ramp from 25°C to 96°C in increments of 1°C with 30 s pauses after each temperature increment. The total assay time is about 2 h. The Tm is calculated by the software GraphPad Prism using a mathematical second derivative method to calculate the inflection point of the curve. The reported Tm is typically an average of three measurements.

The loss in monomer content is as determined by area under the curve calculation of SE-HPLC chromatograms. SE-HPLC is a separation technique based on a solid stationary phase and a liquid mobile phase as outlined by the US Pharmacopeia (USP), chapter 621. This method separates molecules based on their size and shape utilizing a hydrophobic stationary phase and aqueous mobile phase. The separation of molecules is occurring between the void volume (V₀) and the total permeation volume (V_{T}) of a specific column. Measurements by SE-HPLC are performed on a Chromaster HPLC system (Hitachi High-Technologies Corporation) equipped with automated sample injection and a UV detector set to the detection wavelength of 280 nm. The equipment is controlled by the software EZChrom Elite (Agilent Technologies, Version 3.3.2 SP2) which also supports analysis of resulting chromatograms. Protein samples are cleared by centrifugation and kept at a temperature of 4-6°C in the autosampler prior to injection. For the analysis of scFv samples the column Shodex KW403-4F (Showa Denko Inc., #F6989202) is employed with a standardized buffered saline mobile phase (50 mM sodium-phosphate pH 6.5, 300 mM sodium chloride) at the recommended flow rate of 0.35 ml/min. The target sample load per injection was 5 µg. Samples are detected by an UV detector at a wavelength of 280 nm and the data recorded by a suitable software suite. The resulting chromatograms are analyzed in the range of V₀ to V_{T} thereby excluding matrix associated peaks with >10 min elution time.

Suitably, the PDL1-BDs of the multispecific antibodies of the invention are binding domains provided in the present disclosure. The PDL1-BDs of the multispecific antibodies of the invention include, but are not limited to, the humanized PDL1-BDs whose sequences are listed in Table 1.

Suitably, the multispecific antibodies of the invention comprise one PDL1-BD.

The term "PD-L1" or "PDL1" refers in particular to human PDL1 with UniProt ID number Q9NZQ7. Suitably, the PDL1-BD of the multispecific antibodies of the invention targets, in particular human PDL1. Particularly, the multispecific antibodies of the invention comprise one PDL1-BD that targets human and cynomolgus (*Macaca fascicularis*) PDL1.

Suitably, the PDL1-BD used in the present invention is characterized by one or more of the following parameters:
a. when in scFv format, binds to human PDL1 with a monovalent dissociation constant (KD) of 1 to 100 pM, particularly with a KD of 1 to 50 pM, particularly of 1 to 40 pM, as measured by surface plasmon resonance (SPR);
b. when in scFv format, is cross-reactive with *Macaca fascicularis* (cynomolgus) PDL1, in particular binds to cynomolgus PDL1 with a monovalent KD of 1 to 100 pM, particularly with a KD of 1 to 50 pM, particularly of 1 to 40 pM, as measured by SPR;
c. when in scFv format, has the ability to neutralize PDL1/PD-1 interaction, as measured in an NFAT reporter gene assay, with an EC₅₀ value of 0.1 to 10 nM, particularly of 0.1 to 5 pM, particularly of 0.1 to 3 nM;
d. is non-cross-reactive to *Mus musculus* PDL1, as measured by SPR; and/or
e. does not bind to human PDL2, as measured by SPR.

Suitably, the PDL1-BD of the multispecific antibodies of the present invention is further characterized by one or more of the following parameters:
a. when in scFv format, has a melting temperature (Tm), determined by differential scanning fluorimetry, in the range of 60°C to 85°C, particularly in the range of 65°C to 85°C, particularly in the range of 70°C to 85°C, in particular wherein said antibody is formulated in 50 mM phosphate-citrate buffer at pH 6.4, 150 mM NaCl;
b. when in scFv format, has a loss in monomer content, after five consecutive freeze-thaw cycles, of less than 5 %, preferably less than 3 %, more preferably less than 1 %, when the antibody of the invention is at a starting concentration of 10 mg/ml, and in particular wherein the antibody of the invention is formulated in 50 mM phosphate citrate buffer with 150 mM NaCl at pH 6.4; and/or
c. when in scFv format, has a loss in monomer content, after storage for four weeks at 4°C, of less than 15 %, e.g. less than 12 %, less than 10 %, less than 7 %, less than 5 %, less than 4 %, less than 3 %, less than 2 %, preferably less than 1 %, when the antibody of the invention is at a starting concentration of 10 mg/ml, and in particular wherein said antibody is formulated in 50 mM phosphate citrate buffer with 150 mM NaCl at pH 6.4.

Suitably, the PDL1-BDs of the multispecific antibodies of the invention are binding domains provided in the present disclosure. The PDL1-BDs of the multispecific antibodies of the invention include, but are not limited to, the humanized PDL1-BDs whose sequences are listed in Table 1.

The term "multivalent antibody" refers to a single binding molecule with more than one valency, where "valency" is described as the number of antigen-binding moieties that binds to epitopes on target molecules. As such, the single binding molecule can bind to more than one binding site on a target molecule and/or to more than one target molecule due to the presence of more than one copy of the corresponding antigen-binding moieties. Examples of multivalent antibodies include, but are not limited to bivalent antibodies, trivalent antibodies, tetravalent antibodies, pentavalent antibodies, hexavalent antibodies, and the like.

The term "monovalent antibody", as used herein, refers to an antibody that binds to a single target molecule, and more specifically to a single epitope on a target molecule. Also, the term "binding domain" or "monovalent binding domain", as used herein, refers to a binding domain that binds to a single epitope on a target molecule.

The multispecific antibodies of the invention comprise one CD47-BD and one PDL1-BD, *i. e*. the multispecific antibodies of the invention are monovalent for both CD47 and PDL1 specificity.

In particular embodiments, the KD of the PDL1-BD for binding to human PDL1 is at least 500 fold lower, in particular at least 1000 fold lower, in particular at least 1500 fold lower, in particular at least 2000 fold lower, than the KD of the CD47-BD for binding to human CD47.

The multispecific antibodies of the invention do not trigger any visible red blood cell agglutination at a concentration of 5 nM, particularly of 20 nM, particularly of 50 nM, particularly of 100 nM, particularly of 200 nM, particularly of 500 nM, as determined in a standard hemagglutination assay as described in Example 4.2

The expression "hemagglutination" as used herein, refers to a specific form of agglutination that involves red blood cells (RBCs). More specifically, the expression hemagglutination refers to agglutination of RBCs that is caused by antibodies that bind to surface antigens on the RBCs. Hemagglutination is thus a sensitive method to detect RBC-binding antibodies. The assay procedure to detect such hemagglutination is described in Example 4.2.

Likewise, the multispecific antibodies of the invention do not cause the lysis of red blood cells at a concentration of 5 nM, particularly of 20 nM, particularly of 50 nM, as determined in a standard phagocytosis assay, as described in Example 5.4.

Antibody-dependent cell-mediated cytotoxicity (ADCC) of red blood cells, herein also referred to as "lysis of red blood cell" or "red blood cell phagocytosis", is typically determine analogous to the ADCC of other cells where antibodies can bind to. More specifically, the red blood cell lysis was determined analogous to the lysis of the CD47 expressing or CD47/PDL1-coexpressing cell, as described in Example 5.4.

In a particular aspect, the present invention relates to a multispecific antibody comprising:
a) one binding domain, which specifically binds to CD47 (CD47-BD);
   wherein said CD47-BD comprises:
   i. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 5, and
      the LCDR3 sequences of SEQ ID NO: 7; or
   ii. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 6, and
      the LCDR3 sequences of SEQ ID NO: 7;
b) one binding domain, which specifically binds to PDL1 (PDL1-BD);
   wherein said PDL1-BD comprises:
   i. the HCDR1 sequence of SEQ ID NO: 20,
      the HCDR2 sequences of SEQ ID NO: 21,
      the HCDR3 sequence of SEQ ID NO: 23,
      the LCDR1 sequence of SEQ ID NO: 24,
      the LCDR2 sequence of SEQ ID NO: 25, and
      the LCDR3 sequences of SEQ ID NO: 27; or
   ii. the HCDR1 sequence of SEQ ID NO: 20,
      the HCDR2 sequences of SEQ ID NO: 21,
      the HCDR3 sequence of SEQ ID NO: 23,
      the LCDR1 sequence of SEQ ID NO: 24,
      the LCDR2 sequence of SEQ ID NO: 26, and
      the LCDR3 sequences of SEQ ID NO: 27; or
   iii. the HCDR1 sequence of SEQ ID NO: 20,
      the HCDR2 sequences of SEQ ID NO: 22,
      the HCDR3 sequence of SEQ ID NO: 23,
      the LCDR1 sequence of SEQ ID NO: 24,
      the LCDR2 sequence of SEQ ID NO: 25, and
      the LCDR3 sequences of SEQ ID NO: 27; or
   iv. the HCDR1 sequence of SEQ ID NO: 20,
      the HCDR2 sequences of SEQ ID NO: 22,
      the HCDR3 sequence of SEQ ID NO: 23,
      the LCDR1 sequence of SEQ ID NO: 24,
      the LCDR2 sequence of SEQ ID NO: 26, and
      the LCDR3 sequences of SEQ ID NO: 27; or
   v. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 78, and
      the LCDR3 sequences of SEQ ID NO: 80; or
   vi. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 79, and
      the LCDR3 sequences of SEQ ID NO: 80;
c) an IgG-like region comprising an immunoglobulin Fc region, a first Fab arm and a second Fab arm, wherein:
   - the CD47 BD is an scFv fragment replacing the first Fab arm, and
   - the PDL1 BD is located in the second Fab arm or is an scFv fragment replacing said second Fab arm.

In a more particular aspect, the present invention relates to a multispecific antibody comprising:
a) one binding domain, which specifically binds to CD47 (CD47-BD);
   wherein said CD47-BD comprises:
   i. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 5, and
      the LCDR3 sequences of SEQ ID NO: 7; or
   ii. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 6, and
      the LCDR3 sequences of SEQ ID NO: 7;
b) one binding domain, which specifically binds to PDL1 (PDL1-BD);
   wherein said PDL1-BD comprises:
   i. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 78, and
      the LCDR3 sequences of SEQ ID NO: 80; or
   ii. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 79, and
      the LCDR3 sequences of SEQ ID NO: 80;
c) an IgG-like region comprising an immunoglobulin Fc region, a first Fab arm and a second Fab arm, wherein:
   - the CD47 BD is an scFv fragment replacing the first Fab arm, and
   - the PDL1 BD is located in the second Fab arm or is an scFv fragment replacing said second Fab arm.

Other variable regions used in the invention include amino acid sequences that have been mutated, yet have at least 80, 85, 90, 91, 92, 93, 94, 95, 96, 97, 98 or 99 percent identity in the CDR regions with the CDR regions depicted in the sequences described in Table 1. Other variable regions used the invention include mutant amino acid sequences wherein no more than 1, 2, 3, 4 or 5, particularly 1 or 2, amino acids have been mutated in the CDR regions when compared with the CDR regions depicted in the sequence described in Table 1.

Suitably, the VH domains of the binding domains of the multispecific antibodies of the invention, e. g. the CD47-BD, PDL1-BD, hSA-BD or LILRB2-BD, belong to a VH3 or VH4 family. In one embodiment, a binding domain used in the invention comprises a VH domain belonging to the VH3 family.

In the context of the present invention, the term "belonging to VHx family" or "selected from VHx family" means that the respective heavy chain framework sequence or sequences referred to, e. g. FR1, FR2, FR3, FR4, FR1 to FR3, FR1 to FR4, show the highest degree of homology to said VHx family. Likewise, the term "belonging to Vx family" or "selected from Vx family" means that the respective light chain framework sequence or sequences referred to, e. g. FR1, FR2, FR3, FR4, FR1 to FR3, FR1 to FR4, show the highest degree of homology to said Vx family. Examples of VH and VL families are given in Knappik et al., J. Mol. Biol. 296 (2000) 57-86, or in WO 2019/057787.

A specific example of a VH domain belonging to VH3 family is represented by SEQ ID NOs: 151 and 152, and a specific example of a VH domain belonging to VH4 family is represented by SEQ ID NO: 155. Particularly, a VH belonging to VH3 family, as used herein, wherein each FR1 to FR4, independently of each other, have at least 85 %, particularly at least 90 %, more particularly at least 95 % sequence identity to the corresponding FR1 to FR4 of SEQ ID NO: 151 or 152. In particular, heavy chain framework regions FR1 to FR4 are selected from SEQ ID NOs: 151 and 152 (Table 2, regions marked in non-bold). Alternative examples of VH3 and VH4 sequences, and examples of other VHx sequences, may be found in Knappik et al., J. Mol. Biol. 296 (2000) 57-86 or in WO 2019/057787.

Suitably, the VL domains of the binding domains used in the invention comprise: Vκ frameworks FR1 to FR4, particularly Vκ1 or Vκ3 frameworks FR1 to FR4, particularly Vκ1 frameworks FR1 to FR4. In the case where said binding domains are not in a Fab format, for example where said binding domains are in an scFv-format, said binding domains comprise: Vκ frameworks FR1 to FR3, particularly Vκ1 or Vκ3 frameworks FR1 to FR3, particularly Vκ1 frameworks FR1 to FR3, and a framework FR4, which is selected from a Vλ FR4.

A specific example of a VL having Vκ1 frameworks FR1 to FR4 is represented by SEQ ID NO: 158 (Table 2, FR regions are marked in non-bold). Suitable Vκ1 frameworks FR1 to FR3 with an exemplary Vλ FR4 are set forth in SEQ ID NOs: 159 and 160 (Table 2, FR regions are marked in non-bold). Alternative examples of Vκ1 sequences, and examples of Vκ2, Vκ3 or Vκ4 sequences, may be found in Knappik et al., J. Mol. Biol. 296 (2000) 57-86. Suitable Vκ1 frameworks FR1 to FR43 or FR1 to FR4 also comprise, independently of each other, the amino acid sequences having at least 70, 80, 90 percent identity to each of the amino acid sequences corresponding to FR1 to FR3 or FR1 to FR4 of SEQ ID NO: 158 (Table 2, FR regions are marked in non-bold). Suitable Vλ FR4 are as set forth in SEQ ID NO: 161 to SEQ ID NO: 168 and in SEQ ID NO: 169 comprising a single cysteine residue, particular in a case where a second single cysteine is present in the corresponding VH chain, particularly in position 51 (AHo numbering) of VH, for the formation of an inter-domain disulfide bond. In one embodiment, the VL domains of the binding domains of the multispecific antibodies of the invention, when being in Fv or scFv-format, comprises Vλ FR4 comprising the amino acid sequence having at least 80, particularly 90, percent identity to an amino acid sequence selected from any of SEQ ID NO: 161 to SEQ ID NO: 169, or in other words having 1 or 2substitutions when compared to the closest amino acid sequence selected from any of SEQ ID NO: 161 to SEQ ID NO: 169. Particularly, the multispecific antibodies of the invention, when being in Fv or scFv-format, comprises Vλ FR4 comprising an amino acid sequence selected from SEQ ID NOs: 161 or 169.

The binding domains of the multispecific antibodies of the invention comprise a VH domain listed in Tables 1 and 2. Suitably, the binding domains used the invention comprise a VH amino acid sequence listed in Tables 1 and 2, wherein no more than 20 amino acids in the framework sequences (i. e., the sequences which are not CDR sequences) have been mutated (wherein a mutation is, as various non-limiting examples, an addition, substitution or deletion). Suitably, the binding domains of the multispecific antibodies of the invention comprise a VH amino acid sequence listed in one of Tables 1 and 2, wherein no more than 15 amino acids, particularly no more than 10 amino acids, particularly no more than 5 amino acids in the framework sequences (*i. e*., the sequences which are not CDR sequences) have been mutated (wherein a mutation is, as various non-limiting examples, an addition, substitution or deletion). Other binding domains used in the invention include amino acids that have been mutated, yet have at least 80, 85, 90, 91, 92, 93, 94, 95, 96, 97, 98 or 99 percent identity in the VH regions with the VH regions depicted in the corresponding sequences described in Tables 1 and 2, including VH domains comprising at least positions 5 to 140 (AHo numbering), particularly at least positions 3 to 145 of one of the sequences shown in Tables 1 and 2.

In particular, the binding domains of the multispecific antibodies of the invention comprise a VL domain listed in Tables 1 and 2. Suitably, the binding domains of the multispecific antibodies of the invention comprise a VL amino acid sequence listed in Tables 1 and 2, wherein no more than about 20 amino acids in the framework sequences (i. e., the sequences which are not CDR sequences) have been mutated (wherein a mutation is, as various non-limiting examples, an addition, substitution or deletion). Suitably, the binding domains of the multispecific antibodies of the invention comprise a VL amino acid sequence listed in Tables 1 and 2, wherein no more than about 15 amino acids, particularly no more than 10 amino acids, particularly no more than 5 amino acids in the framework sequences (*i*. *e*., the sequences which are not CDR sequences) have been mutated (wherein a mutation is, as various non-limiting examples, an addition, substitution or deletion). Other binding domains used in the invention include amino acids that have been mutated, yet have at least 80, 85, 90, 91, 92, 93, 94, 95, 96, 97, 98 or 99 percent identity in the VL regions with a VL region depicted in the sequences described in Tables 1 and 2, including VL domains comprising at least positions 5 to 140 (AHo numbering), particularly at least positions 3 to 145 of one of the sequences shown in Tables 1 and 2.

In the context of the present invention, the term "binding domain used in the present invention" relates to a binding domain as such, *i. e*. independent of a multispecific context, and, in particular, to a binding domain comprised in a multispecific construct, *e. g*. one of the binding domains comprised in a bispecific, trispecific or tetraspecific construct.

Specific but non-limiting examples of the anti-CD47xPDL1 multispecific antibodies of the invention are PRO4673, PRO4673_without_dummy_LILRB2-BD, PRO3790, PRO4688, PRO4688_without_dummy_LILRB2-BD, PRO5053, PRO5053_without_dummy_LILRB2-BD, PRO5056 and PRO5056_without_dummy_LILRB2-BD, whose sequences are listed in Tables 3 and 4.

In some embodiments, the multispecific antibody additionally comprises one further binding domain, which has specificity for an antigen different from CD47 and PDL1, wherein said one further binding domain is attached to one of the C-termini of the heavy chains of the immunoglobulin Fc region.

In other embodiments, the multispecific antibody additionally comprises two further binding domains, which have specificity for an antigen different from CD47 and PDL1, wherein said two further binding domains are attached to each of the C-termini of the heavy chains of the immunoglobulin Fc region.

In specific embodiments, the one or two further binding domains specifically bind to a tumor associated antigen (TAA) different from CD47 and PDL1. Suitable TAAs are those known in the art. Examples of suitable TAAs are: ADRB3, AFP, ALK, BCMA, beta human chorionic gonadotropin, CA-125 (MUC16), CAIX, CD123, CD133, CD135, CD135 (FLT3), CD138, CD171, CD19, CD20, CD22, CD24, CD276, CD33, CD33, CD38, CD44v6, CD79b, CD97, CDH3 (cadherin 3), CEA, CEACAM6, CLDN6, CLEC12A (CLL1), CSPG4, CYP1B1, EGFR, EGFRvlll, EPCAM, EPHA2, Ephrin B2, ERBBs (e. g. ERBB2), FAP, FGFR1, folate receptor alpha, folate receptor beta, Fos-related antigen, GA733, GD2, GD3, GFRalpha4, globoH, GPC3, GPR20, GPRC5D, HAVCR1, Her2/neu (HER2), HLA-A2, HMWMAA, HPV E6 or E7, human telomerase reverse transcriptase, IL-11Ra, IL-13Ra2, intestinal carboxyl esterase, KIT, Legumain, LewisY, LMP2, Ly6k, MAD-CT-1, MAD-CT-2, ML-IAP, MN-CA IX, MSLN, MUC1, mut hsp 70-2, NA-17, NCAM, neutrophil elastase, NY-BR-1, NY-ESO-1, o-acetyl-GD2, OR51E2, PANX3, PDGFR-beta, PLAC1, Polysialic acid, PSCA, PSMA, RAGE1, ROR1, sLe, sperm protein 17, SSEA-4, SSTR2, sTn antigen, sTn-O-Glycopeptides, TAG72, TARP, TEM1/CD248, TEM7R, thyroglobulin, Tn antigen, Tn-O-Glycopeptides, TPBG (5T4), TRP-2, TSHR, UPK2 and VEGFR2.

In other specific embodiments, the one or two further binding domains specifically bind to an immunomodulatory antigen different from PDL1. Suitable immunomodulatory antigen different from PDL1 are those known in the art. Suitable immunomodulatory antigen different from PDL1 are for example selected from B7-1 (CD80), B7-2 (CD86), B7-DC (CD273), B7-H1 (CD274), B7-H2 (CD275), B7-H3 (CD276), B7-H4 (VTCN1), B7-H5 (VISTA), BTLA (CD272), 4-1BB (CD137), CD137L, CD24, CD27, CD28, CD38, CD40, CD40L (CD154), CD54, CD59, CD70, CTLA-1, CTLA-4 (CD152), CXCL9, GITR (CD357), GITR, HVEM (CD270), ICAM-1 (CD54), ICOS (CD278), LAG-3 (CD223), OX40 (CD134), OX40L (CD252), PD-1 (CD279), TIGIT, CD314, CD334, CD335, CD337, VISTA and TIM-3 (CD366).

In other specific embodiments, the one or two further binding domains specifically bind to an NK cell receptor. Suitable NK cell receptors are those known in the art. Suitable NK cell receptors are for example selected from KIR2DL1, KIR2DL2/3, KIR2DL4, KIR2DL5A, KIR2DL5B, KIR3DL1, KIR3DL2, KIR3DL3, KIR2DS4, KIR3DS, NKG2A, NKG2C, NKG2D, NKG2E, NKp30, NKp44, NKp46, CD2, CD16, CD69, DNAX accessory molecule-1 (DNAM-1), 2B4, NK1.1; a killer immunoglobulin (Ig)-like activating receptors (KAR); ILTs/LIRs (CD85) such as LI LRA1, LlLRA2, LlLRA3, LILRA4, LILRA5, LILRA6, LILRB1, LILRB2, LILRB3, LILRB4, LILRB5, ILT8, ILT10, ILT11 ; NKRP-1; CD94/NKG2A, CD94/NKG2C and CD94/NKG2E heterodimers.

In more specific embodiments, the one or two further binding domains are independently of each other selected from NKp46, LILRB1, LILRB2, LILRB3, LILRB4 and LILRB5, particularly LILRB1, LILRB2, and LILRB4, particularly LILRB1 and LILRB2, particularly LILRB2.

In particular embodiments, the multispecific antibody comprises one further binding domain, which specifically binds to LILRB2 (LILRB2-BDs), wherein said LILRB2-BD is attached to one of the C-termini of the heavy chains of the immunoglobulin Fc region.

In other particular embodiments, the multispecific antibody comprises two further binding domains, which specifically bind to LILRB2 (LILRB2-BDs), wherein said two LILRB2-BDs are attached to each of the C-termini of the heavy chains of the immunoglobulin Fc region. The two LILRB2-BD either bind the same or different epitopes on the extracellular domain of LILRB2. Particularly, the two LILRB2-BDs are identical.

The term "same epitope", as used herein, refers to an individual protein determinant on the protein capable of specific binding to more than one antibody, where that individual protein determinant is identical, *i. e*. consist of identical chemically active surface groupings of molecules such as amino acids or sugar side chains having identical three-dimensional structural characteristics, as well as identical charge characteristics for each of said antibodies. The term "different epitope", as used herein in connection with a specific protein target, refers to individual protein determinants on the protein, each capable of specific binding to a different antibody, where these individual protein determinants are not identical for the different antibodies, *i. e*. consist of non-identical chemically active surface groupings of molecules such as amino acids or sugar side chains having different three-dimensional structural characteristics, as well as different charge characteristics. These different epitopes can be overlapping or non-overlapping.

The term "LILRB2", *i. e*. leukocyte immunoglobulin-like receptor subfamily B member 2, also known as immunoglobulin like transcript 4 (ILT4), refers in particular to human LILRB2 with UniProt ID number A2IXV5. LILRB2 belongs to the subfamily B class of LIR receptors and is expressed on immune cells, such as myeloid cells, including monocytes, macrophages, dendritic cells and granulocytes. It binds classical and nonclassical major histocompatibility complex Class I (MHC-I) proteins on antigen-presenting cells, such as human leukocyte antigen A (HLA-A) and human leukocyte antigen G (HLA-G), and transduces a negative signal that has immunosuppressive effects. It further binds to several angiopoietin-like proteins (ANGPTLs), such as ANGPTL2 that promotes adaptive inflammation in tissue. A wide range of tumors express HLA-G, a nonclassical MHC-I molecule, which has been shown to mediate immunotolerance in cancer through the interaction with LILRB2, and thus allows such tumor cells to escape innate and adaptive immune response. Blocking of LILRB2 would hinder this escape mechanism. Besides, the blocking of LILRB2 can shift suppressed macrophages (M2) to activated state (M1-like).

There are several anti-LILRB2 antibodies known in the art. However, the number of LILRB2-BDs in the form of antibody fragments, such as scFvs, that can be used as universal building blocks for the construction of multispecific antibodies is limited. It is thus desirable to have suitable anti-LILRB2 antibody building blocks at hand that can readily be incorporated into any desirable multispecific antibody format, and that are capable of potently blocking the biological function of LILRB2. Furthermore, said building blocks should have superior biophysical properties, such as in particular a high stability, in order to facilitate their efficient incorporation into multispecific antibodies.

Suitably, the LIRB2-BDs of the multispecific antibodies of the invention target in particular human LILRB2. Particularly, the LIRB2-BDs of the multispecific antibodies of the invention target human and cynomolgus (*Macaca fascicularis*) LILRB2.

Suitably, the LILRB2-BDs used in the present invention are characterized by one or more of the following parameters:
a. when in scFv format, bind to human LILRB2 with a monovalent dissociation constant (KD) of 10 pM to 5 nM, particularly with a KD of 20 pM to 3 nM, particularly of 50 pM to 1 nM, as measured by surface plasmon resonance (SPR);
b. block the interaction of LILRB2 to its ligand HLA-G with an IC₅₀ of 100 pM to 10 nM, particularly of 100 pM to 5 nM, particularly of 100 pM to 2 nM, as measured by flow cytometry;
c. block the interaction of LILRB2 to its ligand HLA-A3 with an IC₅₀ of 100 pM to 10 nM, particularly of 100 pM to 5 nM, particularly of 100 pM to 2 nM, as measured by flow cytometry;
d. are not cross-reactive with members of leukocyte immunoglobulin-like receptor subfamily A (LILRA); and/or
e. are not cross reactive with members of leukocyte immunoglobulin-like receptor subfamily LILRB3, LILRB4 and LILRB5.

The expression "members of leukocyte immunoglobulin-like receptor subfamily A" or "LILRA" refers to leukocyte immunoglobulin-like receptor subfamily A, which, like the LILRBs, are a family of immunoreceptors that belong to leukocyte Ig-like receptors (LIRs) and that are expressed predominantly on monocytes and B cells and at lower levels on dendritic cells and natural killer (NK) cells. Examples are LILRA1, LILRA2, LILRA3, LILRA4, LILRA5 and LILRA6.

The expression "LILRB members other than LILRB2" refer to members of leukocyte immunoglobulin-like receptor subfamily B different from LILRB2. Examples are LILRB1, LILRB3, LILRB4 and LILRB5.

Suitably, the LILRB2-BDs of the multispecific antibodies of the present invention are further characterized by one or more of the following parameters:
a. when in scFv format, have a melting temperature (Tm), determined by differential scanning fluorimetry, in the range of 55°C to 85°C, particularly in the range of 60°C to 85°C, in particular wherein said antibody is formulated in 50 mM phosphate-citrate buffer at pH 6.4, 150 mM NaCl;
b. when in scFv format, have a loss in monomer content, after storage for four weeks, at
   - 80°C, of less than 5 %, less than 4 %, less than 3 %, less than 2 %, particularly less than 1.5 %, when the antibody of the invention is at a starting concentration of 10 mg/ml,
      and in particular wherein said antibody is formulated in 50 mM phosphate citrate buffer with 150 mM NaCl at pH 6.4;
c. when in scFv format, have a loss in monomer content, after storage for four weeks, at 4°C, of less than 15 %, e.g. less than 12 %, less than 10 %, less than 9 %, less than 8 %, particularly less than 7 %, when the antibody of the invention is at a starting concentration of 10 mg/ml, and in particular wherein said antibody is formulated in 50 mM phosphate citrate buffer with 150 mM NaCl at pH 6.4;
d. when in scFv format, have a loss in monomer content, after storage for four weeks, at 40°C, of less than 15 %, e.g. less than 12 %, less than 10 %, less than 9 %, less than 8 %, particularly less than 7 %, when the antibody of the invention is at a starting concentration of 10 mg/ml, and in particular wherein said antibody is formulated in 50 mM phosphate citrate buffer with 150 mM NaCl at pH 6.4.

Suitably, the LILRB2-BDs of the multispecific antibodies of the invention are binding domains provided in the present disclosure. The LILRB2-BDs of the multispecific antibodies of the invention include, but are not limited to, the humanized LILRB2-BDs whose sequences are listed in Table 1.

In a particular aspect, the present invention relates to a multispecific antibody comprising:
a) one binding domain, which specifically binds to CD47 (CD47-BD);
   wherein said CD47-BD comprises:
   i. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 5, and
      the LCDR3 sequences of SEQ ID NO: 7; or
   ii. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 6, and
      the LCDR3 sequences of SEQ ID NO: 7;
b) one binding domain, which specifically binds to PDL1 (PDL1-BD);
   wherein said PDL1-BD comprises:
   i. the HCDR1 sequence of SEQ ID NO: 20,
      the HCDR2 sequences of SEQ ID NO: 21,
      the HCDR3 sequence of SEQ ID NO: 23,
      the LCDR1 sequence of SEQ ID NO: 24,
      the LCDR2 sequence of SEQ ID NO: 25, and
      the LCDR3 sequences of SEQ ID NO: 27; or
   ii. the HCDR1 sequence of SEQ ID NO: 20,
      the HCDR2 sequences of SEQ ID NO: 21,
      the HCDR3 sequence of SEQ ID NO: 23,
      the LCDR1 sequence of SEQ ID NO: 24,
      the LCDR2 sequence of SEQ ID NO: 26, and
      the LCDR3 sequences of SEQ ID NO: 27; or
   iii. the HCDR1 sequence of SEQ ID NO: 20,
      the HCDR2 sequences of SEQ ID NO: 22,
      the HCDR3 sequence of SEQ ID NO: 23,
      the LCDR1 sequence of SEQ ID NO: 24,
      the LCDR2 sequence of SEQ ID NO: 25, and
      the LCDR3 sequences of SEQ ID NO: 27; or
   iv. the HCDR1 sequence of SEQ ID NO: 20,
      the HCDR2 sequences of SEQ ID NO: 22,
      the HCDR3 sequence of SEQ ID NO: 23,
      the LCDR1 sequence of SEQ ID NO: 24,
      the LCDR2 sequence of SEQ ID NO: 26, and
      the LCDR3 sequences of SEQ ID NO: 27; or
   v. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 78, and
      the LCDR3 sequences of SEQ ID NO: 80; or
   vi. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 79, and
      the LCDR3 sequences of SEQ ID NO: 80;
c) an IgG-like region comprising an immunoglobulin Fc region, a first Fab arm and a second Fab arm, wherein:
   - the CD47 BD is an scFv fragment replacing the first Fab arm, and
   - the PDL1 BD is located in the second Fab arm or is an scFv fragment replacing said second Fab arm;
d) two binding domains, which specifically bind to LILRB2 (LILRB2-BDs);
   wherein said LILRB2-BDs comprise:
      i. the HCDR1 sequence of SEQ ID NO: 93,
         the HCDR2 sequences of SEQ ID NO: 94,
         the HCDR3 sequence of SEQ ID NO: 95,
         the LCDR1 sequence of SEQ ID NO: 96,
         the LCDR2 sequence of SEQ ID NO: 97, and
         the LCDR3 sequences of SEQ ID NO: 99; or
      ii. the HCDR1 sequence of SEQ ID NO: 93,
         the HCDR2 sequences of SEQ ID NO: 94,
         the HCDR3 sequence of SEQ ID NO: 95,
         the LCDR1 sequence of SEQ ID NO: 96,
         the LCDR2 sequence of SEQ ID NO: 98, and
         the LCDR3 sequences of SEQ ID NO: 99; or
      iii. the HCDR1 sequence of SEQ ID NO: 112,
         the HCDR2 sequences of SEQ ID NO: 113,
         the HCDR3 sequence of SEQ ID NO: 114,
         the LCDR1 sequence of SEQ ID NO: 115,
         the LCDR2 sequence of SEQ ID NO: 116, and
         the LCDR3 sequences of SEQ ID NO: 118; or
      iv. the HCDR1 sequence of SEQ ID NO: 112,
         the HCDR2 sequences of SEQ ID NO: 113,
         the HCDR3 sequence of SEQ ID NO: 114,
         the LCDR1 sequence of SEQ ID NO: 115,
         the LCDR2 sequence of SEQ ID NO: 117, and
         the LCDR3 sequences of SEQ ID NO: 118 or
      v. the HCDR1 sequence of SEQ ID NO: 128,
         the HCDR2 sequences of SEQ ID NO: 129,
         the HCDR3 sequence of SEQ ID NO: 130,
         the LCDR1 sequence of SEQ ID NO: 131,
         the LCDR2 sequence of SEQ ID NO: 132, and
         the LCDR3 sequences of SEQ ID NO: 134; or
      vi. the HCDR1 sequence of SEQ ID NO: 128,
         the HCDR2 sequences of SEQ ID NO: 129,
         the HCDR3 sequence of SEQ ID NO: 130,
         the LCDR1 sequence of SEQ ID NO: 131,
         the LCDR2 sequence of SEQ ID NO: 133, and
         the LCDR3 sequences of SEQ ID NO: 134;
   and wherein said two LILRB2-BDs are attached to each of the C-termini of the heavy chains of the immunoglobulin Fc region.

In a more particular aspect, the present invention relates to a multispecific antibody comprising:
a) one binding domain, which specifically binds to CD47 (CD47-BD);
   wherein said CD47-BD comprises:
   i. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 5, and
      the LCDR3 sequences of SEQ ID NO: 7; or
   ii. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 6, and
      the LCDR3 sequences of SEQ ID NO: 7;
b) one binding domain, which specifically binds to PDL1 (PDL1-BD);
   wherein said PDL1-BD comprises:
   i. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 78, and
      the LCDR3 sequences of SEQ ID NO: 80; or
   ii. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 79, and
      the LCDR3 sequences of SEQ ID NO: 80;
c) an IgG-like region comprising an immunoglobulin Fc region, a first Fab arm and a second Fab arm, wherein:
   - the CD47 BD is an scFv fragment replacing the first Fab arm, and
   - the PDL1 BD is located in the second Fab arm or is an scFv fragment replacing said second Fab arm;
d) two binding domains, which specifically bind to LILRB2 (LILRB2-BDs), wherein said two LILRB2-BDs are attached to each of the C-termini of the heavy chains of the immunoglobulin Fc region.

In another more particular aspect, the present invention relates to a multispecific antibody comprising:
a) one binding domain, which specifically binds to CD47 (CD47-BD);
   wherein said CD47-BD comprises:
   i. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 5, and
      the LCDR3 sequences of SEQ ID NO: 7; or
   ii. the HCDR1 sequence of SEQ ID NO: 1,
      the HCDR2 sequences of SEQ ID NO: 2,
      the HCDR3 sequence of SEQ ID NO: 3,
      the LCDR1 sequence of SEQ ID NO: 4,
      the LCDR2 sequence of SEQ ID NO: 6, and
      the LCDR3 sequences of SEQ ID NO: 7;
b) one binding domain, which specifically binds to PDL1 (PDL1-BD);
   wherein said PDL1-BD comprises:
   i. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 78, and
      the LCDR3 sequences of SEQ ID NO: 80; or
   ii. the HCDR1 sequence of SEQ ID NO: 74,
      the HCDR2 sequences of SEQ ID NO: 75,
      the HCDR3 sequence of SEQ ID NO: 76,
      the LCDR1 sequence of SEQ ID NO: 77,
      the LCDR2 sequence of SEQ ID NO: 79, and
      the LCDR3 sequences of SEQ ID NO: 80;
c) an IgG-like region comprising an immunoglobulin Fc region, a first Fab arm and a second Fab arm, wherein:
   - the CD47 BD is an scFv fragment replacing the first Fab arm, and
   - the PDL1 BD is located in the second Fab arm or is an scFv fragment replacing said second Fab arm;
d) two binding domains, which specifically bind to LILRB2 (LILRB2-BDs);
   wherein said LILRB2-BDs comprise:
      i. the HCDR1 sequence of SEQ ID NO: 93,
         the HCDR2 sequences of SEQ ID NO: 94,
         the HCDR3 sequence of SEQ ID NO: 95,
         the LCDR1 sequence of SEQ ID NO: 96,
         the LCDR2 sequence of SEQ ID NO: 97, and
         the LCDR3 sequences of SEQ ID NO: 99; or
      ii. the HCDR1 sequence of SEQ ID NO: 93,
         the HCDR2 sequences of SEQ ID NO: 94,
         the HCDR3 sequence of SEQ ID NO: 95,
         the LCDR1 sequence of SEQ ID NO: 96,
         the LCDR2 sequence of SEQ ID NO: 98, and
         the LCDR3 sequences of SEQ ID NO: 99;
   and wherein said two LILRB2-BDs are attached to each of the C-termini of the heavy chains of the immunoglobulin Fc region.

Suitably, the binding domains of the multispecific antibodies of the invention, except for the CD47-BD, are selected from the group consisting of: a Fab, an scFab, an Fv, a dsFv, an scFv and a dsscFv, particularly from a Fab, Fv and scFv.

Suitably, the binding domains of the multispecific antibodies of the invention are operably linked. The binding domains of the multispecific antibodies of the invention are capable of binding to their respective antigens or receptors simultaneously. The term "simultaneously", as used herein refers for example to the simultaneous binding of the CD47-BD and the PDL1-BD or the simultaneous binding of the PDL1-BD and the LILRB2-BD.

The term "operably linked", as used herein, indicates that two molecules (e. *g.,* polypeptides, domains, binding domains) are attached in a way that each molecule retains functional activity. Two molecules can be "operably linked" whether they are attached directly or indirectly (e. g., via a linker, via a moiety, via a linker to a moiety).

The term "linker" refers to a peptide or other moiety that is optionally located between binding domains or antibody fragments used in the invention. A number of strategies may be used to covalently link molecules together. These include, but are not limited to, polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (e. g., whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (e. *g.,* 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, (Gly-Ser)ₙ, (GSGGS)ₙ (SEQ ID NO: 259), (GGGGS)ₙ (SEQ ID NO: 260) and (GGGS)ₙ (SEQ ID NO: 261), where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since oligopeptides comprising these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single-chain antibodies.

In particular embodiments the one or two LILRB2-BD(s) is/are fused to the heavy chain of the immunoglobulin Fc region via a linker L1.

The linker L1 is a peptide of 2-30 amino acids, more particularly 5-25 amino acids, and most particularly 10-20 amino acids. In particular embodiments, said linker L1 comprises one or more units of four (4) glycine amino acid residues and one (1) serine amino acid residue (GGGGS)ₙ (SEQ ID NO:170), wherein n=1, 2, 3, 4 or 5, particularly n=2.

The scFv domains of the multispecific antibodies of the invention comprise a variable heavy chain domain (VH) and a variable light chain domain (VL) connected by a linker L2.

The linker L2 is a peptide of 10-40 amino acids, more particularly 15-30 amino acids, and most particularly 20-25 amino acids. In particular embodiments, said linker L2 comprises one or more units of four (4) glycine amino acid residues and one (1) serine amino acid residue (GGGGS)ₙ (SEQ ID NO:172), wherein n=1, 2, 3, 4, 5, 6, 7 or 8, particularly n=4.

Specific but non-limiting examples of the anti-CD47xPDL1xLILRB2 multispecific antibodies of the invention are PRO4672, PRO4687, PRO5052 and PRO5055, whose sequences are listed in table 4.

The multispecific antibodies of the invention can be produced using any convenient antibody-manufacturing method known in the art (see, e. g., Fischer, N. & Leger, O., Pathobiology 74 (2007) 3-14 with regard to the production of bispecific constructs; Hornig, N. & Färber-Schwarz, A., Methods Mol. Biol. 907 (2012)713-727, and WO 99/57150 with regard to bispecific diabodies and tandem scFvs). Specific examples of suitable methods for the preparation of the bispecific construct further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, e. g., Zhu et al., Cancer Lett. 86 (1994) 127-134); and Suresh et al., Methods Enzymol. 121 (1986) 210-228).

These methods typically involve the generation of monoclonal antibodies, for example by means of fusing myeloma cells with the spleen cells from a mouse that has been immunized with the desired antigen using the hybridoma technology (see, e. g., Yokoyama et al., Curr. Protoc. Immunol. Chapter 2, Unit 2.5, 2006) or by means of recombinant antibody engineering (repertoire cloning or phage display/yeast display) (see, e. g., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The multispecific antibodies of the invention can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl)-cyclohexane-I-carboxylate (sulfo-SMCC) (see e. g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83, and Glennie et al., 1987 J. Immunol. 139: 2367-2375. Conjugating agents are SATA and sulfo-SMCC, both available from Pierce Chemical Co. (Rockford, III).

When the binding specificities are antibodies, they can be conjugated by sulfhydryl bonding of the C-terminus hinge regions of the two heavy chains. In a particular embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb x Fab, a mAb x scFv, a mAb x dsFv or a mAb x Fv fusion protein. Methods for preparing multispecific antibodies and molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific antibodies to their specific targets can be confirmed by, for example, enzyme-linked immunosorbent assay (ELISA), radioimmunoassay (REA), FACS analysis, bioassay (e. g., growth inhibition), or Western Blot assay. Each of these assays generally detects the presence of protein-antibody complexes of particular interest by employing a labeled reagent (e. *g.,* an antibody) specific for the complex of interest.

In a further aspect, the invention provides a nucleic acid or two nucleic acids or three nucleic acids encoding the multispecific antibody of the invention. Such nucleic acids can be optimized for expression in mammalian cells.

The term "nucleic acid" is used herein interchangeably with the term "polynucleotide(s)" and refers to one or more deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e. g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the multispecific antibody described above. When expressed from appropriate expression vectors, polypeptides encoded by these nucleic acid molecules are capable of exhibiting antigen-binding capacities of the multispecific antibody of the present invention.

The polynucleotide sequences can be produced by *de novo* solid-phase DNA synthesis or by PCR mutagenesis of an existing sequence (e. g., sequences as described in the Examples below) encoding the multispecific antibody of the invention or fragments thereof or binding domains thereof. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, e. *g.,* PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the multispecific antibody of the invention or fragments thereof or binding domains thereof.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e. g., bacterial vectors having a bacterial origin of replication and episomal mammalian vectors). Other vectors (e. g., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e. g., replication defective retroviruses, adenoviruses and adeno- associated viruses), which serve equivalent functions. In this particular context, the term "operably linked" refers to a functional relationship between two or more polynucleotide (e. g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i. e., they are cis-acting. However, some transcriptional regulatory sequences, such as enhancers, need not be physically contiguous or located in close proximity to the coding sequences whose transcription they enhance.

Various expression vectors can be employed to express the polynucleotides encoding the multispecific antibody chains. Both viral-based and non-viral expression vectors can be used to produce the antibodies in a mammalian host cell. Non-viral vectors and systems include plasmids, episomal vectors, typically with an expression cassette for expressing a protein or RNA, and human artificial chromosomes (see, e. g., Harrington et al., Nat Genet. 15:345, 1997). For example, non-viral vectors useful for expression of the polynucleotides encoding the multispecific antibody chains in mammalian (e. g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (e. g., enhancers) that are operably linked to the polynucleotides encoding a multispecific antibody chain or a fragment. In one embodiment, an inducible promoter is employed to prevent expression of inserted sequences except under inducing conditions. Inducible promoters include, e. g., arabinose, lacZ, metallothionein promoter or a heat shock promoter. Cultures of transformed organisms can be expanded under non-inducing conditions without biasing the population for coding sequences whose expression products are better tolerated by the host cells. In addition to promoters, other regulatory elements may also be required or desired for efficient expression of a multispecific antibody chain or a fragment. These elements typically include an ATG initiation codon and adjacent ribosome binding site or other sequences. In addition, the efficiency of expression may be enhanced by the inclusion of enhancers appropriate to the cell system in use (see, e. *g.,* Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

Vectors to be used typically encode the multispecific antibody light and heavy chain or chains including constant regions or parts thereof. Such vectors allow expression of the variable regions as fusion proteins with the constant regions thereby leading to production of intact antibodies and antigen-binding fragments thereof. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the multispecific antibody of the invention can be either prokaryotic or eukaryotic. *E. coli* is one prokaryotic host useful for cloning and expressing the polynucleotides of the present invention. Other microbial hosts suitable for use include bacilli, such as *Bacillus subtilis,* and other enterobacteriaceae, such as Salmonella, Serratia, and various Pseudomonas species. In these prokaryotic hosts, one can also make expression vectors, which typically contain expression control sequences compatible with the host cell (e. *g.,* an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express the multispecific antibodies of the invention. Insect cells in combination with baculovirus vectors can also be used.

In one embodiment, mammalian host cells are used to express and produce the multispecific antibody of the invention. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various COS cell lines, HeLa cells, myeloma cell lines, transformed B-cells and hybridomas. The use of mammalian tissue cell culture to express polypeptides is discussed generally in, e. *g.,* Winnacker, FROM GENES TO CLONES, VCH Publishers, N.Y., N.Y., 1987. Expression vectors for mammalian host cells can include expression control sequences, such as an origin of replication, a promoter, and an enhancer (see, e. g., Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP pol III promoter, the constitutive MPS V promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Green, M. R., and Sambrook, J., Molecular Cloning: A Laboratory Manual (Fourth Edition), Cold Spring Harbor Laboratory Press (2012)). Other methods include, e. g., electroporation, calcium phosphate treatment, liposome-mediated transformation, injection and microinjection, ballistic methods, virosomes, immunoliposomes, polycation-nucleic acid conjugates, naked DNA, artificial virions, fusion to the herpes virus structural protein VP22 (Elliot and O'Hare, Cell 88:223, 1997), agent-enhanced uptake of DNA, and ex *vivo* transduction. For long-term, high-yield production of recombinant proteins, stable expression will often be desired. For example, cell lines which stably express the multispecific antibody of the invention can be prepared using expression vectors of the invention which contain viral origins of replication or endogenous expression elements and a selectable marker gene. Following the introduction of the vector, cells may be allowed to grow for 1 to 2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody of the invention or antigen-binding fragment thereof, wherein said method comprises the step of culturing a host cell comprising a nucleic acid or a vector encoding the antibody of the invention or antigen-binding fragment thereof, whereby said antibody of the disclosure or a fragment thereof is expressed.

In one aspect, the present invention relates to a method of producing the multispecific antibody of the invention, the method comprising the step of culturing a host cell expressing a nucleic acid or two nucleic acids or three nucleic acids encoding the multispecific antibody of the invention. In particular, the present invention relates to a method of producing the multispecific antibody of the invention, the method comprising (i) providing a nucleic acid or two nucleic acids or three nucleic acids encoding the multispecific antibody of the invention or a vector or two vectors or three vectors encoding the multispecific antibody of the invention, expressing said nucleic acid or nucleic acids, or said vector or vectors, and collecting said multispecific antibody from the expression system, or (ii) providing a host cell or host cells expressing a nucleic acid or nucleic acids encoding the multispecific antibody of the invention, culturing said host cell or said host cells; and collecting said multispecific antibody from the cell culture.

In a further aspect, the present invention relates to a pharmaceutical composition comprising the multispecific antibody of the invention, and a pharmaceutically acceptable carrier. "Pharmaceutically acceptable carrier" means a medium or diluent that does not interfere with the structure of the antibodies. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

Certain, of such carriers enable pharmaceutical compositions to be formulated as, for example, tablets, pills, dragees, capsules, liquids, gels, syrups, slurries, suspension and lozenges for the oral ingestion by a subject. Certain of such carriers enable pharmaceutical compositions to be formulated for injection, infusion or topical administration. For example, a pharmaceutically acceptable carrier can be a sterile aqueous solution.

The pharmaceutical composition of the invention can be administered by a variety of methods known in the art. The route and/or mode of administration vary depending upon the desired results. Administration can be intravenous, intramuscular, intraperitoneal, or subcutaneous, or administered proximal to the site of the target. In particular embodiments, the administration is intramuscular, or subcutaneous, particularly subcutaneous. The pharmaceutically acceptable carrier should be suitable for intravenous, intramuscular, subcutaneous, parenteral, spinal or epidermal administration (e. *g.,* by injection or infusion), particularly for intramuscular or subcutaneous administration. Depending on the route of administration, the active compound, i. e., the multispecific antibody of the invention, may be coated in a material to protect the compound from the action of acids and other natural conditions that may inactivate the compound.

The pharmaceutical compositions of the invention can be prepared in accordance with methods well known and routinely practiced in the art. See, e. g., Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the multispecific antibody of the invention is employed in the pharmaceutical compositions of the invention. The multispecific antibodies of the invention are formulated into pharmaceutically acceptable dosage forms by conventional methods known to those of skill in the art. Dosage regimens are adjusted to provide the optimum desired response (e. *g.,* a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The multispecific antibody of the invention is usually administered on multiple occasions. Intervals between single dosages can be weekly, monthly or yearly. Intervals can also be irregular as indicated by measuring blood levels of the multispecific antibody of the invention in the patient. Alternatively, the multispecific antibody of the invention can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In certain embodiments, the pharmaceutical composition comprises:
a) a first multispecific antibody that specifically binds to CD47 and PDL1;
b) a second antibody that specifically binds to LILRB2; and
c) a pharmaceutically acceptable carrier.

In particular embodiments, the first multispecific antibody that specifically binds to CD47 and PDL1 is selected from anti-CD47xPDL1 antibodies that are currently tested or have been tested in clinical trials.

The expression "antibodies that have been tested in clinical trials" refers to antibodies that have successfully passed clinical testing and also to antibodies that failed in clinical testing. The expression "antibodies that are currently tested in clinical trials" refers to antibodies that entered clinical trials but have not yet passed clinical trials or have not been withdrawn from clinical trials.

Specific non limiting examples of anti-CD47xPDL1 antibodies that are currently tested or have been tested in clinical trials are: IBI-322 by Innovent Biologics Inc.; PF-07257876 by Pfizer Inc.; 6MW-3211 by Mabwell (Shanghai) Bioscience Co. Ltd.; BAT7104 by Bio-Thera Solutions, Ltd.; QL401 by QLSF Biotherapeutics, Inc.; LB-101 by Centessa Pharmaceuticals plc; Imm2520 by ImmuneOnco Biopharmaceuticals Shanghai Inc.; SHS-009 by Nanjing Sanhome Pharmaceutical Co Ltd.; BCD-106 by Biocad Biopharmaceutical Co.; TJL-1C4 by I-MAB Biopharma Co. Ltd.; SG12473 by Hangzhou Sumgen Biotech Co. Ltd.; XB014 by Exelixis Inc.; PMC-122 by PharmAbcine Inc.; ABP-160 by Abpro Corp.; papiliximab by Shaperon Inc.; JY-207 by Shenzhen Enduring Biotech Ltd.; NY2601 and NY2901 by Light Chain Bioscience AG.

In other particular embodiments, the first multispecific antibody that specifically binds to CD47 and PDL1 is selected from an anti-CD47xPDL1 multispecific antibody according to the invention, as defined herein.

In yet other particular embodiments, the second antibody is a fragment-based antibody further comprising one hSA binding domain.

In further particular embodiments, the second antibody that specifically binds to LILRB2 is selected from anti-LILRB2 antibodies that are currently tested or have been tested in clinical trials.

Specific non limiting examples of anti-LILRB2 antibodies that are currently tested or have been tested in clinical trials are: JTX-8064 by Jounce Therapeutics Inc.; MK-4830 by Merck & Co. Inc.; IO-108 by Immune-One Therapeutics Inc.; NGM-707 by NGM Biopharmaceuticals Inc.; BMS-986406 by Bristol Myers Squibb Company; IOS-1002 by ImmunOs Therapeutics AG; CDX-585 by Celldex Therapeutics Inc.; SPX-303 by SparX Biopharmaceutical Corp.; CHS-1000 by Coherus BioSciences Inc.; ES009 by Elpiscience (Suzhou) Biopharma Ltd.; XBH41 by GV20 Therapeutics and IVS-5001 by Invectys.

In other particular embodiments, the second antibody that specifically binds to LILRB2 is selected from antibodies that comprise at least one LILRB2-BD as defined herein.

In yet other particular embodiments, the second antibody that specifically binds to LILRB2 is selected from antibodies that comprise two LILRB2-BD as defined herein. Specific non-limiting examples of such anti-LILRB2 antibodies are PRO3603, PRO3635 and PRO4710, whose sequences are listed in Table 5.

In one aspect, the present invention relates to the multispecific antibody of the invention or the pharmaceutical composition of the invention for use as a medicament. In a suitable embodiment, the present invention provides the multispecific antibody or the pharmaceutical composition for use in the treatment of a disease, particularly a human disease, more particularly a human disease selected from cancer.

In another aspect, the present invention provides the pharmaceutical composition for use in the manufacture of a medicament for the treatment of a disease, particularly a human disease, more particularly a human disease selected from cancer.

In another aspect, the present invention relates to the use of the multispecific antibody or the pharmaceutical composition for treating a disease, particularly a human disease, more particularly a human disease selected from cancer, in a subject in need thereof.

In another aspect, the present invention relates to a method of treating a subject comprising administering to the subject a therapeutically effective amount of the multispecific antibody of the present invention. In a suitable embodiment, the present invention relates to a method for the treatment of a disease, particularly a human disease, more particularly a human disease selected from cancer, in a subject comprising administering to the subject a therapeutically effective amount of the multispecific antibody of the present invention.

The term "subject" includes human and non-human animals.

The term "animals" include all vertebrates, e. g., non-human mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, e. *g.,* in a human, and includes: (a) inhibiting the disease, i. e., arresting its development; and (b) relieving the disease, i. e., causing regression of the disease.

The term "therapeutically effective amount" or "efficacious amount" refers to the amount of an agent that, when administered to a mammal or other subject for treating a disease, is sufficient to affect such treatment for the disease. The "therapeutically effective amount" will vary depending on the agent, the disease and its severity and the age, weight, etc., of the subject to be treated.

In one embodiment, the disease is a cancer selected from: a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.

There are several anti-CD47 antibodies known in the art. However, the number of available CD47-BDs in the form of antibody fragments, such as scFvs, that can be used as universal building blocks for the construction of multispecific antibodies is limited. It is thus desirable to have suitable anti-CD47 antibody building blocks at hand that can readily be incorporated into any desirable multispecific antibody format, and that are capable to potently block the biological function of CD47, in particular to potently block the interaction between CD47 and SIRPα. Furthermore, said building blocks should have superior biophysical properties, such as in particular a high stability, in order to facilitate their efficient incorporation into multispecific antibodies for pharmaceutical development.

In a further aspect, the present invention relates to an antibody binding domain, having specificity for CD47 (CD47-BD), as defined herein. Preferably, said CD47-BD is in a format selected from an Fv, a dsFv, an scFv and a dsscFv, particularly from an Fv and an scFv. Specific non-limiting examples of such anti-CD47 antibody binding domains are the scFvs with SEQ ID NOs: 16, 17, 18 and 19.

In yet another aspect, the present invention relates to an antibody comprising one or two, preferably one, antibody binding domains having specificity for CD47 (CD47-BD), as defined herein.

In another aspect, the present invention relates to an antibody binding domain having specificity for LILRB2 (LILRB2-BD), as defined herein. Preferably, said LIRB2-BD is in a format selected from an Fv, a dsFv, an scFv and a dsscFv, particularly from an Fv and an scFv.

In yet another aspect, the present invention relates to an antibody comprising one, two, three or four, preferably one or two, antibody binding domains having specificity for LILRB2 (LILRB2-BD), as defined herein. Specific non-limiting examples of such anti-LILRB2 antibodies are PRO3603, PRO3635 and PRO4710, whose sequences are listed in table 5.

### Sequence listing (mutations designated according to AHo numbering scheme; the CDRs defined according to Numab CDR definition, unless specified otherwise)

**Table 1. Examples of binding domains according to the present invention (CDR residues are shown in bold and italic letters).**

| **Anti-CD47 antibody variable domains** | | |
|---|---|---|
| *70-26-804* - *based sequences* | | |
| 1 | **HCDR1** | ***GIDLNSYAM*** |
| | (H27-H42; AHo numbering) | |
| 2 | **HCDR2** | ***YIGSASSTYYASWASG*** |
| | (H57-H76; AHo numbering) | |
| 3 | **HCDR3** | ***RGSTYINI*** |
| | (H108-H138; AHo numbering) | |
| 4 | **LCDR1** | **QASQSISSWVS** |
| | (L24-L42; AHo numbering) | |
| 5 | **LCDR2** | ***RASYLES*** |
| | (L58-L72; AHo numbering) | |
| 6 | **LCDR2** | ***RGGYLES*** |
| | (L58-L72; AHo numbering) | |
| 7 | **LCDR3** | ***QSTDSSTYGGA*** |
| | (L107-L138; AHo numbering) | |
| 8 | **VH** | QQVVESGGGLVQPGGSLRLSCAVSGIDLNSYAMTWVRQAPGKGLEWIGYIGSASS TYYASWASGRFTISKDNSKNTVYLQMNSLRAEDTAVYFCARGSTYINIWGQGTLVT VSS |
| | 70-26-B04-sc03 | |
| 9 | **VH** | QQVVESGGGLVQPGGSLRLSCAVSGIDLNSYAMTWVRQAPGKGLEWIGYIGSASS TYYASWASGRFTISKDNSKNTVYLQMNSLRAEDSAVYFCARGSTYINIWGQGTLVK VSS |
| | 70-26-B04-sc03_(T101S_T146K) | |
| 10 | **VH** | QQVVESGGGLVQPGGSLRLSCAVSGIDLNSYAMTWVRQAPGKCLEWIGYIGSASS TYYASWASGRFTISKDNSKNTVYLQMNSLRAEDSAVYFCARGSTYINWGQGTLVK VSS |
| | 70-26-B04-sc03_(G51C_T101S_T146K) | |
| 11 | **VL** | DVQMTQSPSSLSASVGDRVTITCQASQSISSWVSWYQQKPGKPPKLLIYRASYLES GVPSRFSASGSGTDFTLTISSLQPEDFATYYCQSTDSSTYGGAFGTGTKVTVLG |
| | 70-26-B04-sc03 | |
| 12 | **VL** | DVQMTQSPSSLSASVGDRVTITCQASQSISSWVSWYQQKPGKPPKLLIYRGGYLE SGVPSRFSASGSGTDFTLTISSLQPEDFATYYCQSTDSSTYGGAFGTGTKVTVLG |
| | 70-26-B04-sc03_(A67G_S68G) | |
| 13 | **VL** | DVQMTQSPSSLSASVGDRVTITCQASQSISSWVSWYQQKPGKPPKLLIYRGGYLE SGVPSRFSASGSGTDFTLTISSLQPEDFATYYCQSTDSSTYGGAFGCGTKVTVLG |
| | 70-26-B04-sc03 (A67G_S68G_T141C) | |
| 14 | **VL** | DVQMTQSPSSLSASVGDRVTITCQASQSISSWVSWYQQKPGKPPKLLIYRASYLES GVPSRFSASGSGTDFTLTISSLQPEDFATYYCQSTDSSTYGGAFGQGTKVEIK |
| | 70-26-B04-sc03 (KappaCap) | |
| 15 | **VL** | DVQMTQSPSSLSASVGDRVTITCQASQSISSWVSWYQQKPGKPPKLLIYRGGYLE SGVPSRFSASGSGTDFTLTISSLQPEDFATYYCQSTDSSTYGGAFGQGTKVEIK |
| | 70-26-B04-sc03 (A67G_S68G_KappaCap) | |
| 16 | **scFv (VL-linker-VH)** | DVQMTQSPSSLSASVGDRVTITCQASQSISSWVSWYQQKPGKPPKLLIYRASYLES GVPSRFSASGSGTDFTLTISSLQPEDFATYYCQSTDSSTYGGAFGTGTKVTVLGGG GGSGGGGSGGGGSGGGGSQQVVESGGGLVQPGGSLRLSCAVSGIDLNSYAMT WVRQAPGKGLEWIGYIGSASSTYYASWASGRFTISKDNSKNTVYLQMNSLRAEDT AVYFCARGSTYINIWGQGTLVTVSS |
| | 70-26-B04-sc03 | |
| 17 | **scFv (VL-linker-VH)** | DVQMTQSPSSLSASVGDRVTITCQASQSISSWVSWYQQKPGKPPKLLIYRGGYLE SGVPSRFSASGSGTDFTLTISSLQPEDFATYYCQSTDSSTYGGAFGTGTKVTVLGG GGGSGGGGSGGGGSGGGGSQQVVESGGGLVQPGGSLRLSCAVSGIDLNSYAMT WVRQAPGKGLEWIGYIGSASSTYYASWASGRFTISKDNSKNTVYLQMNSLRAEDT AVYFCARGSTYINIWGQGTLVTVSS |
| | 70-26-B04-sc03_(VL_A67G_S68G) | |
| 18 | **scFv (VL-linker-VH)** | DVQMTQSPSSLSASVGDRVTITCQASQSISSWVSWYQQKPGKPPKLLIYRGGYLE SGVPSRFSASGSGTDFTLTISSLQPEDFATYYCQSTDSSTYGGAFGTGTKVTVLGG GGGSGGGGSGGGGSGGGGSQQVVESGGGLVQPGGSLRLSCAVSGIDLNSYAMT WVRQAPGKGLEWIGYIGSASSTYYASWASGRFTISKDNSKNTVYLQMNSLRAEDS AVYFCARGSTYINIWGQGTLVKVSS |
| | 70-26-B04-sc03_(VL_A67G_S68G; VH_T101S_T146K) | |
| 19 | **scFv (VL-linker-VH)** | DVQMTQSPSSLSASVGDRVTITCQASQSISSWVSWYQQKPGKPPKLLIYRGGYLE SGVPSRFSASGSGTDFTLTISSLQPEDFATYYCQSTDSSTYGGAFGCGTKVTVLGG GGGSGGGGSGGGGSGGGGSQQVVESGGGLVQPGGSLRLSCAVSGIDLNSYAMT WVRQAPGKCLEWIGYIGSASSTYYASWASGRFTISKDNSKNTVYLQMNSLRAEDS AVYFCARGSTYINIWGQGTLVKVSS |
| | 70-26-B04-sc03_(VL_A67G_S68G_T141C; VH_G51C_T101S_T146K) (PRO3703) | |

| **Anti-PD-L1 antibody variable domains** | | |
|---|---|---|
| **SEQ ID NO:** | **Description:** | **Sequence:** |
| | | |

| *37-20-B03* - *based sequences* | | |
|---|---|---|
| 20 | **HCDR1** | ***GFSFNSDYWIY*** |
| | (H27-H42; AHo numbering) | |
| 21 | **HCDR2** | ***SIYGGSSGNTQYASWAQG*** |
| | (H57-H76; AHo numbering) | |
| 22 | **HCDR2** | ***CIYGGSSGNTQYASWAQG*** |
| | (H57-H76; AHo numbering) | |
| 23 | **HCDR3** | ***RGYVDYGGATDL*** |
| | (H108-H138; AHo numbering) | |
| 24 | **LCDR1** | ***QASQSIGTYLA*** |
| | (L24-L42; AHo numbering) | |
| 25 | **LCDR2** | ***RAFILAS*** |
| | (L58-L72; AHo numbering) | |
| 26 | **LCDR2** | ***RGGILAS*** |
| | (L58-L72; AHo numbering) | |
| 27 | **LCDR3** | ***QSNFYSDSTTIGPNA*** |
| | (L107-L138; AHo numbering) | |
| 28 | **VH** | QVQLQESGPGLVKPSETLSLTCKVSGFSFNSDYWIYWIRQPPGKGLEWIGSIYGGS SGNTQYASWAQGRVTISVDSSKNQFSLKLSSVTAADTAVYYCARGYVDYGGATDL WGQGTLVTVSS |
| | 37-20-B03-sc01 | |
| 29 | **VH** | QVQLQESGPGLVKPSETLSLTCKVSGFSFNSDYWIYWIRQPPGKCLEWIGSIYGGS SGNTQYASWAQGRVTISVDSSKNQFSLKLSSVTAADTAVYYCARGYVDYGGATDL WGQGTLVTVSS |
| | 37-20-B03-sc01_(G51C) | |
| 30 | **VH** | QVQLQESGPGLVKPSETLSLTCKVSGFSFNSDYWIYWIRQPPGKGLEWIGSIYGGS SGNTQYASWAQGRVTISVDSSKNQFSLKLSSVTAADSAVYYCARGYVDYGGATDL WGQGTLVKVSS |
| | 37-20-B03-sc01_(T101S_T146K) | |
| 31 | **VH** | QVQLQESGPGLVKPSETLSLTCKVSGFSFNSDYWIYWIRQPPGKCLEWIGSIYGGS SGNTQYASWAQGRVTISVDSSKNQFSLKLSSVTAADSAVYYCARGYVDYGGATDL WGQGTLVKVSS |
| | 37-20-B03-sc01_(G51C_T101S_T146K) | |
| 32 | **VL** | DIQMTQSPSSLSASVGDRVTITCQASQSIGTYLAWYQQKPGKAPKLLIYRAFILASG VPSRFSGSGSGTDFTLTISSLQPEDFATYYCQSNFYSDSTTIGPNAFGTGTKVTVLG |
| | 37-20-B03-sc01 | |
| 33 | **VL** | DIQMTQSPSSLSASVGDRVTITCQASQSIGTYLAWYQQKPGKAPKLLIYRAFILASG VPSRFSGSGSGTDFTLTISSLQPEDFATYYCQSNFYSDSTTIGPNAFGCGTKVTVLG |
| | 37-20-B03-sc01_(T141C) | |
| 34 | **VL** | DIQMTQSPSSLSASVGDRVTITCQASQSIGTYLAWYQQKPGKAPKLLIYRGGILASG VPSRFSGSGSGTDFTLTISSLQPEDFATYYCQSNFYSDSTTIGPNAFGTGTKVTVLG |
| | 37-20-B03-sc01_(A67G_F68G) | |
| 35 | **VL** | DIQMTQSPSSLSASVGDRVTITCQASQSIGTYLAWYQQKPGKAPKLLIYRGGILASG VPSRFSGSGSGTDFTLTISSLQPEDFATYYCQSNFYSDSTTIGPNAFGCGTKVTVLG |
| | 37-20-B03-sc01 (A67G F68G T141C) | |
| 36 | **VL** | DIQMTQSPSSLSASVGDRVTITCQASQSIGTYLAWYQQKPGKAPKLLIYRAFILASG VPSRFSGSGSGTDFTLTISSLQPEDFATYYCQSNFYSDSTTIGPNAFGQGTKVEIK |
| | 37-20-B03-sc01 (KappaCap) | |
| 37 | **VL** | DIQMTQSPSSLSASVGDRVTITCQASQSIGTYLAWYQQKPGKAPKLLIYRGGILASG VPSRFSGSGSGTDFTLTISSLQPEDFATYYCQSNFYSDSTTIGPNAFGQGTKVEIK |
| | 37-20-B03-sc01 (A67G F68G KappaCap) | |
| 38 | **VH** | EVQLVESGGGLVQPGGSLRLSCAASGFSFNSDYWIYWVRQAPGKGLEWIACIYGG SSGNTQYASWAQGRFTISRDNSKNTVYLQMNSLRAEDTAVYFCARGYVDYGGAT DLWGQGTLVTVSS |
| | 37-20-B03-sc09 | |
| 39 | **VH** | EVQLVESGGGLVQPGGSLRLSCAASGFSFNSDYWIYWVRQAPGKCLEWIACIYGG SSGNTQYASWAQGRFTISRDNSKNTVYLQMNSLRAEDTAVYFCARGYVDYGGAT DLWGQGTLVTVSS |
| | 37-20-B03-sc09_(G51C) | |
| 40 | **VH** | EVQLVESGGGLVQPGGSLRLSCAASGFSFNSDYWIYWVRQAPGKGLEWIACIYGG SSGNTQYASWAQGRFTISRDNSKNTVYLQMNSLRAEDSAVYFCARGYVDYGGAT DLWGQGTLVKVSS |
| | 37-20-B03-sc09_(T101S_T146K) | |
| 41 | **VH** | EVQLVESGGGLVQPGGSLRLSCAASGFSFNSDYWIYWVRQAPGKCLEWIACIYGG SSGNTQYASWAQGRFTISRDNSKNTVYLQMNSLRAEDSAVYFCARGYVDYGGAT DLWGQGTLVKVSS |
| | 37-20-B03-sc09_(G51C_T101S_T146K) | |
| 42 | **VH** | EVQLVESGGGLVQPGGSLRLSCAASGFSFNSDYWIYWVRQAPGKGLEWIASIYGG SSGNTQYASWAQGRFTISRDNSKNTVYLQMNSLRAEDTAVYFCARGYVDYGGAT DLWGQGTLVTVSS |
| | 37-20-B03-sc09.1 | |
| 43 | **VH** | EVQLVESGGGLVQPGGSLRLSCAASGFSFNSDYWIYWVRQAPGKCLEWIASIYGG SSGNTQYASWAQGRFTISRDNSKNTVYLQMNSLRAEDTAVYFCARGYVDYGGAT DLWGQGTLVTVSS |
| | 37-20-B03-sc09.1_(G51C) | |
| 44 | **VH** | EVQLVESGGGLVQPGGSLRLSCAASGFSFNSDYWIYWVRQAPGKGLEWIASIYGG SSGNTQYASWAQGRFTISRDNSKNTVYLQMNSLRAEDSAVYFCARGYVDYGGAT DLWGQGTLVKVSS |
| | 37-20-B03-sc09.1_(T101S_T146K) | |
| 45 | **VH** | EVQLVESGGGLVQPGGSLRLSCAASGFSFNSDYWIYWVRQAPGKCLEWIASIYGG SSGNTQYASWAQGRFTISRDNSKNTVYLQMNSLRAEDSAVYFCARGYVDYGGAT DLWGQGTLVKVSS |
| | 37-20-B03-sc09.1_(G51C_T101 S_ T146K) | |
| 46 | **VL** | DIQMTQSPASLSASVGDRVTITCQASQSIGTYLAWYQQKPGKPPKLLIYRAFILASG VPSRFSGSGSGTDFTLTISSLQPEDFATYYCQSNFYSDSTTIGPNAFGTGTKVTVLG |
| | 37-20-B03-sc09.1 | |
| | 37-20-B03-sc09 | |
| 47 | **VL** | DIQMTQSPASLSASVGDRVTITCQASQSIGTYLAWYQQKPGKPPKLLIYRAFILASG VPSRFSGSGSGTDFTLTISSLQPEDFATYYCQSNFYSDSTTIGPNAFGCGTKVTVLG |
| | 37-20-B03-sc09.1_(T141C) | |
| | 37-20-B03-sc09_(T141C) | |
| 48 | **VL** | DIQMTQSPASLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG |
| | 37-20-B03-sc09.1_(A67G F68G) | |
| | 37-20-B03-sc09_(A67G_F68G) | |
| 49 | **VL** | DIQMTQSPASLSASVGDRVTITCQASQSIGTYLAWYQQKPGKPPKLLIYRGGILASG VPSRFSGSGSGTDFTLTISSLQPEDFATYYCQSNFYSDSTTIGPNAFGCGTKVTVLG |
| | 37-20-B03-sc09.1_(A67G_F68G_T141C) | |
| | 37-20-B03-sc09_(A67G F68G T141C) | |
| 50 | **VL** | DIQMTQSPASLSASVGDRVTITCQASQSIGTYLAWYQQKPGKPPKLLIYRAFILASG VPSRFSGSGSGTDFTLTISSLQPEDFATYYCQSNFYSDSTTIGPNAFGQGTKVEIK |
| | 37-20-B03-sc09.1_(KappaCap) | |
| | 37-20-B03-sc09_(KappaCap) | |
| 51 | **VL** | DIQMTQSPASLSASVGDRVTITCQASQSIGTYLAWYQQKPGKPPKLLIYRGGILASG VPSRFSGSGSGTDFTLTISSLQPEDFATYYCQSNFYSDSTTIGPNAFGQGTKVEIK |
| | 37-20-B03-sc09.1_(A67G_F68G_KappaCap) | |
| | 37-20-B03-sc09_(A67G F68G KappaCap) | |
| 52 | **VH** | QQLVESGGGLVQPGGSLRLSCAASGFSFNSDYWIYWVRQAPGKGLEWIASIYGGS SGNTQYASWAQGRFTISKDNSKNTVYLQMNSLRAEDTAVYFCARGYVDYGGATD LWGQGTLVTVSS |
| | 37-20-B03-sc18 | |
| 53 | **VH** | QQLVESGGGLVQPGGSLRLSCAASGFSFNSDYWIYWVRQAPGKCLEWIASIYGGS SGNTQYASWAQGRFTISKDNSKNTVYLQMNSLRAEDTAVYFCARGYVDYGGATD LWGQGTLVTVSS |
| | 37-20-B03-sc18_(G51C) | |
| 54 | **VH** | QQLVESGGGLVQPGGSLRLSCAASGFSFNSDYWIYWVRQAPGKGLEWIASIYGGS SGNTQYASWAQGRFTISKDNSKNTVYLQMNSLRAEDSAVYFCARGYVDYGGATD LWGQGTLVKVSS |
| | 37-20-B03-sc18_(T101S_T146K) | |
| 55 | **VH** | QQLVESGGGLVQPGGSLRLSCAASGFSFNSDYWIYWVRQAPGKCLEWIASIYGGS SGNTQYASWAQGRFTISKDNSKNTVYLQMNSLRAEDSAVYFCARGYVDYGGATD LWGQGTLVKVSS |
| | 37-20-B03-sc18_(G51C_T101S_T146K) | |
| 56 | **VL** | DIQMTQSPSSLSASVGDRVTITCQASQSIGTYLAWYQQKPGKPPKLLIYRAFILASG VPSRFSGSGSGTDFTLTISSLQPEDFATYYCQSNFYSDSTTIGPNAFGTGTKVTVLG |
| | 37-20-B03-sc18 | |
| 57 | **VL** | DIQMTQSPSSLSASVGDRVTITCQASQSIGTYLAWYQQKPGKPPKLLIYRAFILASG VPSRFSGSGSGTDFTLTISSLQPEDFATYYCQSNFYSDSTTIGPNAFGCGTKVTVLG |
| | 37-20-B03-sc18_(T141C) | |
| 58 | **VL** | DIQMTQSPSSLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG |
| | 37-20-B03-sc18_(A67G_F68G) | |
| 59 | **VL** | DIQMTQSPSSLSASVGDRVTITCQASQSIGTYLAWYQQKPGKPPKLLIYRGGILASG VPSRFSGSGSGTDFTLTISSLQPEDFATYYCQSNFYSDSTTIGPNAFGCGTKVTVLG |
| | 37-20-B03-sc18_(A67G_F68G_T141C) | |
| 60 | **VL** | DIQMTQSPSSLSASVGDRVTITCQASQSIGTYLAWYQQKPGKPPKLLIYRAFILASG VPSRFSGSGSGTDFTLTISSLQPEDFATYYCQSNFYSDSTTIGPNAFGQGTKVEIK |
| | 37-20-B03-sc18_(KappaCap) | |
| 61 | **VL** | DIQMTQSPSSLSASVGDRVTITCQASQSIGTYLAWYQQKPGKPPKLLIYRGGILASG VPSRFSGSGSGTDFTLTISSLQPEDFATYYCQSNFYSDSTTIGPNAFGQGTKVEIK |
| | 37-20-B03-sc18_(A67G_F68G_KappaCap) | |
| 62 | **scFv (VL-linker-VH)** | DIQMTQSPSSLSASVGDRVTITCQASQSIGTYLAWYQQKPGKAPKLLIYRAFILASG VPSRFSGSGSGTDFTLTISSLQPEDFATYYCQSNFYSDSTTIGPNAFGTGTKVTVLG GGGGSGGGGSGGGGSGGGGSQVQLQESGPGLVKPSETLSLTCKVSGFSFNSDY WIYWIRQPPGKGLEWIGSIYGGSSGNTQYASWAQGRVTISVDSSKNQFSLKLSSVT AADTAVYYCARGYVDYGGATDLWGQGTLVTVSS |
| | 37-20-B03-sc01 (PRO997) | |
| 63 | **scFv (VL-linker-VH)** | DIQMTQSPSSLSASVGDRVTITCQASQSIGTYLAWYQQKPGKAPKLLIYRGGILASG VPSRFSGSGSGTDFTLTISSLQPEDFATYYCQSNFYSDSTTIGPNAFGTGTKVTVLG GGGGSGGGGSGGGGSGGGGSQVQLQESGPGLVKPSETLSLTCKVSGFSFNSDY WIYWIRQPPGKGLEWIGSIYGGSSGNTQYASWAQGRVTISVDSSKNQFSLKLSSVT AADTAVYYCARGYVDYGGATDLWGQGTLVTVSS |
| | 37-20-B03-sc01_(VL_A67G_F68G) | |
| 64 | **scFv (VL-linker-VH)** | DIQMTQSPSSLSASVGDRVTITCQASQSIGTYLAWYQQKPGKAPKLLIYRGGILASG VPSRFSGSGSGTDFTLTISSLQPEDFATYYCQSNFYSDSTTIGPNAFGTGTKVTVLG GGGGSGGGGSGGGGSGGGGSQVQLQESGPGLVKPSETLSLTCKVSGFSFNSDY WIYWIRQPPGKGLEWIGSIYGGSSGNTQYASWAQGRVTISVDSSKNQFSLKLSSVT AADSAVYYCARGYVDYGGATDLWGQGTLVKVSS |
| | 37-20-B03-sc01_(VL_A67G_F68G; VH_T101S_T146K) | |
| 65 | **scFv (VL-linker-VH)** | DIQMTQSPASLSASVGDRVTITCQASQSIGTYLAWYQQKPGKPPKLLIYRAFILASG VPSRFSGSGSGTDFTLTISSLQPEDFATYYCQSNFYSDSTTIGPNAFGTGTKVTVLG GGGGSGGGGSGGGGSGGGGSEVQLVESGGGLVQPGGSLRLSCAASGFSFNSDY WIYWVRQAPGKGLEWIACIYGGSSGNTQYASWAQGRFTISRDNSKNTVYLQMNSL RAEDTAVYFCARGYVDYGGATDLWGQGTLVTVSS |
| | 37-20-B03-sc09 (PRO1347) | |
| 66 | **scFv (VL-linker-VH)** | DIQMTQSPASLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG GGGGSGGGGSGGGGSGGGGSEVQLVESGGGLVQPGGSLRLSCAASGFSFNSDY WIYWVRQAPGKGLEWIACIYGGSSGNTQYASWAQGRFTISRDNSKNTVYLQMNSL RAEDTAVYFCARGYVDYGGATDLWGQGTLVTVSS |
| | 37-20-B03-sc09_(VL_A67G_F68G) | |
| 67 | **scFv (VL-linker-VH)** | DIQMTQSPASLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG GGGGSGGGGSGGGGSGGGGSEVQLVESGGGLVQPGGSLRLSCAASGFSFNSDY WIYWVRQAPGKGLEWIACIYGGSSGNTQYASWAQGRFTISRDNSKNTVYLQMNSL RAEDSAVYFCARGYVDYGGATDLWGQGTLVKVSS |
| | 37-20-B03-sc09_(VL_A67G_F68G; VH_T101S_T146K) | |
| 68 | **scFv (VL-linker-VH)** | DIQMTQSPASLSASVGDRVTITCQASQSIGTYLAWYQQKPGKPPKLLIYRAFILASG VPSRFSGSGSGTDFTLTISSLQPEDFATYYCQSNFYSDSTTIGPNAFGTGTKVTVLG GGGGSGGGGSGGGGSGGGGSEVQLVESGGGLVQPGGSLRLSCAASGFSFNSDY WIYWVRQAPGKGLEWIASIYGGSSGNTQYASWAQGRFTISRDNSKNTVYLQMNSL RAEDTAVYFCARGYVDYGGATDLWGQGTLVTVSS |
| | 37-20-B03-sc09.1 (PRO2230) | |
| 69 | **scFv (VL-linker-VH)** | DIQMTQSPASLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG GGGGSGGGGSGGGGSGGGGSEVQLVESGGGLVQPGGSLRLSCAASGFSFNSDY WIYWVRQAPGKGLEWIASIYGGSSGNTQYASWAQGRFTISRDNSKNTVYLQMNSL RAEDTAVYFCARGYVDYGGATDLWGQGTLVTVSS |
| | 37-20-B03-sc09.1_(VL_A67G_F68G) | |
| 70 | **scFv (VL-linker-VH)** | DIQMTQSPASLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG GGGGSGGGGSGGGGSGGGGSEVQLVESGGGLVQPGGSLRLSCAASGFSFNSDY WIYWVRQAPGKGLEWIASIYGGSSGNTQYASWAQGRFTISRDNSKNTVYLQMNSL RAEDSAVYFCARGYVDYGGATDLWGQGTLVKVSS |
| | 37-20-B03-sc09.1_(VL_A67G_F68G; VH_T101S_T146K) | |
| 71 | **scFv (VL-linker-VH)** | DIQMTQSPSSLSASVGDRVTITCQASQSIGTYLAWYQQKPGKPPKLLIYRAFILASG VPSRFSGSGSGTDFTLTISSLQPEDFATYYCQSNFYSDSTTIGPNAFGTGTKVTVLG GGGGSGGGGSGGGGSGGGGS-QQLVESGGGLVQPGGSLRLSCAASGFSFNSDY WIYWVRQAPGKGLEWIASIYGGSSGNTQYASWAQGRFTISKDNSKNTVYLQMNSL RAEDTAVYFCARGYVDYGGATDLWGQGTLVTVSS |
| | 37-20-B03-sc18 | |
| 72 | **scFv (VL-linker-VH)** | DIQMTQSPSSLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG GGGGSGGGGSGGGGSGGGGS-QQLVESGGGLVQPGGSLRLSCAASGFSFNSDY WIYWVRQAPGKGLEWIASIYGGSSGNTQYASWAQGRFTISKDNSKNTVYLQMNSL RAEDTAVYFCARGYVDYGGATDLWGQGTLVTVSS |
| | 37-20-B03-sc18_(VL_A67G_F68G) | |
| 73 | **scFv (VL-linker-VH)** | DIQMTQSPSSLSASVGDRVTITCQASQSIGTYLAWYQQKPGKPPKLLIYRGGILASG VPSRFSGSGSGTDFTLTISSLQPEDFATYYCQSNFYSDSTTIGPNAFGTGTKVTVLG GGGGSGGGGSGGGGSGGGGS-QQLVESGGGLVQPGGSLRLSCAASGFSFNSDY WIYWVRQAPGKGLEWIASIYGGSSGNTQYASWAQGRFTISKDNSKNTVYLQMNSL RAEDSAVYFCARGYVDYGGATDLWGQGTLVKVSS |
| | 37-20-B03-sc18_(VL_A67G_F68G; VH_T101S_T146K) (PRO4245) | |

| *33-03-G02* - *based sequences* | | |
|---|---|---|
| 74 | **HCDR1** (H27-H42; AHo numbering) | ***GFSFSSGYDMC*** |
| 75 | **HCDR2** (H57-H76; AHo numbering) | ***CWAGSVDITYYASWAKG*** |
| 76 | **HCDR3** (H108-H138; AHo numbering) | ***RKDAYSDAFNL*** |
| 77 | **LCDR1** (L24-L42; AHo numbering) | ***QASQSINDYLA*** |
| 78 | **LCDR2** (L58-L72; AHo numbering) | ***KASTLAS*** |
| 79 | **LCDR2** (L58-L72; AHo numbering) | ***KGGTLAS*** |
| 80 | **LCDR3** (L107-L138; AHo numbering) | ***QQGYIITDIDNV*** |
| 81 | **VH** | QVQLQESGPGLVKPSETLSLTCKASGFSFSSGYDMCWVRQPPGKGLEWIACVVA GSVDITYYASWAKGRVTISKDSSKNQVSLKLSSVTAADTAVYYCARKDAYSDAFNL WGQGTLVTVSS |
| | 33-03-G02-sc18 (PRO1392) | |
| 82 | **VH** | QVQLQESGPGLVKPSETLSLTCKASGFSFSSGYDMCWVRQPPGKCLEWIACVVA GSVDITYYASWAKGRVTISKDSSKNQVSLKLSSVTAADTAVYYCARKDAYSDAFNL WGQGTLVTVSS |
| | 33-03-G02-sc18_(G51C) | |
| 83 | **VH** | QVQLQESGPGLVKPSETLSLTCKASGFSFSSGYDMCWVRQPPGKGLEWIACVVA GSVDITYYASWAKGRVTISKDSSKNQVSLKLSSVTAADSAVYYCARKDAYSDAFNL WGQGTLVKVSS |
| | 33-03-G02-sc18_(T101S_T146K) (PRO3891) | |
| 84 | **VH** | QVQLQESGPGLVKPSETLSLTCKASGFSFSSGYDMCWVRQPPGKCLEWIACVVA GSVDITYYASWAKGRVTISKDSSKNQVSLKLSSVTAADSAVYYCARKDAYSDAFNL WGQGTLVKVSS |
| | 33-03-G02-sc18_(G51C_T101 S_ T146K) | |
| 85 | **VL** | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLG |
| | 33-03-G02-sc18 (PRO1392) | |
| 86 | **VL** | DIQMTQSPSSLSASVGDRVTITCQASQSINDYLAWYQQKPGKAPKLLIYKGGTLAS GVPSRFSGSGSGTDFTLTISSLQPEDFATYYCQQGYIITDIDNVFGTGTKVTVLG |
| | 33-03-G02-sc18_(A67G_S68G) (PRO3891) | |
| 87 | **VL** | DIQMTQSPSSLSASVGDRVTITCQASQSINDYLAWYQQKPGKAPKLLIYKGGTLAS GVPSRFSGSGSGTDFTLTISSLQPEDFATYYCQQGYIITDIDNVFGCGTKVTVLG |
| | 33-03-G02-sc18 (A67G_S68G_T141C) | |
| 88 | **VL** | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGQGTKVEIK |
| | 33-03-G02-sc18 (KappaCap) | |
| 89 | **VL** | DIQMTQSPSSLSASVGDRVTITCQASQSINDYLAWYQQKPGKAPKLLIYKGGTLAS GVPSRFSGSGSGTDFTLTISSLQPEDFATYYCQQGYIITDIDNVFGQGTKVEIK |
| | 33-03-G02-sc18_(A67G_S68G_KappaCap) | |
| 90 | **scFv (VL-linker-VH)** | DIQMTQSPSSLSASVGDRVTITCQASQSINDYLAWYQQKPGKAPKLLIYKASTLAS GVPSRFSGSGSGTDFTLTISSLQPEDFATYYCQQGYIITDIDNVFGTGTKVTVLGGG GGSGGGGSGGGGSGGGGSQVQLQESGPGLVKPSETLSLTCKASGFSFSSGYDM CWVRQPPGKGLEWIACVVAGSVDITYYASWAKGRVTISKDSSKNQVSLKLSSVTA ADTAVYYCARKDAYSDAFNLWGQGTLVTVSS |
| | 33-03-G02-sc18 (PRO1392) | |
| 91 | **scFv (VL-linker-VH)** | DIQMTQSPSSLSASVGDRVTITCQASQSINDYLAWYQQKPGKAPKLLIYKGGTLAS GVPSRFSGSGSGTDFTLTISSLQPEDFATYYCQQGYIITDIDNVFGTGTKVTVLGGG GGSGGGGSGGGGSGGGGSQVQLQESGPGLVKPSETLSLTCKASGFSFSSGYDM CWVRQPPGKGLEWIACVVAGSVDITYYASWAKGRVTISKDSSKNQVSLKLSSVTA ADTAVYYCARKDAYSDAFNLWGQGTLVTVSS |
| | 33-03-G02-sc18_(VL_A67G_S68G) (PRO3724) | |
| 92 | **scFv (VL-linker-VH)** | DIQMTQSPSSLSASVGDRVTITCQASQSINDYLAWYQQKPGKAPKLLIYKGGTLAS GVPSRFSGSGSGTDFTLTISSLQPEDFATYYCQQGYIITDIDNVFGTGTKVTVLGGG GGSGGGGSGGGGSGGGGSQVQLQESGPGLVKPSETLSLTCKASGFSFSSGYDM CWVRQPPGKGLEWIACVVAGSVDITYYASWAKGRVTISKDSSKNQVSLKLSSVTA ADSAVYYCARKDAYSDAFNLWGQGTLVKVSS |
| | 33-03-G02-sc18_(VL_A67G_S68G; VH_T101S_T146K) (PRO3891) | |

| **Anti-LILRB2 antibody variable domains** | | |
|---|---|---|
| *68-18-E11* - *based sequences* | | |
| 93 | **HCDR1** | ***GFSFTNNYYMC*** |
| | (H27-H42; AHo numbering) | |
| 94 | **HCDR2** | ***CML VGA WGNTYYASWANG*** |
| | (H57-H76; AHo numbering) | |
| 95 | **HCDR3** | ***RARDYTWTLGGDPETSGFNL*** |
| | (H108-H138; AHo numbering) | |
| 96 | **LCDR1** | ***QASQSIGSNLA*** |
| | (L24-L42; AHo numbering) | |
| 97 | **LCDR2** | ***KASTLES*** |
| | (L58-L72; AHo numbering) | |
| 98 | **LCDR2** | ***KGGTLES*** |
| | (L58-L72; AHo numbering) | |
| 99 | **LCDR3** (L107-L138; AHo numbering) | ***QSTAGSSRSGNYGYV*** |
| 100 | **VH** | QQLVESGGGLVQPGGSLRLSCAASGFSFTNNYYMCWVRQAPGKGLEWIACMLVG AWGNTYYASWANGRFTISKDNSKNTVYLQMNSLRAEDTAVYFCARARDYTWTLG GDPETSGFNLWGQGTLVTVSS |
| | 68-18-E11-sc07 | |
| 101 | **VH** | QQLVESGGGLVQPGGSLRLSCAASGFSFTNNYYMCWVRQAPGKGLEWIACMLVG AWGNTYYASWANGRFTISKDNSKNTVYLQMNSLRAEDSAVYFCARARDYTWTLG GDPETSGFNLWGQGTLVKVSS |
| | 68-18-E11-sc07_(T101S T146K) | |
| 102 | **VH** | QQLVESGGGLVQPGGSLRLSCAASGFSFTNNYYMCWVRQAPGKCLEWIACMLVG AWGNTYYASWANGRFTISKDNSKNTVYLQMNSLRAEDSAVYFCARARDYTWTLG GDPETSGFNLWGQGTLVKVSS |
| | 68-18-E11-sc07_(G51C T101S_T146K) | |
| 103 | **VL** | DVQMTQSPSSLSASVGDRVTITCQASQSIGSNLAWYQQKPGKPPKLLIYKASTLES GVPSRFSGSGSGTDFTLTISSLQPEDFATYYCQSTAGSSRSGNYGYVFGTGTKVTV LG |
| | 68-18-E11-sc07 | |
| 104 | **VL** | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGTGTKVTV LG |
| | 68-18-E11-sc07_(A67G S68G) | |
| 105 | **VL** | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGCGTKVT VLG |
| | 68-18-E11-sc07_(A67G_S68G_T141C) | |
| 106 | **VL** | DVQMTQSPSSLSASVGDRVTITCQASQSIGSNLAWYQQKPGKPPKLLIYKASTLES GVPSRFSGSGSGTDFTLTISSLQPEDFATYYCQSTAGSSRSGNYGYVFGQGTKVEI K |
| | 68-18-E11-sc07_(KappaCap) | |
| 107 | **VL** | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGQGTKVEI K |
| | 68-18-E11-sc07_(A67G_S68G_KappaCap) | |
| 108 | **scFv (VL-linker-VH)** | DVQMTQSPSSLSASVGDRVTITCQASQSIGSNLAWYQQKPGKPPKLLIYKASTLES GVPSRFSGSGSGTDFTLTISSLQPEDFATYYCQSTAGSSRSGNYGYVFGTGTKVTV LGGGGGSGGGGSGGGGSGGGGSQQLVESGGGLVQPGGSLRLSCAASGFSFTN NYYMCWVRQAPGKGLEWIACMLVGAWGNTYYASWANGRFTISKDNSKNTVYLQ MNSLRAEDTAVYFCARARDYTWTLGGDPETSGFNLWGQGTLVTVSS |
| | 68-18-E11-sc07 | |
| 109 | **scFv (VL-linker-VH)** | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGTGTKVTV LGGGGGSGGGGSGGGGSGGGGSQQLVESGGGLVQPGGSLRLSCAASGFSFTN NYYMCWVRQAPGKGLEWIACMLVGAWGNTYYASWANGRFTISKDNSKNTVYLQ MNSLRAEDTAVYFCARARDYTWTLGGDPETSGFNLWGQGTLVTVSS |
| | 68-18-E11-sc07_(VL _A67G_S68G) | |
| 110 | **scFv (VL-linker-VH)** | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGTGTKVTV LGGGGGSGGGGSGGGGSGGGGSQQLVESGGGLVQPGGSLRLSCAASGFSFTN NYYMCWVRQAPGKGLEWIACMLVGAWGNTYYASWANGRFTISKDNSKNTVYLQ MNSLRAEDSAVYFCARARDYTWTLGGDPETSGFNLWGQGTLVKVSS |
| | 68-18-E11-sc07_(VL _A67G_S68G; VH_T101S_T146K) | |
| 111 | **scFv (VL-linker-VH)** | DVQMTQSPSSLSASVGDRVTITCQASQSIGSNLAWYQQKPGKPPKLLIYKGGTLES GVPSRFSGSGSGTDFTLTISSLQPEDFATYYCQSTAGSSRSGNYGYVFGCGTKVT VLGGGGGSGGGGSGGGGSGGGGSQQLVESGGGLVQPGGSLRLSCAASGFSFTN NYYMCWVRQAPGKCLEWIACMLVGAWGNTYYASWANGRFTISKDNSKNTVYLQ MNSLRAEDSAVYFCARARDYTWTLGGDPETSGFNLWGQGTLVKVSS |
| | 68-18-E11-sc07_(VL_A67G_S68G_T141C; VH_G51C_T101S_T146K) (PRO3744) | |

| *69-06-G04* - *based sequences* | | |
|---|---|---|
| 112 | **HCDR1** | ***GFDLSADYMC*** |
| | (H27-H42; AHo numbering) | |
| 113 | **HCDR2** | ***CIYIDDDNTYYATWAKG*** |
| | (H57-H76; AHo numbering) | |
| 114 | **HCDR3** | ***RGYGVYTGAITYFTL*** |
| | (H108-H138; AHo numbering) | |
| 115 | **LCDR1** | ***QASQSIGTYLA*** |
| | (L24-L42; AHo numbering) | |
| 116 | **LCDR2** | ***RASTLAS*** |
| | (L58-L72; AHo numbering) | |
| 117 | **LCDR2** | ***RGGTLAS*** |
| | (L58-L72; AHo numbering) | |
| 118 | **LCDR3** | ***QSYDSSNT*** |
| | (L107-L138; AHo numbering) | |
| 119 | **VH** | QEQLVESGGGLVQPGRSLRLSCAASGFDLSADYMCWVRQAPGKGLEWIACIYIDD DNTYYATWAKGRFTISKDNSKNTVYLQMNSLRAEDTAVYFCARGYGVYTGAITYFT LWGQGTLVLVSS |
| | 69-06-G04-sc05 | |
| 120 | **VH** | QEQLVESGGGLVQPGRSLRLSCAASGFDLSADYMCWVRQAPGKGLEWIACIYIDD DNTYYATWAKGRFTISKDNSKNTVYLQMNSLRAEDSAVYFCARGYGVYTGAITYFT LWGQGTLVKVSS |
| | 69-06-G04-sc05_(T101S_T146K) | |
| 121 | **VL** | AFQLTQSPSSLSASVGDRVTITCQASQSIGTYLAWYQQKPGKPPKLLIYRASTLAS GVPSRFSGSGSGTDFTLTISSLQPEDFATYYCQSYDSSNTFGTGTKVTVLG |
| | 69-06-G04-sc05 | |
| 122 | **VL** | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGTGTKVTVLG |
| | 69-06-G04-sc05_(A67G_S68G) | |
| 123 | **VL** | AFQLTQSPSSLSASVGDRVTITCQASQSIGTYLAWYQQKPGKPPKLLIYRASTLAS GVPSRFSGSGSGTDFTLTISSLQPEDFATYYCQSYDSSNTFGQGTKVEIK |
| | 69-06-G04-sc05_(KappaCap) | |
| 124 | **VL** | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGQGTKVEIK |
| | 69-06-G04-sc05_(A67G_S68G_KappaCap) | |
| 125 | **scFv (VL-linker-VH)** | AFQLTQSPSSLSASVGDRVTITCQASQSIGTYLAWYQQKPGKPPKLLIYRASTLAS GVPSRFSGSGSGTDFTLTISSLQPEDFATYYCQSYDSSNTFGTGTKVTVLGGGGG SGGGGSGGGGSGGGGSQEQLVESGGGLVQPGRSLRLSCAASGFDLSADYMCW VRQAPGKGLEWIACIYIDDDNTYYATWAKGRFTISKDNSKNTVYLQMNSLRAEDTA VYFCARGYGVYTGAITYFTLWGQGTLVLVSS |
| | 69-06-G04-sc05 | |
| 126 | **scFv (VL-linker-VH)** | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGTGTKVTVLGGGGG SGGGGSGGGGSGGGGSQEQLVESGGGLVQPGRSLRLSCAASGFDLSADYMCW VRQAPGKGLEWIACIYIDDDNTYYATWAKGRFTISKDNSKNTVYLQMNSLRAEDTA VYFCARGYGVYTGAITYFTLWGQGTLVLVSS |
| | 69-06-G04-sc05_(VL_A67G_S68G) | |
| 127 | **scFv (VL-linker-VH)** | AFQLTQSPSSLSASVGDRVTITCQASQSIGTYLAWYQQKPGKPPKLLIYRGGTLAS GVPSRFSGSGSGTDFTLTISSLQPEDFATYYCQSYDSSNTFGTGTKVTVLGGGGG SGGGGSGGGGSGGGGSQEQLVESGGGLVQPGRSLRLSCAASGFDLSADYMCW VRQAPGKGLEWIACIYIDDDNTYYATWAKGRFTISKDNSKNTVYLQMNSLRAEDSA VYFCARGYGVYTGAITYFTLWGQGTLVKVSS |
| | 69-06-G04-sc05_(VL_A67G_S68G; VH_T101S_L146K) (PRO3697) | |

| *69-01-E02* - *based sequences* | | |
|---|---|---|
| 128 | **HCDR1** | ***GFSFSSSSYMC*** |
| | (H27-H42; AHo numbering) | |
| 129 | **HCDR2** | ***CMVTGSSDDTDYASWAKG*** |
| | (H57-H76; AHo numbering) | |
| 130 | **HCDR3** | ***RAGSAPL*** |
| | (H108-H138; AHo numbering) | |
| 131 | **LCDR1** | ***QASQSIGLNLA*** |
| | (L24-L42; AHo numbering) | |
| 132 | **LCDR2** | ***AASYLAS*** |
| | (L58-L72; AHo numbering) | |
| 133 | **LCDR2** | ***AGGYLAS*** |
| | (L58-L72; AHo numbering) | |
| 134 | **LCDR3** | ***QSTYDGSSFVFA*** |
| | (L107-L138; AHo numbering) | |
| 135 | **VH** | QEQLVESGGGLVQPGGSLRLSCAASGFSFSSSSYMCWVRQAPGKGLEWIACMVT GSSDDTDYASWAKGRFTISKDNSKNTVYLQMNSLRAEDTAVYFCARAGSAPLWG QGTLVLVSS |
| | 69-01-E02-sc06 | |
| 136 | **VH** | QEQLVESGGGLVQPGGSLRLSCAASGFSFSSSSYMCWVRQAPGKGLEWIACMVT GSSDDTDYASWAKGRFTISKDNSKNTVYLQMNSLRAEDSAVYFCARAGSAPLWG QGTLVKVSS |
| | 69-01-E02-sc06_(T101S_T146K) | |
| 137 | **VL** | DVQMTQSPSSLSASVGDRVTITCQASQSIGLNLAWYQQKPGKPPKLLIYAASYLAS GVSSRFSGSGSGTDFTLTISSLQPEDFATYYCQSTYDGSSFVFAFGTGTKVTVLG |
| | 69-01-E02-sc06 | |
| 138 | **VL** | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGTGTKVTVLG |
| | 69-01-E02-sc06_(A67G_S68G) | |
| 139 | **VL** | DVQMTQSPSSLSASVGDRVTITCQASQSIGLNLAWYQQKPGKPPKLLIYAASYLAS GVSSRFSGSGSGTDFTLTISSLQPEDFATYYCQSTYDGSSFVFAFGQGTKVEIKR |
| | 69-01-E02-sc06_(KappaCap) | |
| 140 | **VL** | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGQGTKVEIKR |
| | 69-01-E02-sc06_(A67G_S68G_KappaCap) | |
| 141 | **scFv (VL-linker-VH)** | DVQMTQSPSSLSASVGDRVTITCQASQSIGLNLAWYQQKPGKPPKLLIYAASYLAS GVSSRFSGSGSGTDFTLTISSLQPEDFATYYCQSTYDGSSFVFAFGTGTKVTVLGG GGGSGGGGSGGGGSGGGGSQEQLVESGGGLVQPGGSLRLSCAASGFSFSSSSY MCWVRQAPGKGLEWIACMVTGSSDDTDYASWAKGRFTISKDNSKNTVYLQMNSL RAEDTAVYFCARAGSAPLWGQGTLVLVSS |
| | 69-01-E02-sc06 | |
| 142 | **scFv (VL-linker-VH)** | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGTGTKVTVLGG GGGSGGGGSGGGGSGGGGSQEQLVESGGGLVQPGGSLRLSCAASGFSFSSSSY MCWVRQAPGKGLEWIACMVTGSSDDTDYASWAKGRFTISKDNSKNTVYLQMNSL RAEDTAVYFCARAGSAPLWGQGTLVLVSS |
| | 69-01-E02-sc06_(VL_A67G_S68G) | |
| 143 | **scFv (VL-linker-VH)** | DVQMTQSPSSLSASVGDRVTITCQASQSIGLNLAWYQQKPGKPPKLLIYAGGYLAS GVSSRFSGSGSGTDFTLTISSLQPEDFATYYCQSTYDGSSFVFAFGTGTKVTVLGG GGGSGGGGSGGGGSGGGGSQEQLVESGGGLVQPGGSLRLSCAASGFSFSSSSY MCWVRQAPGKGLEWIACMVTGSSDDTDYASWAKGRFTISKDNSKNTVYLQMNSL RAEDSAVYFCARAGSAPLWGQGTLVKVSS |
| | 69-01-E02-sc06_(VL_A67G_S68G; VH_T101S_L146K) (PRO3696) | |

| **Anti-hSA antibody variable domains (dummy domain)** | | |
|---|---|---|
| *19-01-H0 4-sc03* - *based sequences* | | |
| 144 | **VH** | EVQLVESGGGLVQPGGSLRLSCAASGFSLSSNAMGWVRQAPGKGLEYIGIISVGG FTYYASWAKGRFTISRDNSKNTVYLQMNSLRAEDTATYFCARDRHGGDSSGAFYL WGQGTLVTVSS |
| | 19-01-H04-sc03 | |
| 145 | **VH** | EVQLVESGGGLVQPGGSLRLSCAASGFSLSSNAMGWVRQAPGKCLEYIGIISVGG FTYYASWAKGRFTISRDNSKNTVYLQMNSLRAEDSATYFCARDRHGGDSSGAFYL WGQGTLVKVSS |
| | 19-01-H04-sc03_(G51C_T101S_T146K) | |
| 146 | **VL** | DIQMTQSPSSLSASVGDRVTITCQSSESVYSNNQLSWYQQKPGQPPKLLIYDASDL ASGVPSRFSGSGSGTDFTLTISSLQPEDFATYYCAGGFSSSSDTAFGGGTKLTVLG |
| | 19-01-H04-sc03 | |
| 147 | **VL** | DIQMTQSPSSLSASVGDRVTITCQSSESVYSNNQLSWYQQKPGQPPKLLIYDASDL ASGVPSRFSGSGSGTDFTLTISSLQPEDFATYYCAGGFSSSSDTAFGCGTKLTVLG |
| | 19-01-H04-sc03_(T141C) | |
| 148 | **VL** | DIQMTQSPSSLSASVGDRVTITCQSSESVYSNNQLSWYQQKPGQPPKLLIYDGGDL ASGVPSRFSGSGSGTDFTLTISSLQPEDFATYYCAGGFSSSSDTAFGCGTKLTVLG |
| | 19-01-H04-sc03_(A67G_S68G_T141C) | |
| 149 | **scFv** | DIQMTQSPSSLSASVGDRVTITCQSSESVYSNNQLSWYQQKPGQPPKLLIYDASDL ASGVPSRFSGSGSGTDFTLTISSLQPEDFATYYCAGGFSSSSDTAFGCGTKLTVLG GGGGSGGGGSGGGGSGGGGSEVQLVESGGGLVQPGGSLRLSCAASGFSLSSNA MGWVRQAPGKCLEYIGIISVGGFTYYASWAKGRFTISRDNSKNTVYLQMNSLRAED SATYFCARDRHGGDSSGAFYLWGQGTLVKVSS |
| | 19-01-H04-sc03_(G51C_T141C) (T101S_T146K) | |
| 150 | **scFv** | DIQMTQSPSSLSASVGDRVTITCQSSESVYSNNQLSWYQQKPGQPPKLLIYDGGDL ASGVPSRFSGSGSGTDFTLTISSLQPEDFATYYCAGGFSSSSDTAFGCGTKLTVLG GGGGSGGGGSGGGGSGGGGSEVQLVESGGGLVQPGGSLRLSCAASGFSLSSNA MGWVRQAPGKCLEYIGIISVGGFTYYASWAKGRFTISRDNSKNTVYLQMNSLRAED SATYFCARDRHGGDSSGAFYLWGQGTLVKVSS |
| | 19-01-H04-sc03_(G51C_T141C) (T101S_T146K)_(A67G_S68G) | |

**Table 2. Other sequences related to the present invention.**

| **SEQ ID NO:** | **Description** | **Sequence** |
|---|---|---|
| 151 | VH3 | EVQLVESGGGLVQPGGSLRLSCAASGFSFSANYYPCWVRQAPGKGLEWIGCIYGGS SDITYDANWTKGRFTISRDNSKNTVYLQMNSLRAEDTAVYYCARSAWYSGWGGDLW GQGTLVTVSS |
| 152 | VH3_(51C) | EVQLVESGGGLVQPGGSLRLSCAASGFSFSANYYPCWVRQAPGKCLEWIGCIYGGS SDITYDANWTKGRFTISRDNSKNTVYLQMNSLRAEDTAVYYCARSAWYSGWGGDLW GQGTLVTVSS |
| 153 | VH1a | QVQLVQSGAEVKKPGSSVKVSCKASGIDFNSNYYMCWVRQAPGQGLEWMGCIYVGS HVNTYYANWAKGRVTITADESTSTAYMELSSLRSEDTAVYYCATSGSSVLYFKFWGQ GTLVTVSS |
| 154 | VH1b | QVQLVQSGAEVKKPGASVKVSCKASGIDFNSNYYMCWVRQAPGQGLEWMGCIYVGS HVNTYYANWAKGRVTMTRDTSISTAYMELSSLRSEDTAVYYCATSGSSVLYFKFWGQ GTLVTVSS |
| 155 | VH4 | QVQLQESGPGLVKPSETLSLTCTVSGIDFNSNYYMCWIRQPPGKGLEWIGCIYVGSHV NTYYANWAKGRVTISVDTSKNQFSLKLSSVTAADTAVYYCATSGSSVLYFKFWGQGT LVTVSS |
| 156 | VH5 | EVQLVQSGAEVKKPGESLKISCKGS**GIDFNSNYYM**CWVRQMPGKGLEWMG**CIYVGS** **HVNTYYANWAKG**QVTISADKSISTAYLQWSSLKASDTAMYYCA**TSGSSVLYFKF**WGQ GTLVTVSS |
| 157 | VH6 | QVQLQQSGPGLVKPSQTLSLTCAISGIDFNSNYYMCWIRQSPGRGLEWLGCIYVGSHV NTYYANWAKGRITINPDTSKNQFSLQLNSVTPEDTAVYYCATSGSSVLYFKFWGQGT LVTVSS |
| 158 | Vkappa1_V1 (residue 24 (AHo) is defined here to belonq to LFW1) | DIQMTQSPSSLSASVGDRVTITCQ***ASQSINNVLA***WYQQKPGKAPKLLIY***RASTLAS***GV PSRFSGSGSGTDFTLTISSLQPEDFATYYC***QSSYGNYGD***FGQGTKVEIKR |
| 159 | Vkappa1_V1_λ-LFW4 (residue 24 (AHo) is defined here to belong to LFW1) | DIQMTQSPSSLSASVGDRVTITCQASQSINNVLAWYQQKPGKAPKLLIYRASTLASGV PSRFSGSGSGTDFTLTISSLQPEDFATYYCQSSYGNYGDFGTGTKVTVLG |
| 160 | Vkappa1_V1_λ-LFW4_(141C) (residue 24 (AHo) is defined here to belonq to LFW1) | DIQMTQSPSSLSASVGDRVTITCQASQSINNVLAWYQQKPGKAPKLLIYRASTLASGV PSRFSGSGSGTDFTLTISSLQPEDFATYYCQSSYGNYGDFGCGTKVTVLG |
| 161 | Vλ germline-based FR4 (Sk17) | FGTGTKVTVLG |
| 162 | Vλ germline-based FR4 (Sk12) | FGGGTKLTVLG |
| 163 | Vλ germline-based FR4 | FGGGTQLIILG |
| 164 | Vλ germline-based FR4 | FGEGTELTVLG |
| 165 | Vλ germline-based FR4 | FGSGTKVTVLG |
| 166 | Vλ germline-based FR4 | FGGGTQLTVLG |
| 167 | Vλ germline-based FR4 | FGGGTQLTALG |
| 168 | Vλ germline-based FR4_G141T | FGTGTKLTVLG |
| 169 | Vλ germline-based FR4_G141C | FGCGTKVTVLG |
| 170 | Linker L1 | (GGGGS)ₙ, wherein n=1, 2, 3, 4 or 5 |
| 171 | Linker L1 | GGGGSGGGGS |
| 172 | Linker L2 | (GGGGS)ₙ, wherein n=1, 2, 3, 4, 5, 6, 7 or 8 |
| 173 | Linker L2 | GGGGSGGGGSGGGGSGGGGS |

**Table 3: Examples of anti-PDL1 x CD47 multispecific antibodies, according to the present invention (modifications relative to original sequences, i.e. reference sequences, if present, are shown in bold and underlined; CDR residues are shown in bold and italic letters).**

| **SEQ ID NO:** | **Description** | **Sequence** |
|---|---|---|
| **PRO4673_without_dummy_LILRB2-BD** | | |
| 174 | 70-26-B04-sc03_hole-Fc | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPG |
| 175 | 37-20-B03-sc18_knob-Fc | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGTGTKVTVLGGGGGSGGGGSGGGGSGGG GSQQLVESGGGLVQPGGSLRLSCAASGFSFNSDYWIYWVRQAPGKGLEWIASIYGGSSGNTQYAS WAQGRFTISKDNSKNTVYLQMNSLRAEDSAVYFCARGYVDYGGATDLWGQGTLVKVSSEPKSSD KTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVCTLPPS |
| | | REEMTKNQVSLWCLVKGFYPSDIAVEWESNGQPENNYKTTPPVLDSDGSFFLYSKLTVDKSRWQQ GNVFSCSVMHEALHNHYTQKSLSLSPG |

| **PRO3790** | | |
|---|---|---|
| 176 | 70-26-B04-sc03_hole-Fc | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGK |
| 177 | 37-20-B03-sc9.1-(no S101T, K146T)_knob-Fc | EVQLVESGGGLVQPGGSLRLSCAASGFSFNSDYWIYWVRQAPGKGLEWIASIYGGSSGNTQYASW AQGRFTISRDNSKNTVYLQMNSLRAEDTAVYFCARGYVDYGGATDLWGQGTLVTVSSASTKGPSV FPLAPSSKSTSGGTAALGCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKCKVS NKALPAPIEKTISKAKGQPREPQVCTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGK |
| 178 | 37-20-B03-sc9.1-(kappacap)_Ck | DIQMTQSPASLSASVGDRVTITCQASQSIGTYLAWYQQKPGKPPKLLIYRAFILASGVPSRFSGSGS GTDFTLTISSLQPEDFATYYCQSNFYSDSTTIGPNAFGQGTKVEIKRTVAAPSVFIFPPSDEQLKSGT ASVVCLLNNFYPREAKVQWKVDNALQSGNSQESVTEQDSKDSTYSLSSTLTLSKADYEKHKVYACE VTHQGLSSPVTKSFNRGEC |

| **PRO4688_without_dummy_LILRB2-BD** | | |
|---|---|---|
| 179 | 70-26-B04-sc03_hole-Fc | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPG |
| 180 | 37-20-B03-sc18-(no S101T, K146T)_knob-Fc | QQLVESGGGLVQPGGSLRLSCAASGFSFNSDYWIYWVRQAPGKGLEWIASIYGGSSGNTQYASW AQGRFTISKDNSKNTVYLQMNSLRAEDTAVYFCARGYVDYGGATDLWGQGTLVTVSSASTKGPSV FPLAPSSKSTSGGTAALGCLVKDYFPEPVTVSWNSGALTSGVHTFPAVLQSSGLYSLSSVVTVPSSS |
| | | LGTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKCKVS NKALPAPIEKTISKAKGQPREPQVCTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPG |
| 181 | 37-20-B03-sc18-(kappacap)_Ck | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGQGTKVEIKRTVAAPSVFIFPPSDEQLKSGT ASVVCLLNNFYPREAKVQWKVDNALQSGNSQESVTEQDSKDSTYSLSSTLTLSKADYEKHKVYACE VTHQGLSSPVTKSFNRGEC |

| **PRO5053_without_dummy_LILRB2-BD** | | |
|---|---|---|
| 182 | 70-26-B04-sc03_hole-Fc | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPG |
| 183 | 33-03-G02-sc24_knob-Fc | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGTGTKVTVLGGGGGSGGGGSGGGGSGGGGSQ VQLQESGPGLVKPSETLSLTCKASGFSFSSGYDMCWVRQPPGKGLEWIACVVAGSVDITYYASWA KGRVTISKDSSKNQVSLKLSSVTAADSAVYYCARKDAYSDAFNLWGQGTLVKVSSEPKSSDKTHTC PPCPAPELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVCTLPPSREEMT KNQVSLWCLVKGFYPSDIAVEWESNGQPENNYKTTPPVLDSDGSFFLYSKLTVDKSRWQQGNVFS CSVMHEALHNHYTQKSLSLSPG |

| **PRO505 6_without_dummy_LILRB2-BD** | | |
|---|---|---|
| 184 | 70-26-B04-sc03_hole-Fc | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPG |
| 185 | 33-03-G02-sc24 (no S101T, K146T)_knob-Fc | QVQLQESGPGLVKPSETLSLTCKASGFSFSSGYDMCWVRQPPGKGLEWIACVVAGSVDITYYASW AKGRVTISKDSSKNQVSLKLSSVTAADTAVYYCARKDAYSDAFNLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALPAPIEKTISKAKGQPREPQVCTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPG |
| 186 | 33-03-G02-sc24 (kappacap)_Ck | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |

**Table 4: Examples of anti-PDL1 x CD47 x LILRB2 or anti-PDL1 x CD47 x (dummy domain) multispecific antibodies, according to the present invention (modifications relative to original sequences, i.e. reference sequences, if present, are shown in bold and underlined; CDR residues are shown in bold and italic letters).**

| **SEQ ID NO:** | **Description** | **Sequence** |
|---|---|---|
| **PRO4672 (37-20-B03-based PDL1-BD)** | | |
| 187 | (scFv)2-Fc-(scFv)2, hole | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| | 70-26-B04-sc03_ hole-Fc_(G4S)2_68-18-E11-sc07 | |
| 188 | (scFv)2-Fc-(scFv)2, knob | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGTGTKVTVLGGGGGSGGGGSGGGGSGGG GSQQLVESGGGLVQPGGSLRLSCAASGFSFNSDYWIYWVRQAPGKGLEWIASIYGGSSGNTQYAS |
| | 37-20-B03-sc18_knob-Fc_(G4S)2_68-18-E11-sc07 | WAQGRFTISKDNSKNTVYLQMNSLRAEDSAVYFCARGYVDYGGATDLWGQGTLVKVSSEPKSSD KTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVCTLPPS REEMTKNQVSLWCLVKGFYPSDIAVEWESNGQPENNYKTTPPVLDSDGSFFLYSKLTVDKSRWQQ GNVFSCSVMHEALHNHYTQKSLSLSPGKGGGGSGGGGSDVQMTQSPSSLSASVGDRVTITCQAS QSIGSNLAWYQQKPGKPPKLLIYKGGTLESGVPSRFSGSGSGTDFTLTISSLQPEDFATYYCQSTAG SSRSGNYGYVFGCGTKVTVLGGGGGSGGGGSGGGGSGGGGSQQLVESGGGLVQPGGSLRLSC AASGFSFTNNYYMCWVRQAPGKCLEWIACMLVGAWGNTYYASWANGRFTISKDNSKNTVYLQMN SLRAEDSAVYFCARARDYTWTLGGDPETSGFNLWGQGTLVKVSS |

| **PRO4687 (37-20-B03-based PDL1-BD)** | | |
|---|---|---|
| 189 | 70-26-B04-sc03_hole-Fc_(G4S)2_68-18-E11-sc07 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| 190 | 37-20-B03-sc18 (no S101T, K146T)_knob-Fc_(G4S)2_ 68-18-E11-sc07 | QQLVESGGGLVQPGGSLRLSCAASGFSFNSDYWIYWVRQAPGKGLEWIASIYGGSSGNTQYASW AQGRFTISKDNSKNTVYLQMNSLRAEDTAVYFCARGYVDYGGATDLWGQGTLVTVSSASTKGPSV FPLAPSSKSTSGGTAALGCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKCKVS NKALPAPIEKTISKAKGQPREPQVCTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGKGGGGGSGG GGSDVQMTQSPSSLSASVGDRVTITCQASQSIGSNLAWYQQKPGKPPKLLIYKGGTLESGVPSRFS GSGSGTDFTLTISSLQPEDFATYYCQSTAGSSRSGNYGYVFGCGTKVTVLGGGGGSGGGGSGGG GSGGGGSQQLVESGGGLVQPGGSLRLSCAASGFSFTNNYYMCWVRQAPGKCLEWIACMLVGAW GNTYYASWANGRFTISKDNSKNTVYLQMNSLRAEDSAVYFCARARDYTWTLGGDPETSGFNLWG QGTLVKVSS |
| 191 | 37-20-B03-sc18(kappacap)_Ck | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGQGTKVEIKRTVAAPSVFIFPPSDEQLKSGT ASVVCLLNNFYPREAKVQWKVDNALQSGNSQESVTEQDSKDSTYSLSSTLTLSKADYEKHKVYACE VTHQGLSSPVTKSFNRGEC |

| **PRO5052 (PRO4672 with 33-03-G02-based PDL1 -BD)** | | |
|---|---|---|
| 192 | (scFv)2-Fc-(scFv)2, hole | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| | 70-26-B04-sc03_ hole-Fc_(G4S)2_68-18-E11-sc07 | |
| 193 | (scFv)2-Fc-(scFv)2, knob | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGTGTKVTVLGGGGGSGGGGSGGGGSGGGGSQ VQLQESGPGLVKPSETLSLTCKASGFSFSSGYDMCWVRQPPGKGLEWIACVVAGSVDITYYASWA KGRVTISKDSSKNQVSLKLSSVTAADSAVYYCARKDAYSDAFNLWGQGTLVKVSSEPKSSDKTHTC PPCPAPELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVCTLPPSREEMT KNQVSLWCLVKGFYPSDIAVEWESNGQPENNYKTTPPVLDSDGSFFLYSKLTVDKSRWQQGNVFS CSVMHEALHNHYTQKSLSLSPGKGGGGSGGGGSDVQMTQSPSSLSASVGDRVTITCQASQSIGSN LAWYQQKPGKPPKLLIYKGGTLESGVPSRFSGSGSGTDFTLTISSLQPEDFATYYCQSTAGSSRSG NYGYVFGCGTKVTVLGGGGGSGGGGSGGGGSGGGGSQQLVESGGGLVQPGGSLRLSCAASGFS FTNNYYMCWVRQAPGKCLEWIACMLVGAWGNTYYASWANGRFTISKDNSKNTVYLQMNSLRAED SAVYFCARARDYTWTLGGDPETSGFNLWGQGTLVKVSS |
| | 33-03-G02-sc24_knob-Fc_(G4S)2_68-18-E11-sc07 | |

| **PRO5055 (PRO4687 with 33-03-G02-based PDL1-BD)** | | |
|---|---|---|
| 194 | 70-26-B04-sc03_hole-Fc_(G4S)2_68-18-E11-sc07 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE |
| | | LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDVQMTQSPSSLSASVGDRVTITCQASQSIGSNLAWYQQ KPGKPPKLLIYKGGTLESGVPSRFSGSGSGTDFTLTISSLQPEDFATYYCQSTAGSSRSGNYGYVFG CGTKVTVLGGGGGSGGGGSGGGGSGGGGSQQLVESGGGLVQPGGSLRLSCAASGFSFTNNYYM CWVRQAPGKCLEWIACMLVGAWGNTYYASWANGRFTISKDNSKNTVYLQMNSLRAEDSAVYFCA RARDYTWTLGGDPETSGFNLWGQGTLVKVSS |
| 195 | 33-03-G02-sc24 (no S101T, K146T)_knob-Fc_(G4S)2_ 68-18-E11-sc07 | QVQLQESGPGLVKPSETLSLTCKASGFSFSSGYDMCWVRQPPGKGLEWIACVVAGSVDITYYASW AKGRVTISKDSSKNQVSLKLSSVTAADTAVYYCARKDAYSDAFNLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALPAPIEKTISKAKGQPREPQVCTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGGSGGG GSDVQMTQSPSSLSASVGDRVTITCQASQSIGSNLAWYQQKPGKPPKLLIYKGGTLESGVPSRFSG SGSGTDFTLTISSLQPEDFATYYCQSTAGSSRSGNYGYVFGCGTKVTVLGGGGGSGGGGSGGGG SGGGGSQQLVESGGGLVQPGGSLRLSCAASGFSFTNNYYMCWVRQAPGKCLEWIACMLVGAWG NTYYASWANGRFTISKDNSKNTVYLQMNSLRAEDSAVYFCARARDYTWTLGGDPETSGFNLWGQ GTLVKVSS |
| 196 | 33-03-G02-sc24(kappacap)_Ck | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |

| **PRO4673 (PRO4672 with dummy domain for LILRB2-BD)** | | |
|---|---|---|
| 197 | (scFv)2-Fc-(scFv)2, hole | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDIQMTQSPSSLSASVGDRVTITCQSSESVYSNNQLSWYQ QKPGQPPKLLIYDASDLASGVPSRFSGSGSGTDFTLTISSLQPEDFATYYCAGGFSSSSDTAFGCG |
| | 70-26-B04-sc03_hole-Fc_(G4S)2_19-01-H04-sc02 | |
| | | TKLTVLGGGGGSGGGGSGGGGSGGGGSEVQLVESGGGLVQPGGSLRLSCAASGFSLSSNAMGW VRQAPGKCLEYIGIISVGGFTYYASWAKGRFTISRDNSKNTVYLQMNSLRAEDSATYFCARDRHGG DSSGAFYLWGQGTLVKVSS |
| 198 | (scFv)2-Fc-(scFv)2, knob | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGTGTKVTVLGGGGGSGGGGSGGGGSGGG GSQQLVESGGGLVQPGGSLRLSCAASGFSFNSDYWIYWVRQAPGKGLEWIASIYGGSSGNTQYAS WAQGRFTISKDNSKNTVYLQMNSLRAEDSAVYFCARGYVDYGGATDLWGQGTLVKVSSEPKSSD KTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVCTLPPS REEMTKNQVSLWCLVKGFYPSDIAVEWESNGQPENNYKTTPPVLDSDGSFFLYSKLTVDKSRWQQ GNVFSCSVMHEALHNHYTQKSLSLSPGKGGGGSGGGGSDIQMTQSPSSLSASVGDRVTITCQSSE SVYSNNQLSWYQQKPGQPPKLLIYDASDLASGVPSRFSGSGSGTDFTLTISSLQPEDFATYYCAGG FSSSSDTAFGCGTKLTVLGGGGGSGGGGSGGGGSGGGGSEVQLVESGGGLVQPGGSLRLSCAA SGFSLSSNAMGWVRQAPGKCLEYIGIISVGGFTYYASWAKGRFTISRDNSKNTVYLQMNSLRAEDS ATYFCARDRHGGDSSGAFYLWGQGTLVKVSS |
| | 37-20-B03-sc18_knob-Fc_(G4S)2_19-01-H04-sc02 | |

| **PRO4688 (PRO4687 with dummy domain for LILRB2-BD)** | | |
|---|---|---|
| 199 | 70-26-B04-sc03_hole-Fc_(G4S)2_19-01-H04-sc02 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDIQMTQSPSSLSASVGDRVTITCQSSESVYSNNQLSWYQ QKPGQPPKLLIYDASDLASGVPSRFSGSGSGTDFTLTISSLQPEDFATYYCAGGFSSSSDTAFGCG TKLTVLGGGGGSGGGGSGGGGSGGGGSEVQLVESGGGLVQPGGSLRLSCAASGFSLSSNAMGW VRQAPGKCLEYIGIISVGGFTYYASWAKGRFTISRDNSKNTVYLQMNSLRAEDSATYFCARDRHGG DSSGAFYLWGQGTLVKVSS |
| 200 | 37-20-B03-sc18 (no S101T, K146T)_knob-Fc_(G4S)2_19-01-H04-sc02 | QQLVESGGGLVQPGGSLRLSCAASGFSFNSDYWIYWVRQAPGKGLEWIASIYGGSSGNTQYASW AQGRFTISKDNSKNTVYLQMNSLRAEDTAVYFCARGYVDYGGATDLWGQGTLVTVSSASTKGPSV FPLAPSSKSTSGGTAALGCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKCKVS NKALPAPIEKTISKAKGQPREPQVCTLPPSREEMTKNQVSLWCLVKGFYPSDIAVEWESNGQPENN |
| | | YKTTPPVLDSDGSFFLYSKLTVDKSRWQQGNVFSCSVMHEALHNHYTQKSLSLSPGKGGGGGSGG GGSDIQMTQSPSSLSASVGDRVTITCQSSESVYSNNQLSWYQQKPGQPPKLLIYDASDLASGVPSR FSGSGSGTDFTLTISSLQPEDFATYYCAGGFSSSSDTAFGCGTKLTVLGGGGGSGGGGSGGGGSG GGGSEVQLVESGGGLVQPGGSLRLSCAASGFSLSSNAMGWVRQAPGKCLEYIGIISVGGFTYYAS WAKGRFTISRDNSKNTVYLQMNSLRAEDSATYFCARDRHGGDSSGAFYLWGQGTLVKVSS |
| 201 | 37-20-B03-sc18 (kappacap)_Ck | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGQGTKVEIKRTVAAPSVFIFPPSDEQLKSGT ASVVCLLNNFYPREAKVQWKVDNALQSGNSQESVTEQDSKDSTYSLSSTLTLSKADYEKHKVYACE VTHQGLSSPVTKSFNRGEC |
| | | |

| **PRO5053 (PRO5052 with dummy domain for LILRB2-BD)** | | |
|---|---|---|
| 202 | (scFv)2-Fc-(scFv)2, hole | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDIQMTQSPSSLSASVGDRVTITCQSSESVYSNNQLSWYQ QKPGQPPKLLIYDASDLASGVPSRFSGSGSGTDFTLTISSLQPEDFATYYCAGGFSSSSDTAFGCG TKLTVLGGGGGSGGGGSGGGGSGGGGSEVQLVESGGGLVQPGGSLRLSCAASGFSLSSNAMGW VRQAPGKCLEYIGIISVGGFTYYASWAKGRFTISRDNSKNTVYLQMNSLRAEDSATYFCARDRHGG DSSGAFYLWGQGTLVKVSS |
| | 70-26-B04-sc03_hole-Fc_(G4S)2_19-01-H04-sc02 | |
| 203 | (scFv)2-Fc-(scFv)2, knob | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGTGTKVTVLGGGGGSGGGGSGGGGSGGGGSQ VQLQESGPGLVKPSETLSLTCKASGFSFSSGYDMCWVRQPPGKGLEWIACVVAGSVDITYYASWA KGRVTISKDSSKNQVSLKLSSVTAADSAVYYCARKDAYSDAFNLWGQGTLVKVSSEPKSSDKTHTC PPCPAPELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVCTLPPSREEMT KNQVSLWCLVKGFYPSDIAVEWESNGQPENNYKTTPPVLDSDGSFFLYSKLTVDKSRWQQGNVFS CSVMHEALHNHYTQKSLSLSPGKGGGGSGGGGSDIQMTQSPSSLSASVGDRVTITCQSSESVYSN NQLSWYQQKPGQPPKLLIYDASDLASGVPSRFSGSGSGTDFTLTISSLQPEDFATYYCAGGFSSSS DTAFGCGTKLTVLGGGGGSGGGGSGGGGSGGGGSEVQLVESGGGLVQPGGSLRLSCAASGFSL |
| | 33-03-G02-sc24_knob-Fc_(G4S)2_19-01-H04-sc02 | |
| | | SSNAMGWVRQAPGKCLEYIGIISVGGFTYYASWAKGRFTISRDNSKNTVYLQMNSLRAEDSATYFC ARDRHGGDSSGAFYLWGQGTLVKVSS |

| **PRO5056 (PRO5055 with dummy domain for LILRB2-BD)** | | |
|---|---|---|
| 204 | 70-26-B04-sc03_hole-Fc_(G4S)2_19-01-H04-sc02 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE LLGGPSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKNQVSLS CAVKGFYPSDIAVEWESNGQPENNYKTTPPVLDSDGSFFLVSKLTVDKSRWQQGNVFSCSVMHEA LHNHYTQKSLSLSPGKGGGGSGGGGSDIQMTQSPSSLSASVGDRVTITCQSSESVYSNNQLSWYQ QKPGQPPKLLIYDASDLASGVPSRFSGSGSGTDFTLTISSLQPEDFATYYCAGGFSSSSDTAFGCG TKLTVLGGGGGSGGGGSGGGGSGGGGSEVQLVESGGGLVQPGGSLRLSCAASGFSLSSNAMGW VRQAPGKCLEYIGIISVGGFTYYASWAKGRFTISRDNSKNTVYLQMNSLRAEDSATYFCARDRHGG DSSGAFYLWGQGTLVKVSS |
| 205 | 33-03-G02-sc24 (no S101T, K146T)_knob-Fc_(G4S)2_19-01-H04-sc02 | QVQLQESGPGLVKPSETLSLTCKASGFSFSSGYDMCWVRQPPGKGLEWIACVVAGSVDITYYASW AKGRVTISKDSSKNQVSLKLSSVTAADTAVYYCARKDAYSDAFNLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALPAPIEKTISKAKGQPREPQVCTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGGSGGG GSDIQMTQSPSSLSASVGDRVTITCQSSESVYSNNQLSWYQQKPGQPPKLLIYDASDLASGVPSRF SGSGSGTDFTLTISSLQPEDFATYYCAGGFSSSSDTAFGCGTKLTVLGGGGGSGGGGSGGGGSG GGGSEVQLVESGGGLVQPGGSLRLSCAASGFSLSSNAMGWVRQAPGKCLEYIGIISVGGFTYYAS WAKGRFTISRDNSKNTVYLQMNSLRAEDSATYFCARDRHGGDSSGAFYLWGQGTLVKVSS |
| 206 | 33-03-G02-sc24 (kappacap)_Ck | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |

**Table 5: LILRB2 antibodies used in the examples of the present invention (modifications relative to original sequences, i.e. reference sequences, if present, are shown in bold and underlined; CDR residues are shown in bold and italic letters).**

| **SEQ ID NO:** | **Description** | **Sequence** |
|---|---|---|
| ***PRO3603*** | | |
| 207 | 68-18-E11-sc02_IgG1_VH (2x) | QQLVESGGGRVQPGGSLRLSCAASGFSFTNNYYMCWVRQAPGKGLEWIACMLVGAWGNTYYAS WANGRFTISKDNSKNTVYLQMNSLRAEDTATYFCARARDYTWTLGGDPETSGFNLWGQGTQVTV SSASTKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQYNSTYRVVSVLTVLHQDWL NGKEYKCKVSNKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHYTQKSLSLSPG K |
| 208 | 68-18-E11-sc02_IgG1_VL (2x) | DVQMTQSPSSLSASVGDRVTITCQASQSIGSNLAWYQQKPGKPPKLLIYKASTLESGVPSRFSGSG SGTDFTLTISSLQPEDFATYYCQSTAGSSRSGNYGYVFGQGTKVEIKRTVAAPSVFIFPPSDEQLKS GTASVVCLLNNFYPREAKVQWKVDNALQSGNSQESVTEQDSKDSTYSLSSTLTLSKADYEKHKVYA CEVTHQGLSSPVTKSFNRGEC |

| ***PRO3635*** | | |
|---|---|---|
| 209 | 68-18-E11-sc02_IgG4_VH (2x) | QQLVESGGGRVQPGGSLRLSCAASGFSFTNNYYMCWVRQAPGKGLEWIACMLVGAWGNTYYAS WANGRFTISKDNSKNTVYLQMNSLRAEDTATYFCARARDYTWTLGGDPETSGFNLWGQGTQVTV SSASTKGPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPPCPAPEFLGGPSVFLFPPKPKDTL MISRTPEVTCVVVDVSQEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSLTCLVKGFYPSDIAVEWES NGQPENNYKTTPPVLDSDGSFFLYSRLTVDKSRWQEGNVFSCSVMHEALHNHYTQKSLSLSLGK |
| 210 | 68-18-E11-sc02_IgG4_VL (2x) | DVQMTQSPSSLSASVGDRVTITCQASQSIGSNLAWYQQKPGKPPKLLIYKASTLESGVPSRFSGSG SGTDFTLTISSLQPEDFATYYCQSTAGSSRSGNYGYVFGQGTKVEIKRTVAAPSVFIFPPSDEQLKS GTASVVCLLNNFYPREAKVQWKVDNALQSGNSQESVTEQDSKDSTYSLSSTLTLSKADYEKHKVYA CEVTHQGLSSPVTKSFNRGEC |

| ***PRO4710*** | | |
|---|---|---|
| 211 | 68-18-E11-sc07_IgG4_VH (2x) | QQLVESGGGLVQPGGSLRLSCAASGFSFTNNYYMCWVRQAPGKGLEWIACMLVGAWGNTYYAS WANGRFTISKDNSKNTVYLQMNSLRAEDTAVYFCARARDYTWTLGGDPETSGFNLWGQGTLVTVS SASTKGPSVFPLAPCSRSTSESTAALGCLVKDYFPEPVTVSWNSGALTSGVHTFPAVLQSSGLYSLS SVVTVPSSSLGTKTYTCNVDHKPSNTKVDKRVESKYGPPCPPCPAPEFLGGPSVFLFPPKPKDTLMI SRTPEVTCVVVDVSQEDPEVQFNWYVDGVEVHNAKTKPREEQFNSTYRVVSVLTVLHQDWLNGKE |
| | | YKCKVSNKGLPSSIEKTISKAKGQPREPQVYTLPPSQEEMTKNQVSLTCLVKGFYPSDIAVEWESNG QPENNYKTTPPVLDSDGSFFLYSRLTVDKSRWQEGNVFSCSVMHEALHNHYTQKSLSLSLGK |
| 212 | 68-18-E11-sc07_IgG4_VL (2x) | DVQMTQSPSSLSASVGDRVTITCQASQSIGSNLAWYQQKPGKPPKLLIYKGGTLESGVPSRFSGSG SGTDFTLTISSLQPEDFATYYCQSTAGSSRSGNYGYVFGQGTKVEIKRTVAAPSVFIFPPSDEQLKS GTASVVCLLNNFYPREAKVQWKVDNALQSGNSQESVTEQDSKDSTYSLSSTLTLSKADYEKHKVYA CEVTHQGLSSPVTKSFNRGEC |

**Table 6: Reference antibodies used in the examples of the present invention (modifications relative to original sequences, i.e. reference sequences, if present, are shown in bold and underlined; CDR residues are shown in bold and italic letters).**

| **SEQ ID NO:** | **Description** | **Sequence** |
|---|---|---|
| **PRO4682 (Format with CD47 in the Fab arm and PRO4245 as PDL1-BD)** | | |
| 213 | 37-20-B03-sc18_knob-Fc_(G4S)2_68-18-E11-sc07 | QQVVESGGGLVQPGGSLRLSCAVSGIDLNSYAMTWVRQAPGKGLEWIGYIGSASSTYYASWASG RFTISKDNSKNTVYLQMNSLRAEDTAVYFCARGSTYINIWGQGTLVTVSSASTKGPSVFPLAPSSKS TSGGTAALGCLVKDYFPEPVTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKALPAPIEK TISKAKGQPREPQVYTLPPCREEMTKNQVSLSCAVKGFYPSDIAVEWESNGQPENNYKTTPPVLDS DGSFFLVSKLTVDKSRWQQGNVFSCSVMHEALHNHYTQKSLSLSPGKGGGGGSGGGGSDVQMT QSPSSLSASVGDRVTITCQASQSIGSNLAWYQQKPGKPPKLLIYKGGTLESGVPSRFSGSGSGTDF TLTISSLQPEDFATYYCQSTAGSSRSGNYGYVFGCGTKVTVLGGGGGSGGGGSGGGGSGGGGSQ QLVESGGGLVQPGGSLRLSCAASGFSFTNNYYMCWVRQAPGKCLEWIACMLVGAWGNTYYASW ANGRFTISKDNSKNTVYLQMNSLRAEDSAVYFCARARDYTWTLGGDPETSGFNLWGQGTLVKVSS |
| 214 | 70-26-B04-sc03 Hole-Fc_(G4S)2_68-18-E11-sc07 | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGTGTKVTVLGGGGGSGGGGSGGGGSGGG GSQQLVESGGGLVQPGGSLRLSCAASGFSFNSDYWIYWVRQAPGKGLEWIASIYGGSSGNTQYAS WAQGRFTISKDNSKNTVYLQMNSLRAEDSAVYFCARGYVDYGGATDLWGQGTLVKVSSEPKSSD KTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVCTLPPS REEMTKNQVSLWCLVKGFYPSDIAVEWESNGQPENNYKTTPPVLDSDGSFFLYSKLTVDKSRWQQ GNVFSCSVMHEALHNHYTQKSLSLSPGKGGGGSGGGGSDVQMTQSPSSLSASVGDRVTITCQAS QSIGSNLAWYQQKPGKPPKLLIYKGGTLESGVPSRFSGSGSGTDFTLTISSLQPEDFATYYCQSTAG SSRSGNYGYVFGCGTKVTVLGGGGGSGGGGSGGGGSGGGGSQQLVESGGGLVQPGGSLRLSC |
| | | AASGFSFTNNYYMCWVRQAPGKCLEWIACMLVGAWGNTYYASWANGRFTISKDNSKNTVYLQMN SLRAEDSAVYFCARARDYTWTLGGDPETSGFNLWGQGTLVKVSS |
| 215 | 70-26-B04-sc03_Ck | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |

| **PRO4683 (PRO4682 with dummy domain for LILRB2-BD)** | | |
|---|---|---|
| 216 | 37-20-B03-sc18_knob-Fc_(G4S)2_19-01-H04-sc02 | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGTGTKVTVLGGGGGSGGGGSGGGGSGGG GSQQLVESGGGLVQPGGSLRLSCAASGFSFNSDYWIYWVRQAPGKGLEWIASIYGGSSGNTQYAS WAQGRFTISKDNSKNTVYLQMNSLRAEDSAVYFCARGYVDYGGATDLWGQGTLVKVSSEPKSSD KTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVCTLPPS REEMTKNQVSLWCLVKGFYPSDIAVEWESNGQPENNYKTTPPVLDSDGSFFLYSKLTVDKSRWQQ GNVFSCSVMHEALHNHYTQKSLSLSPGKGGGGSGGGGSDIQMTQSPSSLSASVGDRVTITCQSSE SVYSNNQLSWYQQKPGQPPKLLIYDASDLASGVPSRFSGSGSGTDFTLTISSLQPEDFATYYCAGG FSSSSDTAFGCGTKLTVLGGGGGSGGGGSGGGGSGGGGSEVQLVESGGGLVQPGGSLRLSCAA SGFSLSSNAMGWVRQAPGKCLEYIGIISVGGFTYYASWAKGRFTISRDNSKNTVYLQMNSLRAEDS ATYFCARDRHGGDSSGAFYLWGQGTLVKVSS |
| 217 | 70-26-B04-sc03 Hole-Fc_(G4S)2_19-01-H04-sc02_HC | QQVVESGGGLVQPGGSLRLSCAVSGIDLNSYAMTWVRQAPGKGLEWIGYIGSASSTYYASWASG RFTISKDNSKNTVYLQMNSLRAEDTAVYFCARGSTYINIWGQGTLVTVSSASTKGPSVFPLAPSSKS TSGGTAALGCLVKDYFPEPVTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKALPAPIEK TISKAKGQPREPQVYTLPPCREEMTKNQVSLSCAVKGFYPSDIAVEWESNGQPENNYKTTPPVLDS DGSFFLVSKLTVDKSRWQQGNVFSCSVMHEALHNHYTQKSLSLSPGKGGGGGSGGGGSDIQMTQ SPSSLSASVGDRVTITCQSSESVYSNNQLSWYQQKPGQPPKLLIYDASDLASGVPSRFSGSGSGTD FTLTISSLQPEDFATYYCAGGFSSSSDTAFGCGTKLTVLGGGGGSGGGGSGGGGSGGGGSEVQLV ESGGGLVQPGGSLRLSCAASGFSLSSNAMGWVRQAPGKCLEYIGIISVGGFTYYASWAKGRFTISR DNSKNTVYLQMNSLRAEDSATYFCARDRHGGDSSGAFYLWGQGTLVKVSS |
| 218 | 70-26-B04-sc03_Ck | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |

| **PRO4677 (Morrison-H format with CD47-BD and PDL1-BD (PRO4245) as C-terminal scFvs and Fab LILRB2-BD's)** | | |
|---|---|---|
| 219 | 68-18-E11-sc07 Hole-Fc_(G4S)2_70-26-B04-sc03 | QQLVESGGGLVQPGGSLRLSCAASGFSFTNNYYMCWVRQAPGKGLEWIACMLVGAWGNTYYAS WANGRFTISKDNSKNTVYLQMNSLRAEDTAVYFCARARDYTWTLGGDPETSGFNLWGQGTLVTVS SASTKGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVYTLPPCREEMTKNQVSLSCAVKGFYPSDIAVEWE SNGQPENNYKTTPPVLDSDGSFFLVSKLTVDKSRWQQGNVFSCSVMHEALHNHYTQKSLSLSPGK GGGGSGGGGSDVQMTQSPSSLSASVGDRVTITCQASQSISSWVSWYQQKPGKPPKLLIYRGGY LESGVPSRFSASGSGTDFTLTISSLQPEDFATYYCQSTDSSTYGGAFGCGTKVTVLGGGGGSGGG GSGGGGSGGGGSQQVVESGGGLVQPGGSLRLSCAVSGIDLNSYAMTWVRQAPGKCLEWIGYIGS ASSTYYASWASGRFTISKDNSKNTVYLQMNSLRAEDSAVYFCARGSTYINIWGQGTLVKVSS |
| 220 | 68-18-E11-sc07 knob-Fc_(G4S)2_37-20-B03-sc18 | QQLVESGGGLVQPGGSLRLSCAASGFSFTNNYYMCWVRQAPGKGLEWIACMLVGAWGNTYYAS WANGRFTISKDNSKNTVYLQMNSLRAEDTAVYFCARARDYTWTLGGDPETSGFNLWGQGTLVTVS SASTKGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTYRVVSVLTVLHQDWLN GKEYKCKVSNKALPAPIEKTISKAKGQPREPQVCTLPPSREEMTKNQVSLWCLVKGFYPSDIAVEWE SNGQPENNYKTTPPVLDSDGSFFLYSKLTVDKSRWQQGNVFSCSVMHEALHNHYTQKSLSLSPGK GGGGGSGGGGSDIQMTQSPSSLSASVGDRVTITCQASQSIGTYLAWYQQKPGKPPKLLIYRGGILA SGVPSRFSGSGSGTDFTLTISSLQPEDFATYYCQSNFYSDSTTIGPNAFGTGTKVTVLGGGGGSGG GGSGGGGSGGGGSQQLVESGGGLVQPGGSLRLSCAASGFSFNSDYWIYWVRQAPGKGLEWIASI YGGSSGNTQYASWAQGRFTISKDNSKNTVYLQMNSLRAEDSAVYFCARGYVDYGGATDLWGQGT LVKVSS |
| 221 | 68-18-E11-sc07_Ck (2x) | DVQMTQSPSSLSASVGDRVTITCQASQSIGSNLAWYQQKPGKPPKLLIYKGGTLESGVPSRFSGSG SGTDFTLTISSLQPEDFATYYCQSTAGSSRSGNYGYVFGQGTKVEIKRTVAAPSVFIFPPSDEQLKS GTASVVCLLNNFYPREAKVQWKVDNALQSGNSQESVTEQDSKDSTYSLSSTLTLSKADYEKHKVYA CEVTHQGLSSPVTKSFNRGEC |

| **PRO4678 (PRO4677 with dummu domain for LILRB2-BD)** | | |
|---|---|---|
| 222 | 19-01-H04-sc02 knob-Fc_(G4S)2_37-20-B03-sc18 | EVQLVESGGGLVQPGGSLRLSCAASGFSLSSNAMGWVRQAPGKGLEYIGIISVGGFTYYASWAKG RFTISRDNSKNTVYLQMNSLRAEDTATYFCARDRHGGDSSGAFYLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK |
| | | ALPAPIEKTISKAKGQPREPQVCTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGGSGGG GSDIQMTQSPSSLSASVGDRVTITCQASQSIGTYLAWYQQKPGKPPKLLIYRGGILASGVPSRFSGS GSGTDFTLTISSLQPEDFATYYCQSNFYSDSTTIGPNAFGTGTKVTVLGGGGGSGGGGSGGGGSG GGGSQQLVESGGGLVQPGGSLRLSCAASGFSFNSDYWIYWVRQAPGKGLEWIASIYGGSSGNTQY ASWAQGRFTISKDNSKNTVYLQMNSLRAEDSAVYFCARGYVDYGGATDLWGQGTLVKVSS |
| 223 | 19-01-H04-sc02 Hole-Fc_(G4S)2_70-26-B04-sc03 | EVQLVESGGGLVQPGGSLRLSCAASGFSLSSNAMGWVRQAPGKGLEYIGIISVGGFTYYASWAKG RFTISRDNSKNTVYLQMNSLRAEDTATYFCARDRHGGDSSGAFYLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALPAPIEKTISKAKGQPREPQVYTLPPCREEMTKNQVSLSCAVKGFYPSDIAVEWESNGQPENNYKT TPPVLDSDGSFFLVSKLTVDKSRWQQGNVFSCSVMHEALHNHYTQKSLSLSPGKGGGGGSGGGG SDVQMTQSPSSLSASVGDRVTITCQASQSISSWVSWYQQKPGKPPKLLIYRGGYLESGVPSRFSAS GSGTDFTLTISSLQPEDFATYYCQSTDSSTYGGAFGCGTKVTVLGGGGGSGGGGSGGGGSGGGG SQQVVESGGGLVQPGGSLRLSCAVSGIDLNSYAMTWVRQAPGKCLEWIGYIGSASSTYYASWASG RFTISKDNSKNTVYLQMNSLRAEDSAVYFCARGSTYINIWGQGTLVKVSS |
| 224 | 19-01-H04-sc02_Ck | DIQMTQSPSSLSASVGDRVTITCQSSESVYSNNQLSWYQQKPGQPPKLLIYDASDLASGVPSRFSG SGSGTDFTLTISSLQPEDFATYYCAGGFSSSSDTAFGQGTKVEIKRTVAAPSVFIFPPSDEQLKSGTA SVVCLLNNFYPREAKVQWKVDNALQSGNSQESVTEQDSKDSTYSLSSTLTLSKADYEKHKVYACEV THQGLSSPVTKSFNRGEC |

| **PRO4711 (CD47 from** WO 2021124073 **in Fab arm and PRO2230 as PDL1-BD)** | | |
|---|---|---|
| 225 | 37-20-B03-sc09.1-knob-Fc-(G4S)2-68-18-E11-sc02 DIS | DIQMTQSPASLSASVGDRVTITCQASQSIGTYLAWYQQKPGKPPKLLIYRAFILASGVPSRFSGSGS GTDFTLTISSLQPEDFATYYCQSNFYSDSTTIGPNAFGTGTKVTVLGGGGGSGGGGSGGGGSGGG GSEVQLVESGGGLVQPGGSLRLSCAASGFSFNSDYWIYWVRQAPGKGLEWIASIYGGSSGNTQYA SWAQGRFTISRDNSKNTVYLQMNSLRAEDTAVYFCARGYVDYGGATDLWGQGTLVTVSSEPKSSD KTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVCTLPPS REEMTKNQVSLWCLVKGFYPSDIAVEWESNGQPENNYKTTPPVLDSDGSFFLYSKLTVDKSRWQQ GNVFSCSVMHEALHNHYTQKSLSLSPGKGGGGSGGGGSDVQMTQSPSSLSASVGDRVTITCQAS QSIGSNLAWYQQKPGKPPKLLIYKASTLESGVPSRFSGSGSGTDFTLTISSLQPEDFATYYCQSTAG SSRSGNYGYVFGCGTKVTVLGGGGGSGGGGSGGGGSGGGGSQQLVESGGGRVQPGGSLRLSC AASGFSFTNNYYMCWVRQAPGKCLEWIACMLVGAWGNTYYASWANGRFTISKDNSKNTVYLQMN SLRAEDTATYFCARARDYTWTLGGDPETSGFNLWGQGTQVTVSS |
| 226 | WO 2021124073_61_hole -Fc_(G4S)2_68-18-E11-sc02 DIS | QVQLVQSGAEVKKPGSSVKVSCKASGYTFSSYAISWVRQAPGQGLEWMGGISPIFGTANYAQKFQ GRVTITADESTSTAYMELSSLRSEDTAVYYCARDAGRSSDVGWYVGAIDVWGQGTLVTVSSASTKG PSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGGPSVFLFPPKPKDTLMISRT PEVTCVVVDVSHEDPEVKFNWYVDGVEVHNAKTKPREEQYNSTYRVVSVLTVLHQDWLNGKEYKC KVSNKALPAPIEKTISKAKGQPREPQVYTLPPCREEMTKNQVSLSCAVKGFYPSDIAVEWESNGQPE NNYKTTPPVLDSDGSFFLVSKLTVDKSRWQQGNVFSCSVMHEALHNHYTQKSLSLSPGKGGGGSG GGGSDVQMTQSPSSLSASVGDRVTITCQASQSIGSNLAWYQQKPGKPPKLLIYKASTLESGVPSRF SGSGSGTDFTLTISSLQPEDFATYYCQSTAGSSRSGNYGYVFGCGTKVTVLGGGGGSGGGGSGG GGSGGGGSQQLVESGGGRVQPGGSLRLSCAASGFSFTNNYYMCWVRQAPGKCLEWIACMLVGA WGNTYYASWANGRFTISKDNSKNTVYLQMNSLRAEDTATYFCARARDYTWTLGGDPETSGFNLW GQGTQVTVSS |
| 227 | WO 2021124073_62 | QSVLTQPPSASGTPGQRVTISCSGSSSNIGSNYVYWYQQLPGTAPKLLIYRNNQRPSGVPDRFSGS KSGTSASLAISGLRSEDEADYYCAAWDDSLSGVVFGGGTKLTVLGQPKAAPSVTLFPPSSEELQAN KATLVCLISDFYPGAVTVAWKADSSPVKAGVETTTPSKQSNNKYAASSYLSLTPEQWKSHRSYSCQ VTHEGSTVEKTVAPTECS |

| **PRO3843 (CD47 and PDL1 from** WO 2021124073 **in Fab arms)** | | |
|---|---|---|
| 228 | WO 2021124073_61_(G4 S)2_68-18-E11-sc02 | QVQLVQSGAEVKKPGSSVKVSCKASGYTFSSYAISWVRQAPGQGLEWMGGISPIFGTANYAQKFQ GRVTITADESTSTAYMELSSLRSEDTAVYYCARDAGRSSDVGWYVGAIDVWGQGTLVTVSSASTKG PSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGGPSVFLFPPKPKDTLMISRT PEVTCVVVDVSHEDPEVKFNWYVDGVEVHNAKTKPREEQYNSTYRVVSVLTVLHQDWLNGKEYKC KVSNKALPAPIEKTISKAKGQPREPQVYTLPPCREEMTKNQVSLSCAVKGFYPSDIAVEWESNGQPE NNYKTTPPVLDSDGSFFLVSKLTVDKSRWQQGNVFSCSVMHEALHNHYTQKSLSLSPGKGGGGSG GGGSDVQMTQSPSSLSASVGDRVTITCQASQSIGSNLAWYQQKPGKPPKLLIYKASTLESGVPSRF SGSGSGTDFTLTISSLQPEDFATYYCQSTAGSSRSGNYGYVFGCGTKVTVLGGGGGSGGGGSGG GGSGGGGSQQLVESGGGRVQPGGSLRLSCAASGFSFTNNYYMCWVRQAPGKCLEWIACMLVGA WGNTYYASWANGRFTISKDNSKNTVYLQMNSLRAEDTATYFCARARDYTWTLGGDPETSGFNLW GQGTQVTVSS |
| 229 | WO 2021124073_64_(G4 S)2_68-18-E11-sc02 DIS | EVQLVESGGGLVKPGGSLRLSCAASGFTFSNAWMNWVRQAPGKGLEWVGRIKTKADGGTTDYAA PVKGRFTISRDDSKNTLYLQMNSLKTEDTAVYYCTTDPGEYWDSVYGGMDYWGQGTLVTVSSAST KGPSVFPLAPSSKSTSGGTAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPELLGGPSVFLFPPKPKDTLMI SRTPEVTCVVVDVSHEDPEVKFNWYVDGVEVHNAKTKPREEQYNSTYRVVSVLTVLHQDWLNGKE |
| | | YKCKVSNKALPAPIEKTISKAKGQPREPQVCTLPPSREEMTKNQVSLWCLVKGFYPSDIAVEWESNG QPENNYKTTPPVLDSDGSFFLYSKLTVDKSRWQQGNVFSCSVMHEALHNHYTQKSLSLSPGKGGG GSGGGGSDVQMTQSPSSLSASVGDRVTITCQASQSIGSNLAWYQQKPGKPPKLLIYKASTLESGV PSRFSGSGSGTDFTLTISSLQPEDFATYYCQSTAGSSRSGNYGYVFGCGTKVTVLGGGGGSGGGG SGGGGSGGGGSQQLVESGGGRVQPGGSLRLSCAASGFSFTNNYYMCWVRQAPGKCLEWIACML VGAWGNTYYASWANGRFTISKDNSKNTVYLQMNSLRAEDTATYFCARARDYTWTLGGDPETSGF NLWGQGTQVTVSS |
| 230 | WO 2021124073_62 | QSVLTQPPSASGTPGQRVTISCSGSSSNIGSNYVYWYQQLPGTAPKLLIYRNNQRPSGVPDRFSGS KSGTSASLAISGLRSEDEADYYCAAWDDSLSGVVFGGGTKLTVLGQPKAAPSVTLFPPSSEELQAN KATLVCLISDFYPGAVTVAWKADSSPVKAGVETTTPSKQSNNKYAASSYLSLTPEQWKSHRSYSCQ VTHEGSTVEKTVAPTECS |

| **PRO4914 (CrossmAb format with Fab CD47-BD and Fab PDL1-BD)** | | |
|---|---|---|
| 231 | 70-26-B04-sc03 crossed_hole-Fc_-(G4S)2-68-18-E11-sc07 | QQVVESGGGLVQPGGSLRLSCAVSGIDLNSYAMTWVRQAPGKGLEWIGYIGSASSTYYASWASG RFTISKDNSKNTVYLQMNSLRAEDTAVYFCARGSTYINIWGQGTLVTVSSASVAAPSVFIFPPSDEQL KSGTASVVCLLNNFYPREAKVQWKVDNALQSGNSQESVTEQDSKDSTYSLSSTLTLSKADYEKHKV YACEVTHQGLSSPVTKSFNRGECDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGKEYKCKVSNKALP APIEKTISKAKGQPREPQVYTLPPCREEMTKNQVSLSCAVKGFYPSDIAVEWESNGQPENNYKTTPP VLDSDGSFFLVSKLTVDKSRWQQGNVFSCSVMHEALHNHYTQKSLSLSPGKGGGGSGGGGSDVQ MTQSPSSLSASVGDRVTITCQASQSIGSNLAWYQQKPGKPPKLLIYKGGTLESGVPSRFSGSGSGT DFTLTISSLQPEDFATYYCQSTAGSSRSGNYGYVFGCGTKVTVLGGGGGSGGGGSGGGGSGGGG SQQLVESGGGLVQPGGSLRLSCAASGFSFTNNYYMCWVRQAPGKCLEWIACMLVGAWGNTYYAS WANGRFTISKDNSKNTVYLQMNSLRAEDSAVYFCARARDYTWTLGGDPETSGFNLWGQGTLVKV SS |
| 232 | 37-20-B03-sc18 knob-Fc-(G4S)2-68-18-E11-sc07 | QQLVESGGGLVQPGGSLRLSCAASGFSFNSDYWIYWVRQAPGKGLEWIASIYGGSSGNTQYASW AQGRFTISKDNSKNTVYLQMNSLRAEDTAVYFCARGYVDYGGATDLWGQGTLVTVSSASTKGPSV FPLAPSSKSTSGGTAALGCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPSVFLFPPKPKDTLMISRTPEV TCVVVDVSHEDPEVKFNWYVDGVEVHNAKTKPREEQYNSTYRVVSVLTVLHQDWLNGKEYKCKVS NKALPAPIEKTISKAKGQPREPQVCTLPPSREEMTKNQVSLWCLVKGFYPSDIAVEWESNGQPENN YKTTPPVLDSDGSFFLYSKLTVDKSRWQQGNVFSCSVMHEALHNHYTQKSLSLSPGKGGGGSGGG GSDVQMTQSPSSLSASVGDRVTITCQASQSIGSNLAWYQQKPGKPPKLLIYKGGTLESGVPSRFSG SGSGTDFTLTISSLQPEDFATYYCQSTAGSSRSGNYGYVFGCGTKVTVLGGGGGSGGGGSGGGG SGGGGSQQLVESGGGLVQPGGSLRLSCAASGFSFTNNYYMCWVRQAPGKCLEWIACMLVGAWG |
| | | NTYYASWANGRFTISKDNSKNTVYLQMNSLRAEDSAVYFCARARDYTWTLGGDPETSGFNLWGQ GTLVKVSS |
| 233 | 70-26-B04-sc03_ CH1_crossed | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGQGTKVEIKSSASTKGPSVFPLAPSSKSTSGGT AALGCLVKDYFPEPVTVSWNSGALTSGVHTFPAVLQSSGLYSLSSVVTVPSSSLGTQTYICNVNHKP SNTKVDKKVEPKSC |
| 234 | 37-20-B03-sc18_ck | DIQMTQSPSSLSASVGDRVTITCQASQSIGTYLAWYQQKPGKPPKLLIYRGGILASGVPSRFSGSGS GTDFTLTISSLQPEDFATYYCQSNFYSDSTTIGPNAFGQGTKVEIKRTVAAPSVFIFPPSDEQLKSGT ASVVCLLNNFYPREAKVQWKVDNALQSGNSQESVTEQDSKDSTYSLSSTLTLSKADYEKHKVYACE VTHQGLSSPVTKSFNRGEC |

| **PRO5054 (PRO5052 with dummy domain for CD47-BD)** | | |
|---|---|---|
| 235 | 19-01-H04-sc02_hole-Fc_(G4S)2_68-18-E11-sc07 | DIQMTQSPSSLSASVGDRVTITCQSSESVYSNNQLSWYQQKPGQPPKLLIYDASDLASGVPSRFSG SGSGTDFTLTISSLQPEDFATYYCAGGFSSSDTAFGCGTKLTVLGGGGGSGGGGSGGGGSGGG GSEVQLVESGGGLVQPGGSLRLSCAASGFSLSSNAMGWVRQAPGKCLEYIGIISVGGFTYYASWA KGRFTISRDNSKNTVYLQMNSLRAEDSATYFCARDRHGGDSSGAFYLWGQGTLVKVSSEPKSSDK THTCPPCPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQVYTLPPCR EEMTKNQVSLSCAVKGFYPSDIAVEWESNGQPENNYKTTPPVLDSDGSFFLVSKLTVDKSRWQQG NVFSCSVMHEALHNHYTQKSLSLSPGKGGGGSGGGGSDVQMTQSPSSLSASVGDRVTITCQASQ SIGSNLAWYQQKPGKPPKLLIYKGGTLESGVPSRFSGSGSGTDFTLTISSLQPEDFATYYCQSTAGS SRSGNYGYVFGCGTKVTVLGGGGGSGGGGSGGGGSGGGGSQQLVESGGGLVQPGGSLRLSCA ASGFSFTNNYYMCWVRQAPGKCLEWIACMLVGAWGNTYYASWANGRFTISKDNSKNTVYLQMNS LRAEDSAVYFCARARDYTWTLGGDPETSGFNLWGQGTLVKVSS |
| 236 | 33-03-G02-sc24_knob-Fc_(G4S)2_68-18-E11-sc07 | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGTGTKVTVLGGGGGSGGGGSGGGGSGGGGSQ VQLQESGPGLVKPSETLSLTCKASGFSFSSGYDMCWVRQPPGKGLEWIACVVAGSVDITYYASWA KGRVTISKDSSKNQVSLKLSSVTAADSAVYYCARKDAYSDAFNLWGQGTLVKVSSEPKSSDKTHTC PPCPAPELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVCTLPPSREEMT KNQVSLWCLVKGFYPSDIAVEWESNGQPENNYKTTPPVLDSDGSFFLYSKLTVDKSRWQQGNVFS CSVMHEALHNHYTQKSLSLSPGKGGGGSGGGGSDVQMTQSPSSLSASVGDRVTITCQASQSIGSN LAWYQQKPGKPPKLLIYKGGTLESGVPSRFSGSGSGTDFTLTISSLQPEDFATYYCQSTAGSSRSG NYGYVFGCGTKVTVLGGGGGSGGGGSGGGGSGGGGSQQLVESGGGLVQPGGSLRLSCAASGFS |
| | | FTNNYYMCWVRQAPGKCLEWIACMLVGAWGNTYYASWANGRFTISKDNSKNTVYLQMNSLRAED SAVYFCARARDYTWTLGGDPETSGFNLWGQGTLVKVSS |

| **PRO5057 (PRO5055 with dummy domain for CD47-BD)** | | |
|---|---|---|
| 237 | 19-01-H04-sc02_hole-Fc_(G4S)2_68-18-E11-sc07 | DIQMTQSPSSLSASVGDRVTITCQSSESVYSNNQLSWYQQKPGQPPKLLIYDASDLASGVPSRFSG SGSGTDFTLTISSLQPEDFATYYCAGGFSSSDTAFGCGTKLTVLGGGGGSGGGGSGGGGSGGG GSEVQLVESGGGLVQPGGSLRLSCAASGFSLSSNAMGWVRQAPGKCLEYIGIISVGGFTYYASWA KGRFTISRDNSKNTVYLQMNSLRAEDSATYFCARDRHGGDSSGAFYLWGQGTLVKVSSEPKSSDK THTCPPCPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQVYTLPPCR EEMTKNQVSLSCAVKGFYPSDIAVEWESNGQPENNYKTTPPVLDSDGSFFLVSKLTVDKSRWQQG NVFSCSVMHEALHNHYTQKSLSLSPGKGGGGSGGGGSDVQMTQSPSSLSASVGDRVTITCQASQ SIGSNLAWYQQKPGKPPKLLIYKGGTLESGVPSRFSGSGSGTDFTLTISSLQPEDFATYYCQSTAGS SRSGNYGYVFGCGTKVTVLGGGGGSGGGGSGGGGSGGGGSQQLVESGGGLVQPGGSLRLSCA ASGFSFTNNYYMCWVRQAPGKCLEWIACMLVGAWGNTYYASWANGRFTISKDNSKNTVYLQMNS LRAEDSAVYFCARARDYTWTLGGDPETSGFNLWGQGTLVKVSS |
| 238 | 33-03-G02-sc24 Knob-Fc_(G4S)2_68-18-E11-sc07 | QVQLQESGPGLVKPSETLSLTCKASGFSFSSGYDMCWVRQPPGKGLEWIACVVAGSVDITYYASW AKGRVTISKDSSKNQVSLKLSSVTAADTAVYYCARKDAYSDAFNLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALPAPIEKTISKAKGQPREPQVCTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGGSGGG GSDVQMTQSPSSLSASVGDRVTITCQASQSIGSNLAWYQQKPGKPPKLLIYKGGTLESGVPSRFSG SGSGTDFTLTISSLQPEDFATYYCQSTAGSSRSGNYGYVFGCGTKVTVLGGGGGSGGGGSGGGG SGGGGSQQLVESGGGLVQPGGSLRLSCAASGFSFTNNYYMCWVRQAPGKCLEWIACMLVGAWG NTYYASWANGRFTISKDNSKNTVYLQMNSLRAEDSAVYFCARARDYTWTLGGDPETSGFNLWGQ GTLVKVSS |
| 239 | 33-03-G02-sc24_Ck | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |

| **PRO5423 (PRO5052 with silenced Fc)** | | |
|---|---|---|
| 240 | 70-26-B04-sc03_hole-Fc_(G4S)2 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS |
| | 68-18-E11-sc07 | QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE AAGGPSVFLFPPKPKDTLMISRTPEVTCVVVDVSHEDPEVKFNWYVDGVEVHNAKTKPREEQYNST YRVVSVLTVLHQDWLNGKEYKCKVSNKALGAPIEKTISKAKGQPREPQVYTLPPCREEMTKNQVSL SCAVKGFYPSDIAVEWESNGQPENNYKTTPPVLDSDGSFFLVSKLTVDKSRWQQGNVFSCSVMHE ALHNHYTQKSLSLSPGKGGGGSGGGGSDVQMTQSPSSLSASVGDRVTITCQASQSIGSNLAWYQ QKPGKPPKLLIYKGGTLESGVPSRFSGSGSGTDFTLTISSLQPEDFATYYCQSTAGSSRSGNYGYVF GCGTKVTVLGGGGGSGGGGSGGGGSGGGGSQQLVESGGGLVQPGGSLRLSCAASGFSFTNNYY MCWVRQAPGKCLEWIACMLVGAWGNTYYASWANGRFTISKDNSKNTVYLQMNSLRAEDSAVYFC ARARDYTWTLGGDPETSGFNLWGQGTLVKVSS |
| 241 | 33-03-G02 sc24 knob-Fc_LALA-PG_(G4S)2 68-18-E11-sc07 | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGTGTKVTVLGGGGGSGGGGSGGGGSGGGGSQ VQLQESGPGLVKPSETLSLTCKASGFSFSSGYDMCWVRQPPGKGLEWIACVVAGSVDITYYASWA KGRVTISKDSSKNQVSLKLSSVTAADSAVYYCARKDAYSDAFNLWGQGTLVKVSSEPKSSDKTHTC PPCPAPEAAGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALGAPIEKTISKAKGQPREPQVCTLPPSREEM TKNQVSLWCLVKGFYPSDIAVEWESNGQPENNYKTTPPVLDSDGSFFLYSKLTVDKSRWQQGNVF SCSVMHEALHNHYTQKSLSLSPGKGGGGSGGGGSDVQMTQSPSSLSASVGDRVTITCQASQSIGS NLAWYQQKPGKPPKLLIYKGGTLESGVPSRFSGSGSGTDFTLTISSLQPEDFATYYCQSTAGSSRS GNYGYVFGCGTKVTVLGGGGGSGGGGSGGGGSGGGGSQQLVESGGGLVQPGGSLRLSCAASG FSFTNNYYMCWVRQAPGKCLEWIACMLVGAWGNTYYASWANGRFTISKDNSKNTVYLQMNSLRA EDSAVYFCARARDYTWTLGGDPETSGFNLWGQGTLVKVSS |

| **PRO5134 (PRO5055 with silenced Fc)** | | |
|---|---|---|
| 242 | 33-03-G02 sc24 Knob-Fc_LALA-PG_(G4S)2 68-18-E11-sc07 | QVQLQESGPGLVKPSETLSLTCKASGFSFSSGYDMCWVRQPPGKGLEWIACVVAGSVDITYYASW AKGRVTISKDSSKNQVSLKLSSVTAADTAVYYCARKDAYSDAFNLWGQGTLVTVSSASTKGPSVFP LAPSSKSTSGGTAALGCLVKDYFPEPVTVSWNSGALTSGVHTFPAVLQSSGLYSLSSVVTVPSSSLG TQTYICNVNHKPSNTKVDKKVEPKSCDKTHTCPPCPAPEAAGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALGAPIEKTISKAKGQPREPQVCTLPPSREEMTKNQVSLWCLVKGFYPSDIAVEWESNGQPENNYK TTPPVLDSDGSFFLYSKLTVDKSRWQQGNVFSCSVMHEALHNHYTQKSLSLSPGKGGGGGSGGG GSDVQMTQSPSSLSASVGDRVTITCQASQSIGSNLAWYQQKPGKPPKLLIYKGGTLESGVPSRFSG SGSGTDFTLTISSLQPEDFATYYCQSTAGSSRSGNYGYVFGCGTKVTVLGGGGGSGGGGSGGGG SGGGGSQQLVESGGGLVQPGGSLRLSCAASGFSFTNNYYMCWVRQAPGKCLEWIACMLVGAWG |
| | | NTYYASWANGRFTISKDNSKNTVYLQMNSLRAEDSAVYFCARARDYTWTLGGDPETSGFNLWGQ GTLVKVSS |
| 243 | 70-26-B04-sc03 | DVQMTQSPSSLSASVGDRVTITCQASQSISSWVSWYQQKPGKPPKLLIYRGGYLESGVPSRFSASG SGTDFTLTISSLQPEDFATYYCQSTDSSTYGGAFGCGTKVTVLGGGGGSGGGGSGGGGSGGGGS QQVVESGGGLVQPGGSLRLSCAVSGIDLNSYAMTWVRQAPGKCLEWIGYIGSASSTYYASWASGR FTISKDNSKNTVYLQMNSLRAEDSAVYFCARGSTYINIWGQGTLVKVSSEPKSSDKTHTCPPCPAPE AAGGPSVFLFPPKPKDTLMISRTPEVTCVVVDVSHEDPEVKFNWYVDGVEVHNAKTKPREEQYNST YRVVSVLTVLHQDWLNGKEYKCKVSNKALGAPIEKTISKAKGQPREPQVYTLPPCREEMTKNQVSL SCAVKGFYPSDIAVEWESNGQPENNYKTTPPVLDSDGSFFLVSKLTVDKSRWQQGNVFSCSVMHE ALHNHYTQKSLSLSPGKGGGGSGGGGSDVQMTQSPSSLSASVGDRVTITCQASQSIGSNLAWYQ QKPGKPPKLLIYKGGTLESGVPSRFSGSGSGTDFTLTISSLQPEDFATYYCQSTAGSSRSGNYGYVF GCGTKVTVLGGGGGSGGGGSGGGGSGGGGSQQLVESGGGLVQPGGSLRLSCAASGFSFTNNYY MCWVRQAPGKCLEWIACMLVGAWGNTYYASWANGRFTISKDNSKNTVYLQMNSLRAEDSAVYFC ARARDYTWTLGGDPETSGFNLWGQGTLVKVSS |
| | hole-Fc_LALA-PG_(G4S)2 68-18-E11-sc07 | |
| 244 | 33-03-G02-sc24 | DIQMTQSPSSLSASVGDRVTITCQASQSINDYLAWYQQKPGKAPKLLIYKGGTLASGVPSRFSGSG SGTDFTLTISSLQPEDFATYYCQQGYIITDIDNVFGQGTKVEIKRTVAAPSVFIFPPSDEQLKSGTASV VCLLNNFYPREAKVQWKVDNALQSGNSQESVTEQDSKDSTYSLSSTLTLSKADYEKHKVYACEVTH QGLSSPVTKSFNRGEC |
| | Ck_VL | |

| **Reference anti-CD47 antibodies** | | |
|---|---|---|
| ***PRO2777 [magrolimab]* - *CD47, 5F9 clone*** | | |
| 245 | IgG4 | QVQLVQSGAEVKKPGASVKVSCKASGYTFTNYNMHWVRQAPGQRLEWMGTIYPGNDDTSYNQKF KDRVTITADTSASTAYMELSSLRSEDTAVYYCARGGYRAMDYWGQGTLVTVSSASTKGPSVFPLAP CSRSTSESTAALGCLVKDYFPEPVTVSWNSGALTSGVHTFPAVLQSSGLYSLSSVVTVPSSSLGTKT YTCNVDHKPSNTKVDKRVESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNGKEYKCKVSNKGLPSSI EKTISKAKGQPREPQVYTLPPSQEEMTKNQVSLTCLVKGFYPSDIAVEWESNGQPENNYKTTPPVL DSDGSFFLYSRLTVDKSRWQEGNVFSCSVMHEALHNHYTQKSLSLSLGK |
| | Hu5F9 (US_9382320) | |
| | Heavy chain | |
| 246 | IgG4 | DIVMTQSPLSLPVTPGEPASISCRSSQSIVYSNGNTYLGWYLQKPGQSPQLLIYKVSNRFSGVPDRF SGSGSGTDFTLKISRVEAEDVGVYYCFQGSHVPYTFGQGTKLEIKRTVAAPSVFIFPPSDEQLKSGT ASVVCLLNNFYPREAKVQWKVDNALQSGNSQESVTEQDSKDSTYSLSSTLTLSKADYEKHKVYACE VTHQGLSSPVTKSFNRGEC |
| | Hu5F9 (US_9382320) | |
| | Light chain | |

| **Reference anti-LILRB2 antibodies** | | |
|---|---|---|
| ***PRO2734 [Merck]* - *LILRB2*** | | |
| 247 | IgG4 | EVQLQQWGAGLLKPSETLSLTCAVYGGSFSGYYWSWIRQPPGKGLEWIGEINHAGSTNYNPSLKS RVTISVDTSKNQFSLKLSSVTAADTAVYYCARLPTRWVTTRYFDLWGRGTLVTVSSASTKGPSVFPL APCSRSTSESTAALGCLVKDYFPEPVTVSWNSGALTSGVHTFPAVLQSSGLYSLSSVVTVPSSSLGT KTYTCNVDHKPSNTKVDKRVESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWESNGQPENNYKTTPPV LDSDGSFFLYSRLTVDKSRWQEGNVFSCSVMHEALHNHYTQKSLSLSLGK |
| | US20180298096A1 | |
| | Heavy chain | |
| 248 | IgG4 | ESVLTQPPSVSGAPGQRVTISCTGSSSNIGAGYDVHWYQQLPGTAPKLLIYGDSNRPSGVPDRFSV SKSGASASLAITGLQAEDEADYYCQSFDNSLSAYVFGGGTQLTVLGQPKAAPSVTLFPPSSEELQAN KATLVCLISDFYPGAVTVAWKADSSPVKAGVETTTPSKQSNNKYAASSYLSLTPEQWKSHRSYSCQ VTHEGSTVEKTVAPTECS |
| | US20180298096A1 | |
| | Light chain | |

| ***PRO2518 [Jounce]* - *LILRB2*** | | |
|---|---|---|
| 249 | IgG4 | QITLKESGPTLVKPTQTLTLTCTFSGFSLNTYAMGVSWIRQPPGKALEWLASIWWNGNKYNNPSLKS RLTVTKDTSKNQVVLTMTNMDPVDTATYYCAHSRIIRFTDYVMDAWGQGTLVTVSSASTKGPSVFPL APCSRSTSESTAALGCLVKDYFPEPVTVSWNSGALTSGVHTFPAVLQSSGLYSLSSVVTVPSSSLGT KTYTCNVDHKPSNTKVDKRVESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWESNGQPENNYKTTPPV LDSDGSFFLYSRLTVDKSRWQEGNVFSCSVMHEALHNHYTQKSLSLSLGK |
| | J19.H1 | |
| | Heavy chain | |
| 250 | IgG4 | DIQMTQSPSSLSTSVGDRVTITCRASEDIYNDLAWYQQKPGKAPKLLIYNANSLHTGVASRFSGSGS GTDFTFTISSLQPEDVATYFCQQYYDYPLTFGQGTKLEIKRTVAAPSVFIFPPSDEQLKSGTASVVCL LNNFYPREAKVQWKVDNALQSGNSQESVTEQDSKDSTYSLSSTLTLSKADYEKHKVYACEVTHQGL SSPVTKSFNRGEC |
| | J19.H1 | |
| | Light chain | |

| ***PRO3483 [MGM]* - *LILRB2*** | | |
|---|---|---|
| 251 | IgG4 | QVQLVQSGAEVKKPGASVKVSCKASGYTFTDYYINWVRQAPGQGLEWMGNVNPNDGGTTYNQKF KGRVTMTTDTSTSTAYMELRSLRSDDTAVYYCARREIYFYGTIYYYAMDYWGQGTLVTVSSASTKG PSVFPLAPCSRSTSESTAALGCLVKDYFPEPVTVSWNSGALTSGVHTFPAVLQSSGLYSLSSVVTVP SSSLGTKTYTCNVDHKPSNTKVDKRVESKYGPPCPPCPAPEFLGGPSVFLFPPKPKDTLMISRTPEV TCVVVDVSQEDPEVQFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAVEWESNGQPENNY KTTPPVLDSDGSFFLYSRLTVDKSRWQEGNVFSCSVMHEALHNHYTQKSLSLSLGK |
| | NGM707 | |
| | (US20210355211A1, SEQ ID 144/145) | |
| | Heavy chain | |
| 252 | IgG4 | DIQLTQSPSFLSASVGDRVTITCRASESVDYYGNSFMYWYQQKPGKAPKLLIYFASNLESGVPSRFS GSGSGTEFTLTISSLQPEDFATYYCQQNNEDPWTFGGGTKVEIKRTVAAPSVFIFPPSDEQLKSGTA SVVCLLNNFYPREAKVQWKVDNALQSGNSQESVTEQDSKDSTYSLSSTLTLSKADYEKHKVYACEV THQGLSSPVTKSFNRGEC |
| | NGM707 | |
| | (US20210355211A1, SEQ ID 144/145) | |
| | Light chain | |

| **Reference anti-CD47-PDL1 antibodies** | | |
|---|---|---|
| ***PRO3599 [Pfizer]* - *CD47-PDL1 bispecific*** | | |
| 253 | IgG | QVQLVQSGAEVKKPGSSVKVSCKASGYTFSSYAISWVRQAPGQGLEWMGGISPIFGTANYAQKFQ GRVTITADESTSTAYMELSSLRSEDTAVYYCARDAGRSSDVGWYVGAIDVWGQGTLVTVSSASTKG PSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGGPSVFLFPPKPKDTLMISRT PEVTCVVVDVSHEDPEVKFNWYVDGVEVHNAKTKPREEQYNSTYRVVSVLTVLHQDWLNGKEYKC KVSNKALPAPIEKTISKAKGQPREPQVYTLPPCREEMTKNQVSLSCAVKGFYPSDIAVEWESNGQPE NNYKTTPPVLDSDGSFFLVSKLTVDKSRWQQGNVFSCSVMHEALHNHYTQKSLSLSPGK |
| | WO 2021124073_62_ /WO 2021124073_61 /WO 2021124073_64 | |
| | Heavy chain 1 | |
| 254 | IgG | EVQLVESGGGLVKPGGSLRLSCAASGFTFSNAWMNWVRQAPGKGLEWVGRIKTKADGGTTDYAA PVKGRFTISRDDSKNTLYLQMNSLKTEDTAVYYCTTDPGEYWDSVYGGMDYWGQGTLVTVSSAST KGPSVFPLAPSSKSTSGGTAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPELLGGPSVFLFPPKPKDTLMI SRTPEVTCVVVDVSHEDPEVKFNWYVDGVEVHNAKTKPREEQYNSTYRVVSVLTVLHQDWLNGKE YKCKVSNKALPAPIEKTISKAKGQPREPQVCTLPPSREEMTKNQVSLWCLVKGFYPSDIAVEWESNG QPENNYKTTPPVLDSDGSFFLYSKLTVDKSRWQQGNVFSCSVMHEALHNHYTQKSLSLSPGK |
| | WO 2021124073_62_ /WO 2021124073_61 /WO 2021124073_64 | |
| | Heavy chain 2 | |
| 255 | IgG | QSVLTQPPSASGTPGQRVTISCSGSSSNIGSNYVYWYQQLPGTAPKLLIYRNNQRPSGVPDRFSGS KSGTSASLAISGLRSEDEADYYCAAWDDSLSGVVFGGGTKLTVLGQPKAAPSVTLFPPSSEELQAN KATLVCLISDFYPGAVTVAWKADSSPVKAGVETTTPSKQSNNKYAASSYLSLTPEQWKSHRSYSCQ VTHEGSTVEKTVAPTECS |
| | WO 2021124073_62_ /WO 2021124073_61 /WO 2021124073_64 | |
| | Light chain 1 and 2 | |

| ***PRO3851 [Innovent] - CD47-PDL1 bispecific*** | | |
|---|---|---|
| 256 | EP_3733715_A1 | QVQLQESGPGLVKPSETLSLTCTVSGGSIEHYYWSWIRQPPGKGLEWIGYIYYSGSTNYNPSLKSRV TISVDTSKNQFSLKLSSVTAADTAVYYCARGKTGSAAWGQGTLVTVSSASTKGPSVFPLAPSSKSTS GGTAALGCLVKDYFPEPVTVSWNSGALTSGVHTFPAVLQSSGLYSLSSVVTVPSSSLGTQTYICNVN HKPSNTKVDKKVEPKSCDKTHTCPPCPAPEAAGGPSVFLFPPKPKDTLMISRTPEVTCVVVDVSHE DPEVKFNWYVDGVEVHNAKTKPREEQYNSTYRVVSVLTVLHQDWLNGKEYKCKVSNKALPAPIEKT |
| | (CD47xPD-L1, SEQ ID 1,8,14) | |
| | Heavy chain 1 | |
| | | ISKAKGQPREPQVYTLPPCRDELTKNQVSLWCLVKGFYPSDIAVEWESNGQPENNYKTTPPVLDSD GSFFLYSKLTVDKSRWQQGNVFSCSVMHEALHNHYTQKSLSLSPG |
| 257 | EP_3733715_A1 | QVQLQESGGGLVQPGGSLRLSCAASAYTISRNSMGWFRQAPGKGLEGVAAIESDGSTSYSDSVKG RFTISLDNSKNTLYLEMNSLRAEDTAVYYCAAPKVGLGPRTALGHLAFMTLPALNYWGQGTLVTVSS GGGGSGGGGSGGGGSGGGGSQVQLQESGGGLVQPGGSLRLSCAASAYTISRNSMGWFRQAPG KGLEGVAAIESDGSTSYSDSVKGRFTISLDNSKNTLYLEMNSLRAEDTAVYYCAAPKVGLGPRTALG HLAFMTLPALNYWGQGTLVTVSSDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDWLNGKEYKCKVSNKALP APIEKTISKAKGQPREPQVCTLPPSRDELTKNQVSLSCAVKGFYPSDIAVEWESNGQPENNYKTTPP VLDSDGSFFLVSKLTVDKSRWQQGNVFSCSVMHEALHNHYTQKSLSLSPG |
| | (CD47xPD-L1, SEQ ID 1,8,14) | |
| | Heavy chain 2 | |
| 258 | EP_3733715_A1 | DIQMTQSPSSVSASVGDRVTITCRASQGISRWLAWYQQKPGKAPKLLIYAASSLQSGVPSRFSGSG SGTDFTLTISSLQPEDFATYYCQQTVSFPITFGGGTKVEIKRTVAAPSVFIFPPSDEQLKSGTASVVCL LNNFYPREAKVQWKVDNALQSGNSQESVTEQDSKDSTYSLSSTLTLSKADYEKHKVYACEVTHQGL SSPVTKSFNRGEC |
| | (CD47xPD-L1, SEQ ID 1,8,14) | |
| | Light chain | |

Throughout the text of this application, should there be a discrepancy between the text of the specification (*e. g*., Tables 1 to 6) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all subcombinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### Examples

### Example 1: Generation and testing of antibodies directed against human PDL1:

The PDL1-BDs disclosed herein have been generated, produced and tested following the methods described in detail in the patent applications WO 2019/072868 and WO 2019/072869.

### 1.1 Generation, identification, screening and testing

Briefly, rabbits were immunized with recombinant human PDL1 extracellular domain. B cells from these immunized rabbits were then subjected to flow-cytometry-based sorting procedure. In this sorting procedure PD1/PDL1 neutralizing high-affinity anti-PDL1 antibodies were identified and isolated through detecting their binding to PD-1 extracellular domain, immobilized on protein G beads. The isolated B cells expressing the neutralizing anti-PDL1 antibodies were cultured, and the cultured supernatants collected. The obtained rabbit monoclonal antibodies in each cell culture supernatant were characterized in a high-throughput ELISA for binding to recombinant human PDL1 and cynomolgus PDL1. In addition, neutralization potential of the PDL1/PD-1 interaction as well as of PDL1/B7-1 interaction was determined by competition ELISA as well as by a cell-based reporter gene assay. In addition, binding affinities of antibodies towards human PDL1 were measured by surface plasmon resonance (SPR). The methods used for direct ELISA, competition ELISA and SPR, as well as the results of the characterization are described in detail in the patent applications WO 2019/072868 and WO 2019/072869.

### 1.2 Cloning and production of rabbit IgGs

Following the identification of the clones, rabbit IgG antibodies were cloned, expressed and purified for further characterization (hit confirmation), as described in the patent applications WO 2019/072868 and WO 2019/072869.

### 1.3 Humanization and scFv production

The CDRs of the identified rabbit clones were grafted onto Numab's proprietary VH/VL frameworks to produce stable scFvs, as described in the patent applications WO 2019/072868 and WO 2019/072869. Different grafting variants were designed and produced. Examples of production details for some of them are summarized in Table 7.

### 1.4 Pharmacodynamics Characterization of humanized scFvs

### Affinity to human PDL1

Affinity of the humanized scFvs to human PDL1 was determined by SPR analysis as described in WO 2019/072869. Results are summarized in Table 8.

### Neutralization of PDL1/PD-1 interaction by NFAT reporter gene assay

Potency of some PDL1 scFvs to neutralize PDL1-binding to PD-1 was assessed by NFAT cell-based reporter gene assay analogous to the method described in WO 2019/072869. Potencies are summarized in Table 9, which shows that the tested anti-PDL1 scFvs can efficiently block ligand binding.

### Species cross-reactivity (binding to cynomolgus monkey and mouse PDL1 by SPR)

Cross-reactivity to cynomolgus PDL1 was measured in a similar assay as used to measure binding to human PDL1, with the recombinant PDL1 produced by Sino Biological. Table 10 summarizes the affinities obtained for all tested scFvs. All tested scFvs that showed binding to human PDL1 also showed binding to cynomolgus PDL1.

### Selectivity for PDL1 versus PDL2 by SPR

Humanized scFvs were tested for binding to PDL2 by SPR analysis on a T200 device (Biacore, GE Healthcare). In this experiment, Fc-tagged human PDL2 was captured using the Human Antibody Capture kit from GE HealthCare. After each analyte injection cycle the CM5 sensor chip was regenerated, and new antigen was captured. The scFvs were injected as analyte at a concentration of 180 nM diluted in running buffer for 5 min and dissociation of the protein was allowed to proceed for 12 min. No binding to PDL2 was observed for PRO1392 (33-03-G02-sc18) and PRO1347 (37-20-B03-sc09).

### 1.5 Biophysical characterization of the humanized anti-PDL1 scFvs

Manufacture of material for stability assessment and storage stability study of exemplary humanized anti-PDL1 scFvs has been performed as described in the patent applications WO 2019/072868 and WO 2019/072869. Results of the storage stability study are summarized in Table 11.

**Table 7: Production summary table of exemplary anti-PDL1 scFvs.**

| **Clone ID** | **Protein ID** | **VH Framework** | **Final yield [mg]** | **Final titer [mg/L]** | **Final monomeric content [%]** | **Tm1 [°C]** | **Tm2 [°C]** |
|---|---|---|---|---|---|---|---|
| 33-03-G02-sc18 | PRO1392 | VH4 | 7.44 | 37 | 97 | 70.9 | 79.2 |
| 33-03-G02-sc18 (VL_A67G_F68G) | PRO3724 | VH4 | 0.30 | 26 | 89 | 66.2 | 74.9 |
| 33-03-G02-sc18 (VL_A67G_F68G; VH_T101S_T146K) | PRO3891 | VH4 | 10.7 | 216 | 99 | 64.9 | 74.1 |
| 37-20-B03-sc09 | PRO1347 | VH3 | 2.30 | 11.5 | 98 | 77.2 | - |
| 37-20-B03 sc09 (VL_A67G_F68G) | PRO3723 | VH3 | 0.64 | 50 | 99 | 67.4 | 76.5 |
| 37-20-B03 sc09.1 | PRO2230 | VH3 | 53.0 | 242 | 100 | 72.8 | 81.7 |
| 37-20-B03 sc18 (VL_A67G_F68G; VH_T101S_T146K) | PRO4245 | VH3 | 38.7 | 484.3 | 99.9 | 72.5. | - |

**Table 8: Affinities of exemplary anti-PDL1 scFv to human PDL1.**

| **Clone ID** | **Protein ID** | **Framework** | **kₐ [M⁻¹ s⁻¹]** | **k_{d} [s⁻¹]** | **K_{D} [M]** |
|---|---|---|---|---|---|
| 33-03-G02-sc18 | PRO1392 | VH4 | 5.6 ±1.2E+06 | 4.8 ±1.7E-05 | 8.6 ±1.9E-12 |
| 33-03-G02-sc18 (VL_A67G_F68G) | PRO3724 | VH4 | 4.7E+06 | 5.7E-05 | 1.2E-11 |
| 33-03-G02-sc18 (VL_A67G_F68G; VH_T101S_T146K) | PRO3891 | VH4 | 6.0 ±0.6E+06 | 3.9 ±0.4E-05 | 4.1 ±3.1E-12 |
| 37-20-B03-sc09 | PRO1347 | VH3 | 7.38E+06 | 6.64E-05 | 1.09E-11 |
| 37-20-B03 sc09 (VL_A67G_F68G) | PRO3723 | VH3 | 3.08E+06 | 7.95E-05 | 2.58E-11 |
| 37-20-B03 sc09.1 | PRO2230 | VH3 | 6.3 ±1.3E+06 | 9.3 ±2.8E-05 | 1.5 ±0.2E-11 |
| 37-20-B03 sc18 (VL_A67G_F68G) | PRO5036 | VH3 | 4.0E+06 | 1.4E-04 | 3.4E-11 |
| 37-20-B03 sc18 (VL_A67G_F68G; VH_T101_S_T146K) | PRO4245 | VH3 | 4.0 ±0.3E+06 | 1.4 ±0.2E-04 | 3.6 ±0.3E-11 |

**Table 9: Potencies of exemplary anti-PDL1 scFvs to inhibit the interaction between human PDL1 and PD-1.**

| **Clone ID** | **Protein ID** | **PDL1/PD-1 EC₅₀ [nM]** | **PDL1/PD-1 Max. inhibition (rel. to avelumab) [%]** | |
|---|---|---|---|---|
| 33-03-G02-sc18 | PRO1392 | 0.97 | 104.5 | |
| 33-03-G02-sc18 (VL_A67G_F68G; VH_T101S_T146K) | PRO3891 | 1.36 | 94.6 | |
| 37-20-B03-sc09 | PRO1347 | 0.28 | 100.1 | |
| 37-20-B03 sc09.1 | PRO2230 | 0.93 | 91.8 | |
| 37-20-B03 sc18 (VL_A67G_F68G; VH_T101_S_T146K) | PRO4245 | 1.81 | 105.6 | |

**Table 10: Affinities of exemplary anti-PDL1 scFvs to cynomolgus PDL1**

| **Clone ID** | **Protein ID** | **VH Framework** | **kₐ [M⁻¹ s⁻¹]** | **k_{d} [s⁻¹]** | **K_{D} [M]** |
|---|---|---|---|---|---|
| 33-03-G02-sc18 | PRO1392 | VH4 | 5.2 ±0.2E+06 | 3.4 ±1.9E-05 | 6.5 ±3.7E-12 |
| 33-03-G02-sc18 (VL_A67G_F68G; VH_T101S_T146K) | PRO3891 | VH4 | 5.9 ±0.5E+06 | 2.9 ±0.4E-05 | 3.4 ±2.1E-12 |
| 37-20-B03-sc09 | PRO1347 | VH3 | 6.88E+06 | 8.77E-05 | 1.27E-11 |
| 37-20-B03 sc09.1 | PRO2230 | VH3 | 7.0 ±1.1E+06 | 8.4±2.1E-05 | 1.2 ±0.2E-11 |
| 37-20-B03 sc18 (VL_A67G_F68G; VH_T101S_T146K) | PRO4245 | VH3 | 4.6 ±0.9E+06 | 1.4 ±0.2E-04 | 3.2 ±0.9E-11 |

**Table 11: 4w stability study of the selected domains.**

| **Clone ID** | **Protein ID** | **Temp . [°C]** | **Monomeric content [%]** | | | | **Monomeric content loss [%]** | | | **Protein concentration [mg/mL]** | | | | **Protein content loss [%]** | | |
|---|---|---|---|---|---|---|---|---|---|---|---|---|---|---|---|---|
| | | | **d0** | **d7** | **d14** | **d28** | **d7** | **d14** | **d28** | **d0** | **d7** | **d14** | **d28** | **d7** | **d14** | **d28** |
| 33-03-G02-sc18 | PRO1392 | -80 | 97.4 | 97.4 | n.a. | 97.2 | 0.0 | n.a. | 0.2 | 10.6 | 9.4 | n.a. | 11.0 | 11.7 | n.a. | -3.9 |
| | | 4 | 97.4 | 97.1 | n.a. | 96.9 | 0.2 | n.a. | 0.5 | 10.6 | 10.7 | n.a. | 10.7 | -0.9 | n.a. | -1.3 |
| | | 40 | 97.4 | 94.2 | 91.1 | 84.7 | 3.2 | 6.4 | 13.0 | 10.6 | 10.7 | n.a. | 11.2 | -0.8 | n.a. | -5.9 |
| 33-03-G02-sc18 (VL_A67G_S68G) | PRO3724 | -80 | 99.8 | n.a. | n.a. | 99.8 | n.a. | n.a. | 0.0 | 9.8 | n.a. | n.a. | 9.4 | n.a. | n.a. | 3.7 |
| | | 4 | 99.8 | n.a. | 99.5 | 99.3 | n.a. | 0.3 | 0.6 | 9.8 | n.a. | 9.7 | 9.8 | n.a. | 0.7 | -0.2 |
| | | 40 | 99.8 | n.a. | 90.9 | 85.4 | n.a. | 9.0 | 14.5 | 9.8 | n.a. | 10.1 | 10.2 | n.a. | -3.0 | -3.9 |
| 33-03-G02-sc18 (VL_A67G_F68G; VH_T101S_T146K) | PRO3891 | -80 | 98.7 | n.a. | n.a. | 98.7 | n.a. | n.a. | 0.0 | 10.5 | n.a. | n.a. | 10.5 | n.a. | n.a. | 0.8 |
| | | 4 | 98.7 | n.a. | 98.2 | 97.8 | n.a. | 0.5 | 0.9 | 10.5 | n.a. | 11.1 | 10.4 | n.a. | -4.9 | 1.2 |
| | | 40 | 98.7 | n.a. | 87.6 | 83.4 | n.a. | 11.2 | 15.5 | 10.5 | n.a. | 11.1 | 12.4 | n.a. | -5.7 | -18.0 |
| 37-20-B03-sc09 | PRO1347 | 4 | 98.5 | 97.1 | n.a. | 94.6 | 1.4 | n.a. | 4.0 | 10.6 | 11.4 | 11.1 | 11.5 | -7.6 | -4.4 | -8.0 |
| | | 40 | 98.5 | 83.1 | n.a. | 75.6 | 15.6 | n.a. | 23.3 | 10.6 | 11.9 | 11.7 | 11.7 | -12.1 | -10.5 | -10.0 |
| 37-20-B03-sc09.1 | PRO2230 | -80 | 98.6 | n.a. | 98.6 | 98.6 | n.a. | 0.0 | 0.0 | 9.7 | n.a. | n.a. | 9.8 | n.a. | NA | -0.9 |
| | | 4 | 98.6 | n.a. | 98.4 | 98.4 | n.a. | 0.2 | 0.2 | 9.7 | n.a. | 9.9 | 10.2 | n.a. | -1.4 | -4.5 |
| | | 40 | 98.6 | n.a. | 95.9 | 92.9 | n.a. | 2.8 | 5.8 | 9.7 | n.a. | 9.9 | 10.0 | n.a. | -2.0 | -2.5 |
| 37-20-B03-sc18 | PRO4245 | -80 | 99.8 | n.a. | n.a. | 99.6 | n.a. | n.a. | 0.2 | 10.9 | n.a. | n.a. | 11.2 | n.a. | NA | -2.9 |
| | | 4 | 99.8 | n.a. | 100.0 | 99.2 | n.a. | -0.2 | 0.6 | 10.9 | n.a. | 11.9 | 11.1 | n.a. | -9.3 | -1.4 |
| | | 40 | 99.8 | n.a. | 95.7 | 89.8 | n.a. | 4.1 | 10.0 | 10.9 | n.a. | 12.0 | 11.4 | n.a. | -10.4 | -4.2 |

### Example 2: Generation and testing of antibodies directed against human CD47:

### Aim of the project

The goal of the project was to generate humanized monoclonal antibody variable domains that specifically bind to and neutralize the biological effect of human CD47. A further goal was to identify anti-CD47 antibody variable domains that are potent and stable enough for incorporation into multispecific antibody formats and have a different range of affinities.

### 2.1. Immunization

Two different immunization protocols were applied to a total of nine rabbits to obtain an optimal immune response against human CD47. Rabbits were immunized with recombinantly produced and purified CD47 extracellular domain (ECD) (Sino Biological, Cat. No. 12283-HCCH). Prior to immunization, the quality of recombinant human CD47 was analyzed by the manufacturer 1) for purity by SDS-page and SEC-HPLC, and 2) for biological activity by measuring the ability to bind to SIRPα in a functional ELISA. The first group of six rabbits (protocol 1) received 4 injections of 200 µg each throughout 70 days. The second group of three rabbits (protocol 2) received 5 injections of 200 µg each through a period of 112 days. During the course of the immunization, the strength of the humoral immune response against the antigen was qualitatively assessed by determining the maximal dilution (titer) for the serum of each rabbit that still results in detectable binding of the polyclonal serum antibodies to the antigen. Serum antibody titers against the immobilized antigen (recombinant human CD47 as well as recombinant cynomolgus monkey CD47) were assessed using an enzyme-linked immunosorbent assay (ELISA). All nine rabbits immunized with purified human CD47 ECD showed high titers for the human antigen with EC₅ up to 4.6 × 10⁷, as well as for cynomolgus CD47 with EC₅ up to 4.0 × 10⁷. Additionally, the titers of neutralizing antibodies to block CD47 binding to SIRPα were determined. The nine rabbits showed neutralizing titers with EC₅ up to 4.6 × 10⁶.

### 2.2. Sorting

Prior to the hit identification procedure, a flow-cytometry-based sorting procedure was performed in the presence R-Phycoerythrin (RPE)-labelled human or cynomolgus monkey CD47 ECD, which allows the specific detection and isolation of CD47-binding B-cells. A total of 1.4 × 10⁸ lymphocytes derived from seven rabbits were analyzed in this sorting campaign. Of these, a total of 7,040 B-cells expressing CD47-specific antibodies (IgG) were isolated and individually cultured as single clones for three to four weeks.

### 2.3. Hit identification

### General remarks

For hit identification, direct ELISA screens were performed to assess binding to human CD47. Binding to cynomolgus monkey CD47 was evaluated by direct ELISA as well as CELISA using transiently transfected cells expressing cynomolgus CD47.

### Binding to human CD47 by ELISA

For hit identification, *i. e*. the identification of B cell clones that produce antibodies binding to CD47, cell culture supernatants of the above 7,040 B cell clones were screened for the presence of antibodies that bind to human CD47 by ELISA. This primary hit identification procedure was performed with non-purified antibodies from the cell culture supernatants, as the scale of the high-throughput culture does not allow for purification of the individual rabbit antibodies. The use of cell supernatants allows for ranking of large numbers of antibodies. The ELISA method used assesses the "quantity" of rabbit IgGs bound to recombinant human CD47, however it does not provide any information regarding the affinity or the concentration of the antibodies. In this assay, supernatants from 1807 B cell clones produced a signal that was above background.

### Inhibition of SIPRα binding to CD47 in a cellular assay and in a competition ELISA

All 1807 human CD47-binding supernatants were further analyzed for their potential to inhibit the binding of SIPRα to CD47 expressing cells by flow-cytometry and for blockage of the interaction of human CD47 with human SIRPα in a competition ELISA.

For this purpose, a flow-cytometry based assay and a receptor ligand competition-ELISA were developed and adapted for use with B cell supernatant. For the flow-cytometry based assay, inhibition of binding of biotinylated SIPRα-Fc fusion protein to Raji cells expressing CD47 by B-cell supernatants was assessed by flow-cytometry. Since the assays could be performed with B cell supernatant as matrix and were sensitive and precise enough, both assays were used for screening. The inhibitory activity of each B cell supernatant was tested using single well analysis (no dose-responses were performed). Therefore, the extent of inhibition observed is not only dependent on the IgG properties, but also on the concentration of rabbit IgGs in the B cell supernatant.

905 out of 1807 clones blocked the interaction of human CD47 and human SIRPα by more than 50 % and 811 clones inhibited above 70 % in the flow-cytometry based assay. 1335 clones blocked the interaction of human CD47 and human SIPRα by more than 50 % and 1056 clones inhibited above 70% in the competition ELISA. 850 hits show at least 50 % inhibition in both assays.

### Species cross-reactivity (binding to cynomolgus monkey CD47 by ELISA and CELISA)

All 1,807 hits identified in the primary screening were analyzed for species cross-reactivity to cynomolgus monkey by ELISA and by a cell-based ELISA (CELISA). The ability of the antibodies to cross-react with cynomolgus CD47 by CELISA was assessed using transiently transfected CHO cells expressing cynomolgus CD47.

987 clones (52 %) showed binding to cynomolgus CD47 in the direct ELISA and 1034 clones (57 %) showed binding to cynomolgus monkey CD47 by CELISA, respectively. Overall, many antibodies were identified showing cross-reactivity to cynomolgus monkey species.

### Binding to red blood cells by flow-cytometry

The ability of the rabbit antibodies to bind to human CD47 expressed on red blood cells (RBCs) was assessed by flow cytometry. Human RBCs were incubated with rlgG positive B cell supernatants at 4 °C for 1 hour, followed by the addition of APC-conjugated anti-rabbit Fc secondary antibody at 4 °C for 30 minutes.

939 clones (55 %) showed binding to RBC. Consequently, 868 (45 %) antibodies did not show any binding to RBCs. These antibodies might have a beneficial safety profile *in vivo.*

### Determination of binding affinity to human CD47

In a secondary hit identification procedure, information on the binding affinities to human CD47 of the 1053 monoclonal rabbit antibodies that were qualified as positive during the primary screening and were blocking the interaction between SIRPα and CD47 and were cyno CD47 cross-reactive, were determined by surface plasmon resonance (SPR).

For the affinity screening by SPR, an antibody specific for the Fc region of rabbit IgGs was immobilized on a sensor chip (SPR-24 Pro Affinity Sensor, High Capacity Amine, Bruker) using a standard amine-coupling procedure. Rabbit monoclonal antibodies in B cell supernatants were captured by the immobilized anti-rabbit IgG antibody. A minimal IgG concentration in the B cell supernatants is required to allow sufficient capture. After capturing of the monoclonal antibodies, human CD47 ECD (extracellular domain) was injected into the flow cells for 3 min at a concentration of 45 nM, and dissociation of the protein from the IgG captured on the sensor chip was allowed to proceed for 5 min. The assay was run at 37°C with a buffer containing 400 mM NaCl to allow better resolution of the off-rates and therefore improve the ranking of the antibodies. The apparent dissociation (k_{d}) and association (kₐ) rate constants and the apparent dissociation equilibrium constant (K_{D}) were calculated with the analysis software (Analyzer, Bruker) using the 1:1 Langmuir binding model.

Binding affinities to human CD47 could be measured for 656 anti-CD47 antibodies. They showed dissociation constants (K_{D}) ranging from 1.7 × 10⁻¹² M to 5.8 × 10⁻⁷ M. 27 % of the antibodies analyzed had a K_{D} below 0.5 nM.

### Competition with CC-90002 for binding to Raji cells by flow-cytometry

The ability of anti-CD47 rlgGs in B cell supernatants to compete with the anti-CD47 antibody from Celgene (clone CC-9002) for binding to CD47 expressed on the cell surface was tested by flow-cytometry. For this, inhibition of binding of saturating amounts of Celgene antibody to Raji cells expressing CD47 by B-cell supernatants was assessed by flow-cytometry.

Out of 1054 clones tested 576 clones (55 %) compete with CC-9002 for CD47 binding and 45% of the clones did not compete for binding. The best non-competing rabbit clones were selected for humanization and scFv production.

### 2.4 Humanization and scFv production

The CDRs of the identified rabbit clones were grafted onto Numab's proprietary VH/VL frameworks to produce stable scFvs, as described in the patent application WO 2022/136675. Different grafting variants were designed and produced.

### 2.5 Manufacture of suitable humanized anti-CD47 binding domains for characterization (scFv format)

The CDRs of the identified rabbit clones were grafted onto Numab's proprietary VH/VL frameworks to produce stable scFvs, as described in the patent applications WO 2019/072868 and WO 2019/072869. Different grafting variants were designed and produced. From the scFvs that exhibited the desired properties, the best one was selected (PRO3703). Production details of PRO3703 are summarized in Table12.

Briefly, the expression of the scFv was performed in CHO cells using the ExpiCHO Expression System (Thermo Fisher Scientific). Expression was conducted according to manufacturer's instructions. Proteins were purified from clarified harvest by affinity chromatography (Protein L and/or Protein A). If necessary, variant scFvs were polished by SE-chromatography to a final monomeric content > 95 %. For quality control of the manufactured material, standard analytical methods such as ESI-MS, SE-HPLC, UV₂₈₀ and SDS-PAGE were applied.

The mass of the scFvs has for example been verified by the following ESI-MS standard method. Manufactured scFvs were 5 fold diluted with 1 % TFA. Two µl of sample were injected into an ACQUITY UPLC@ BioResolve-RP-mAb 2.7 µm 2.1x150 mm, 450 Å column (Waters, USA) and desalted using a gradient from 15 % to 85 % buffer B (0.1 % formic acid, 75 % propan-2-ol in acetonitrile) at a flow rate of 200 µl/min at 50°C. The MS analysis was performed on a Synapt G2 mass spectrometer directly coupled to the UPLC station. Mass spectra were acquired in the positive-ion mode by scanning the m/z range from 400 to 5,000 Da with a scan duration of 1 s and an interscan delay of 0.1 s. The data were recorded with the MassLynx 4.2 Software (both Waters, UK). Where possible, the recorded m/z data of single peaks were deconvoluted into mass spectra by applying the maximum entropy algorithm MaxEnt1 (MaxLynx).

The monomeric content of the manufactured scFvs has been determined for example by using the following standard SE-HPLC method. Five µg of the respective scFv, typically present at a concentration of from 0.1 to 10 mg/ml , were injected onto a Shodex KW402.5-4F column using a Hitachi Chromaster HPLC system at 25°C and a flow rate of 0.35 ml/min. The mobile phase was 50 mM sodium acetate, 250 mM sodium chloride in water at pH 6.0. Elution profile was monitored at 280 nm.

**Table 12: Manufacture of scFvs**

| **Protein ID** | **Construct ID** | **Final titer [mg/L]** | **Final purity by SE-HPLC [% monomer]** |
|---|---|---|---|
| PRO3703 | 70-26-B04-sc03 | 66.6 | 99.5 |

### 2.6 Pharmacodynamic characterization of suitable humanized anti-CD47 binding domains (scFvs format)

### Binding potency to human CD47

Affinity of PRO3703 to human CD47 was determined by SPR analysis using an SPR 24 system (Sierra Sensor - Bruker). Human CD47 was immobilized on a CMD200M SPR sensor prism chip (Xantec). Then PRO3703 was injected at concentrations of 10 nM, 2 nM and 0.4 nM on the sensor chip over spots with immobilized human CD47. An empty spot was used as reference. After each analyte cycle, the surface was regenerated with 3 M MgCl₂ solution, thereby allowing the next analyte cycle to have only free ligand available. Binding affinity K_{D} was globally calculated by the Bruker SPR software based on the binding association (*k*ₒₙ) and dissociation (*k*_{off}) rate, by fitting a 1:1 Langmuir model to the curves obtained by the cycles with the three different concentrations of the analyte. The results are shown in Table 13.

**Table 13: Affinity of PRO3703 to human CD47.**

| **Protein ID** | **scFv** | **Affinity to human CD47** | | |
|---|---|---|---|---|
| | Clone ID | kₐ (M⁻¹ s⁻¹) | k_{d} (s⁻¹) | K_{D} (M) |
| PRO3703 | 70-26-B04-sc03 | 2.44 ±0.21E+06 | 2.90 ±0.17E-02 | 1.19 ±0.034E-08 |

### Binding to human and cynomolgus PBMCs

The ability of the anti-CD47 scFvs to bind to specific cells within the subset of PBMCs was investigated. It was found that PRO3703 strongly binds to HLA-DR+ CD14+ monocytes and HLA-DR+ CD14- APCs. No binding to any other of the tested PBMC cell types was observed (see Table 14).

**Table 14: Binding to human and cynomolgus PBMCs.**

| | **Human / Cynomolgus Monkey** | | | |
|---|---|---|---|---|
| **PRO ID** | **HLA-DR+ CD14+ EC₅₀ [nM]** | **HLA-DR+ CD14-EC₅₀ [nM]** | **B cells (CD20+) EC₅₀ [nM]** | **T cells (CD3+) EC₅₀ [nM]** |
| PRO3703 | 5.084 / >40nM | 3.350 / >40nM | >40nM / >40nM | >40nM / >40nM |

### 2.7 Biophysical characterization of anti-CD47 binding domains (scFv format)

### Storage stability study

PRO3703 was subjected to stability studies, such as a four-week stability study, in which the scFvs were formulated in an aqueous buffer (50 mM phosphate citrate buffer (NaCiP), 150 mM NaCl, pH 6.4) at 10 mg/ml and stored at < -80°C, 4°C and 40°C for four weeks. At the minimum, the fractions of monomers and oligomers in the formulation were evaluated by integration of SE-HPLC peak areas after two weeks and at the end of each study. Additional time points were recorded for most of the molecules. Table 15 (monomer content) and Table 16 (protein content) compare day 14 (d14) and endpoint measurements obtained at day 28 (d28) of the study.

The tested anti-CD47 scFv PRO3703 appears to be very stable, exhibiting no measurable loss in the monomer content upon storage for 28 days at -80°C and 4°C and only minimal loss in monomeric content upon storage for 28 days at 40°C. Likewise, PRO3703 exhibited no protein content loss at any tested storage temperature and time point.

### Thermal unfolding

Thermal stability of PRO3703 was analyzed by nano dynamic scanning fluorimetry (nDSF) using a NanoTemper, allowing the determination of the onset of unfolding (Tₒₙₛₑₜ), midpoint of unfolding (Tₘ). Tₘ and Tₒₙₛₑₜ results of duplicate/triplicate measurements are summarized in Table 17. As can be deduced from Table 17, all molecules exhibit high melting temperatures of at least 59°C, most of the molecules exhibit melting temperatures of >60°C or >70°C.

**Table 15: Course of monomeric content and monomeric content loss upon sample storage at a concentration of 10 g/L and temperatures of -80°C, 4°C and 40°C for up to 28 days.**

| **PRO ID** | **Description** | **Day 0** | **Day 14 (MC -80°C)** | **Day 28 (MC -80°C)** | **Day 14 (MC 4°C)** | **Day 28 (MC 4°C)** | **Day 14 (MC 40°C)** | **Day 28 (MC 40°C)** | **Day 14 (MC% -80°C)** | **Day 28 (MC% -80°C)** | **Day 14 (MC% 4°C)** | **Day 28 (MC% 4°C)** | **Day 14 (MC% 40°C)** | **Day 28 (MC% 40°C)** |
|---|---|---|---|---|---|---|---|---|---|---|---|---|---|---|
| PRO3703 | 70-26-B04-sc03 | 99.6 | n.a. | 99.5 | 99.6 | 99.5 | 99.5 | 99.3 | n.a. | 0.0 | 0.0 | 0.0 | 0.1 | 0.3 |

**Table 16: Course of protein content and protein content loss upon sample storage at a concentration of 10 g/L and temperatures of -80°C, 4°C and 40°C for up to 28 days.**

| **PRO ID** | **Description** | **Day 0 (PC)** | **Day 14 (PC -80°C)** | **Day 28 (PC -80°C)** | **Day 14 (PC 4°C)** | **Day 28 (PC 4°C)** | **Day 14 (PC 40°C)** | **Day 28 (PC 40°C)** | **Day 14 (PC% -80°C)** | **Day 28 (PC% -80°C)** | **Day 14 (PC% 4°C)** | **Day 28 (PC% 4°C)** | **Day 14 (PC% 40°C)** | **Day 28 (PC% 40°C)** |
|---|---|---|---|---|---|---|---|---|---|---|---|---|---|---|
| PRO3703 | 70-26-B04-sc03 | 9.2 | n.a. | 8.6 | 9.3 | 9.2 | 9.5 | 10.5 | n.a. | 6.6 | -0.8 | 0.3 | -3.1 | -13.9 |

**Table 17: nDSF measurement of scFvs using NanoTemper device for determination of Tₘ and Tₒₙₛₑₜ.**

| **PRO ID** | **Description** | **Tₒₙₛₑₜ [°C]** | **Tₘ [°C]** |
|---|---|---|---|
| PRO3703 | 70-26-B04-sc03 | 54.3 | 59.2 |

| | | | |
|---|---|---|---|
| PC: protein content [mg/mL] MC: monomer content [%] | | | |

### Example 3: Generation and testing of antibodies directed against human LILRB2:

### Aim of the project

The goal of the project was to generate humanized monoclonal antibody variable domains that specifically bind to and neutralize the biological effect of human LILRB2. A further goal was to identify anti-LILRB2 antibody variable domains that are potent and stable enough for incorporation into multispecific antibody formats.

### 3.1. Immunization

Three different immunization protocols were applied to a total of 12 rabbits to obtain an optimal immune response against human LILRB2. Rabbits were immunized with recombinantly produced and purified LILRB2 extracellular domain (ECD) (Sino Biological, Cat. No. 14132-H08H). Prior to immunization, the quality of recombinant human LILRB2 was analyzed by the manufacturer for purity by SDS-page. The first group of three rabbits (protocol 1) received 4 injections of 200 µg each throughout 33 days. The second group of six rabbits (protocol 2) received 4 injections of 200 µg each throughout 70 days. The third group of three rabbits (protocol 3) received 5 injections of 200 µg each through a period of 108 days. During the course of the immunization, the strength of the humoral immune response against the antigen was qualitatively assessed by determining the maximal dilution (titer) for the serum of each rabbit that still results in detectable binding of the polyclonal serum antibodies to the antigen. Serum antibody titers against the immobilized antigen (recombinant human LILRB2) were assessed using an enzyme-linked immunosorbent assay (ELISA). All nine rabbits immunized with purified human LILRB2 showed high titers with EC₅ up to 1:1.5 × 10⁷.

### 3.2. Sorting

Prior to the hit identification procedure, a flow-cytometry-based sorting procedure was performed in the presence of R-Phycoerythrin (RPE)-labelled human or cynomolgus monkey LILRB2 ECD, which allows the specific detection and isolation of high-affinity LILRB2-binding B-cells. A total of 9.05 × 10⁷ lymphocytes derived from nine rabbits were analyzed in this sorting campaign. Of these, a total of 10,560 B-cells expressing LILRB2-specific antibodies (IgG) were isolated and individually cultured as single clones for three to four weeks.

### 3.3. Hit identification

### General remarks

For hit identification, direct ELISA screens were performed to assess binding to human LILRB2. Binding to cynomolgus and rhesus monkey LILRB2 was evaluated by CELISA using transiently transfected cells expressing cynomolgus or rhesus LILRB2.

### Binding to human LILRB2 by ELISA

For hit identification, i. e. the identification of B cell clones that produce antibodies binding to LILRB2, cell culture supernatants of the above 10,560 B cell clones were screened for the presence of antibodies that bind to human LILRB2 ECD by ELISA. This primary hit identification procedure was performed with non-purified antibodies from the cell culture supernatants, as the scale of the high-throughput culture does not allow for purification of the individual rabbit antibodies. The use of cell supernatants allows for ranking of large numbers of antibodies. The ELISA method used assesses the "quantity" of rabbit IgGs bound to recombinant human LILRB2, however it does not provide any information regarding the affinity or the concentration of the antibodies. In this assay, supernatants from 1,423 B cell clones produced a signal that was above background.

### Inhibition of HLA-G and HLA-A02 binding to human LILRB2 expressing cells

All 1,423 human LILRB2-binding supernatants were further analyzed for their potential to inhibit the binding of HLA-G tetramers to LILRB2-expressing cells. For some of the supernatants also the potential to inhibit binding of HLA-A02 tetramers to LILRB2-expressing cells was analyzed.

For this purpose, a flow-cytometry based assay was developed. Inhibition of binding of PE-labelled HLA-G tetramers or HLA-A02 multimers, respectively, to CHO cells stably transfected with human LILRB2 by B-cell supernatants was assessed by flow-cytometry. Since the assays could be performed with B cell supernatant as matrix and were sensitive and precise enough, both assays were used for screening. The blocking activity of each B cell supernatant was tested using single well analysis (no dose-responses were performed). Therefore, the extent of inhibition observed is not only dependent on the IgG properties, but also on the concentration of rabbit IgGs in the B cell supernatant.

661 out of 1,423 clones blocked the interaction of human LILRB2 and human HLA-G tetramer by more than 30 %. 94 out of 931 clones blocked the interaction of human LILRB2 and HLA-A02 by more than 50 %.

### Species cross-reactivity (binding to cynomolgus monkey and rhesus monkey LILRB2 by CELISA)

All 1,423 hits identified in the primary screening were analyzed for species cross-reactivity to cynomolgus monkey and rhesus monkey LILRB2 by a cell-based ELISA (CELISA). The ability of the antibodies to cross-react with cyno and rhesus LILRB2 was assessed by CELISA using transiently transfected HEK293T cells expressing rhesus or cynomolgus LILRB2 with an N-terminal V5-tag.

141 (9.9 %) and 51 clones (3.6 %) showed binding to cynomolgus monkey and rhesus monkey LILRB2, respectively. Overall, there were only a few antibodies identified showing cross-reactivity to either of the two monkey species.

### Specificity assessment and mapping of binding region (binding to human LILRA1 and to human LILRB2/LILRA1 chimeras by CELISA)

All 1,423 hits identified in the primary screening were analyzed for cross-reactivity to LILRA1, the LILR family member with the highest sequence identity to LILRB2, by a cell-based ELISA (CELISA). The ability of the antibodies to selectively bind to LILRB2 versus LILRA1 was performed using full-length LILRA1 transiently transfected HEK293T cells. 1,177 clones show binding with an optical density (OD) below 1 and are therefore potentially not binding to LILRA1.

LILRB2 (UniProt: Q8N423) is a single-pass transmembrane protein possessing an extracellular domain of four Ig-like domain and an intracellular domain with 3 ITIM-containing motifs. To map the binding regions of the anti-LILRB2 antibodies, chimeric cDNA constructs were generated where each of the extracellular Ig-like domains of LILRB2 was replaced individually by the corresponding Ig-like domain of LILRA1. The clones potentially not showing binding to LILRA1 were tested for binding to cells transiently transfected with the 4 different LILRB2/LILRA1 chimeras. Domain specificity was used to group the LILRB2 specific antibodies into bins.

86 antibodies showed binding to the N-terminal Ig-like domain 1 of LILRB2, 80 to Ig-like domain 2, 2 to Ig-like domain 3 and 323 to Ig-like domain 4. For the rest of the antibodies no binding region was identified.

### Determination of binding affinity to human LILRB2

In a secondary hit identification procedure, information on the binding affinities to human LI LRB2 of the approximately 1,177 monoclonal rabbit antibodies that were qualified as positive during the primary screening and were not binding to LILRA1 were determined by surface plasmon resonance (SPR).

For this affinity screening by SPR, an antibody specific for the Fc region of rabbit IgGs was immobilized on a sensor chip (SPR-24 Pro Affinity Sensor, High Capacity Amine, Bruker) using a standard amine-coupling procedure. Rabbit monoclonal antibodies in B cell supernatants were captured by the immobilized anti-rabbit IgG antibody. After capturing of the monoclonal antibodies, human LILRB2 ECD (extracellular domain) was injected into the flow cells for 3 min at a concentration of 45 nM, and dissociation of the protein from the IgG captured on the sensor chip was allowed to proceed for 5 min. The apparent dissociation (k_{d}) and association (kₐ) rate constants and the apparent dissociation equilibrium constant (K_{D}) were calculated with the analysis software (Analyzer, Bruker) using the 1:1 Langmuir binding model.

For 817 anti-LILRB2 antibodies binding affinities to human LILRB2 could be measured. They showed dissociation constants (K_{D}) ranging from 4.8 × 10⁻¹³ M to 1.2 × 10⁻⁷ M. 50 % of the antibodies analyzed had a K_{D} below 0.5 nM.

### 3.4 Humanization and scFv production

The CDRs of the identified rabbit clones were grafted onto Numab's proprietary VH/VL frameworks to produce stable scFvs, as described in the patent applications WO 2022/136675. Different grafting variants were designed and produced.

### 3.5 Manufacture of suitable humanized anti-LILRB2 binding domains for characterization (scFv format)

Humanized scFvs have been produced analogous to the method described in Example 2.5. Production details of the LILRB2 scFvs are summarized in Table 18.

For additional specific characterization of the 68-18-E11 clone, some IgG variants of this clone have been prepared. Production procedure was analogous to that described below for the IgG-based multispecific anti-CD47xPDL1 and anti-CD47xPDL1xLILRB2 antibodies, with the only difference that for PRO3603, no SEC-polishing step was necessary due to high post-capture monomeric purity. For PRO3603, a simple dialysis against the respective final buffer in a dialysis membrane was performed instead. PRO3635 and PRO4710 on the other hand were polished by SEC, as described below. The manufacturing details of produced anti-LILRB2 IgGs are also summarized in Table 18.

**Table 18: Manufacture of anti-LILRB2 scFvs and IgGs**

| **Protein ID** | **Construct ID** | **Final titer [mg/L]** | **Final purity by SE-HPLC [% monomer]** |
|---|---|---|---|
| PRO3744 | 68-18-E11-sc07 | 166.8 | 99.5 |
| PRO3696 | 69-01-E02-sc06 | 655.8 | 99.4 |
| PRO3697 | 69-06-G04-sc05 | 365.6 | 99.2 |
| PRO3603 (IgG1) | 68-18-E11-sc02 | 108 | 99.7 (after capture) |
| PRO3635 (IgG4) | 68-18-E11-sc02 | 116 | 99.7 |
| PRO4710 (IgG4) | 68-18-E11-sc07 | 165 | 97.8 |
| PRO2518 (Reference) | J19.H1 (Jounce) | 130 | 98.9 (after capture) |
| PRO2734 (Reference) | Merck | 146 | 96.4 (after capture) |
| PRO3483 (Reference) | NGM | 77 | 94.7 |

### 3.6 Pharmacodynamic characterization of suitable humanized anti-LILRB2 binding domains (scFv format)

### Binding affinity to LILRB2

Affinities of the selected anti-LILRB2 scFvs to human and cynomolgus LILRB2 were determined by SPR analysis using an SPR 24 system (Sierra Sensor - Bruker). Human or cynomolgus LILRB2 were immobilized on a CMD200M SPR sensor prism chip (Xantec). Then LILRB2 scFvs were injected at concentrations of 10 nM, 2 nM and 0.4 nM on the sensor chip over spots with immobilized human LILRB2. Empty spots were used as references. After each analyte cycle, the surface was regenerated with 3 M MgCl₂ solution, thereby allowing the next analyte cycle to have only free ligand available. Binding affinity K_{D} was globally calculated by the Bruker SPR software based on the binding association (*k*ₒₙ) and dissociation (*k*_{off}) rate, by fitting a 1:1 Langmuir model to the curves obtained by the cycles with the three different concentrations of the analyte. The binding affinities towards human LILRB2 are summarized in Table 19. None of the tested anti-LILRB2 scFvs showed measurable binding to cynomolgus LILRB2.

**Table 19: Affinity of anti-LILRB2 scFvs to human LILRB2.**

| **Protein ID** | **scFv** | **Affinity to human LILRB2** | | |
|---|---|---|---|---|
| | Clone ID | kₐ (M⁻¹ s⁻¹) | k_{d} (s⁻¹) | K_{D} (M) |
| PRO3744 | 44-03-A03 -sc01 | 7.71E+05 | 1.14E-04 | 1.48E-10 |
| PRO3696 | 69-01-E02-sc06 | 7.36E+05 | 1.66E-04 | 2.26E-10 |
| PRO3697 | 69-06-G04-sc05 | 9.74E+05 | 1.28E-04 | 1.31E-10 |

### Binding of anti-LILRB2 scFvs to CHO K1 cells expressing human LILRB2

To test binding of Numab's anti-LILRB2 scFvs PRO3696 and PRO3744 to CHO K1 cells constitutively expressing human LILRB2, a flow cytometry-based assay was developed.

For cell binding experiments, CHO K1 cells expressing human LILRB2 were seeded in a 96-well plate (50,000 cells per well) and 3-fold serial dilutions of the molecules to test were added at concentrations starting at 100 nM to the plates and incubated for 60 min at 4°C on a shaker. Supernatants were removed and detection reagent (Numab's anti-framework antibody conjugated to Alexa Fluor 647, in-house) was added for flow cytometry analysis. The plates were measured with the flow cytometer Novocyte with a flow rate of 120 µl/min and the geometric mean of the fluorescence intensity of single cells was calculated. Obtained MFI values were plotted as function of log-transformed concentrations and ECso values were calculated by using GraphPad prism software.

Table 20 summarises the cell-binding potency data, presenting the ECso and maximum binding values for PRO3696 and PRO3744. The data revealed robust binding of the scFvs PRO3744 and PRO3696 to CHO cells that express human LILRB2, with ECso values in the range of 1 nM.

**Table 20: Binding of anti-LILRB2 scFvs to CHO K1 cells expressing human LILRB2**

| **PRO ID** | **EC₅₀ [nM]** | **Maximum binding (max. MFI sample)** |
|---|---|---|
| PRO3744 | 0.881 | 554'603 |
| PRO3696 | 0.796 | 760'006 |

### Binding of anti-LILRB2 scFvs to different human and cynomolgus PBMC cell types

Binding of the anti-LILRB2 scFv PRO3744 to different PBMC cell types from human as well as from cynomolgus monkeys was examined within the specified subset of T cells, B cells, and myeloid cells. In particular, the ability of anti-LILRB2 scFv PRO3744 to target HLA-DR+ CD14+ monocytes and HLA-DR+ CD14- APCs from human and cynomolgus monkey was investigated. The method used to assess the binding to these cell-types was analogous to the method described in the preceding section.

The results of the cell-binding measurements are summarized in Table 21. Besides binding to human HLA-DR+ CD14+ monocytes and human HLA-DR+ CD14- APCs, no binding to any other human PBMC cell types was observed. Also, no significant binding to cynomolgus monkey PBMC cell types was observed.

### Neutralization of LILRB2 ligand interaction (HLA-G, HLA-A3)

To test the potency of the anti-LILRB2 scFvs to block the binding of known LILRB2 ligands to LILRB2, the binding of HLA-G (complex with human H2AFX, RIIPRHLQL, APC labeled, from Creative Biolabs) and HLA-A3 (HLA-A0301 dextramer, PE labelled, from ImmuDex) to CHO cells expressing human LILRB2 was tested in the presence of the anti LILRB2 scFvs PRO3744, PRO3696 and PRO3697 using flow cytometry.

LILRB2 expressing CHO cells (stably transduced to express human LILRB2 sequence, Q8N423) were seeded in a 96-well plate (50,000 cells per well). 4-fold serial dilutions of the molecules to test including benchmark anti-LILRB2 antibody J-19 analogue (positive control) were added at concentrations ranging from 160 nM to 0.2 pM to the plates and incubated for 60 min at 4°C on a shaker. Supernatants were removed and APC-labelled HLA-G tetramer (from CreativeBiolabs) or HLA-A0301 dextramer labeled with PE (from ImmuDex) were added to the plates at a final concentration of 100 pM (HLA-A0301 dextramer) or at a final dilution of 1:5000 (HLA-G tetramer).
After 30 min incubation at 4°C, cells were washed and 50 µl/well of pre-diluted Cytofix solution (1:10 in ddH₂0, from BD Bioscience) were added for flow cytometry analysis. The plates were measured with the flow cytometer Novocyte with a flow rate of 120 µl/min and the geometric mean of the PE and APC fluorescence intensity of bound LILRB2 ligand, HLA-G or HLA-A3, to single cells was calculated. Obtained MFI values were plotted as function of log-transformed concentrations and ECso values were calculated by using GraphPad prism software.

The potency data for inhibition of LILRB2/HLA-G and LILRB2/HLA-A3 are summarized in Table 22. The IC₅₀ as well as maximum inhibition values for the anti-LILRB2 scFvs PRO3744, PRO3696 and PRO3697 were normalized to the control IgG antibody J-19 (PRO2518), which exhibits high affinity binding to human LILRB2.

Although being monovalent, all tested scFvs, namely PRO3744, PRO3696, and PRO3697, demonstrated potent and comprehensive inhibition of the LILRB2/HLA-G interaction with potencies similar to the bivalent IgG antibody J-19 (PRO2518). Notably, PRO3744 also achieved complete neutralization of the HLA-A3/LILRB2 interaction, while PRO3696 exhibited partial inhibition, and PRO3697 did not impede HLA-A3 binding.

### LILRB2 selectivity assessment

To assess whether the anti-LILRB2 antibodies selectively bind to LILRB2 and not to other members of protein family LILRA and LILRB, a cellular ELISA (CELISA) using transiently transfected CHO cells was developed.

The selectivity assessment was performed as follows. The day before CELISA, CHO cells seeded in a 96-well cell culture plate were transiently transfected with plasmids encoding LILRA1 (identifier: 075019-1), LILRA2 (Q8N149-1), LILRA3 (Q8N6C8-1), LILRA4 (P59901-1), LILRA5 (A6NI73-1), LILRA6 (Q6PI73-1), LILRB1 (Q8NHL6-1), LILRB2 (Q8N423-2), LILRB3 (075022-1), LILRB4 (Q8NHJ6-1) or LILRB5 (075023-1). Next day, dilution series of molecules to test ranging from 100 nM to 6.4 pM were added to the plates of transfected CHO cells and incubated for 1 h at 37°C in the incubator. As a positive control to confirm successful expression of proteins of interest, transfected CHO cells were also stained with an antibody directed against V5 tag (R&D Systems, cat. MAB8926), as all LILRA and LILRB constructs were designed to express V5 tag at the N-terminus of the proteins of interest. Supernatants were removed and detection reagent (rabbit anti-human Fc fragment, HRP-conjugated, from Jackson ImmunoResearch, cat. 309-035-008) was added to the wells. Plates were incubated for 45 min at 37°C in the incubator. Next, cells were washed and 50 µl of TMB substrate was added to each well. HRP reaction was stopped until sufficient color development was achieved by adding 50 µl of stopping solution (1M HCl) to each well. Plates were read by optical density (OD) measurement at 450 nm and at 690 nm as reference wavelength using BioTek Synergy Neo2 multimode reader. Obtained OD values were plotted as function of log-transformed concentrations and ECso values were calculated by using GraphPad prism software.

**Table 21: Binding to human and cyno PBMCs**

| | | **Human (2 donors)** | | | | **Cynomolgus Monkey (2 donors)** | | | |
|---|---|---|---|---|---|---|---|---|---|
| **PRO ID** | **ConcenTration [nM]** | **HLA-DR+ CD14+ [geoMFI × 1000]** | **HLA-DR+ CD14[geoMFI × 1000]** | **B cells (CD20+) [geoMFI × 1000]** | **T cells (CD3+) [geoMFI × 1000]** | **HLA-DR+ CD14+ [geoMFI × 1000]** | **HLA-DR+ CD14[geoMFI × 1000]** | **B cells (CD20+) [geoMFI × 1000]** | **T cells (CD3+) [geoMFI × 1000]** |
| PRO3744 | 1.6 | 16.0 ± 5.1 | 3.5 ± 2.7 | No binding | No binding | 0.38 ± 0.04 | No binding | No binding | No binding |
| | 200 | 21.1 ± 5.8 | 3.6 ± 2.9 | No binding | No binding | 1.12 ± 0.50 | No binding | No binding | No binding |
| PRO3744 x-linked *) | 1.6 | 27.1 ± 10.9 | 5.4 ± 4.5 | No binding | No binding | 0.43 ± 0.16 | No binding | No binding | No binding |
| | 200 | 37.2 ± 12.4 | 7.5 ± 6.3 | No binding | No binding | 7.68 ± 2.79 | No binding | No binding | No binding |

| | | | | | | | | | |
|---|---|---|---|---|---|---|---|---|---|
| *) X-linked: cross linked with an anti-framework antibody | | | | | | | | | |

**Table 22: LILRB2 neutralization potency**

| | **LILRB2 / HLA-G** | | | **LILRB2 / HLA-A3** | | |
|---|---|---|---|---|---|---|
| **PRO ID** | **IC₅₀ [nM]** | **Rel. IC₅₀ (IC₅₀ J-19 / IC₅₀ sample)** | **Rel. max. inhibition % (rel. to J-19)** | **IC₅₀ [nM]** | **Rel. IC₅₀ (IC₅₀ J-19 / IC₅₀ sample)** | **Rel. max. inhibition % (rel. to J-19)** |
| PRO3744 | 0.973 | 0.684 | 99.1 | 0.893 | 0.683 | 96.7 |
| PRO3696 | 1.085 | 0.635 | 98.4 | 0.867 | 0.679 | 76.2 |
| PRO3697 | 0.593 | 1.160 | 99.5 | no inhibition | NA | 9.5 |

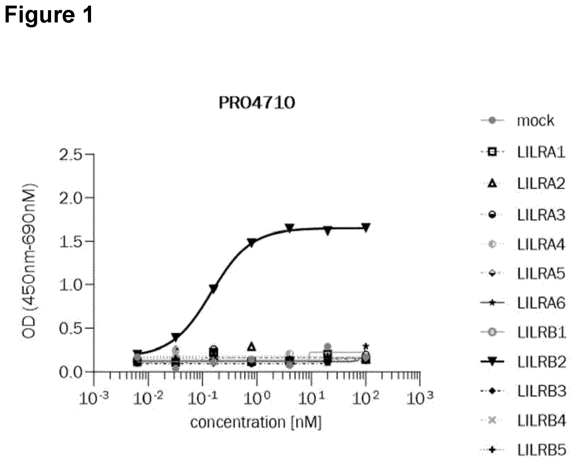

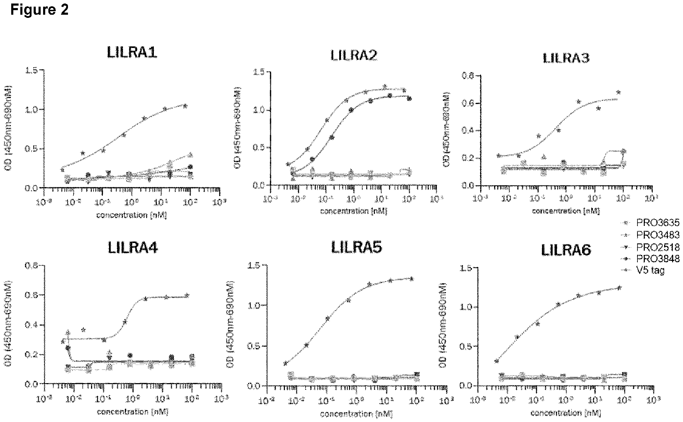

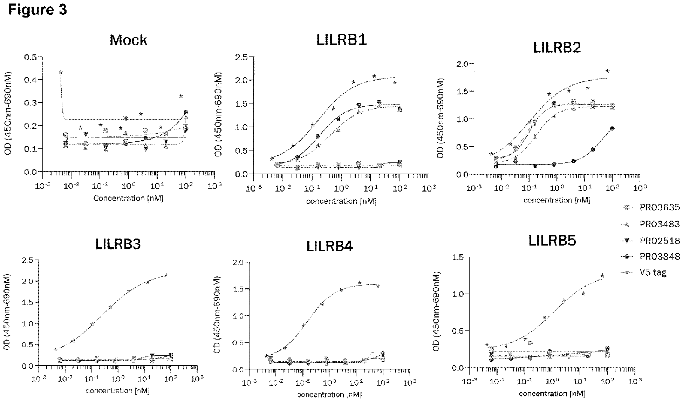

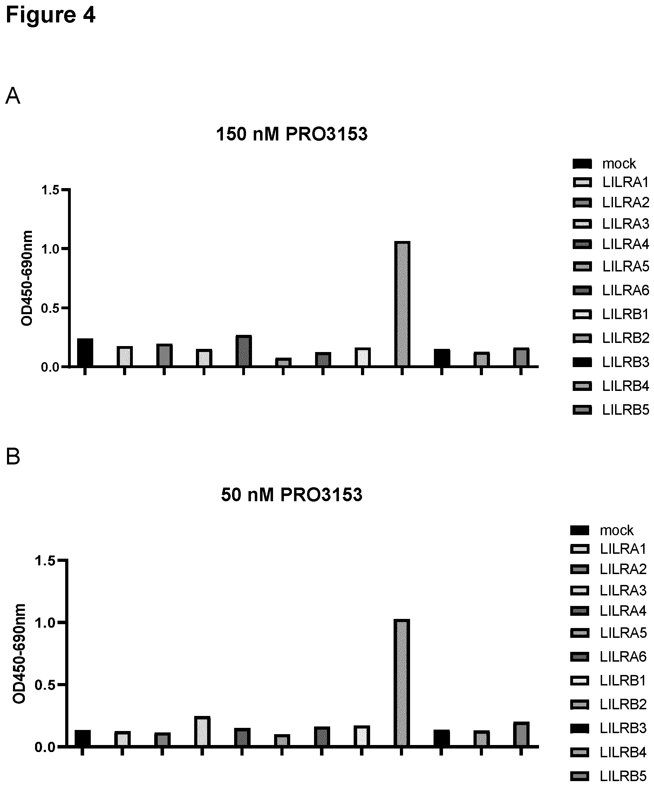

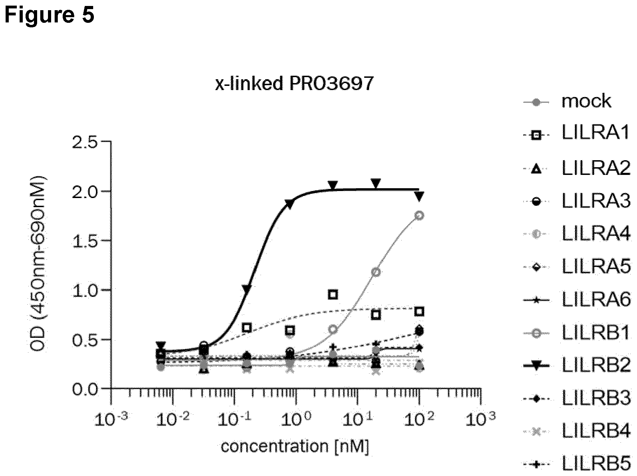

As shown in Figures 1, 2 and 3, PRO4710, the IgG4 variant of scFv PRO3744 (68-18-E11-sc07) as well as PRO3635, the (IgG variant of scFv PRO3064 (68-18-E11-sc02), showed selective binding to LILRB2 while exhibiting no binding to any one of the tested LILRA family members or to LILRB1, LILRB3, LILRB4 or LILRB5. The scFv PRO3153 (69-01-E02-sc03), i. e. a precursor of PRO3696 (69-01-E02-sc06), exhibited the same selectivity for LILRB2 as PRO4710, when tested at a concentration of 150 nM (see Figure 4). On the other hand, cross-linked PRO3697 (69-06-G04-sc05) showed, besides strong binding to LILRB2, weak binding to LILRB1, but no binding to any of the tested LILRBA family members or to LILRB3, LILRB4 or LILRB5, as seen for the other clones (see Figure 5).

### LPS-driven cytokine secretion

To evaluate the effect of the LILRB2-BDs of the invention on the cytokine secretion levels of human monocyte-derived macrophages (HMDM), an LPS-driven cytokine secretion assay was performed as described in the following.

HMDMs were generated by culturing CD14+ cells for 6-8 days in complete medium (RPMI 1640, 10 % heat-inactivated bovine serum, Sigma Aldrich) in the presence of 50 ng/ml human macrophage colony stimulating factor (M-CSF) (Peprotech) at 37°C and 5 % CO₂. Medium was exchanged and M-CSF replenished on day 3 of the culture. 50 000 - 100 000 HMDM cells were seeded in a flat-bottom 96-well plate and exposed to 100 ng/mL LPS in the presence of a serial dilution of PRO3744, PRO3696, and PRO3697. After 24 h of incubation at 37°C and 5 % CO₂, cell culture supernatants were harvested and human interleukin 1β (IL-1β), human interleukin 6 (IL-6) and tumor necrosis cell factor alpha (TNF-α) levels were quantified using the cytometric bead array method (CBA) from LegendPlex kit provided by Biolegend according to kit instructions (cat no.: 740930). Cytokine concentrations were interpolated from a standard curve generated for each cytokine and plotted against control conditions for calculation of EC₅₀ values. Data was acquired on Attune NxT Flow Cytometer (Thermo Fisher Scientific) and NovoCyte Quanteon 4025 Flow Cytometer (Agilent) and analyzed by Biolegend's LegendPLEX Data Analysis Software.

As shown in Table 23, IL-1β, IL-6 and TNF-α levels increased in the dose-dependent manner, indicating the activation of HMDM cells by PRO3744, PRO3696, and PRO3697 in the presence of LPS.

### Re-polarization of M2 macrophages

Further, to evaluate the potency of the LILRB2-BDs of the invention to stimulate repolarization of M2 macrophages to their M1 state, a re-polarization assay has been performed, which follows the level of several surface markers that are indicative for repolarization, such as CD86 (marker for M1 state) and CD163 and CD206 (markers for M2 state). The re-polarization assay was performed as follows.

Human peripheral blood mononuclear cells (PBMCs) were isolated from buffy coats of healthy donors by density gradient centrifugation (STEMCELL Technologies). CD14+ cells were isolated from PBMCs by positive selection with magnetic beads (Miltenyi Biotech, 130-050-201). HMDMs were generated by culturing CD14+ cells for 6-8 days in complete medium (RPMI 1640, 10 % heat-inactivated bovine serum, Sigma Aldrich) in the presence of 50 ng/ml human macrophage colony stimulating factor (M-CSF) (Peprotech) at 37°C and 5 % CO₂. Medium was exchanged and M-CSF replenished on day 3 of the culture.

HMDM cells were exposed to 25 ng/mL IL-4 in the presence of a serial dilution of PRO3744, PRO3696, and PRO3697 at 37°C and 5 % CO₂. After 24 h, flow cytometry assessment for the surface markers CD86, CD163 and CD206 was performed.

HMDMs were harvested from culturing flasks on day 6-8 using Accutase (Biowest), seeded at 50'000 cells per well in 96-well flat bottom plates (TPP) and treated with 25 ng/mL IL-4 (Peprotech, 200-04 - 50 µg) together with a serial dilution of test compounds or isotype antibodies (dose range 100 nM - 0.0006 nM) for 24 h at 37°C and 5 % CO2. After 24 h, cells were stained with Zombie Aqua Fixable Viability Kit (BioLegend) for 30 min at 4°C. Cells were incubated with the Fc receptor block (Human TruStain FcX, BioLegend, 422302) for 10 min at room temperature, to prevent nonspecific binding of the staining antibodies. Staining antibodies (all from BioLegend) were added to the cells for 30 min at 4°C: CD163-APC (cat. no. 333610), CD206-BV711 (cat. no. 321136), CD86-AF488 (cat. no. 305414), CD80-PE (cat. no. 305208) and CD14-PerCp-Cy5.5 (cat. no. 325622). Following washing step to remove primary antibodies, samples were fixed with a BD CellFix kit (BD, 340181): Cells were incubated in 50 µL of fixing solution for 20-30 min at 4°C in the dark. Samples were washed, resuspended in PBS and acquired with Attune NxT Flow Cytometer (Thermo Fisher Scientific). Data was analysed with the FlowJo software (Tree Star).

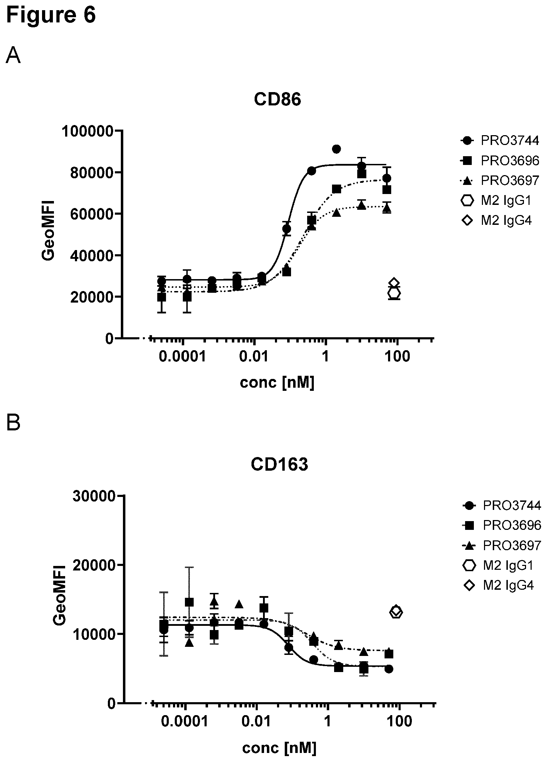

Figure 6 and Table 23, summarize the results obtained for CD86, CD163 and CD206 levels, i. e. markers associated with M1 (pro-inflammatory) and M2 (anti-inflammatory) macrophage polarization, in the presence of the anti-LILRB2 scFvs PRO3744, PRO3696, and PRO3697. For all anti-LILRB2-BDs, a dose dependent increase in the M1 marker CD86 and dose dependent decrease in the M2 markers CD163 and CD206 could be observed. These results indicate a shift of the macrophages towards M1 state (re-polarization), potentially suggesting an enhanced immune response.

**Table 23: LPS-driven cytokine secretion and repolarization of M2 macrophages**

| | **LPS-driven cytokine secretion** | | | **Re-polarization** | | |
|---|---|---|---|---|---|---|
| **PRO ID** | **IL-1β EC₅₀ [nM]** | **TNF-α EC₅₀ [nM]** | **IL-6 EC₅₀ [nM]** | **CD86 EC₅₀ [nM]** | **CD163 EC₅₀ [nM]** | **CD206 EC₅₀ [nM]** |
| PRO3744 | 0.7788 | 1.1031 | 0.8035 | 0.1171 | 0.1070 | 0.1361 |
| PRO3696 | 0.4462 | 0.8912 | 0.8036 | 0.2433 | 0.3715 | 0.5867 |
| PRO3697 | 0.2788 | 2.2044 | 0.4500 | 0.2140 | 0.1726 | 0.5117 |

### 3.7 Biophysical characterization of suitable anti-LILRB2 binding domains (scFv format)

### Storage stability study

Humanized scFvs were subjected to stability studies, such as a four-week stability study, in which the scFvs were formulated in an aqueous buffer (50 mM NaCiP, 150 mM NaCl, pH 6.4) at 10 mg/ml and stored at < -80°C, 4°C and 40°C for four weeks. At the minimum, the fractions of monomers and oligomers in the formulation were evaluated by integration of SE-HPLC peak areas after two weeks and at the end of each study. Additional time points were recorded for most of the molecules. Table 24 (monomer content) and Table 25 (protein content) compare day 14 (d14) and endpoint measurements obtained on day 28 (d28) of the study.

Most molecules exhibited >5 % monomer loss upon storage for 28 days at 4°C. None of the scFvs exhibited considerable protein content loss at any tested storage temperature and time point. Most pronounced differences in monomeric content between molecules were observed in samples stored for 14/28 days at 40°C.

### Thermal unfolding

Thermal stability of scFvs was analyzed by nano dynamic scanning fluorimetry (nDSF) using a NanoTemper, allowing the determination of the onset of unfolding (Tonset), midpoint of unfolding (Tm). Tm and Tonset results of duplicate/triplicate measurements are summarized in Table 26. As can be deduced from Table 26, all molecules exhibit high melting temperatures of at least 59°C, most of the molecules exhibit melting temperatures of >60°C or >70°C.

**Table 24: Course of monomeric content and monomeric content loss upon sample storage at a concentration of 10 g/L and temperatures of -80°C, 4°C and 40°C for up to 28 days.**

| **PRO ID** | **Description** | **Day 0** | **Day 14 (MC -80°C)** | **Day 28 (MC -80°C)** | **Day 14 (MC 4°C)** | **Day 28 (MC 4°C)** | **Day 14 (MC 40°C)** | **Day 28 (MC 40°C)** | **Day 14 (MC% -80°C)** | **Day 28 (MC% -80°C)** | **Day 14 (MC% 4°C)** | **Day 28 (MC% 4°C)** | **Day 14 (MC% 40°C)** | **Day 28 (MC% 40°C)** |
|---|---|---|---|---|---|---|---|---|---|---|---|---|---|---|
| PRO3744 | 68-18-E11-sc07 | 98.9 | n.a. | 99.9 | 99.9 | 99.9 | 99.8 | 98.5 | n.a. | -1.0 | -1.0 | -1.0 | -0.9 | 0.4 |
| PRO3697 | 69-06-G04-sc05 | 98.1 | n.a. | 97.1 | 93.2 | 92.2 | 92.3 | 91.8 | n.a. | 1.0 | 5.0 | 6.0 | 5.9 | 6.5 |
| PRO3696 | 69-01-E02-sc06 | 99.8 | n.a. | 99.0 | 98.0 | 98.9 | 97.6 | 95.9 | n.a. | 0.9 | 1.8 | 0.9 | 2.2 | 3.9 |

**Table 25: Course of protein content and protein content loss upon sample storage at a concentration of 10 g/L and temperatures of -80°C, 4°C and 40°C for up to 28 days.**

| **PRO ID** | **Description** | **Day 0 (PC)** | **Day 14 (PC -80°C)** | **Day 28 (PC -80°C)** | **Day 14 (PC 4°C)** | **Day 28 (PC 4°C)** | **Day 14 (PC 40°C)** | **Day 28 (PC 40°C)** | **Day 14 (PC% -80°C)** | **Day 28 (PC% -80°C)** | **Day 14 (PC% 4°C)** | **Day 28 (PC% 4°C)** | **Day 14 (PC% 40°C)** | **Day 28 (PC% 40°C)** |
|---|---|---|---|---|---|---|---|---|---|---|---|---|---|---|
| PRO3744 | 68-18-E11-sc07 | 9.9 | n.a. | 9.9 | 9.8 | 9.9 | 10.1 | 10.2 | n.a. | 0.3 | 0.9 | 0.4 | -1.7 | -3.5 |
| PRO3697 | 69-06-G04-sc05 | 10.5 | n.a. | 10.7 | 10.3 | 10.7 | 10.5 | 11.0 | n.a. | -1.8 | 1.4 | -1.9 | -0.1 | -4.5 |
| PRO3696 | 69-01-E02-sc06 | 10.5 | n.a. | 11.0 | 10.6 | 10.6 | 10.8 | 10.8 | n.a. | -4.4 | -1.2 | -0.9 | -2.4 | -2.7 |

**Table 26: nDSF measurement of scFvs using NanoTemper device for determination of Tm and Tonset.**

| **PROID** | **Description** | **Tₒₙₛₑₜ [°C]** | **Tₘ [°C]** |
|---|---|---|---|
| PRO3744 | 68-18-E11-sc07 | 64.1 | 74.6 |
| PRO3697 | 69-06-G04-sc05 | 57.9 | 65.5 |
| PRO3696 | 69-01-E02-sc06 | 53.2 | 61.1 |

| | | | |
|---|---|---|---|
| PC: protein content [mg/mL] MC: monomer content [%] | | | |

### Example 4: Manufacture and characterization of anti-CD47 × PDL1 bispecific antibodies:

### 4.1 Manufacture of anti-CD47 × PDL1 bispecific antibodies

Manufacture anti-CD47 × PDL1 bispecific antibodies was performed as follows. Expression of multispecific constructs was performed in ExpiCHO-S cells using the transient ExpiCHO expression system (Gibco). Expression cultures were cultivated in batch using shaking flasks for 8 days at 37°C. The culture supernatants were separated from cells by centrifugation followed by a 0.22 µm sterile filtration.

Target proteins were captured from the clarified culture supernatants by protein A affinity chromatography and polished by a size-exclusion chromatography (SEC) step, except for the reference molecule PRO3851 (Innovent), which was polished by a cation-exchange chromatography (CIEX) step. For some molecules, no post-capture polishing step was necessary.

For the quality control of the manufactured material standard analytical methods such as SE-HPLC, SDS-PAGE, and UV₂₈₀ were used. Manufacturing details of anti-CD47 × PDL1 bispecific antibodies according to the invention as well as of reference anti-CD47 × PDL1 bispecific antibodies are summarized in **Table 27.** The reference PRO3609 has an (scFv)₂-Fc antibody format, which is a format according to the invention, but comprises an CD47-BD that has an intermediate (higher) binding affinity.

**Table 27: Manufacture of anti-CD47 × PDL1 bispecific antibodies according to the invention and reference constructs**

| **PRO ID** | **Description** | | **Post-capture titer [mg/L]*** | **Post-SEC monomeric purity [%]** |
|---|---|---|---|---|
| | **PD-L1 binder** | **CD47 binder** | | |
| **PRO3790** | 37-20-B03-sc09.1 | 70-26-B04-sc03 | 84.3 | 100.0 |
| **PRO3609 (Reference)** | 37-20-B03-sc09.1 | 70-36-C05-sc02 (intermediate affinity) | 245 | 98.6 |
| **PRO3599 (Reference)** | CD47xPDL1 (Pfizer) | | 145 | 99.8 |
| **PRO3851 (Reference)** | CD47xPDL1 (Innovent) | | 442 | 97.3 |

### 4.2 Pharmacodynamic characterization of suitable anti-CD47 binding domains

### Binding affinity to human CD47 and PDL1

Affinity of PRO3790 to human CD47 and human PDL1 was determined by SPR analysis as described in Examples 2.6 and 3.6. The results are shown in Table 28.

**Table 28: Affinity of PRO3790 to human CD47 and human PDL1.**

| **Protein ID** | **Target** | **Affinity** | | |
|---|---|---|---|---|
| | | kₐ (M⁻¹ s⁻¹) | k_{d} (s⁻¹) | K_{D} (M) |
| PRO3790 | human CD47 | 6.72E+05 | 2.35E-02 | 3.50E-08 |
| | human PDL1 | 5.52E+05 | 4.19E-05 | 7.59E-11 |

### Binding to cancer cell lines

To test binding of PRO3790 and reference compounds PRO3599 (anti-CD47xPDL1 AB from Pfizer) and PRO2777 (magrolimab; hu5F9 clone; IgG4) to plasma membranous CD47, cancer cells expressing human CD47 and PD-L1 were tested in flow cytometry.

50,000 cells per well of cancer cell line HT-1080 (from ATCC, cat. CCL-121, double positive for CD47 and PD-L1) or HT-29 (from ATCC, cat. HTB-38, positive for CD47) were seeded in a 96-well plate. Threefold serial dilutions of the molecules to test were added at concentrations ranging from 33 nM to 0.6 pM (for PRO2777 on HT-29 and HT-1080 and for PRO3790 and PRO3599 on HT-1080) or from 500 nM to 8.5 pM (PRO3790 and PRO3599 on HT-29 cells) to the plates and incubated for 60 min at 4°C with shaking. Supernatants were removed and detection reagent (goat anti-human IgG, PE conjugated, from Jackson ImmunoResearch, cat. 109-116-170) was added for flow cytometry analysis. The plates were measured with the flow cytometer Novocyte with a flow rate of 120 µl/min and the geometric mean of the PE fluorescence intensity of single cells was calculated.

The cell binding potency data are summarized in Table 29. The ECso as well as maximum binding values for PRO3790 and PRO3599 were normalized to the control antibody PRO2777, which exhibits high affinity binding to human CD47.

Both PRO3790 and PRO3599 showed potent binding to PD-L1 co-expressing HT-1080 cells, and weak binding to HT-29 cells which do not express PD-L1. High affinity anti-CD47 molecule PRO2777 showed a PD-L1-independent highly potent binding to both cancer cell lines tested.

### Neutralization of CD47/SIRPα interaction

To test the potency of PRO3790, PRO3599 and PRO2777 to neutralize CD47/SIRPα interaction, binding of SIRPα ligand to cancer cells expressing human CD47 and PD-L1 in presence of molecules of interest was tested in flow cytometry.

50,000 cells per well of cancer cell line NCI-H292 (from ATCC, cat. CRL-1848, double positive for human CD47 and PD-L1) or CHO CD47 (in-house cell line, positive for human CD47) were seeded in a 96-well plate. Threefold serial dilutions of the molecules to test were added at concentrations ranging from 100 nM to 2 pM (PRO2777 on NCI-H292 and CHO CD47), from 300 nM to 5 pM (for PRO3790 and PRO3599 on NCI-H292) or from 500 nM to 9 pM (for PRO3790 and PRO3599 on CHO CD47) to the plates and incubated for 60 min at 4°C on a shaker. Supernatants were removed and biotinylated SIRPα (from Sino Biological, cat. 11612-H02H1, in-house biotinylated) was added to the plates at a final concentration of 0.5 µg/ml. After 30 min incubation at 4°C, cells were washed and detection reagent (streptavidin, PE conjugated, from BioLegend, cat. 405204) was added for flow cytometry analysis. The plates were measured with the flow cytometer Novocyte with a flow rate of 120 µl/min, and the geometric mean of the PE fluorescence intensity of bound SIRPα to single cells was calculated.

The potency data for inhibition of CD47/SIRPα are summarized in Table 30. The IC₅₀ as well as maximum inhibition values for PRO3790 and PRO3599 were normalized to the control antibody PRO2777, which exhibits high affinity binding to human CD47.

PRO3790 and PRO3599 showed potent inhibition of CD47/SIRPα interaction to PD-L1 co-expressing NCI-H292 cells, and weak inhibition of SIRPα binding to CHO CD47 cells which do not express PD-L1. PRO3790 showed a better potency to inhibit SIRPα binding to NCI-H292 cells than the Pfizer molecule (PRO3599), while the potency was comparable for the two molecules when CHO CD47 cells were used. High affinity anti-CD47 molecule PRO2777 demonstrated potent inhibition of SIRPα binding to both cell lines tested.

**Table 29: Binding to cancer cell lines**

| | | | **HT-1080 cells** | | **HT-29 cells** | |
|---|---|---|---|---|---|---|
| **PRO ID** | **PD-L1 binder** | **CD47 binder** | **EC₅₀ [nM]** | **Rel. maximum binding (max. MFI sample / max. MFI magrolimab)** | **EC₅₀ [nM]** | **Rel. maximum binding (max. MFI sample / max. MFI magrolimab)** |
| PRO2777 | n.a. | magrolimab | 0.271 | 1 | 0.2697 | 1 |
| PRO3790 | 37-20-B03-sc09.1 | 70-26-B04-sc03 | 0.150 | 0.45 | > 100 | 0.57 (at 200 nM) |
| PRO3599 | Pfizer | Pfizer | 0.293 | 0.42 | > 100 | 0.59 (at 200 nM) |

**Table 30: Neutralization of CD47/SIRPα interaction**

| | | | **NCI-H292 cells** | | **CHO CD47 cells** | |
|---|---|---|---|---|---|---|
| **PRO ID** | **PD-L1 binder** | **CD47 binder** | **EC₅₀ [nM]** | **Rel. maximum binding (max. MFI sample / max. MFI magrolimab)** | **EC₅₀ [nM]** | **Rel. maximum binding (max. MFI sample / max. MFI magrolimab)** |
| PRO2777 | n.a. | magrolimab | 0.072 | 100 | 0.066 | 100.00 |
| PRO3790 | 37-20-B03-sc09.1 | 70-26-B04-sc03 | 0.068 | 92.5 | 109.8 | 87.0 |
| PRO3599 | Pfizer | Pfizer | 0.234 | 85.9 | 149.9 | 72.1 |

### Cell binding to RBCs

To test the binding of PRO3790 and PRO2777 to human red blood cells (RBCs), a flow cytometry-based assay was used.

Human RBCs were isolated from peripheral blood of healthy donors. Human RBCs were seeded in a 96-well plate and 3-fold serial dilutions of the molecules to test were added at concentrations ranging from 100 nM to 1.7 pM (for PRO2777) or from 500 nM to 8.5 pM (PRO3790) to the plates and incubated for 60 min at 4°C with shaking. Supernatants were removed and detection reagent (goat anti-human IgG, PE conjugated, from Jackson ImmunoResearch, cat. 109-116-170) was added for flow cytometry analysis. The plates were measured with the flow cytometer Novocyte with a flow rate of 120 µl/min and the geometric mean of the PE fluorescence intensity of single cells was calculated.

The cell-binding potency data are summarized in Table 31. The ECso as well as maximum binding values for PRO3790 were normalized to the control antibody PRO2777, which exhibits high affinity binding to human CD47.

PRO3790 exhibited a remarkably lower binding potency than the magrolimab analog (PRO2777) to human erythrocytes as indicated by a calculated rel. ECso of 0.003 fold.

**Table 31: Cell binding to RBC & hemagglutination**

| | | | **Human erythrocytes** | | |
|---|---|---|---|---|---|
| **PRO ID** | **PD-L1 binder** | **CD47 binder** | **EC₅₀ [nM]** | **Rel. EC₅₀ (EC₅₀ magrolimab / EC₅₀ sample)** | **Rel. maximum binding (max. MFI sample / max. MFI magrolimab)** |
| PRO2777 | n.a. | magrolimab | 0.325 | 1 | 1 |
| PRO3790 | 37-20-B03-sc09.1 | 70-26-B04-sc03 | 98.76 | 0.003 | 0.18 |

### Hemagglutination

To test whether PRO3790 triggers agglutination of human erythrocytes, a hemagglutination assay based on optical density measurement was performed.

The hemagglutination assay was performed as follows. Human erythrocytes were isolated from peripheral blood of healthy volunteer and 1 mio. human erythrocytes were seeded in a 96-well plate. Threefold serial dilutions of the molecules to test were added at concentrations ranging from 100 nM to 1.7 pM (for PRO2777) or from 500 nM to 8.5 pM (PRO3790) to the plates and incubated for 120 min at 37°C and afterwards at 4°C overnight. The optical density (OD) of each well was measured at 800 nm using BioTek Synergy Neo2 multi-plate reader. Obtained OD values were plotted as function of log-transformed concentrations and ECso values were calculated by using GraphPad prism software.

The data of hemagglutination assay are summarized in Table 32. The ECso as well as maximum OD values for PRO3790 were normalized to the control antibody PRO2777, which exhibits high affinity binding to human CD47. PRO3790 showed no sign of hemagglutination at all tested concentrations (highest concentration was 500 nM), while the magrolimab analog (PRO2777) was able to trigger agglutination of human erythrocytes with EC₅₀ value of 0.182 nM.

**Table 32: Hemagglutination**

| | | | **Hemagglutination assay** | | |
|---|---|---|---|---|---|
| **PRO ID** | **PD-L1 binder** | **CD47 binder** | **EC₅₀ [nM]** | **Rel. EC₅₀ (EC₅₀ magrolimab / EC₅₀ sample)** | **Rel. maximum hemagglutination (OD800nm sample / OD800nm magrolimab)** |
| PRO2777 | n.a. | magrolimab | 0.182 | 1 | 1 |
| PRO3790 | 37-20-B03-sc09.1 | 70-26-B04-sc03 | No hemagglutination observed at 500 nM | | |

### PD-1 / PD-L1 neutralization assay

The potency to inhibit PD-1 / PD-L1 interaction was assessed in a cell-based reporter gene assay employing human PD-1-expressing Jurkat NFAT reporter cells. In this assay, CHO cells stably expressing PD-L1 and a T cell receptor (TCR) activator were incubated with Jurkat T cells stably expressing PD-1 and as a reporter gene to monitor T cell activation, firefly luciferase under the control of the NFAT response elements (target and effector cells both from BPS Bioscience). Upon binding of the TCR activator on CHO cells to the Jurkat T cells, TCR signaling triggers NFAT-induced expression of firefly luciferase. The interaction between PD-L1 and PD-1 negatively regulates TCR signaling and decreases firefly luciferase expression. Consequently, blockade of the PD-L1/PD-1 interaction in this system restores luciferase activity.

35'000 CHO/PD-L1/TCR activator cells (BPS Bioscience) in 100 µl of cell culture medium (DMEM/F12, 10 % FCS) were added to the inner wells of a white cell culture plate and incubated for 16-20 h at 37°C and 5 % CO₂. On the next day, 95 µl of cell culture medium was removed from each well and 50 µl of 2-fold concentrated 3-fold serial dilutions of the multispecific antibody test compounds comprising a PD-L1-BD and of reference antibody avelumab ranging from 50 nM to 8 pM (final concentration in the well) were added. 50 µl of effector Jurkat cells (BPS Bioscience) diluted at 400'000 cells/ml in assay buffer (RPMI1640 with 10 % FCS) were added to each well, and plates were incubated for 6 h at 37°C and 5 % CO₂. Finally, 50 µL luciferase substrate (BPS Bioscience) prepared according to manufacturer's protocol, were added per well, and plates were incubated 30 min in the dark, whereafter luminescence was measured using microplate reader BioTek Synergy Neo2. Concentration-response curves were fitted by using a four-parameter logistic fit (GraphPad prism software) and the half-maximal effective concentration (EC₅₀) was calculated. For comparison of multispecific antibody test compounds from different assay plates, relative EC₅₀ values were used. These values were determined by calibrating the EC₅₀ value of the molecules against the reference molecule avelumab included on each assay plate (relative EC₅₀: EC₅₀, avelumab / EC₅₀, test). In addition, the maximum activation of each multispecific antibody test compound was calculated and normalized to the maximum activation seen for avelumab (relative maximum activation: maximum activation, test / maximum activation, avelumab).

PRO3790 showed potent blockade of PD-1 / PD-L1 interaction in transgenic Jurkat NFAT reporter gene assay, comparable to benchmark antibody avelumab and reference molecule PRO3599 (Table 33).

**Table 33: PD-L1 / PD-1 transgenic NFAT reporter gene assay**

| **PRO ID** | **PD-L1 binder** | **CD47 binder** | **EC₅₀ [nM]** | **Rel. EC₅₀ (EC₅₀ avelumab / EC₅₀ sample)** | **Rel. maximum activation (max. activation sample / max. activation aveluamb)** |
|---|---|---|---|---|---|
| - | avelumab | - | 0.5070 | 1.000 | 1 |
| PRO3599 | WO 2021124073 | WO 2021124073 | 0.5254 | 0.965 | 0.97 |
| PRO3790 | 37-20-B03-sc09.1 | 70-26-B04-sc03 | 0.2720 | 1.864 | 0.95 |

### Phagocytosis assay

Human peripheral blood mononuclear cells (PBMCs) were isolated from buffy coats of healthy donors by density gradient centrifugation (STEMCELL Technologies). CD14+ cells were isolated from PBMCs by positive selection with magnetic beads (Miltenyi Biotech, 130-050-201). HMDMs were generated by culturing CD14+ cells for 6-8 days in complete medium (RPMI 1640, 10 % heat-inactivated bovine serum, Sigma Aldrich) in the presence of 50 ng/ml human macrophage colony stimulating factor (M-CSF) (Peprotech) at 37°C and 5 % CO₂. Medium was exchanged and M-CSF replenished on day 3 of the culture.

HMDMs were harvested from culturing flasks on day 6-8 using Accutase (Biowest), seeded at 50'000 per well in 96-well flat bottom plates (TPP) and treated with 10 ng/ml IL-10 (Peprotech) overnight. The next day HMDMs were co-incubated with CFSE-labelled target cells at an effector:target ratio of 1:1 for 2.5 h at 37°C in the presence of a serial dilution of test compounds (dose range 200 nM - 0.02 pM). In the case of RBCs, the effector:target ratio was 1:50. At the end of the incubation period, cells were stained with a viability marker (Zombie NIR, Biolegend) and an anti-CD14-APC staining antibody (Biolegend). Samples were fixed and acquired by flow cytometry (Attune NXT, Thermo Fisher Scientific). Phagocytic activity was determined as double positive events (CD14+ HMDMs that have taken up CFSE positive target cells) and phagocytosis is presented as % CFSE+ macrophages.

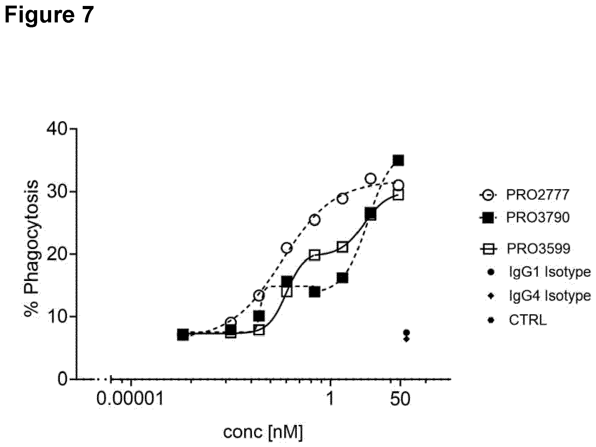

Figure 7 shows effective phagocytosis of tumors with bi-specific compounds necessitates the inhibition of both PD-L1 and CD47. In the case of CD47 × PD-L1 bi-specific compounds, the phagocytosis curve exhibits a bi-phasic pattern, in contrast to magrolimab, which demonstrated a straightforward dose-dependent phagocytosis. The initial part of the curve depends on PD-L1 inhibition, whereas the second part is driven by CD47 blockade. Given that the PD-L1 inhibitor is the same across all three tested molecules, the EC₅₀ for this phase remains comparable.

Phagocytosis of tumor cell by PD-L1 × CD47 bi-specific antibodies is more pronounced with high affinity CD47 binder (PRO3791) when compared to those with low affinity CD47 moieties (PRO3790 and PRO3802). This is reflected by EC₅₀ of the second part of the curve, highlighting the variation between compounds featuring high and low CD47 binders.

### Example 5: Anti-CD47 × PDL1x LILRB2 trispecific antibodies

### 5.1 Manufacture of anti-CD47 × PDL1x LILRB2 trispecific antibodies

The trispecific antibodies according to the present invention have been produced analogous to the methods described in Example 4.1. Briefly, expression of multispecific constructs was performed in ExpiCHO-S cells using the transient ExpiCHO expression system (Gibco). Expression cultures were cultivated in batch using shaking flasks for 8 days at 37°C. The culture supernatants were separated from cells by centrifugation followed by a 0.22 µm sterile filtration. All target proteins were captured from the clarified culture supernatants by protein A affinity chromatography and were polished by a size-exclusion chromatography (SEC) step, except for PRO3851, which was polished by cation-exchange chromatography (ClEX). Some target proteins with a high post-capture monomeric purity (i.e. PRO2518, PRO3483, PRO3603) were not SEC-polished and, instead, dialyzed against the respective final buffer in a dialysis membrane. For the quality control of the manufactured material, standard analytical methods such as SE-HPLC, SDS-PAGE, and UV280 were used. Table 34 shows a production overview of these molecules. All molecules were finalized to a monomeric content ≥95 % after polishing or dialysis, respectively.

**Table 34: Description of produced anti-CD47 × PDL1x LILRB2/hSA trispecific antibodies and trispecific reference molecules**

| | **Identifier** | **PD-L1 binder** | **CD47 binder** | **LILRB2 binder** | **Post-capture titer [mg/L]*** | **Post-SEC monomeric purity [%]** |
|---|---|---|---|---|---|---|
| **anti-PDL1 × CD47 × LILRB2** | | | | | | |
| | **PRO5052** | 33-03-G02-sc24 | 70-26-B04-sc03 | 68-18-E11-sc07 | 290 | 97.7 |
| | **PRO5053** | 33-03-G02-sc24 | 70-26-B04-sc03 | HSA dummy domain | 274 | 97.2 |
| | **PRO5055** | 33-03-G02-sc24 | 70-26-B04-sc03 | 68-18-E11-sc07 | 143 | 97.6 |
| | **PRO5056** | 33-03-G02-sc24 | 70-26-B04-sc03 | HSA dummy domain | 179 | 98.8 |
| | **PRO4672** | 37-20-B03-sc18 | 70-26-B04-sc03 | 68-18-E11-sc07 | 278 | 99.1 |
| | **PRO4673** | 37-20-B03-sc18 | 70-26-B04-sc03 | HSA dummy domain | 163 | 98.3 |
| | **PRO4687** | 37-20-B03-sc18 | 70-26-B04-sc03 | 68-18-E11-sc07 | 148 | 99.2 |
| | **PRO4688** | 37-20-B03-sc18 | 70-26-B04-sc03 | HSA dummy domain | 108 | 98.7 |

| **Reference molecules** | | | | | | |
|---|---|---|---|---|---|---|
| CD47-BD in Fab | **PRO4682** | 37-20-B03-sc18 | 70-26-B04-sc03 | 68-18-E11-sc07 | 220 | 99.2 |
| CD47-BD in Fab | **PRO4683** | 37-20-B03-sc18 | 70-26-B04-sc03 | HSA dummy domain | 118 | 98.4 |
| Inverse Morrison-H | **PRO4677** | 37-20-B03-sc18 | 70-26-B04-sc03 | 68-18-E11-sc07 | 170 | 98.9 |
| Inverse Morrison-H | **PRO4678** | 37-20-B03-sc18 | 70-26-B04-sc03 | HSA dummy domain | 32 | 99.7 |
| No CD47-BD | **PRO5054** | 33-03-G02-sc24 | HSA dummy domain | 68-18-E11-sc07 | 346 | 97.4 |
| No CD47-BD | **PRO5057** | 33-03-G02-sc24 | HSA dummy domain | 68-18-E11-sc07 | 434 | 95.0 |
| Prior art CD47-BD | **PRO4711** | 37-20-B03-sc18 | Pfizer | 68-18-E11-sc02 | 73 | 99.3 |
| Prior art CD47- and PDL1-BD | **PRO3843** | Pfizer | Pfizer | 68-18-E11-sc02 | 186 | 98.7 |
| CrossmAB | **PRO4914** | 37-20-B03-sc18 | 70-26-B04-sc03 | 68-18-E11-sc02 | 246 | 97.5 |
| CD47-BD intermediate affinity | **PRO3613** | 37-20-B03-sc09.1 | 70-36-C05-sc02 (Int. CD47) | 68-18-E11-sc02 | 138 | 100.0 |
| Fc silenced | **PRO5423** | 33-03-G02-sc24 | 70-26-B04-sc03 | 68-18-E11-sc07 | 258 | 97.2 |
| Fc silenced | **PRO5134** | 33-03-G02-sc24 | 70-26-B04-sc03 | 68-18-E11-sc07 | 248 | 98.6 |

### 5.2 Biophysical characterization of anti-CD47 × PDL1x LILRB2 trispecific antibodies according to the invention

### Storage stability study

PRO4672, PRO5052, PRO4687 and PRO5055 were subjected to stability studies such as a four-week stability study, in which the scFvs were formulated in an aqueous buffer (20 mM histidine, 125 mM NaCl, 0.02 % Poloxamer 188, pH 6.0) at 10 mg/ml and stored at 4°C, 25°C and 40°C for 2 weeks, 4 weeks and up to 8 weeks. At the minimum, the fractions of monomers and oligomers in the formulation were evaluated by integration of SE-HPLC peak areas after two weeks, four weeks and at the end of each study. Table 35 summarizes stability results obtained at t2w (2 weeks), t4w (4 weeks) and endpoint measurements obtained at t8w (8 weeks) of the study.

As shown in Table 35, PRO4672, PRO5052, PRO4687 and PRO5055 showed good storage stability at 4°C, 25°C and 40°C. The loss of monomeric content after storage at 25°C for 4 weeks or at 4°C for 8 weeks was less than 1 %. More surprisingly, the loss of monomeric content after storage at 40°C for 4 weeks was still less than 5 %. None of the stable molecules showed considerable loss of protein content at any temperature.

### Freeze-thaw stability

As in the early development stages the drug substance is usually stored frozen, formulation and fill/finish processing will require some freezing and thawing operations. The compatibility of the top performing trispecific molecules with respect to freeze-thawing (F/T) cycles (colloidal stability) was assessed.

For the F/T stability assessment the same analytical methods (SE-HPLC, cGE) and parameters (% monomer content and % monomer loss) as for the storage stability study were applied to monitor the quality of the molecules over five F/T cycles. Table 35 illustrates that after five F/T cycles (F/T) the loss of monomer content was less than 1 %. None of the stable molecules showed considerable loss of protein content at any temperature (data not shown).

**Table 35: Stability studies**

| PRO-ID | t0 | Monom er conten t by SE-HPLC [rel. %] | | | | | | Δ Mono mer con tent by SE-HPLC [rel. %] | | | | | | nDSF | | |
|---|---|---|---|---|---|---|---|---|---|---|---|---|---|---|---|---|
| | | FIT | 40°C | | 25°C | 4°C | | F/T | 40°C | | 25°C | 4°C | | Ton | Tm1 | Tm2 |
| | | 5 | t2w | t4w | t4w | t4w | t8w | 5 | t2w | t4w | t4w | t4w | t8w | [°C] | [°C] | [°C] |
| PRO4672 | 98.5 | 98.6 | 98.3 | 97.4 | 98.9 | 98.9 | 98.4 | 0.1 | -0.2 | -1.1 | 0.4 | 0.4 | -0.1 | 52.3 | 59.9 | 72.3 |
| PRO5052 | 98.9 | 98.6 | 97.9 | 96.5 | 98.5 | 98.7 | 98.3 | -0.3 | -1.0 | -2.4 | -0.4 | -0.2 | -0.6 | 56.5 | 60.3 | 67.8 |
| PRO4687 | 98.5 | 98.1 | 96.3 | 94.0 | 97.9 | 98.3 | 98.0 | -0.4 | -2.2 | -4.4 | -0.6 | -0.2 | -0.5 | 55.4 | 60.3 | 70.6 |
| PRO5055 | 99.2 | 98.9 | 97.9 | 97.0 | 98.8 | 98.9 | 99.0 | -0.3 | -1.3 | -2.2 | -0.4 | -0.3 | -0.2 | 57.4 | 60.4 | 70.6 |

### 5.3 Pharmacodynamic characterization of anti-CD47 × PDL1 × LILRB2 trispecific antibodies according to the invention

### Binding affinity to human CD47, PDL1 and LILRB2

Affinity of trispecific molecules to human and cynomolgus CD47, and human and cynomolgus PDL1 was determined by SPR analysis as described in Examples 2.6 and 3.6. For affinity measurements to human LILRB2, a different SPR measurement procedure was applied to determine the binding affinity without avidity effects.

Briefly, binding affinity without avidity was determined by using a proprietary anti-framework rabbit IgG (rlgG21-57-20-B11) immobilized to a carboxymethylated dextran surface (CM5 sensorchip; Biacore, Cytiva) to capture the multispecific antibodies according to the present invention on the surface. For each antigen, a titration series of the respective antigen was injected as analyte. After each capture-analyte injection cycle, every flow channel on the sensor chip was regenerated (3 M MgCl₂), and a multispecific antibody according to the present invention was captured again followed by a different concentration of the antigen. The binding kinetics to the human antigens were measured using a multicycle kinetic assay, with eleven analyte concentrations ranging from 0.088 to 90 nM, 1:2 diluted in running buffer (HEPES, 150 mM NaCl, 0.05 % Tween-20, pH 7.5; Bioconcept).

The results are shown in Table 36.

**Table 36: Affinity of different multispecific test constructs to human and cynomolgus CD47, human and cynomolgus PDL1, and human and cynomolgus LILRB2.**

| **Protein ID** | **Target** | **Affinity** | | |
|---|---|---|---|---|
| | | kₐ (M⁻¹ s⁻¹) | k_{d} (s⁻¹) | K_{D} (M) |
| PRO4672 | human CD47 | 4.48E+05 | 2.56E-02 | 5.72E-08 |
| | human PDL1 | 1.05E+06 | 1.26E-04 | 1.20E-10 |
| | human LILRB2 *⁾ | 2.4E+05 | 8.9E-05 | 3.7E-10 |
| PRO4687 | human CD47 | 8.11E+05 | 2.04E-02 | 2.52E-08 |
| | human PDL1 | 1.31E+06 | 1.16E-04 | 8.85E-11 |
| | human LILRB2 *⁾ | 2.6E+05 | 9.3E-05 | 3.6E-10 |
| PRO5052 | human CD47 | 6.6E+05 | 2.2E-02 | 3.4 ±0.2E-08 |
| | cynom. CD47 | | | 3.2 ±0.1E-08 |
| | human PDL1 | 2.3E+06 | 2.9E-05 | 1.3 ±0.4E-11 |
| | cynom. PDL1 | | | 1.2 ±0.2E-11 |
| | human LILRB2 *⁾ | 2.9E+05 | 5.4E-05 | 1.9 ±0.8E-10 |
| | cynom. LILRB2 *⁾ | | | no binding |
| PRO5053 | human CD47 | 5.33E+05 | 2.65E-02 | 4.97E-08 |
| | human PDL1 | 2.05E+06 | 2.72E-05 | 1.33E-11 |
| | human LILRB2 *⁾ | n.a. | n.a. | n.a. |
| PRO5055 | human CD47 | 5.1E+05 | 1.1E-02 | 2.1 ±0.04E-08 |
| | cynom. CD47 | | | 2.1 ±0.1E-08 |
| | human PDL1 | 2.2E+06 | 3.3E-05 | 1.6 ±0.7E-11 |
| | cynom. PDL1 | | | 8.4 ±0.9E-12 |
| | human LILRB2 *⁾ | 2.6E+05 | 9.2E-05 | 3.5E-10 |
| | cynom. LILRB2 *⁾ | | | no binding |
| PRO5056 | human CD47 | 4.39E+05 | 2.49E-02 | 5.66E-08 |
| | human PDL1 | 2.02E+06 | 2.13E-05 | 1.05E-11 |
| | human LILRB2 *⁾ | n.a. | n.a. | n.a. |

| | | | | |
|---|---|---|---|---|
| *⁾ Affinity to human and cynomolgus LILRB2 has been measured without avidity. | | | | |

### Binding of trispecific antibodies to different human and cyno PBMCs cell types

Binding of the multispecific antibodies of the invention to different PBMC cell types from human as well as from cynomolgus monkeys was examined within the specified subset of T cells, B cells, and myeloid cells. In particular, the ability of the anti-CD47 × PDL1 × LILRB2 antibodies to target HLA-DR+ CD14+ monocytes and HLA-DR+ CD14- APCs, both from human and cynomolgus monkey, was investigated. The method used to assess the binding to these cell-types was analogous to the method described in the preceding section. The results are summarized in Table 37. Binding is detected by an increase in fluorescence signal indicated in [GeoMFI × 1000].

### Re-polarization of M2 macrophages and LPS-driven cytokine secretion

The ability of the anti-CD47 × PDL1 × LILRB2 antibodies to re-polarize M2 macrophages and to foster LPS-driven cytokine secretion was assessed using the methods disclosed in Example 3.6.

It was observed that the anti-CD47 × PDL1 × LILRB2 antibodies of the invention could inhibit LILRB2, causing a dosage-dependent rise in the M1 marker CD86 and a decline in the M2 markers CD163 and CD206, with a similar potency to that of the monospecific anti-LILRB2 IgG4 PRO4710 (see Table 38). On the other hand, the reference anti-PD-L1 × CD47 bi-specific antibodies from Pfizer (PRO3599), the LILRB2-BD-devoid trispecific variants PRO5053 and PRO5056 (HSA-BD as LILRB2 mock binding domain), as well as the anti-CD47 antibody magrolimab and the anti-PDL1 antibody atezolizumab (tested at a 50 nM dose), showed no re-polarization activity. Results of the cytokine secretion are summarized in Table 39.

**Table 38: Re-polarization potency of M2 macrophages for the anti-LILRB2 IgG PRO4710, the trispecific antibodies PRO4672, PRO4687, PRO5052, PRO5053, PRO5055 and PRO5056, and reference compounds magrolimab, atezolizumab, and PRO3599**

| **PRO ID** | **CD86 EC₅₀ [nM]** | **CD163 EC₅₀ [nM]** | **CD206 EC₅₀ [nM]** |
|---|---|---|---|
| **PRO4710** | 0.119 ± 0.070 | 0.072 ± 0.008 | 0.203 ± 0.129 |
| **magrolimab** | n.a. | n.a. | n.a. |
| **atezolizumab** | n.a. | n.a. | n.a. |
| **PRO3599** | n.a. | n.a. | n.a. |
| **PRO4672** | 0.079 ± 0.009 | 0.067 ± 0.004 | 0.120 ± 0.043 |
| **PRO4687** | 0.078 ± 0.009 | 0.070 ± 0.009 | 0.124 ± 0.077 |
| **PRO5052** | 0.082 ± 0.006 | 0.073 ± 0.001 | 0.134 ± 0.082 |
| **PRO5053** | n.a. | n.a. | n.a. |
| **PRO5055** | 0.085 ± 0.012 | 0.058 ± 0.003 | 0.128 ± 0.071 |
| **PRO5056** | n.a. | n.a. | n.a. |

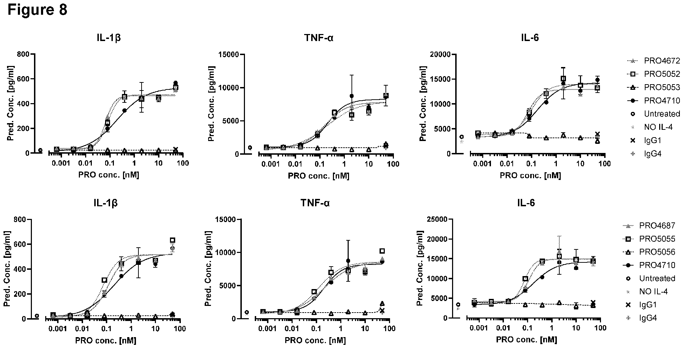

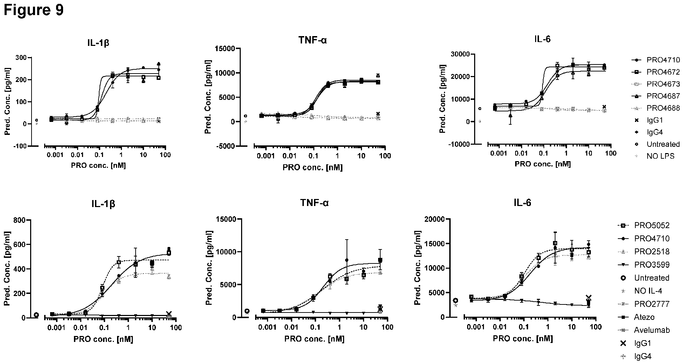

The trispecific antibodies according to the invention further exhibit the ability to foster LPS-driven cytokine secretion (see Figures 8 and 9, and Table 39), due to the presence of the LILRB2 arm, which is confirmed through comparison with the respective mock compounds. The tested multispecific antibodies demonstrate similar effects obtained with the reference anti-LILRB2 antibody J19.H1 from Jounce (PRO2518, see Figure 9), while there is no observed activity for the LILRB2-BD-devoid trispecific variants PRO5053 and PRO5056 (HSA-BD as LILRB2 mock binding domain) and the reference antibodies PRO3599, magrolimab and atezolizumab (at the top dose).

**Table 39: Induction of cytokine secretion for the anti-LILRB2 IgG PRO4710, the trispecific antibodies PRO4672, PRO4687, PRO5052, PRO5053, PRO5055 and PRO5056, and reference compounds magrolimab, atezolizumab, and PRO3599**

| **PRO ID** | **IL-1β EC₅₀ [nM]** | **TNF-α EC₅₀ [nM]** | **IL-6 EC₅₀ [nM]** |
|---|---|---|---|
| **PRO4710** | 0.248 ± 0.136 | 0.169 ± 0.026 | 0.153 ± 0.043 |
| **magrolimab** | n.a. | n.a. | n.a. |
| **atezolizumab** | n.a. | n.a. | n.a. |
| **PRO3599** | n.a. | n.a. | n.a. |
| **PRO4672** | 0.096 ± 0.026 | 0.123 ± 0.037 | 0.078 ± 0.007 |
| **PRO4687** | 0.205 ± 0.166 | 0.175 ± 0.038 | 0.097 ± 0.020 |
| **PRO5052** | 0.082 ± 0.028 | 0.117 ± 0.052 | 0.077 ± 0.024 |
| **PRO5053** | n.a. | n.a. | n.a. |
| **PRO5055** | 0.0705 | 0.141 ± 0.066 | 0.063 ± 0.019 |
| **PRO5056** | n.a. | n.a. | n.a. |

Both assays demonstrate the efficacy of LILRB2 inhibition through the absence of activity in PD-L1 × CD47 bi-specific compounds, and Pfizer compound. Furthermore, comparable activity is observed between multispecific constructs comprising a high-affinity CD47-BD and a low-affinity CD47-BD. Notably, the trispecific antibodies of the invention having different PDL1-BDs exhibit an activity comparable with established benchmarks from Jounce, Merck, and NGM.

### 5.4 Phagocytosis

The *in vitro* phagocytic activity induced by bi-/multispecific antibodies of the invention was assessed using the H292, A375, Nalm6, tumor cell lines and primary red blood cells (RBCs). The assay relies on the uptake of labelled target cells by macrophages and analysis by flow cytometry.

Human peripheral blood mononuclear cells (PBMCs) were isolated from buffy coats of healthy donors by density gradient centrifugation (STEMCELL Technologies). CD14+ cells were isolated from PBMCs by positive selection with magnetic beads (Miltenyi Biotech, 130-050-201). HMDMs were generated by culturing CD14+ cells for 6-8 days in complete medium (RPMI 1640, 10 % heat-inactivated bovine serum, Sigma Aldrich) in the presence of 50 ng/ml human macrophage colony stimulating factor (M-CSF) (Peprotech) at 37°C and 5 % CO₂. Medium was exchanged and M-CSF replenished on day 3 of the culture.

HMDMs were harvested from culturing flasks on day 6-8 using Accutase (Biowest), seeded at 50'000 cells per well in 96-well flat bottom plates (TPP) and treated with 10 ng/ml IL-10 (Peprotech) overnight. The next day HMDMs were co-incubated with CFSE-labelled target cells at an effector:target ratio of 1:1 for 2.5 h at 37°C in the presence of a serial dilution of test compounds (dose range 200 nM - 0.02 pM). In the case of RBCs, the effector:target ratio was 1:50. At the end of the incubation period, cells were stained with a viability marker (Zombie NIR, Biolegend) and an anti-CD14-APC staining antibody (Biolegend). Samples were fixed and acquired by flow cytometry (Attune NXT, Thermo Fisher Scientific). Phagocytic activity was determined as double positive events (CD14+ HMDMs that have taken up CFSE positive target cells) and phagocytosis is presented as % CFSE+ macrophages.

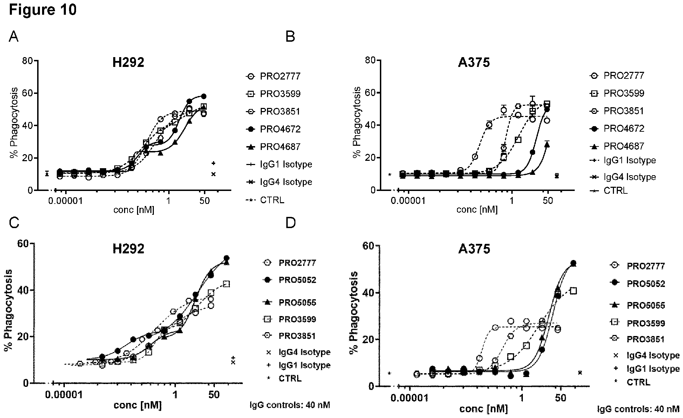

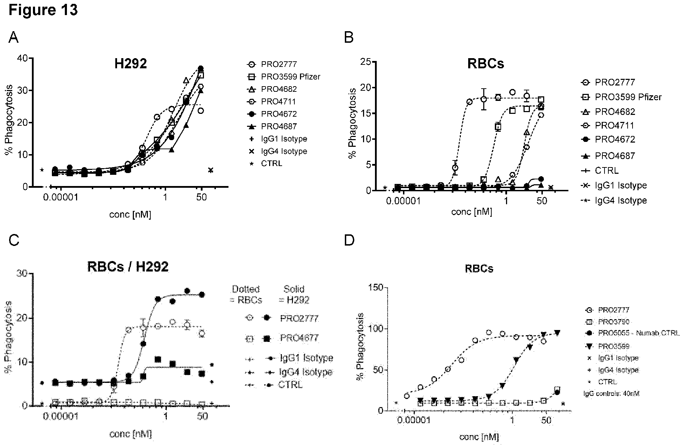

The results of the phagocytosis potency analysis are summarized in Tables 40, 41, 42 and 43 as well as in Figures 10, 11, 12 and 13.The multispecific antibodies induced a bi-phasic response curve, with an initial part driven by the anti-PD-L1 arm, confirmed through comparison with avelumab (anti-PDL1 IgG1). This initial increase in phagocytosis was absent in PDL1-negative cell lines, confirming PDL1 dependency. The second part of the dose-response curve is driven by anti-CD47 engagement, which was confirmed by PRO5054 and PRO5057, two compounds in which anti-CD47 was replaced by an unspecific human serum albumin (hSA) binder (hSA-BD), herein also referred to as mock binding domain. In PDL1-positive cell lines the herein described antibodies showed similar potency but higher efficacy as the benchmark compounds magrolimab, PRO3599 (anti-CD47xPDL1 bispecific antibody of Pfizer), PRO3851 (anti-CD47xPDL1 bispecific antibody of Innovent) and a combination of magrolimab & avelumab. In PDL1-negative cell lines potency was reduced compared to benchmark compounds, highlighting the PDL1 dependency of the described antibodies. Modification of the Fc-effector functions (PRO5134), herein also referred to as Fc-silent version, abolished phagocytosis.

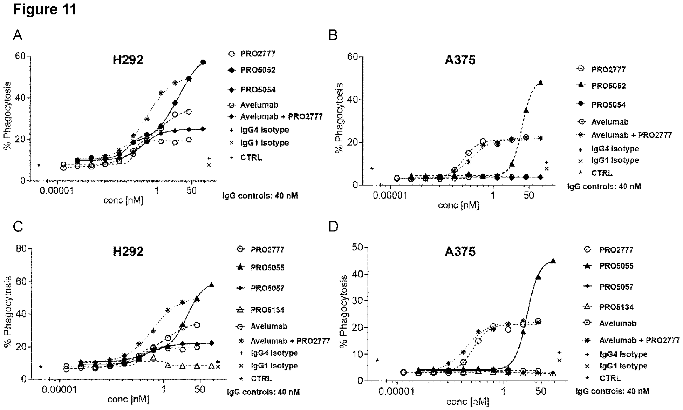

As apparent from Figure 11 and the data in Tables 40 and 41, the trispecific antibodies of the invention PRO5052, PRO5055, and PRO4672 surpasses the efficacy of the magrolimab and avelumab combination in phagocytosis. PRO4687 showed high efficacy against H292 cell but not against Nalm6 cells. For the comparative molecules PRO5054 and PRO5057, where the CD47-BD has been replaced by a mock binding domain, significantly reduced phagocytosis potency for H292 and A375 cancer cells could be observed, as expected.

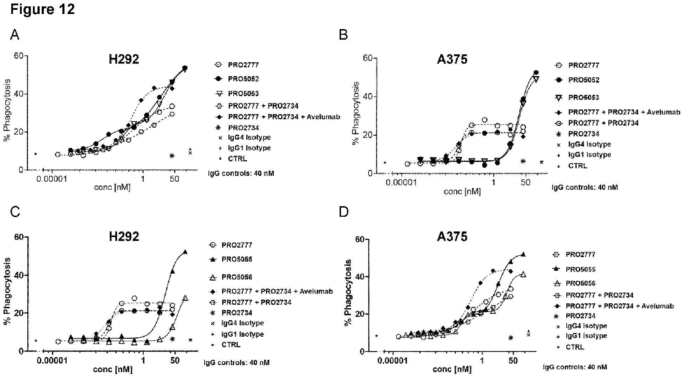

From Table 42 and Figure 12, it is further evident that phagocytosis potency may also be increased by the presence of the LILRB2-BDs, as indicated by the somewhat reduced activity observed in the trispecific molecules, where the LILRB2-BDs have been replaced by mock binding domains (PRO5053 and PRO5056).

From Tables 42 and 43, it is further evident that re-formatting the multispecific antibodies by placing the anti-LILRB2 domains in the Fab arms, opposite of the Fc-region, completely abolished tumor cell phagocytosis (PRO4677). Combination of magrolimab (PRO2777) with PRO2734, an anti-LILRB2 IgG4 antibody (Merck), did not enhance phagocytosis, indicating that the activity/efficiency of the LILRB2-BDs is also significantly format-dependent, and not only signalling-dependent. It is believed by the inventors that LILRB2-engaging domains in proximity to the Fc-region improve orientation of the compounds enhancing trans-bridging of macrophages and tumor cells (in absence and presence of PDL1 expression on tumor cells).

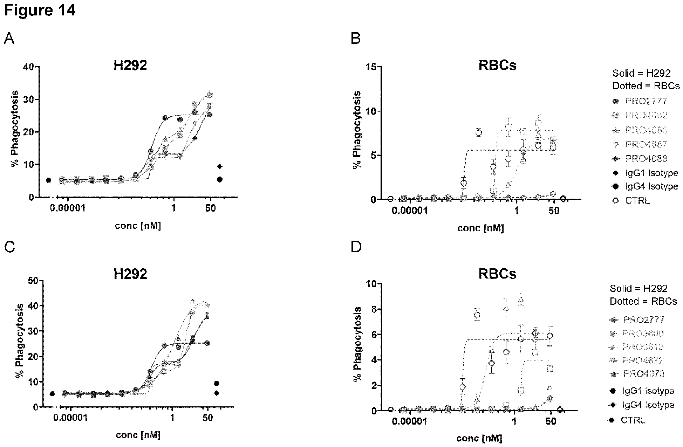

Many different multispecific antibody formats have been tested for H292 cancer cell phagocytosis and red blood cells (RBC) phagocytosis (see Table 43 and also Figures 10, 11, 12, 13, 14 and 15). The data illustrate that the following multispecific antibodies and reference antibodies exhibit high levels of cancer cell phagocytosis, but at the same time also significant levels of RBS phagocytosis:
- trispecific antibodies PRO3843, PRO4682, PRO4683 and PRO4711, where the CD47-BD is located in one of the Fab arms in the form of a Fab;
- bispecific antibodies PRO3609 and trispecific antibody PRO3613, where the CD47-BD has an intermediate binding affinity for CD47;
- the reference anti-CD47 x PDL1 antibodies PRO3599 (Pfizer), PRO3851 (Innovent) and magrolimab.

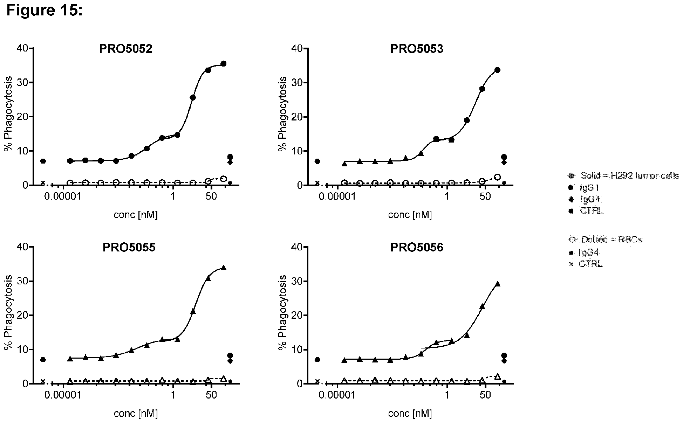

On the other hand, the bispecific antibody PRO3790 and the trispecific antibodies PRO4672, PRO4673, PRO4687, PRO4688, RO5052, PRO5053, PRO5055 and PRO5056 exhibit high cancer cell phagocytosis and, at the same time, only minimal or no measurable RBC phagocytosis. The bispecific and trispecific antibodies of the invention thus have a significantly larger therapeutic window than the reference antibodies PRO3599, PRO3851 and magrolimab. The phagocytosis data in Table 43 further illustrates that placing the CD47-BD at the C-terminus of the Fc-region (PRO4677, PRO4678) or replacing the CD47-BD by a mock domain (PRO5054), also lead to minimal or no measurable RBC phagocytosis, but also dramatically decrease cancer cell phagocytosis. Generally, all tested multispecific antibodies of the invention containing a CD47-BD in scFv format in one of the Fab arms did not induce noteworthy RBC phagocytosis at all concentrations tested and, thus, exhibit a large potential therapeutic window, as illustrated in Figure 15. Phagocytosis of PD-L1 positive tumor cells was unaffected by the different format and positioning of the CD47-BD, except for PRO4677, where tumor and RBC phagocytosis were abolished. The reference anti-CD47 x PDL1 antibodies PRO3599 (Pfizer) and PRO3851 (Innovent), and magrolimab exhibit the highest maximal levels of RBC phagocytosis.

**Table 37: Binding of anti-CD47 × PDL1 × LILRB2 antibodies to human and cyno PBMCs.**

| | | **Human (2 donors)** | | | | **Cynomolgus Monkey (2 donors)** | | | |
|---|---|---|---|---|---|---|---|---|---|
| **PROID** | **Concentration [nM]** | **HLA-DR+ CD14+ [geoMFI × 1000]** | **HLA-DR+ CD14-[geoMFI × 1000]** | **B cells (CD20+) [geoMFI × 1000]** | **T cells (CD3+) [geoMFI × 1000]** | **HLA-DR+ CD14+ [geoMFI × 1000]** | **HLA-DR+ CD14-[geoMFI × 1000]** | **B cells (CD20+) [geoMFI × 1000]** | **T cells (CD3+) [geoMFI × 1000]** |
| PRO5052 | 1.6 | 65.0 ± 5.4 | 13.4 ± 7.3 | 3.9 ± 0.5 | 1.6 ± 0.2 | 122.6 ± 8.2 | 37.9 ± 2.4 | 15.9 ± 0.9 | 1.5 ± 0.1 |
| | 200 | 145.1 ± 6.1 | 41.6 ± 7.5 | 33.0 ± 4.0 | 32.7 ± 2.3 | 244.1 ± 12.3 | 90.4 ± 4.0 | 70.5 ± 1.1 | 16.2 ± 0.7 |
| PRO5053 | 1.6 | 60.0 ± 5.0 | 13.5 ± 3.2 | 7.4 ± 0.8 | 5.1 ± 0.1 | 131.1 ± 7.2 | 51.2 ± 1.3 | 26.0 ± 1.6 | 3.8 ± 0.3 |
| | 200 | 179.0 ± 41.4 | 41.0 ± 4.2 | 40.1 ± 4.1 | 55.7 ± 7.3 | 238.5 ± 17.0 | 121.9 ± 2.1 | 83.5 ± 0.9 | 31.0 ± 1.8 |
| PRO5054 | 1.6 | 36.6 ± 11.1 | 8.2 ± 6.4 | 0.8 ± 0.1 | 0.7 ± 0.2 | 87.4 ± 54.7 | 19.5 ± 7.3 | 2.6 ± 0.4 | 0.5 ± 0.1 |
| | 200 | 52.7 ± 11.4 | 8.2 ± 6.5 | 1.0 ± 0.1 | 0.8 ± 0.3 | 101.1 ± 15.3 | 23.9 ± 3.3 | 4.8 ± 1.2 | 0.7 ± 0.0 |
| PRO5055 | 1.6 | 76.4 ± 7.1 | 20.2 ± 6.8 | 10.8 ± 1.1 | 7.3 ± 0.1 | 128.1 ± 4.3 | 57.9 ± 2.2 | 36.0 ± 2.3 | 7.1 ± 0.6 |
| | 200 | 188.9 ± 22.8 | 46.9 ± 2.0 | 45.0 ± 5.4 | 56.4 ± 6.8 | 241.8 ± 8.6 | 104.9 ± 8.1 | 89.6 ± 2.6 | 31.0 ± 1.8 |
| PRO5056 | 1.6 | 46.9 ± 1.1 | 9.1 ± 3.2 | 3.8 ± 0.5 | 1.1 ± 0.1 | 120.9 ± 4.0 | 36.8 ± 4.2 | 15.9 ± 0.7 | 0.9 ± 0.1 |
| | 200 | 108.3 ± 8.6 | 25.0 ± 4.5 | 16.3 ± 2.4 | 12.6 ± 0.6 | 211.2 ± 22.7 | 65.8 ± 4.3 | 42.5 ± 3.1 | 5.4 ± 0.3 |
| PRO5057 | 1.6 | 26.2 ± 7.8 | 5.9 ± 4.7 | 0.6 ± 0.1 | 0.5 ± 0.2 | 41.7 ± 5.3 | 8.9 ± 1.1 | 1.8 ± 0.0 | 0.3 ± 0.0 |
| | 200 | 40.4 ± 10.9 | 7.3 ± 5.7 | 1.0 ± 0.1 | 0.7 ± 0.2 | 76.9 ± 8.0 | 19.8 ±1.5 | 4.0 ± 0.6 | 0.6 ± 0.0 |
| PRO5134 | 1.6 | 65.0 ± 5.4 | 13.4 ± 7.3 | 3.9 ± 0.5 | 1.6 ± 0.2 | 122.6 ± 8.2 | 37.9 ± 2.4 | 15.9 ± 0.9 | 1.5 ± 0.1 |
| | 200 | 145.1 ± 6.1 | 41.6 ± 7.5 | 33.0 ± 4.0 | 32.7 ± 2.3 | 244.1 ± 12.3 | 90.4 ± 4.0 | 70.5 ± 1.1 | 16.2 ± 0.7 |

**Table 40: PDL1-dependent tumor cell phagocytosis of multispecific antibodies PRO4672 and PRO4687 and reference antibodies PRO2777, PRO3599 and PRO3851.**

| | | | **H292 cells** | | | **Nalm6 cells** | |
|---|---|---|---|---|---|---|---|
| **PRO ID** | **PDL1 binder** | **CD47 binder** | **PDL1 driven EC₅₀ [nM]** | **CD47 driven EC₅₀ [nM]** | **Maximum phagocytosis level *⁾** | **CD47 driven EC₅₀ [nM]** | **Maximum phagocytosis level *⁾** |
| **PRO2777** | - | magrolimab | - | 0.22 ± 0.15 | 29.37 ± 6.78 | 0.02 ± 0.01 | 29.42 ± 10.76 |
| **PRO3599 (Pfizer)** | PRO3599 | PRO3599 | 0.35 ± 0.31 | 8.63 ± 5.44 | 37.86 ± 6.09 | 17.67 ± 14.61 | 45.76 ± 8.33 |
| **PRO3851 (Innovent)** | PRO3851 | PRO3851 | n.a. | 1.82 ± 1.46 | 32.58 ± 7.27 | 0.99 ± 0.5 | 34.4 ± 11.29 |
| **PRO4672** | 37-20-B03-sc18 | 70-26-B04-sc03 | 0.32 ± 0.29 | 8.32 ± 2.90 | 42.71 ± 7.19 | 15.29 | 49.7 |
| **PRO4687** | 37-20-B03-sc18 | 70-26-B04-sc03 | 0.043 ± 0.027 | 8.62 ± 1.93 | 36.09 ± 8.55 | n.a. | 28.4 |

| | | | | | | | |
|---|---|---|---|---|---|---|---|
| *) Maximum phagocytosis level was determined at 40 nM unless otherwise stated. | | | | | | | |

**Table 41: PDL1- and CD47-dependent tumor cell phagocytosis of multispecific antibodies PRO5052 and PRO5055, comparative antibodies PRO5054, PRO5057 and PRO5134, and reference antibodies PRO2777, avelumab, PRO3599 and PRO3851.**

| | | | **H292 cells** | | | **A375 cells** | |
|---|---|---|---|---|---|---|---|
| **PRO ID** | **PDL1 binder** | **CD47 binder** | **PDL1 driven EC₅₀ [nM]** | **CD47 driven EC₅₀ [nM]** | **Maximum phagocytosis level *⁾** | **CD47 driven EC₅₀ [nM]** | **Maximum phagocytosis level *⁾** |
| **PRO2777** | - | magrolimab | - | 0.22 ± 0.15 | 29.37 ± 6.78 | 0.02 ± 0.01 | 23.65 ± 5.94 |
| **avelumab** | avelumab | - | 0.15 ± 0.14 | - | 19.9 ± 0.86 | n.a. | 4.4 ± 1.04 |
| **PRO3599 (Pfizer)** | PRO3599 | PRO3599 | 0.35 ± 0.31 | 8.63 ± 5.44 | 37.86 ± 6.09 **⁾ | 8.54 ± 5.99 | 36.03 +-/ 6.53 **⁾ |
| **PRO3851 (Innovent)** | PRO3851 | PRO3851 | n.a. | 1.82 ± 1.46 | 32.58 ± 7.27 | 0.9 ± 0.76 | 23.6 +-/ 1.3 |
| **PRO5052** | 33-03-G02-sc24 | 70-26-B04-sc03 | 0.2 ± 0.3 | 10.91 ± 2.96 | 47.47 ± 6.53 **⁾ | 19.29 ± 7.52 | 48.63 ± 4.6 **⁾ |
| **PRO5054** | 33-03-G02-sc24 | - | 0.09 ± 0.08 | n.a. | 20.05 ± 5.91 **⁾ | n.a. | 4.08 ± 0.3 **⁾ |
| **PRO5055** | 33-03-G02-sc24 | 70-26-B04-sc03 | 0.19 ± 0.3 | 10.94 ± 3.58 | 46.77 ± 7.04 **⁾ | 12.09 ± 3.23 | 44.07 ± 7.26 **⁾ |
| **PRO5057** | 33-03-G02-sc24 | - | 0.13 ± 0.04 | n.a. | 21.13 ± 4.0 **⁾ | n.a. | 3.9 ± 0.85 **⁾ |
| **PRO5134** | 33-03-G02-sc24 | 70-26-B04-sc03 | n.a. | n.a. | 7.84 ± 0.69 **⁾ | n.a. | 4.06 ± 1.13 **⁾ |

| | | | | | | | |
|---|---|---|---|---|---|---|---|
| *) Maximum phagocytosis level was determined at 40 nM unless otherwise stated. **) Maximum phagocytosis level determined at 80 nM. | | | | | | | |

**Table 42: Cancer cell phagocytosis of trispecific antibodies with and without LILRB2-BD, and of reference antibodies PRO2777, PRO2734 and avelumab alone and in combination.**

| | | | | **H292 cells** | | | **A375 cells** | |
|---|---|---|---|---|---|---|---|---|
| **PRO ID** | **PDL1-BD** | **CD47-BD** | **LILRB2-BD** | **PD-L1 driven EC₅₀ [nM]** | **CD47 driven EC₅₀ [nM]** | **Maximum phagocytosis level *⁾** | **CD47 driven EC₅₀ [nM]** | **Maximum phagocytosis level *⁾** |
| **PRO2777 (magrolimab)** | - | magrolimab | - | - | 0.22 ± 0.15 | 29.37 ± 6.78 | 0.02 ± 0.01 | 23.65 ± 5.94 |
| **PRO2734 (Merck)** | - | - | PRO2734 | - | - | 7.73 ± 0.18 | - | 4.89 ± 1.42 |
| **PRO2777 + avelumab** | avelumab | magrolimab | - | n.a. | 0.27 ± 0.1 | 37.25 ± 6.87 | 0.014 ± 0.003 | 22.55 ± 0.55 **⁾ |
| **PRO2777 + PRO2734** | - | magrolimab | PRO2734 | - | 2.25 ± 1.7 | 28.75 ± 0.65 | 0.02 ± 0.01 | 20.3 ± 1.6 |
| **PRO2777 + PRO2734 + avelumab** | avelumab | magrolimab | PRO2734 | - | 0.29 ± 0.05 | 46 ± 3.2 | 0.02 ± 0.01 | 18.65 ± 0.55 |
| **PRO5052** | 33-03-G02-sc24 | 70-26-B04-sc03 | 68-18-E11-sc07 | 0.2 ± 0.32 | 10.91 ± 3.22 | 47.47 ± 6.53 **⁾ | 19.29 ± 7.52 | 48.63 ± 4.6 **⁾ |
| **PRO5053** | 33-03-G02-sc24 | 70-26-B04-sc03 | - | 0.18 ± 0.22 | 28.45 ± 16.15 | 44.94 ± 9.38 **⁾ | 27.49 ± 2.29 | 31.6 ± 43.69 **⁾ |
| **PRO5055** | 33-03-G02-sc24 | 70-26-B04-sc03 | 68-18-E11-sc07 | 0.19 ± 0.3 | 10.94 ± 3.58 | 46.77 ± 7.04 **⁾ | 12.09 ± 3.23 | 44.07 ± 7.26 **⁾ |
| **PRO5056** | 33-03-G02-sc24 | 70-26-B04-sc03 | - | 0.13 ± 0.14 | 54.30 ± 15.05 | 34.32 ± 5.16 **⁾ | 76.9 ± 21.63 | 23.03 ± 5.15 **⁾ |

| | | | | | | | | |
|---|---|---|---|---|---|---|---|---|
| *) Maximum phagocytosis level was determined at 40 nM unless otherwise stated. **) Maximum phagocytosis level determined at 80 nM. | | | | | | | | |

**Table 43: H292 cancer cell phagocytosis and RBC phagocytosis of multispecific antibodies of the invention and reference antibodies.**

| | | | | **H292 cells** | | | **RBCs** | |
|---|---|---|---|---|---|---|---|---|
| **PRO ID** | **PD-L1-BD** | **CD47-BD** | **LILRB2-BD** | **PD-L1 driven EC₅₀ [nM]** | **CD47 driven EC₅₀ [nM]** | **Maximum phagocytosis level** | **CD47 driven EC₅₀ [nM]** | **Maximum phagocytosis level relative to PRO2777 *⁾** |
| PRO2777 | - | magrolimab | - | - | 0.22 ± 0.15 | 29.37 ± 6.78**⁾ | 0.01 ± 0.003 | 1 **⁾ |
| PRO3599 | PRO3599 | PRO3599 | - | 0.35 ± 0.31 | 8.63 ± 5.44 | 37.86 ± 6.09**⁾ | 3.0 ± 2.56 | 1.05 ± 0.18 **⁾ |
| PRO3851 | PRO3851 | PRO3851 | - | n.a. | 1.82 ± 1.46 | 32.58 ± 7.27 | 3.0 ± 1.94 | 0.68 ± 0.13 |
| PRO3609 | 37-20-B03-sc09.1 | 70-36-C05-sc02 | - | 0.12 ± 0.04 | 4.24 ± 1.61 | 39.3 ± 14.41 | 4.1 ± 2.86 | 0.89 ± 0.51 |
| PRO3613 | 37-20-B03-sc09.1 | 70-36-C05-sc02 | 68-18-E11-sc02 | 0.25 ± 0.20 | 2.13 ± 0.65 | 48.16 ± 6.16 | 0.03 ± 0.024 | 0.88 ± 0.66 |
| PRO4682 | 37-20-B03-sc18 | 70-26-B04-sc03 | 68-18-E11-sc07 | 0.14 ± 0.14 | 9.3 ± 8.9 | 34.94 ± 6.28 | 2.78 ± 3.34 | 0.96 ± 0.17 **⁾ |
| PRO4683 | 37-20-B03-sc18 | 70-26-B04-sc03 | - | 0.19 ± 0.06 | 5.33 ± 0.75 | 37.45 ± 5.45 | 2.687 | 0.94 |
| PRO4711 | 37-20-B03-sc18 | PRO3599 | 68-18-E11-sc02 | 0.11 ± 0.02 | 6.76 ± 2.15 | 31.97 ± 5.13 | 6.25 ± 5.19 | 0.92 ± 0.11 |
| PRO3843 | PRO3599 | PRO3599 | 68-18-E11-sc02 | 0.08 ± 0.02 | 10.21 ± 0.82 | 32.16 ± 5.25 | 10.44 ± 8.37 | 0.92 |
| PRO3790 | 37-20-B03-sc09.1 | 70-26-B04-sc03 | - | 0.04 ± 0.016 | 31.2 ± 16.3 | 35 **) | n.a. | 0.25 ± 0.07 **⁾ |
| PRO4672 | 37-20-B03-sc18 | 70-26-B04-sc03 | 68-18-E11-sc07 | 0.32 ± 0.29 | 8.32 ± 2.90 | 42.71 ± 7.19 | 39.43 ± 21.98 | 0.12 ± 0.05 |
| PRO4673 | 37-20-B03-sc18 | 70-26-B04-sc03 | - | 0.18 ± 0.12 | 10.96 ± 2.95 | 35.1 ± 1.3 | n.a. | 0.19 |
| PRO4687 | 37-20-B03-sc18 | 70-26-B04-sc03 | 68-18-E11-sc07 | 0.043 ± 0.027 | 8.62 ± 1.93 | 36.09 ± 8.55 | n.a. | 0.11 ± 0.07 |
| PRO4688 | 37-20-B03-sc18 | 70-26-B04-sc03 | - | 0.08 ± 0.025 | 13.5 ± 3.13 | 28.65 ± 1.69 | n.a. | 0.11 |
| PRO4677 | 37-20-B03-sc18 | 70-26-B04-sc03 | 68-18-E11-sc07 | n.a. | n.a. | 8.68 ± 1.3 | n.a. | 0.02 |
| PRO4678 | 37-20-B03-sc18 | 70-26-B04-sc03 | - | n.a. | n.a. | n.a. | n.a. | 0.04 |
| PRO5052 | 33-03-G02-sc24 | 70-26-B04-sc03 | 68-18-E11-sc07 | 0.2 ± 0.3 | 10.91 ± 2.96 | 47.47 ± 7.21 **⁾ | 39.79 ± 3.17 | 0.14 ± 0.03 **⁾ |
| PRO5053 | 33-03-G02-sc24 | 70-26-B04-sc03 | - | 0.19 ± 0.22 | 28.25 ± 16.15 | 44.94 ± 9.38 **⁾ | n.a. | 0.14 ± 0.01 **⁾ |
| PRO5054 | 33-03-G02-sc24 | - | 68-18-E11-sc07 | 0.09 ± 0.08 | n.a. | 20.05 ± 5.91 **⁾ | n.a. | 0.03 ± 0.002 **⁾ |
| PRO5055 | 33-03-G02-sc24 | 70-26-B04-sc03 | 68-18-E11-sc07 | 0.19 ± 0.3 | 10.94 ± 3.58 | 46.77 ± 7.04 **⁾ | 44.64 ± 6.82 | 0.11 ± 0.04 **⁾ |
| PRO5056 | 33-03-G02-sc24 | 70-26-B04-sc03 | - | 0.13 ± 0.14 | 54.30 ± 15.05 | 34.32 ± 5.16 **⁾ | n.a. | 0.12 ± 0.05 **⁾ |
| PRO5057 | 33-03-G02-sc24 | - | 68-18-E11-sc07 | 0.13 +/- 0.04 | n.a. | 21.13 +/- 4.0 **⁾ | n.d. | n.d. |
| PRO5134 | 33-03-G02-sc24 | 70-26-B04-sc03 | 68-18-E11-sc07 | n.a. | n.a. | n.a. | n.d. | n.d. |
| PRO5423 | 33-03-G02-sc24 | 70-26-B04-sc03 | 68-18-E11-sc07 | n.a. | n.a. | n.a. | n.d. | n.d. |
| PRO4914 | 37-20-B03-sc18 | 70-26-B04-sc03 | 68-18-E11-sc07 | 0.071 ± 0.08 | 25.24 ± 15.37 | 34.07 ± 7.14 **⁾ | 37.43 ± 9.67 | 0.84 ± 0.21 **⁾ |

| | | | | | | | | |
|---|---|---|---|---|---|---|---|---|
| N.d. : Not determined, as there is no phagocytosis of RBCs expected. *) Maximum phagocytosis level was determined at 40 nM unless otherwise stated. **) Maximum phagocytosis level determined at 80 nM. | | | | | | | | |

### 5.5 Multispecific antibodies activate NK cells to tumor cell killing via ADCC

The *in vitro* cytotoxic activity of primary NK cells induced by the multispecific antibodies according to the invention was assessed using the H226 tumor cell line, transfected with the IncuCyte NucLight Orange Lentivirus Reagent (Sartorious, 4625). The assay makes use of the IncuCyte live imaging platform (Sartorius) and quantifies the killing of H226 NucRed tumor cells as measured by the loss of the NucRed signal over time.

Human peripheral blood mononuclear cells (PBMCs) were isolated from buffy coats of healthy donors by density gradient centrifugation (STEMCELL Technologies). NK cells were subsequently isolated by negative selection with magnetic beads (Miltenyi Biotech, 130-092-657). NK cells were pre-activated overnight in (RPMI 1640, 10 % heat-inactivated bovine serum, Sigma Aldrich) in the presence of 0.2 - 1 nM human IL-2 (R&D Systems) at 37°C and 5 % CO₂.

H226 NucRed tumor cells were seeded at 7'000 cells per well in 96-well flat bottom plates (TPP) on day -1 and cultured in RPMI+10 %FCS. The next day, pre-activated NK cells were harvested, washed with medium and co-incubated with target cells at an effector:target ratio of 5:1 for 24 h at 37°C in the presence of a serial dilution of test compounds (dose range 20 nM - 0.03 pM). Images of the tumor cell - NK cell co-culture were taken every two hours and the count of H226 NucRed tumor cells was assessed over time. Data is presented as fold change to T0 for each compound and is an average of four images per well.

The trispecific antibodies PRO5052 and PRO5055 exhibited a potent ADCC with ECso values that are similar to that of avelumab and higher than that of the reference PRO3599. Magrolimab (anti-CD47 IgG4) and atezolizumab (anti-PD-L1 modified IgG1) failed to induce ADCC. Also, the Fc-silent variant PRO5134 failed to induce ADCC. The trispecific antibody PRO5054, where the CD47-BD is replaced by a mock binding domain, shows a significantly reduced ADCC, highlighting the importance of simultaneous targeting of PDL1 and CD47. The presence or absence of LILRB2-BDs in the trispecific molecules had no effect on ADCC potency.

### 5.6 Induction of T cell proliferation

To evaluate whether the trispecific antibodies of the invention are capable to activate T cell proliferation, these antibodies were tested in an immunosuppressive assay. The immunosuppressive assay used aims to track recovery of T cell proliferation in the co-culture with suppressive macrophages in the presence of T cell stimulation and test compounds. The assay was performed as follows.

T cells were isolated from PBMC fraction with a magnetic Pan T Cell negative isolation kit (Miltenyi Biotec, cat no. 130-096-535). Bulk T cell population was used containing CD4+ and CD8+ T cells. T cells were stained with a cell proliferation tracking dye (CTV CellTrace dye, Thermo Fisher Scientific, cat no. C34557) by incubation of T cells with the CTV dye (5 µM working solution) for 20 min at 37°C. To stop the labelling reaction, 5x volume of complete medium containing 10 % FBS was added for 5 min at room temperature followed by extensive washing.

T cells were stimulated to proliferate by a co-culture in 96 flat-bottom well plates (TPP) with coated anti-CD3 (at 2 ug/mL, BioLegend, cat no. 317326) and soluble anti-CD28 mAbs (4 µg/mL, BioLegend, cat no. 302934). Suppressive human monocyte-derived macrophages (HMDM) were generated by treatment with IL-10 (10 ng/mL, BioLegend cat no. 571004,) and TGF-β (20 ng/mL, Peprotech, cat no. 100-21) for 24 h at 5 % CO₂ and 37°C prior to co-culture with T cells. HMDM were added to the culture in different ratios, e.g. 1:1, 2.5:1 and 5:1 (T cell : HMDM). Total number of cells varied from 120 k to 150 k per well. Co-culture of T cells and macrophages lasted for 5 days at 37°C and 5 % CO₂. As a positive control, stimulated T cells cultured without HMDM were used (max. proliferation observed). As a negative control CTV-labelled, unstimulated T cells were used (population showed no proliferation).

T cell proliferation was measured by quantification of CTV dilution, which increases with every cell division. Data was collected with flow cytometry method, where T cells and HMDM were identified by staining with mAb (all from BioLegend: CD14-APC cat. no. 325608, CD4-FITC 317408, CD8 PercP cat no. 344708). Live/dead stain (Zombie NIR Fixable Viability Kit, BioLegend, cat. No. 423106) was employed to exclude dead cells from subsequent analysis. Data was acquired with Attune NxT Flow Cytometer (Thermo Fisher Scientific) and analysed with the FlowJo software (Tree Star) and T cell proliferation was quantified by calculating the proliferation index, which is the total number of divisions divided by the number of cells that went into division.

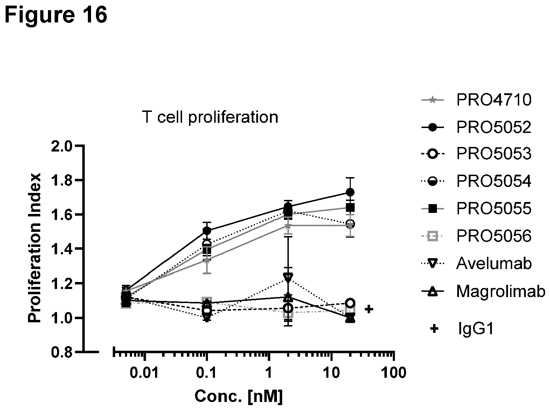

Trispecific molecules of the invention demonstrate a dose-dependent induction of T-cell proliferation, as indicated by the significant CTV dye dilution and proliferation index (see Figure 16). This heightened activity is attributed to the inhibition of LILRB2, as evidenced by the singular effectiveness of the IgG compound as a standalone agent. Magrolimab, avelumab, and atezolizumab IgGs, as well as PRO5053 and PRO5056 showed no significant impact after treatment.

The induction of T cell proliferation is specifically propelled by the inhibition of LILRB2, with no discernible involvement of the PDL1- and CD47-BDs in this assay. Additionally, the trispecific antibodies of the invention exhibit dose-dependent activity in this context.

### 5.7 SEA Assay

An SEA (Staphylococcal Enterotoxin A) assay was further performed to evaluate the potential usability of the multispecific antibodies of the invention in conditions characterized by excessive inflammation.

Peripheral blood mononuclear cells (PBMC) were isolated from fresh human whole blood employing density gradient centrifugation. PBMCs were depleted of NK cells using anti-CD56 microbeads (Miltenyi Biotec, 130-097-042) and the MACS cell separation kit (Miltenyi Biotec). 200,000 NK-cell depleted PBMCs per well were added to the 96-well plate (flat bottom, TPP), followed by the addition of serial dilutions of experimental compounds in assay buffer containing Staphylococcal enterotoxin A (SEA, Toxin Technology, AT 101) at a concentration of 10 ng/mL. After 76 h of incubation at 37°C and 5 % CO₂, cell supernatants were harvested and human Interleukin 1β (IL-1β) and tumor necrosis cell factor alpha (TNF-α) levels in the culture supernatants were quantified using the cytometric bead array method (CBA) from LegendPlex kit provided by BioLegend according to kit instructions (cat no.: 740930).

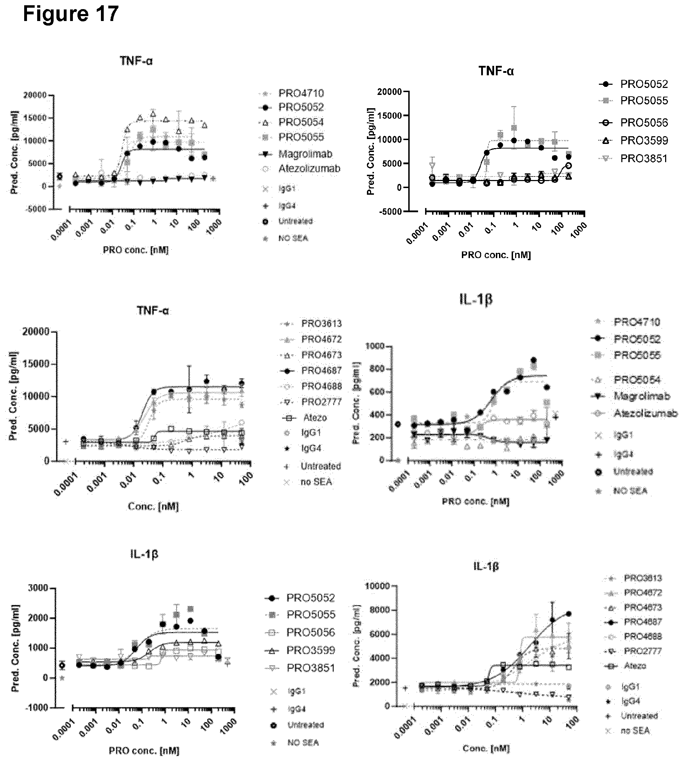

As shown in Figure 17, the inhibition of LILRB2 is responsible for mediating TNFα secretion, and the tested multispecific antibodies demonstrated comparable potency to the anti-LILRB2 IgG4 format from Numab. Treatment with magrolimab or atezolizumab did not lead to an increase in TNFα levels, and anti-PDL1 × CD47 bispecific antibodies also failed to enhance TNFα release.

IL-1β induction is primarily attributed to PDL1 binding, as demonstrated by the response to atezolizumab, with no discernible impact from CD47 and LILRB2 blockade. Interestingly, the multispecific compound exhibited activity on par with the combination of three IgGs, considering data from five donors. However, it is important to note a significant variability among donors in the observed outcomes.

### 5.8 Hemagglutination assay

To test whether the multispecific antibodies of the invention trigger agglutination of human erythrocytes, a hemagglutination assay based on optical density measurement was performed. The method used to assess agglutination was the same as the method described in the Example 4.2.

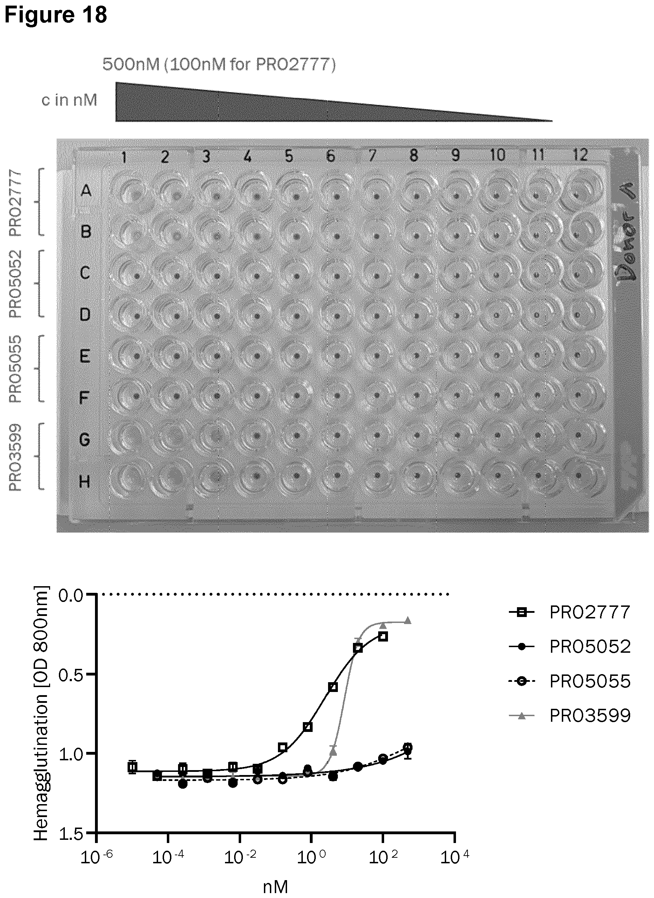

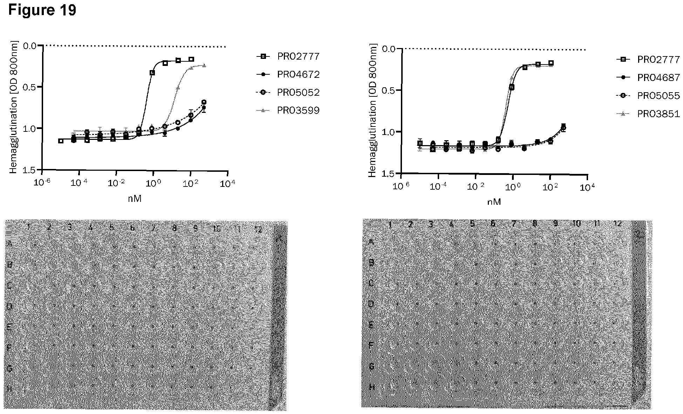

There was no apparent hemagglutination observed for trispecific molecules PRO5052 and PRO5055 within the tested concentration range, which extended up to 500 nM, as seen for the anti-CD47 x PDL1 bispecific antibody PRO3790. Conversely, treatment with PRO2777 (anti-CD47 antibody of clone hu5F9) and PRO3599 (anti-CD47 x PDL1 bispecific antibody from Pfizer) resulted in noticeable agglutination of red blood cells (RBCs), although PRO3599 exhibited larger ECso values than PRO2777 (Figures 18 and 19).

### 5.9 Cell binding to cancer cell lines

To test binding of the trispecific antibodies of the invention to CD47 on the surface of cancer cells, cancer cells expressing human CD47 and PD-L1 were tested in flow cytometry. The method used to determine cell binding to cancer cell lines was the same as described in Example 4.2.

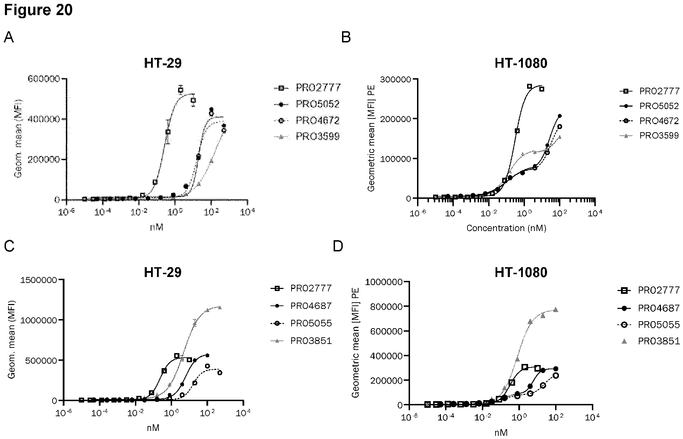

The results of the binding tests are summarized in Figure 20. Biphasic binding curves were obtained on HT-1080 cells, due to sequential binding of the POL 1- and CD47-BDs to the cells. For HT-29 cells, only monophasic binding curves were obtained, as these cells do not express PDL1. PRO4672, PRO4687, PRO5052 and PRO5055 demonstrated potent binding to HT-1080 and weaker binding to HT-29 cells. The reference antibody PRO3599 exhibited a similar binding profile to both cancer cell lines, albeit with a somewhat weaker potency for HT-29 cancer cells. The reference antibodies PRO2777 and PRO3851 showed a monophasic binding profile to both cell lines with PRO3851 exhibiting the highest Emax of all tested antibodies.

### 5.10 Neutralization of CD47/SIRPα interaction

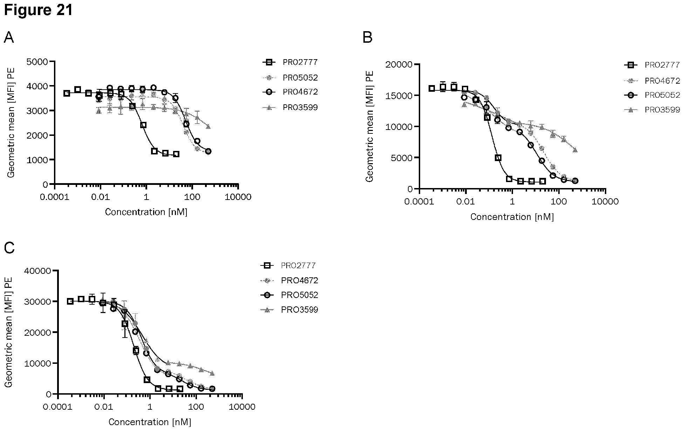

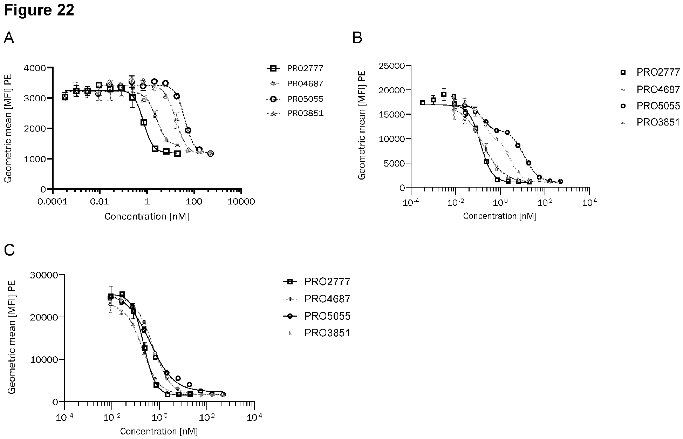

The potency of the trispecific antibodies of the present invention to inhibit the interaction of CD47/SIRPα on NCI-H292 and CHO CD47 cells was assessed analogous to the procedure disclosed in Example 4.2. The results of the inhibition study are summarized in Figures 21 and 22.

As apparent from these Figures, PRO5052 and PRO5055 are able to completely inhibit CD47/SIRPα interaction, with IC₅₀ values comparable to PRO4672 and PRO4687, respectively. On the other hand, PRO3599 showed only partial SIRPα neutralization, irrespective of IFNy stimulation of NCI-H292 cells. The other reference antibodies PRO2777 (magrolimab) and PRO3851 showed similar SIRPα neutralization potencies on NCI-H292 cells.

### 5.11 LILRB2 selectivity assessment

To assess whether the trispecific antibodies according to the invention selectively bind to LILRB2 and not to other members of protein family LILRA and LILRB, a cellular ELISA (CELISA) using transiently transfected CHO cells was developed. The method applied for this assessment was the same as described in Example 3.6.

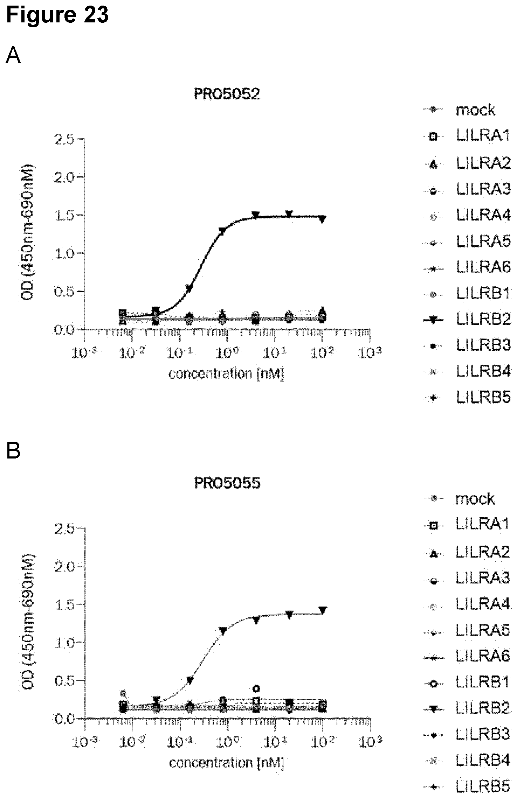

As shown in Figure 23, trispecific antibodies PRO5052 and PRO5055 demonstrated potent and selective binding to LILRB2.

## Claims

1. A multispecific antibody comprising:
a) one binding domain, which specifically binds to CD47 (CD47-BD),
wherein said CD47-BD comprises:
i. the HCDR1 sequence of SEQ ID NO: 1,
the HCDR2 sequences of SEQ ID NO: 2,
the HCDR3 sequence of SEQ ID NO: 3,
the LCDR1 sequence of SEQ ID NO: 4,
the LCDR2 sequence of SEQ ID NO: 6, and
the LCDR3 sequences of SEQ ID NO: 7; or
ii. the HCDR1 sequence of SEQ ID NO: 1,
the HCDR2 sequences of SEQ ID NO: 2,
the HCDR3 sequence of SEQ ID NO: 3,
the LCDR1 sequence of SEQ ID NO: 4,
the LCDR2 sequence of SEQ ID NO: 5, and
the LCDR3 sequences of SEQ ID NO: 7;
b) one binding domain, which specifically binds to PDL1 (PDL1-BD); and
c) an IgG-like region comprising an immunoglobulin Fc region, a first Fab arm and a second Fab arm, wherein:
- the CD47 BD is an scFv fragment replacing the first Fab arm, and
- the PDL1 BD is in the binding domain of the second Fab arm or is an scFv fragment replacing said second Fab arm.

2. The multispecific antibody of claim 1, wherein said CD47-BD comprises:
the HCDR1 sequence of SEQ ID NO: 1,
the HCDR2 sequences of SEQ ID NO: 2,
the HCDR3 sequence of SEQ ID NO: 3,
the LCDR1 sequence of SEQ ID NO: 4,
the LCDR2 sequence of SEQ ID NO: 6, and
the LCDR3 sequences of SEQ ID NO: 7.

3. The multispecific antibody of claim 1 or 2, wherein said PDL1-BD comprises:
i. the HCDR1 sequence of SEQ ID NO: 74,
the HCDR2 sequences of SEQ ID NO: 75,
the HCDR3 sequence of SEQ ID NO: 76,
the LCDR1 sequence of SEQ ID NO: 77,
the LCDR2 sequence of SEQ ID NO: 78, and
the LCDR3 sequences of SEQ ID NO: 80; or
ii. the HCDR1 sequence of SEQ ID NO: 74,
the HCDR2 sequences of SEQ ID NO: 75,
the HCDR3 sequence of SEQ ID NO: 76,
the LCDR1 sequence of SEQ ID NO: 77,
the LCDR2 sequence of SEQ ID NO: 79, and
the LCDR3 sequences of SEQ ID NO: 80;
iii. the HCDR1 sequence of SEQ ID NO: 20,
the HCDR2 sequences of SEQ ID NO: 21,
the HCDR3 sequence of SEQ ID NO: 23,
the LCDR1 sequence of SEQ ID NO: 24,
the LCDR2 sequence of SEQ ID NO: 25, and
the LCDR3 sequences of SEQ ID NO: 27; or
iv. the HCDR1 sequence of SEQ ID NO: 20,
the HCDR2 sequences of SEQ ID NO: 21,
the HCDR3 sequence of SEQ ID NO: 23,
the LCDR1 sequence of SEQ ID NO: 24,
the LCDR2 sequence of SEQ ID NO: 26, and
the LCDR3 sequences of SEQ ID NO: 27; or
v. the HCDR1 sequence of SEQ ID NO: 20,
the HCDR2 sequences of SEQ ID NO: 22,
the HCDR3 sequence of SEQ ID NO: 23,
the LCDR1 sequence of SEQ ID NO: 24,
the LCDR2 sequence of SEQ ID NO: 25, and
the LCDR3 sequences of SEQ ID NO: 27; or
vi. the HCDR1 sequence of SEQ ID NO: 20,
the HCDR2 sequences of SEQ ID NO: 22,
the HCDR3 sequence of SEQ ID NO: 23,
the LCDR1 sequence of SEQ ID NO: 24,
the LCDR2 sequence of SEQ ID NO: 26, and
the LCDR3 sequences of SEQ ID NO: 27; or
particularly, wherein said PDL1-BD comprises:
i. the HCDR1 sequence of SEQ ID NO: 74,
the HCDR2 sequences of SEQ ID NO: 75,
the HCDR3 sequence of SEQ ID NO: 76,
the LCDR1 sequence of SEQ ID NO: 77,
the LCDR2 sequence of SEQ ID NO: 78, and
the LCDR3 sequences of SEQ ID NO: 80; or
ii. the HCDR1 sequence of SEQ ID NO: 74,
the HCDR2 sequences of SEQ ID NO: 75,
the HCDR3 sequence of SEQ ID NO: 76,
the LCDR1 sequence of SEQ ID NO: 77,
the LCDR2 sequence of SEQ ID NO: 79, and
the LCDR3 sequences of SEQ ID NO: 80.

4. The multispecific antibody of any of the preceding claims, wherein the KD of the PDL1-BD for binding to human PDL1 is at least 500 fold lower, in particular at least 1000 fold lower, in particular at least 1500 fold lower, in particular at least 2000 fold lower, than the KD of the CD47 BD for binding to human CD47.

5. The multispecific antibody of any one of the preceding claims, wherein said CD47-BD comprises
a) a VH sequence at least 90, 91, 92, 93, 94, 95, 96, 97, 98 or 99 percent identical to any one of the amino acid sequences selected from SEQ ID NOs: 8 and 9; and
a VL sequence at least 90, 91, 92, 93, 94, 95, 96, 97, 98 or 99 percent identical to any one of the amino acid sequences selected from SEQ ID NOs: 11 and 12, particularly to SEQ ID NO: 12; or
b) a VH sequence at least 90, 91, 92, 93, 94, 95, 96, 97, 98 or 99 percent identical to the amino acid sequence of SEQ ID NO: 10; and
a VL sequence at least 90, 91, 92, 93, 94, 95, 96, 97, 98 or 99 percent identical to the amino acid sequence of SEQ ID NO: 13.

6. The multispecific antibody of any one of the preceding claims, wherein said PDL1-BD comprises
a) a VH sequence at least 90, 91, 92, 93, 94, 95, 96, 97, 98 or 99 percent identical to the amino acid sequence of SEQ ID NO: 28; and
a VL sequence at least 90, 91, 92, 93, 94, 95, 96, 97, 98 or 99 percent identical to any one of the amino acid sequences selected from SEQ ID NOs: 34, 36 and 37, particularly selected from SEQ ID NOs: 34 and 37, particularly to SEQ ID NO:37; or
b) a VH sequence at least 90, 91, 92, 93, 94, 95, 96, 97, 98 or 99 percent identical to any one of the amino acid sequences selected from SEQ ID NOs: 29 and 31; and a VL sequence at least 90, 91, 92, 93, 94, 95, 96, 97, 98 or 99 percent identical to any one of the amino acid sequences selected from SEQ ID NOs: 33 and 35, particularly to SEQ ID NO: 35; or
c) a VH sequence at least 90, 91, 92, 93, 94, 95, 96, 97, 98 or 99 percent identical to the amino acid sequence of SEQ ID NO: 30; and
a VL sequence at least 90, 91, 92, 93, 94, 95, 96, 97, 98 or 99 percent identical to any one of the amino acid sequences selected from SEQ ID NOs: 32 and 34, particularly to SEQ ID NO: 34; or
d) a VH sequence at least 90, 91, 92, 93, 94, 95, 96, 97, 98 or 99 percent identical to any one of the amino acid sequences selected from SEQ ID NOs: 38 and 42; and a VL sequence at least 90, 91, 92, 93, 94, 95, 96, 97, 98 or 99 percent identical to any one of the amino acid sequences selected from SEQ ID NOs: 48, 50 and 51, particularly selected from SEQ ID NOs: 48 and 51, particularly to SEQ ID NO: 51; or
e) a VH sequence at least 90, 91, 92, 93, 94, 95, 96, 97, 98 or 99 percent identical to any one of the amino acid sequences selected from SEQ ID NOs: 39, 41, 43 and 45; and
a VL sequence at least 90, 91, 92, 93, 94, 95, 96, 97, 98 or 99 percent identical to any one of the amino acid sequences selected from SEQ ID NOs: 47 and 49, particularly to SEQ ID NO: 49; or
f) a VH sequence at least 90, 91, 92, 93, 94, 95, 96, 97, 98 or 99 percent identical to any one of the amino acid sequences selected from SEQ ID NOs: 40 and 44; and a VL sequence at least 90, 91, 92, 93, 94, 95, 96, 97, 98 or 99 percent identical to any one of the amino acid sequences selected from SEQ ID NOs: 46 and 48, particularly to SEQ ID NO: 48; or
g) a VH sequence at least 90, 91, 92, 93, 94, 95, 96, 97, 98 or 99 percent identical to the amino acid sequence of SEQ ID NO: 52; and
a VL sequence at least 90, 91, 92, 93, 94, 95, 96, 97, 98 or 99 percent identical to any one of the amino acid sequences selected from SEQ ID NOs: 58, 60 and 61, particularly selected from SEQ ID NOs: 58 and 61, particularly to SEQ ID NO: 61; or
h) a VH sequence at least 90, 91, 92, 93, 94, 95, 96, 97, 98 or 99 percent identical to any one of the amino acid sequences selected from SEQ ID NOs: 53 and 55; and a VL sequence at least 90, 91, 92, 93, 94, 95, 96, 97, 98 or 99 percent identical to any one of the amino acid sequences selected from SEQ ID NOs: 57 and 59, particularly to SEQ ID NO: 59; or
i) a VH sequence at least 90, 91, 92, 93, 94, 95, 96, 97, 98 or 99 percent identical to the amino acid sequence of SEQ ID NO: 54; and
a VL sequence at least 90, 91, 92, 93, 94, 95, 96, 97, 98 or 99 percent identical to any one of the amino acid sequences selected from SEQ ID NOs: 56 and 58, particularly to SEQ ID NO: 58; or
j) a VH sequence at least 90, 91, 92, 93, 94, 95, 96, 97, 98 or 99 percent identical to the amino acid sequence of SEQ ID NO: 81; and
a VL sequence at least 90, 91, 92, 93, 94, 95, 96, 97, 98 or 99 percent identical to any one of the amino acid sequences selected from SEQ ID NOs: 86, 88 and 89, particularly selected from SEQ ID NOs: 86 and 89, particularly to SEQ ID NO: 89; or
k) a VH sequence at least 90, 91, 92, 93, 94, 95, 96, 97, 98 or 99 percent identical to the amino acid sequence of SEQ ID NO: 83; and
a VL sequence at least 90, 91, 92, 93, 94, 95, 96, 97, 98 or 99 percent identical to any one of the amino acid sequences selected from SEQ ID NOs: 85 and 86, particularly to SEQ ID NO: 86; or
l) a VH sequence at least 90, 91, 92, 93, 94, 95, 96, 97, 98 or 99 percent identical to any one of the amino acid sequences selected from SEQ ID NOs: 82 and 84, particularly to SEQ ID NO: 84; and
a VL sequence at least 90, 91, 92, 93, 94, 95, 96, 97, 98 or 99 percent identical to the amino acid sequences of SEQ ID NO: 86.

7. The multispecific antibody of any one of the preceding claims, wherein said multispecific antibody further comprises:
d) one further binding domain, which has specificity for an antigen different from CD47 and PDL1, particularly one further binding domain, which specifically binds to LILRB2 (LILRB2-BD),
wherein said one further binding domain is attached to one of the C-termini of the heavy chains of the immunoglobulin Fc region; or
d) two further binding domains, which have specificity for an antigen different from CD47 and PDL1, particularly two further binding domains, which specifically bind to LILRB2 (LILRB2-BD),
wherein said two further binding domains are attached to each of the C-termini of the heavy chains of the immunoglobulin Fc region.

8. The multispecific antibody of claim 7, wherein said LILRB2-BD comprises:
i. the HCDR1 sequence of SEQ ID NO: 93,
the HCDR2 sequences of SEQ ID NO: 94,
the HCDR3 sequence of SEQ ID NO: 95,
the LCDR1 sequence of SEQ ID NO: 96,
the LCDR2 sequence of SEQ ID NO: 97, and
the LCDR3 sequences of SEQ ID NO: 99; or
ii. the HCDR1 sequence of SEQ ID NO: 93,
the HCDR2 sequences of SEQ ID NO: 94,
the HCDR3 sequence of SEQ ID NO: 95,
the LCDR1 sequence of SEQ ID NO: 96,
the LCDR2 sequence of SEQ ID NO: 98, and
the LCDR3 sequences of SEQ ID NO: 99; or
iii. the HCDR1 sequence of SEQ ID NO: 112,
the HCDR2 sequences of SEQ ID NO: 113,
the HCDR3 sequence of SEQ ID NO: 114,
the LCDR1 sequence of SEQ ID NO: 115,
the LCDR2 sequence of SEQ ID NO: 116, and
the LCDR3 sequences of SEQ ID NO: 118; or
iv. the HCDR1 sequence of SEQ ID NO: 112,
the HCDR2 sequences of SEQ ID NO: 113,
the HCDR3 sequence of SEQ ID NO: 114,
the LCDR1 sequence of SEQ ID NO: 115,
the LCDR2 sequence of SEQ ID NO: 117, and
the LCDR3 sequences of SEQ ID NO: 118 or
v. the HCDR1 sequence of SEQ ID NO: 128,
the HCDR2 sequences of SEQ ID NO: 129,
the HCDR3 sequence of SEQ ID NO: 130,
the LCDR1 sequence of SEQ ID NO: 131,
the LCDR2 sequence of SEQ ID NO: 132, and
the LCDR3 sequences of SEQ ID NO: 134; or
vi. the HCDR1 sequence of SEQ ID NO: 128,
the HCDR2 sequences of SEQ ID NO: 129,
the HCDR3 sequence of SEQ ID NO: 130,
the LCDR1 sequence of SEQ ID NO: 131,
the LCDR2 sequence of SEQ ID NO: 133, and
the LCDR3 sequences of SEQ ID NO: 134.
particularly, wherein said LILRB2-BD comprises
a) a VH sequence at least 90, 91, 92, 93, 94, 95, 96, 97, 98 or 99 percent identical to the amino acid sequence of SEQ ID NO: 100; and
a VL sequence at least 90, 91, 92, 93, 94, 95, 96, 97, 98 or 99 percent identical to any one of the amino acid sequences selected from SEQ ID NOs: 104, 106 and 107, particularly selected from SEQ ID NOs: 104 and 107, particularly to SEQ ID NO: 107; or
b) a VH sequence at least 90, 91, 92, 93, 94, 95, 96, 97, 98 or 99 percent identical to the amino acid sequence of SEQ ID NO: 101; and
a VL sequence at least 90, 91, 92, 93, 94, 95, 96, 97, 98 or 99 percent identical to any one of the amino acid sequences selected from SEQ ID NOs: 103 and 104, particularly to SEQ ID NO: 104; or
c) a VH sequence at least 90, 91, 92, 93, 94, 95, 96, 97, 98 or 99 percent identical to the amino acid sequence of SEQ ID NO: 102; and
a VL sequence at least 90, 91, 92, 93, 94, 95, 96, 97, 98 or 99 percent identical to the amino acid sequence of SEQ ID NO: 105; or
d) a VH sequence at least 90, 91, 92, 93, 94, 95, 96, 97, 98 or 99 percent identical to the amino acid sequence of SEQ ID NO: 119; and
a VL sequence at least 90, 91, 92, 93, 94, 95, 96, 97, 98 or 99 percent identical to any one of the amino acid sequences selected from SEQ ID NOs: 122, 123 and 124, particularly selected from SEQ ID NOs: 122 and 124, particularly to SEQ ID NO: 124; or
e) a VH sequence at least 90, 91, 92, 93, 94, 95, 96, 97, 98 or 99 percent identical to the amino acid sequence of SEQ ID NO: 120; and
a VL sequence at least 90, 91, 92, 93, 94, 95, 96, 97, 98 or 99 percent identical to any one of the amino acid sequences selected from SEQ ID NOs: 121 and 122, particularly to SEQ ID NO: 122; or
f) a VH sequence at least 90, 91, 92, 93, 94, 95, 96, 97, 98 or 99 percent identical to the amino acid sequence of SEQ ID NO: 135; and
a VL sequence at least 90, 91, 92, 93, 94, 95, 96, 97, 98 or 99 percent identical to any one of the amino acid sequences selected from SEQ ID NOs: 138, 139 and 140, particularly selected from SEQ ID NOs: 138 and 140, particularly to SEQ ID NO: 140; or
g) a VH sequence at least 90, 91, 92, 93, 94, 95, 96, 97, 98 or 99 percent identical to the amino acid sequence of SEQ ID NO: 136; and
a VL sequence at least 90, 91, 92, 93, 94, 95, 96, 97, 98 or 99 percent identical to the amino acid sequence of SEQ ID NO: 138.

9. A nucleic acid or two nucleic acids or three nucleic acids encoding the multispecific antibody of any one of claims 1 to 8.

10. A host cell or host cells comprising a vector or two vectors or three vectors comprising the nucleic acid or two nucleic acids or three nucleic acids of claim 9.

11. A pharmaceutical composition comprising the multispecific antibody of any one of claims 1 to 8 and a pharmaceutically acceptable carrier.

12. A pharmaceutical composition comprising:
i. a first multispecific antibody that specifically binds to CD47 and PDL1;
ii. a second antibody that specifically binds to LILRB2; and
iii. a pharmaceutically acceptable carrier.

13. The pharmaceutical composition of claim 12, wherein
a) the first antibody that specifically binds to CD47 and PDL1 is selected from the group consisting of: IBI-322 by Innovent Biologics Inc.; PF-07257876 by Pfizer Inc.; 6MW-3211 by Mabwell (Shanghai) Bioscience Co. Ltd.; BAT7104 by Bio-Thera Solutions, Ltd.; QL401 by QLSF Biotherapeutics, Inc.; LB-101 by Centessa Pharmaceuticals plc; Imm2520 by ImmuneOnco Biopharmaceuticals Shanghai Inc.; SHS-009 by Nanjing Sanhome Pharmaceutical Co Ltd.; BCD-106 by Biocad Biopharmaceutical Co.; TJL-1C4 by I-MAB Biopharma Co. Ltd.; SG12473 by Hangzhou Sumgen Biotech Co. Ltd.; XB014 by Exelixis Inc.; PMC-122 by PharmAbcine Inc.; ABP-160 by Abpro Corp.; papiliximab by Shaperon Inc.; JY-207 by Shenzhen Enduring Biotech Ltd.; NY2601 and NY2901 by Light Chain Bioscience AG; or
the first antibody that specifically binds to CD47 and PDL1 is selected from a multispecific antibody as defined in any one of the claims 1 to 6;
b) the second antibody that specifically binds to LILRB2 is selected from the group consisting of: JTX-8064 by Jounce Therapeutics Inc.; MK-4830 by Merck & Co. Inc.; IO-108 by Immune-One Therapeutics Inc.; NGM-707 by NGM Biopharmaceuticals Inc.; BMS-986406 by Bristol Myers Squibb Company; IOS-1002 by ImmunOs Therapeutics AG; CDX-585 by Celldex Therapeutics Inc.; SPX-303 by SparX Biopharmaceutical Corp.; CHS-1000 by Coherus BioSciences Inc.; ES009 by Elpiscience (Suzhou) Biopharma Ltd.; XBH41 by GV20 Therapeutics and IVS-5001 by Invectys; or
the second antibody that specifically binds to LILRB2 comprises at least one LILRB2-BD as defined in claim 9.

14. The multispecific antibody of any one of claims 1 to 8 or the pharmaceutical composition of any one of claims 11 to 13 for use in the treatment of a disease, particularly a human disease, more particularly a human disease selected from cancer, more particularly a human disease selected from a benign or especially malignant tumor, solid tumors, mesothelioma, brain cancer, kidney cancer, liver cancer, adrenal gland cancer, bladder cancer, breast cancer, stomach cancer (e. g., gastric tumors), esophageal cancer, ovarian cancer, cervical cancer, thymic cancer, choriocarcinoma, oral cancer, salivary cancer, colon cancer, rectum cancer, prostate cancer, pancreatic cancer, lung cancer (e. g., non-small cell lung cancer and small cell lung cancer), vaginal cancer, thyroid cancer, melanoma (e. g., unresectable or metastatic melanoma), renal cell carcinoma, sarcoma, glioma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e. g., Philadelphia chromosome-positive chronic myelogenous leukemia), acute lymphoblastic leukemia (e. g., Philadelphia chromosome-positive acute lymphoblastic leukemia), non-Hodgkin's lymphoma, plasma cell myeloma, Hodgkin's lymphoma, a leukemia, and any combination thereof.

15. An antibody binding domain, having specificity for CD47 (CD47-BD), as defined in any one of claims 1, 2 and 5, wherein said CD47-BD is in a format selected from an Fv, a dsFv, an scFv and a dsscFv, particularly from an Fv and an scFv, particularly wherein said CD47-BD is in the form of an scFv.

16. An antibody comprising one or two, preferably one, antibody binding domains according to claim 15.
